# EUROPEAN PATENT SPECIFICATION

(11) **EP 2 522 746 B1**
(45) Date of publication and mention of the grant of the patent: **12.11.2014**
(21) Application number: 12168648.9
(22) Date of filing: 02.03.2007
(51) Int. Cl.: C12Q 1/68, C07H 21/02

(54) **MicroRNA expression profile associated with pancreatic cancer**
Mikro-RNA-Expressionsprofil im Zusammenhang mit Bauchspeicheldrüsenkrebs
Profil d'expression de micro-ARN associé au cancer du pancréas

(30) Priority: 02.03.2006 US 778271 P
(43) Date of publication of application: 14.11.2012
(62) Divisional of application: 07757822.7
(73) Proprietor: The Ohio State University Research Foundation, Columbus, OH 43201-1063 (US); The Board of Regents of the University of Oklahoma, Norman, OK 73019 (US)
(72) Inventor: Schmittgen, Thomas D., Granville, OH 43023 (US); Brackett, Daniel J., Seminole, OK 74868 (US)
(74) Representative: Denison, Christopher Marcus

(56) References cited:
- WO-A2-2005/078139
- WO-A2-2007/081680
- JIANG JINMAI ET AL: "Real-time expression profiling of microRNA precursors in human cancer cell lines", NUCLEIC ACIDS RESEARCH, OXFORD UNIVERSITY PRESS, GB, vol. 33, no. 17, 1 January 2005 (2005-01-01), pages 5394-5403, XP002489966, ISSN: 1362-4962, DOI: 10.1093/NAR/GKI863 [retrieved on 2005-09-28]
- VOLINIA STEFANO ET AL: "A microRNA expression signature of human solid tumors defines cancer gene targets", PROCEEDINGS OF THE NATIONAL ACADEMY OF SCIENCES, NATIONAL ACADEMY OF SCIENCES, US, vol. 103, no. 7, 14 February 2006 (2006-02-14), pages 2257-2261, XP002460699, ISSN: 0027-8424, DOI: 10.1073/PNAS.0510565103

## Description

### BACKGROUND

Pancreatic cancer is the fourth leading cause of cancer-related death in the United States. The annual death rate over the last five years has been approximately 30,000 with a similar number of new cases diagnosed each year. The prognosis for pancreatic cancer is the worst of all cancers with a mortality/incidence ratio of 0.99. The incidence of pancreatic cancer in the United States is approximately 9 per 100,000. These discouraging numbers, reflecting the increasing rates of incidence and death, are due to the lack of improvement in detection and diagnosis strategies and the paucity of breakthroughs in treatment regimens.

MicroRNAs (miRNAs) are short noncoding RNAs that have been identified in the genome of a wide range of species. miRNAs were first discovered in *C*. *elegans* in 1993 and have subsequently been discovered in all multicellular organisms. miRNAs are negative regulators of gene expression and are believed to function primarily through imperfect base pair interactions to sequences within the 3' untranslated region of protein coding mRNAs. By 2006, 326 miRNAs had been discovered in humans. While the role for each of these miRNAs is unknown, specific miRNAs have been implicated in the regulation of a diverse number of cellular processes including differentiation of adipocytes, maturation of oocytes, maintenance of the pluripotent cell state and regulation of insulin secretion.

A growing number of direct and indirect evidence suggests a relationship between altered miRNA expression and cancer. These include miR-15a and miR-16-1 in chronic lymphocytic leukemia, miR-143 and miR-145 in colorectal cancer, let-7 in lung cancer and miR-155 in diffuse large B cell lymphoma. Expression profiling has identified other cancers with differential expression of several miRNAs including breast cancer, glioblastoma and papillary thyroid cancer. A polycistron encoding five miRNAs is amplified in human B-cell lymphomas and forced expression of the polycistron along with c-myc was tumorigenic, suggesting that this group of miRNAs may function as oncogenes.

A method for reliably and accurately diagnosing, or for screening individuals for a predisposition to, pancreatic cancer is needed. A method of treating pancreatic cancer is also highly desirable.

### SUMMARY

The present invention is based, in part, on the identification of miRNAs that have altered expression in pancreatic adenocarcinoma.

The invention provides methods, as defined in the claims, of diagnosing whether a subject has, or is at risk of developing, pancreatic ductal adenocarcinoma, comprising measuring the level of an miR gene product which is a precursor or mature form of MIR-135b.

As defined in the claims, the methods may comprise measuring combinations of miR gene products and/or additional miR gene products.

Also disclosed are miR gene products with altered expression selected from the following group: MIR-034b, MIR-092-2-P, MIR-096-P, MIR-129-2, MIR-130a-P, MIR-133b, MIR-139, MIR-188b-P, MIR-192, MIR-200a-P, MIR-204, MIR-210, MIR-299-P, MIR-302d, MIR-337, MIR-371, MIR-378, MIR-383, MIR-422b, MIR-423, MIR-375, let-7a-2-P, let-7b, let-7c, let-7d, let-7f-1, let-7i, MIR-001-2, MIR-007-1, MIR-015a, MIR-015b, MIR-016-1, MIR-019b-1-P, MIR-021, MIR-023a, MIR-024-1,2, MIR-027a, b, MIR-029a,c, MIR-030d, MIR-032, MIR-092-1, MIR-098, MIR-099a, MIR-100, MIR-107, MIR-125b-1, MIR-126, MIR-128a, MIR-132, MIR-136, MIR-142-P, MIR-145-P, MIR-152, MIR-155, MIR-181a,c, MIR-196a-2, MIR-212, MIR-213, MIR-215, MIR-218-1,2, MIR-221, MIR-222-P, MIR-301, MIR-328, MIR-331-P, MIR-345, MIR-367, MIR-376, MIR-424 and combinations thereof.

In some cases, the level of the gene product in the biological sample is less than the level of its corresponding miR gene product in the control sample. Such under-expressed gene products include: MIR-092-2-P, MIR-096-P, MIR-129-2, MIR-133b, MIR-139, MIR-188b-P, MIR-204, MIR-299-P, MIR-337, MIR-371, MIR-383, MIR-375 and combinations thereof.

In some cases , the level of the miR gene product in the biological sample is greater than the level of its corresponding miR gene product in the control sample. Such over-expressed miR gene products include: let-7a-2-P, let-7b, let-7c, let-7d, let-7f-1, let-7i, MIR-001-2, MIR-007-1, MIR-015a, MIR-015b, MIR-016-1, MIR-019b-1-P, MIR-021, MIR-023a, MIR-024-1,2, MIR-027a, b, MIR-029a,c, MIR-030d, MIR-032, MIR-092-1, MIR-098, MIR-099a, MIR-100, MIR-107, MIR-125b-1, MIR-126, MIR-128a, MIR-132, MIR-136, MIR-142-P, MIR-145-P, MIR-152, MIR-155, MIR-181a,c, MIR-196a-2, MIR-212, MIR-213, MIR-215, MIR-218-1,2, MIR-221, MIR-222-P, MIR-301, MIR-328, MIR-331-P, MIR-345, MIR-367, MIR-376, MIR-424, and combinations thereof.

The methods of the present disclosure may include: (a) assaying the expression levels of a plurality of miR gene products in the test sample to provide an miR expression profile for the test sample; and (b) comparing the miR expression profile of the test sample to a corresponding miR expression profile generated from the control sample. A difference between the miR expression profile of the test sample and the miR expression profile of the control sample is indicative of the subject either having, or being at risk for developing, pancreatic cancer.

The multiple miR gene products may correspond to a substantial portion of the full complement of miR genes in a cell. In other embodiments, the multiple miR gene products correspond to about 95%, 90%, 80%, 70% or 60% of the full complement of miR genes in a cell.

The the multiple miR gene products may include one or more miR gene products selected from the group consisting of: MIR-139, MIR096-P, MIR-375, let-7b, let-7d, let-7f-1, let-7i, MIR-155, MIR-181a, MIR-212, MIR-301, MIR-007-1, and MIR-021.

The level of said miR gene product can be measured using a variety of techniques that are well known in the art. These techniques include amplification-based assays, hybridization-based assays, and microarray analyses. The level of the miR gene product can be determined by measuring the corresponding miR gene copy in the sample.

The biological sample obtained from the subject can include pancreatic tissue, pancreatic tumor or pancreatic cells. The biological sample can also include pancreatic juice.

A kit for diagnosing pancreatic cancer in a subject suspected of having, or being at risk for developing, pancreatic cancer is also disclosed. Such a kit includes: (a) a means for measuring the level of at least one miR gene product in a biological sample derived from the subject's pancreas, and (b) a means for comparing the level of the miR gene product in the biological sample to the level of a corresponding miR gene product in a control sample. A detected difference between the level of the miR gene product in the biological sample as compared with the level of the corresponding miR gene product in the control sample is indicative of the subject either having, or being at risk for developing, pancreatic cancer.

A method of screening a subject who is at risk of developing pancreatic cancer is also disclosed. Such a method includes evaluating the level of at least one miR gene product, or a combination of miR gene products, associated with pancreatic cancer in a biological sample obtained form the subject's pancreas, wherein an alteration in the level of the miR gene product, or combination of miR gene products, in the biological sample as compared to the level of a corresponding miR gene product in a control sample, is indicative of the subject being at risk for developing pancreatic cancer.

In one embodiment, the biological sample includes pancreatic tissue that is either normal or suspected to be precancerous.

A method of inhibiting the progression of pancreatic cancer in a subject whose pancreatic cancer cells contain a greater amount of an miR gene product relative to control cells is also disclosed. Such a method includes administering to the subject an effective amount of an inhibitor molecule that is capable of reducing the amount of the miR gene product in the pancreatic cancer cells.

In some cases, the inhibitor molecule causes post-transcriptional silencing of the up-regulated miR gene product or inhibits maturation of the up-regulated miR gene product. In some cases, the inhibitor molecule is an antisense oligonucleotide of said up-regulated miR gene product, a ribozyme, a small interfering RNA (siRNA), or a molecule capable of forming a triple helix with a gene coding for the up-regulated miR gene product. In some embodiments, the inhibitor compound causes methylation of the miR gene product promoter, resulting in reduced expression of the miR gene.

In one case the inhibitor molecule is administered as naked RNA, in conjunction with a delivery agent. In another case the inhibitor molecule is administered as a nucleic acid encoding the inhibitor molecule.

A method of inhibiting the progression of pancreatic cancer in a subject whose pancreatic cancer cells contain a lesser amount of an miR gene product relative to control cells is also disclosed. Such a method includes administering to the subject an effective amount of an isolated miR gene product corresponding to the miR gene product.

In one case the isolated miR gene product is the functional mature miR. In other case the isolated miR gene product is an oligonucleotide comprising miR precursor hairpin sequence containing the looped portion of the hairpin, or an oligonucleotide comprising a duplex miR precursor lacking the hairpin.

In one case the isolated gene product is administered as naked RNA, in conjunction with a delivery agent. In another case the isolated gene product is administered as a nucleic acid encoding the isolated miR gene product.

In another method disclosed herein, progression of cancer is inhibiting by administration of a compound that causes hypo-methylation of the promoter region of a down-regulated miR gene product.

A pharmaceutical composition for treating pancreatic cancer in a subject, wherein the subject presents at least one under-expressed miR gene product is also disclosed. In some embodiments, the pharmaceutical composition comprises an isolated miR gene product that corresponds to the under-expressed miR gene product, and a pharmaceutically-acceptable carrier. In other embodiments, the pharmaceutical composition comprises at least one nucleic acid encoding the under-expressed miR gene product and a pharmaceutically-acceptable carrier.

The invention also provides for a pharmaceutical compound as defined in the claims for treating pancreatic cancer in a subject, wherein the subject presents at least one over-expressed miR gene product. The pharmaceutical compound comprises at least one antisense oligonucleotide that is specific for the over-expressed miR gene product and a pharmaceutically-acceptable carrier.

The invention also provides for a method as defined in the claims for determining the efficacy of a therapeutic regimen inhibiting progression of pancreatic cancer in a subject.

The invention also provides for a method as defined in the claims of identifying an anti-pancreatic cancer agent. This method comprises the steps of: (a) determining the expression level of at least one miR gene product which is over-expressed in a biological sample containing pancreatic cancer cells, thereby generating data for a pre-test expression level of said miR gene product; (b) contacting the biological sample with a test agent; (c) determining the expression level of the miR gene product in the biological sample after step (b), thereby generating data for a post-test expression level; and (d) comparing the post-test expression level to the pre-test expression level of the miR gene product, wherein a decrease in the post-test expression level of the miR gene product is indicative that the test agent has anti-pancreatic cancer properties.

The invention also provides for another method of as defined in the claims identifying an anti-pancreatic cancer agent, comprising the steps of: (a) determining the expression level of at least one miR gene product which is under-expressed in a biological sample containing pancreatic cancer cells, thereby generating data for a pre-test level expression of said miR gene product; (b) contacting the biological sample with a test agent; (c) determining the expression level of the miR gene product in the biological sample, thereby generating data for a post-test level; and (d) comparing the post-test expression level to the pre-test expression level of said miR gene product, wherein an increase in the post-test expression level of the miR gene product is indicative that the test agent has anti-pancreatic cancer properties.

### BRIEF DESCRIPTION OF THE FIGURES

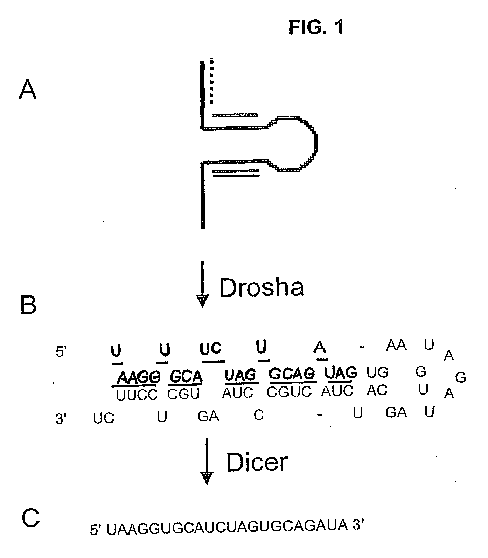

Figure 1. miRNA processing and primer design. miRNAs such as human miR-18 are transcribed as a (A) large primary precursor (pri-miRNA) that is processed by the nuclear enzyme Drosha to produce the (B) putative 62 nt precursor miRNA (pre-miRNA). Both the pri-miRNA and pre-miRNA contain the hairpin structure. The underlined portion of the pre-miRNA represents the sequence of the (C) 22 nt mature miRNA that is processed from the pre-miRNA by the ribonuclease Dicer. Single line denotes forward primer; Double line denotes reverse primer; Dashed line denotes sense primer used along with the reverse (black) primer to amplify the pri-miRNA only.

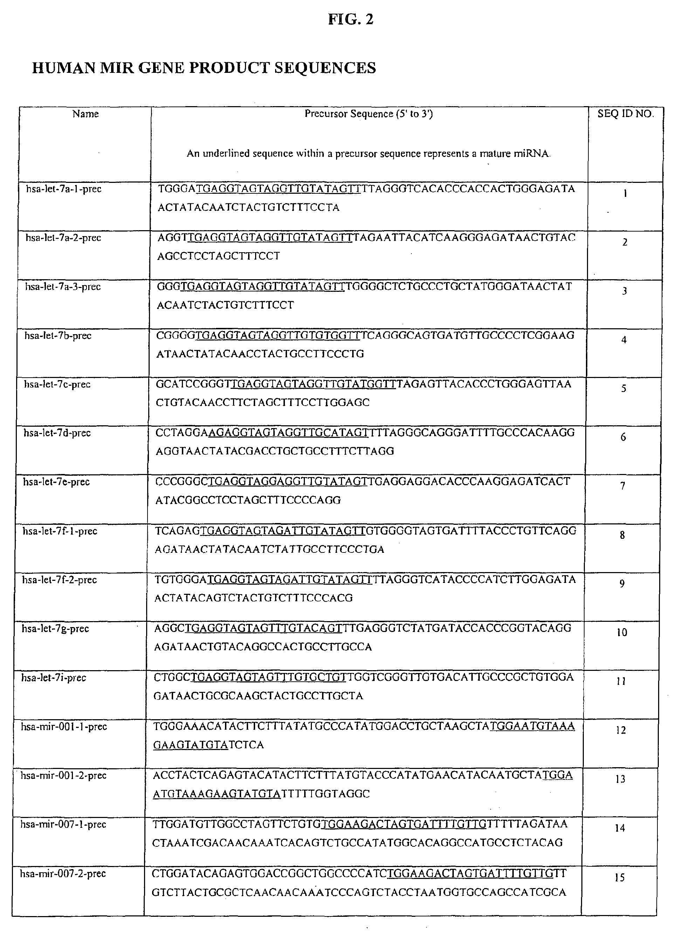

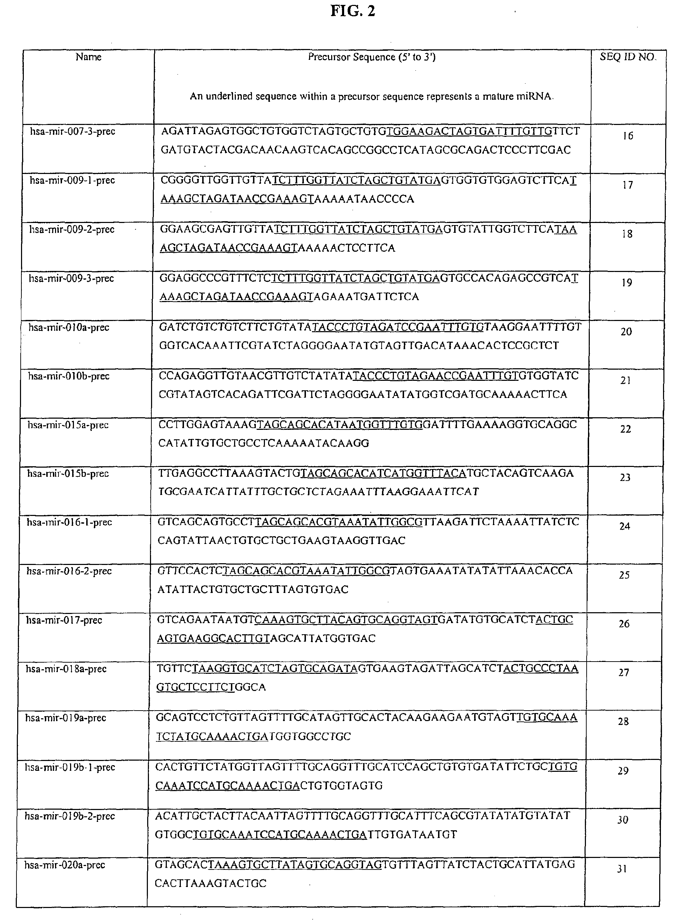

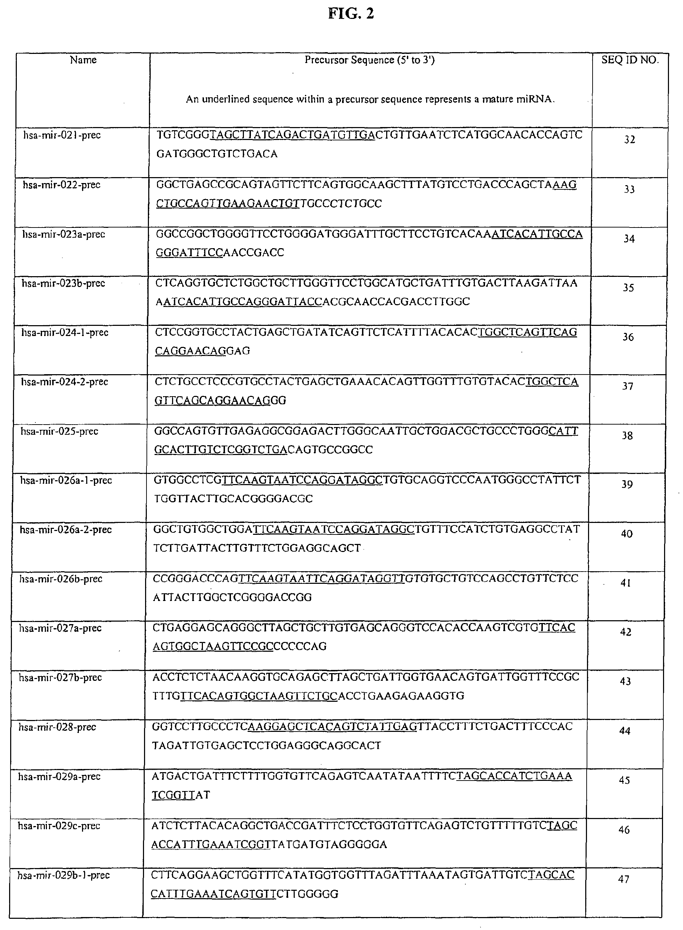

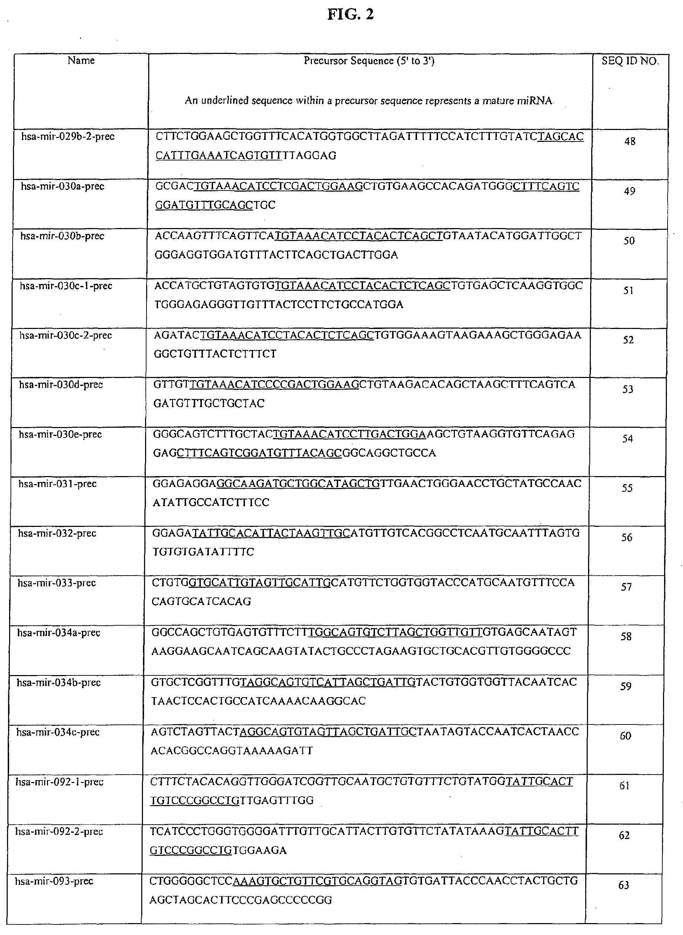

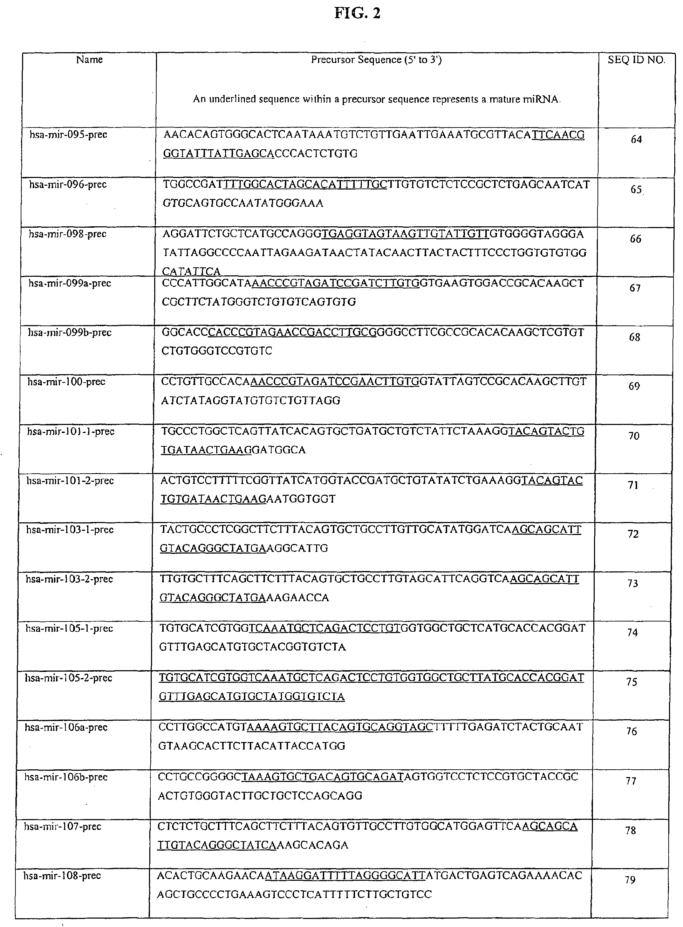

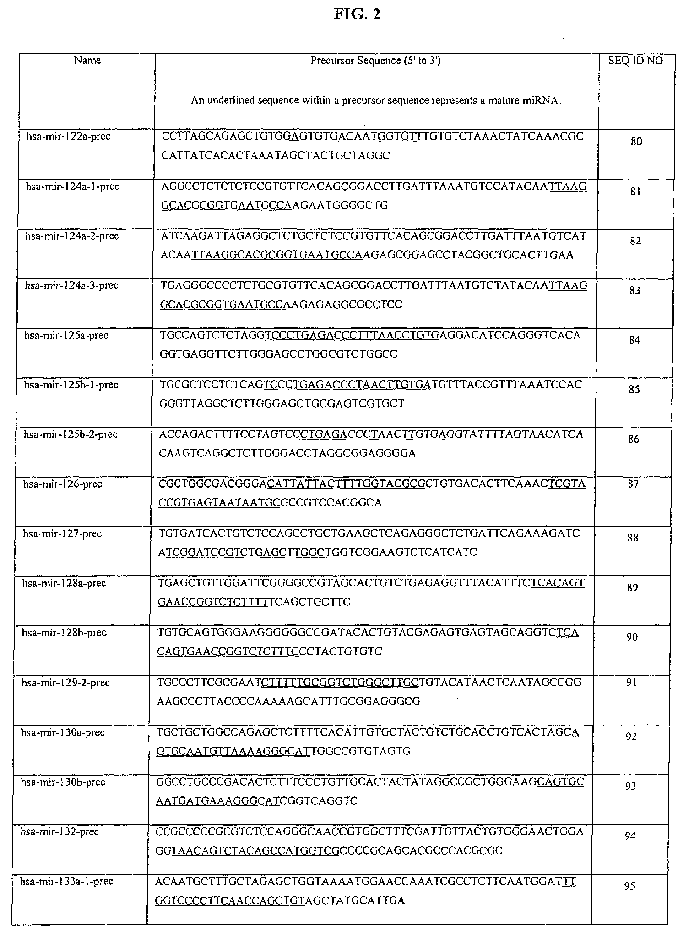

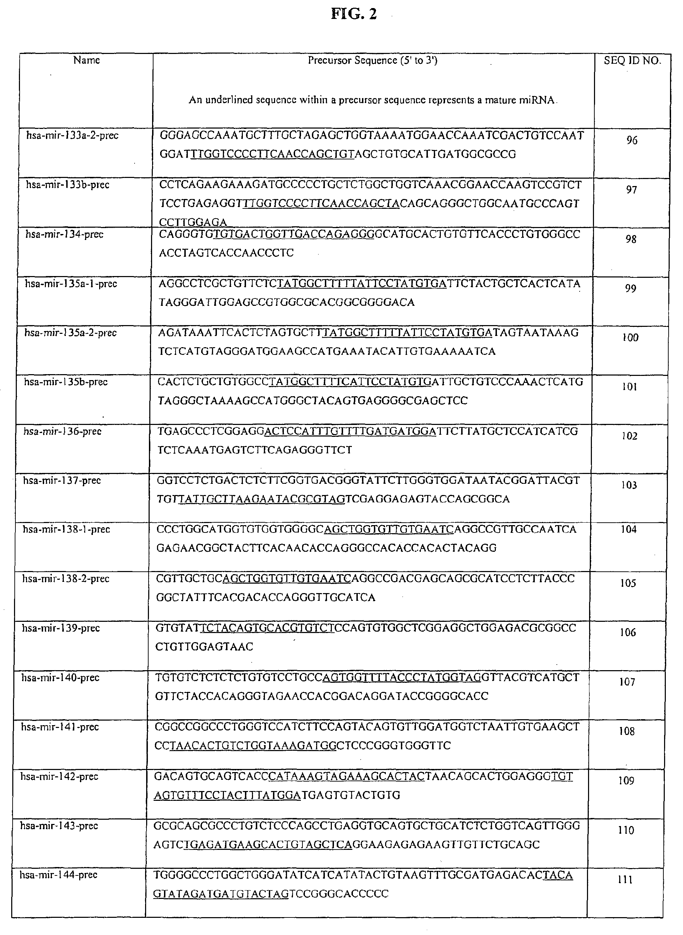

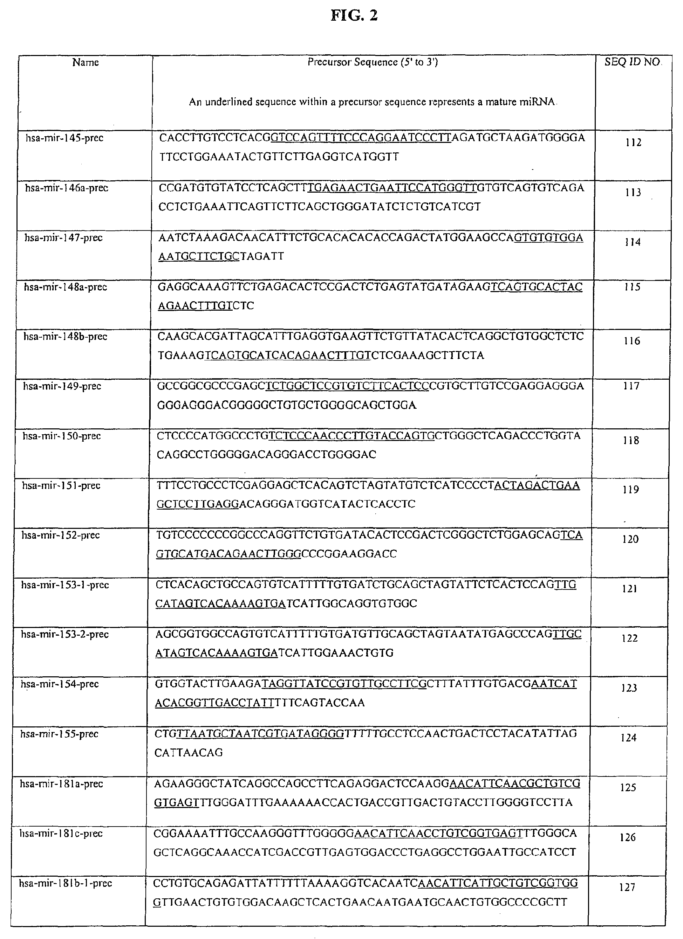

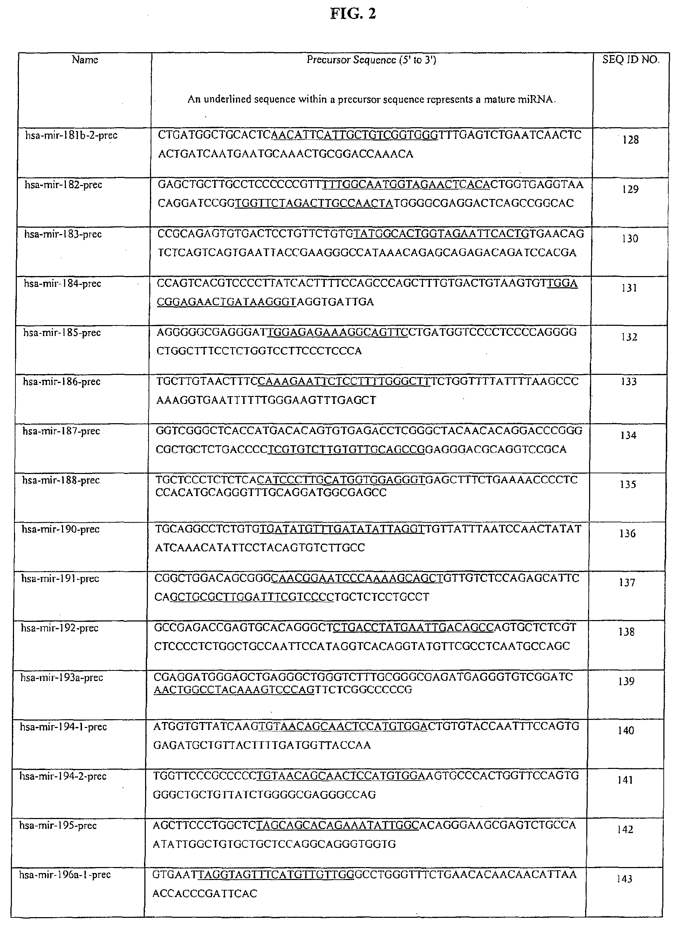

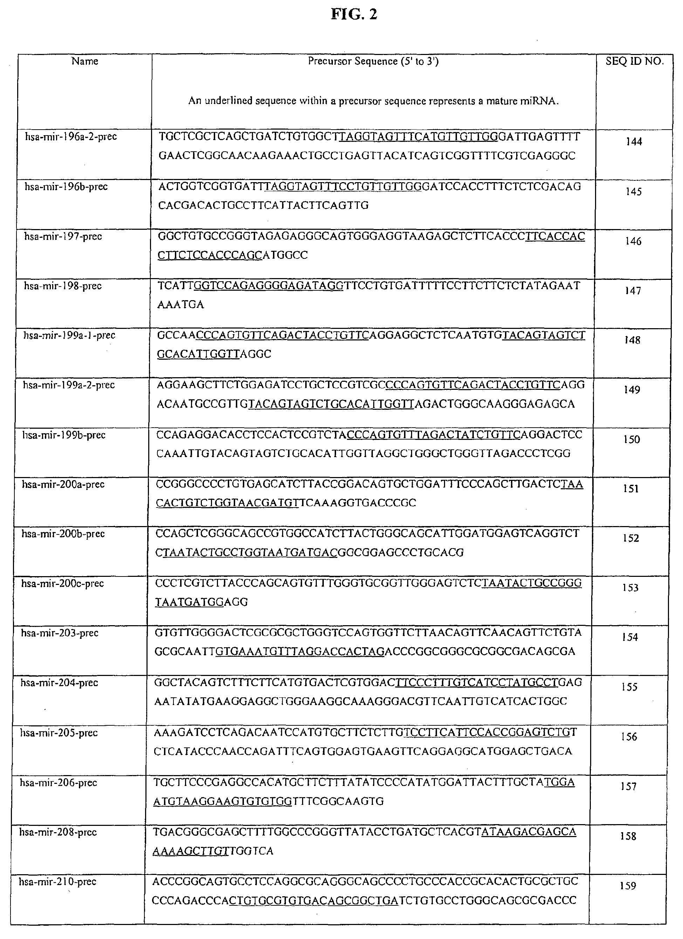

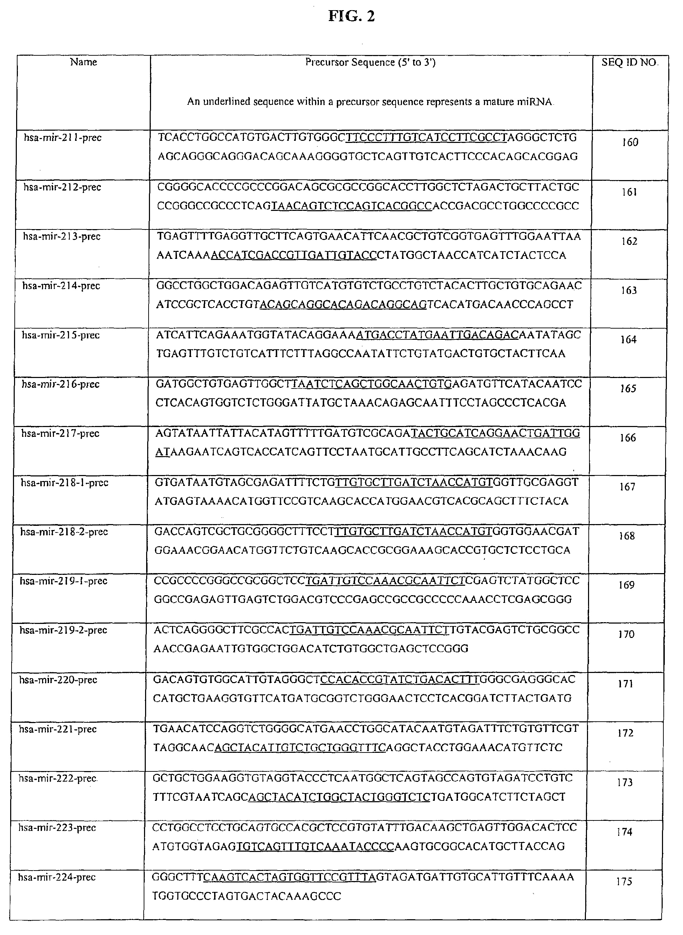

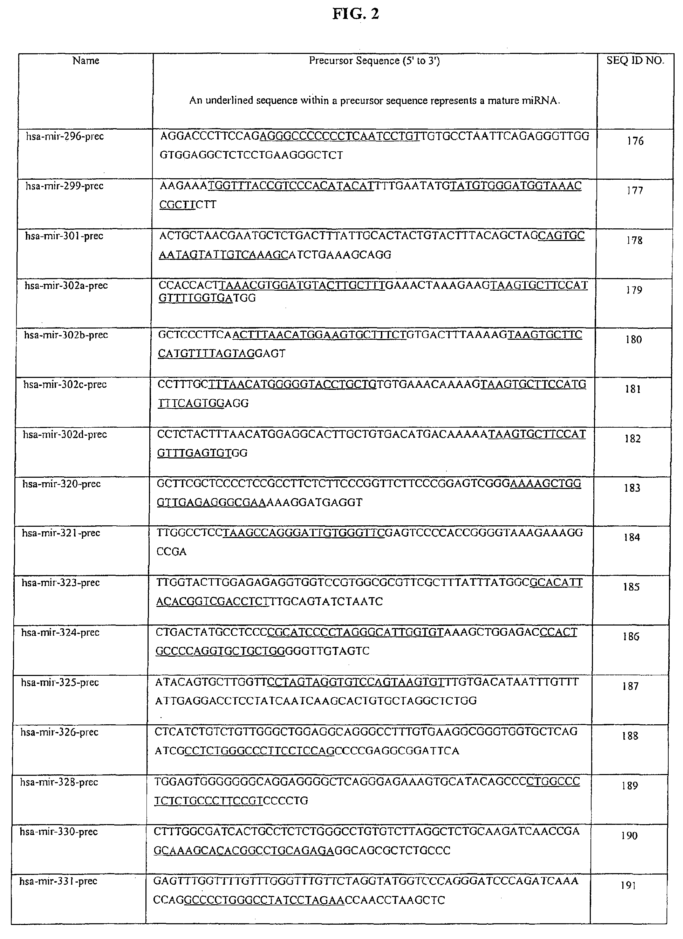

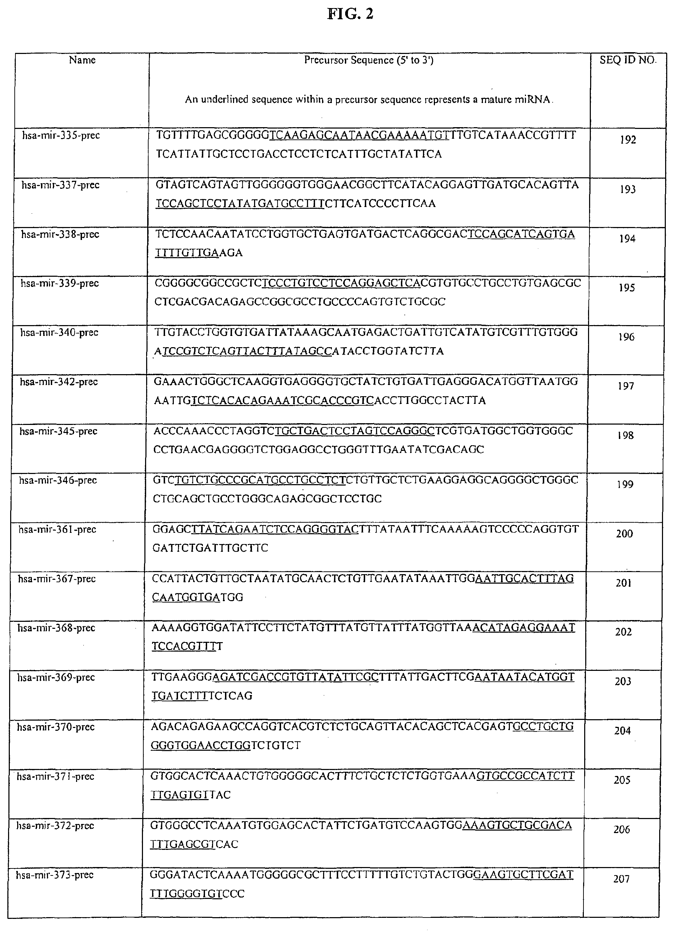

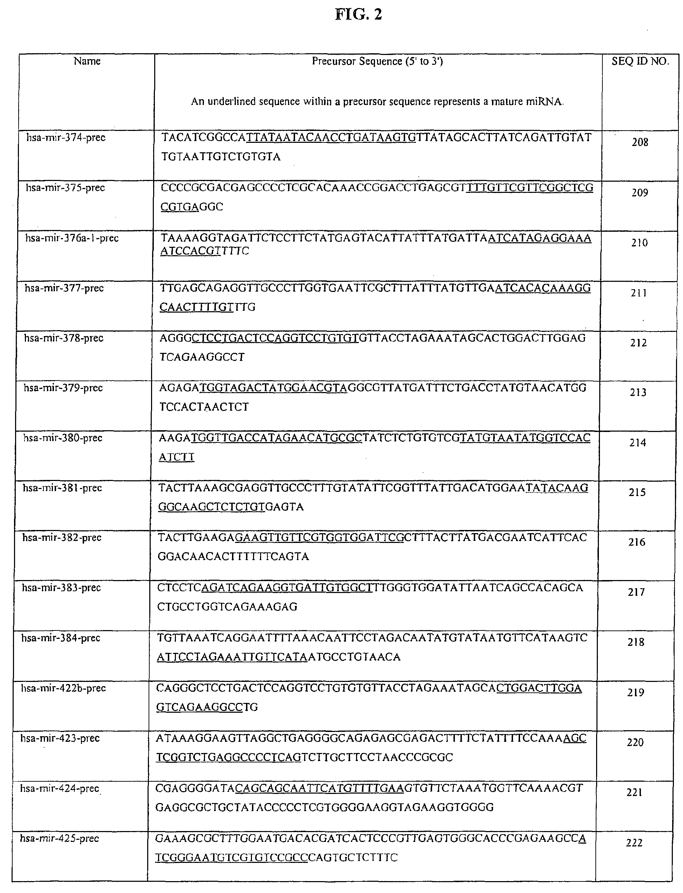

Figure 2. Table of human miR gene product sequences. The sequences represent miRNA precursors and the underlined sequence within a precursor sequence represents a mature miRNA. All sequences are presented in the 5' to 3' orientation.

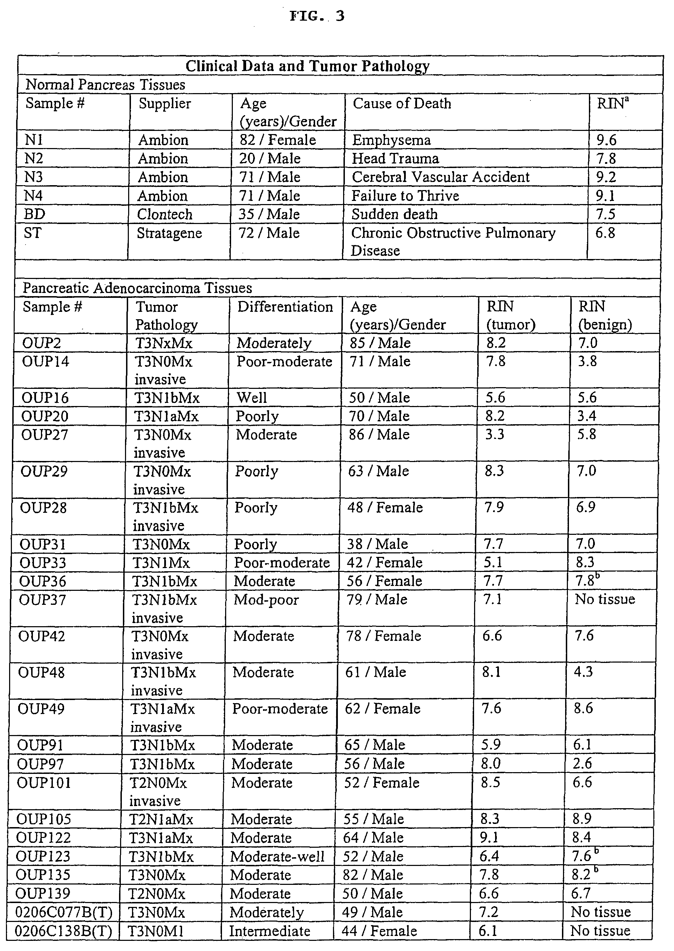

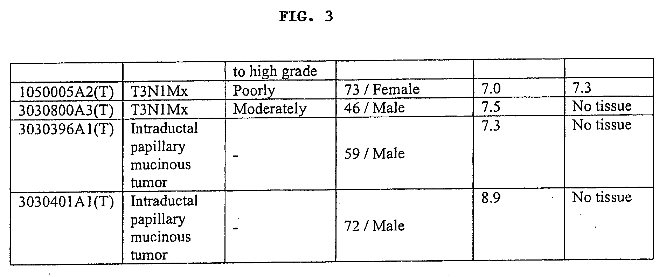

Figure 3. Clinical data and tumor pathology.

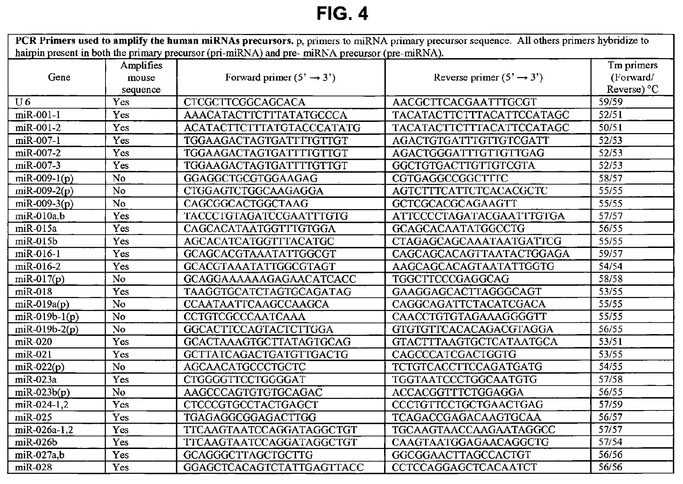

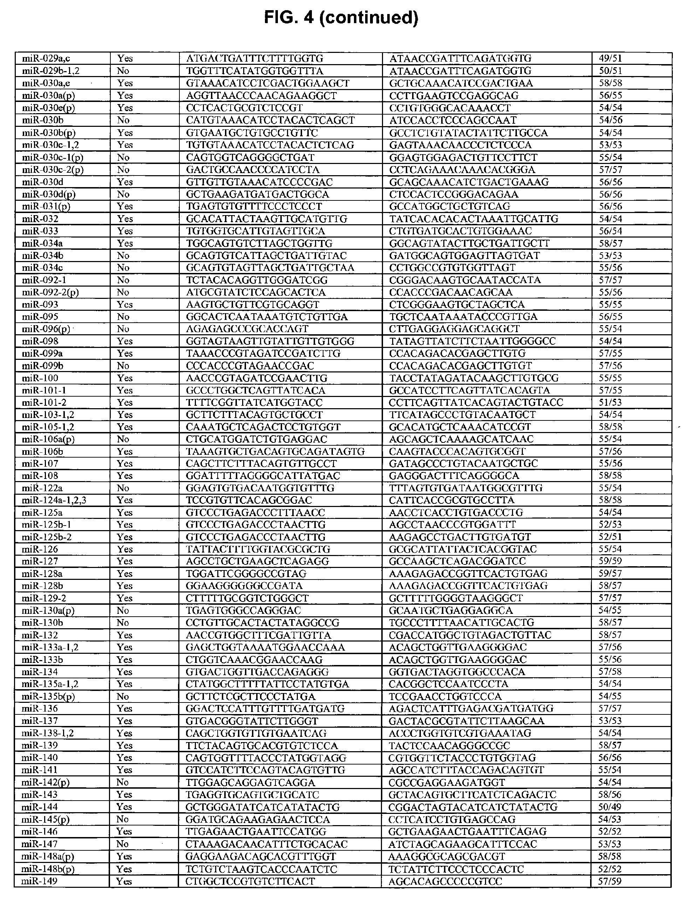

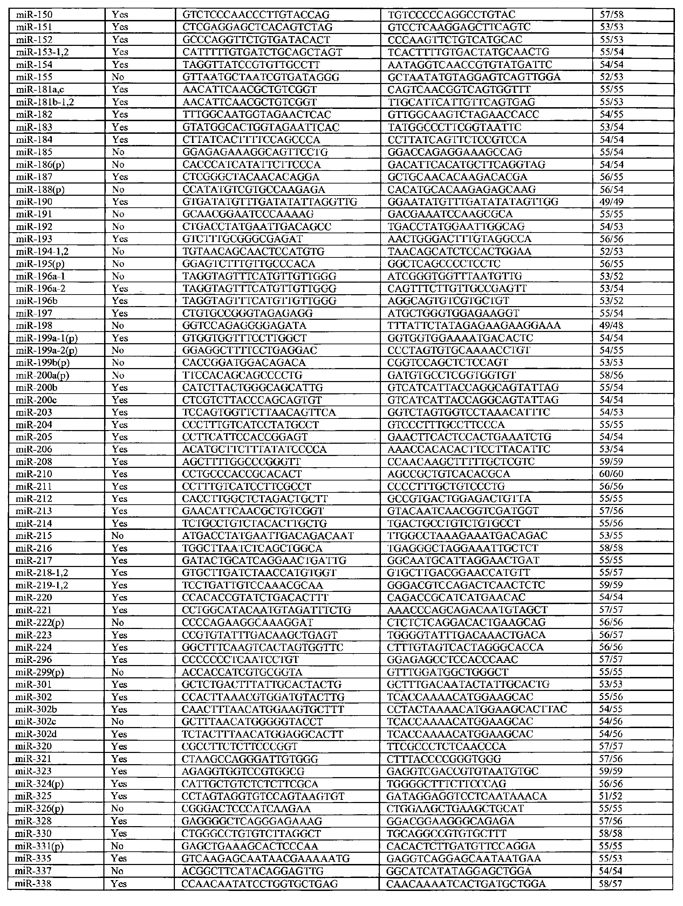

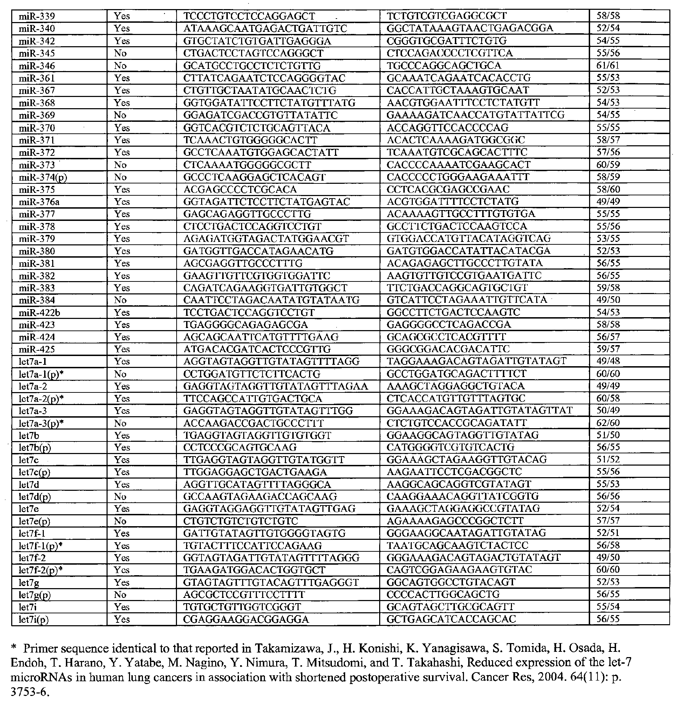

Figure 4. PCR Primers used to amplify the human miRNAs precursors. p, primers to miRNA primary precursor sequence. All other primers hybridize to hairpin present in both the primary precursor and precursor miRNA.

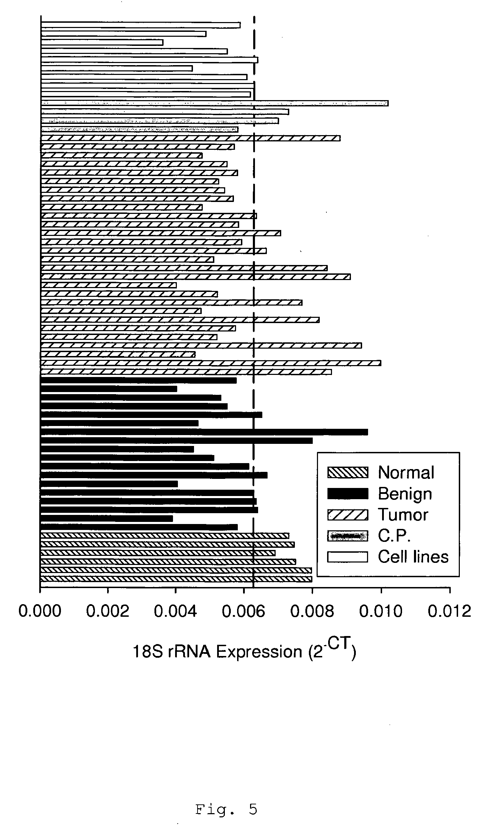

Figure 5. 18S rRNA expression in pancreatic tissue. The expression of the 18S rRNA internal control is shown in pancreatic tumors, adjacent benign tissue, normal pancreas, chronic pancreatitis and pancreatic cancer cell lines. 18S rRNA expression, determined using real-time PCR as described herein, is presented as 2-CT. Dashed line, mean value.

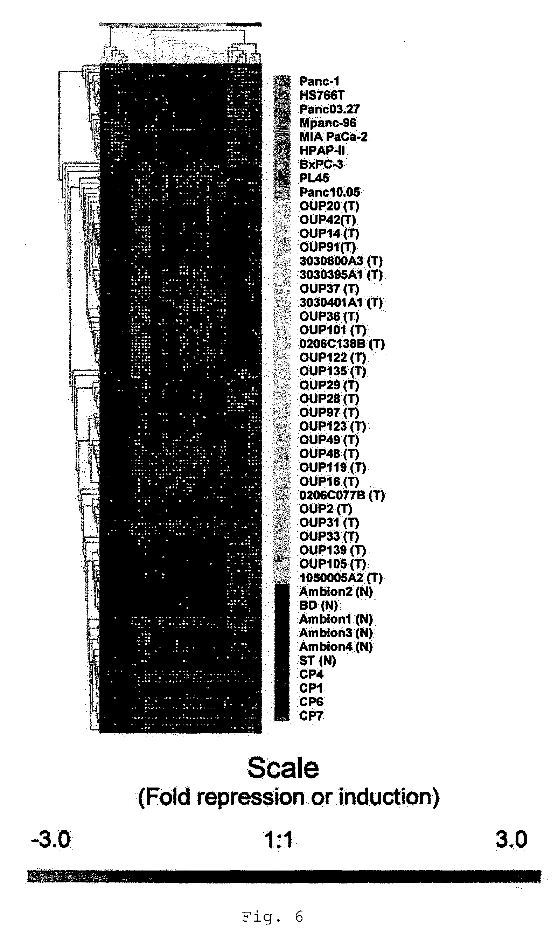

Figure 6. Heatmap of miRNA precursor expression in pancreatic samples. A. The relative expression of 201 miRNA precursors was determined by realtime PCR; data are presented as Δ CT. Unsupervised hierarchical clustering was performed on a subset of the entire data set; data are unfiltered. A median expression value equal to one was designated black; red increased expression; green, reduced expression; grey, undetectable expression. B. Dendrogram representing the results of hierarchical clustering analysis of the miRNA precursor expression pattern in 47 samples. Sample include primary pancreatic tumors (yellow, N = 28), normal pancreatic tissues (blue, N = 6), chronic pancreatitis (orange, N = 4) and pancreatic cancer cell lines (turquoise, N = 9).

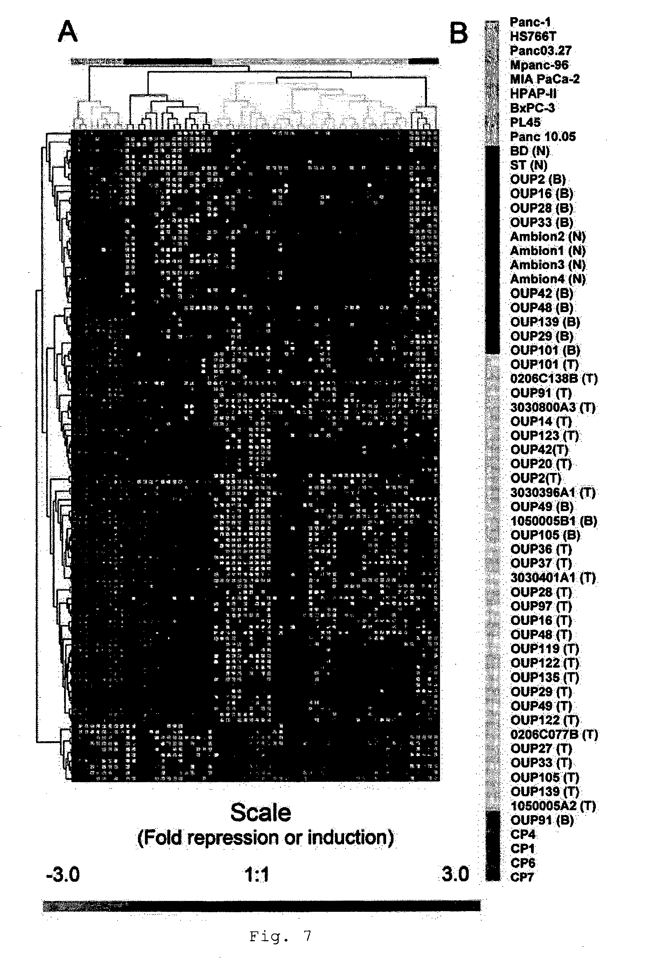

Figure 7. miRNA precursor expression in pancreatic samples. A. The relative expression of each miRNA precursor was determined by real-time PCR; data are presented as ΔCT. Unsupervised hierarchical clustering was performed on a subset of 108 genes that are differentially expressed (P < 0.001) among groups (tumor, chronic pancreatitis, cell lines and normal tissue) as determined by ANOVA multi-group comparison test. A median expression value equal to one was designated black; red increased expression; green, reduced expression; grey, undetectable expression. B. Dendrogram representing the results of hierarchical clustering analysis of the miRNA precursor expression pattern in 47 samples. Samples include primary pancreatic tumors (N = 28), adjacent benign tissue (N = 15), normal pancreatic tissues (N = 6), chronic pancreatitis (N = 4) and pancreatic cancer cell lines (N = 9).

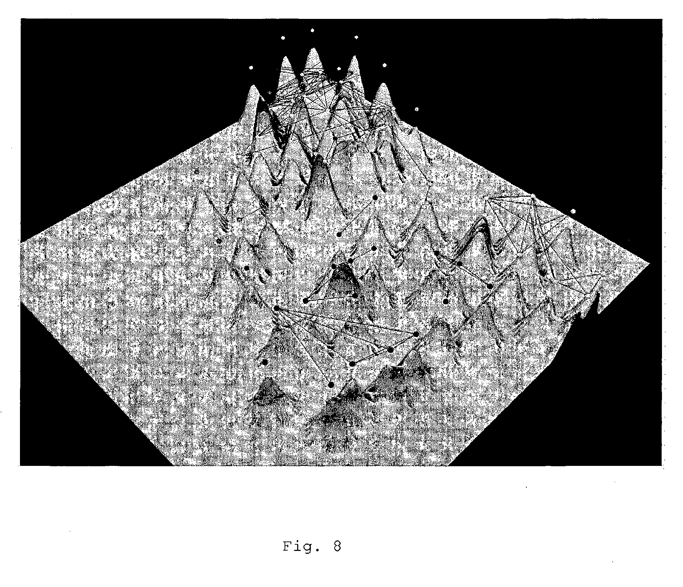

Figure 8. Three-dimensional expression terrain map was created from the filtered miRNA precursor expression data presented in Fig. 7. Each mountain represents an individual sample (tumor, adjacent benign, chronic pancreatitis, normal pancreas or pancreatic cancer cell line). The individual mountains sort into small groups based upon their similarities or differences to each other. Colored dots represent the types of sample: tumors, yellow; adjacent benign tissue, blue; normal pancreatic tissues, black; chronic pancreatitis, orange; and pancreatic cancer cell lines, turquoise. The lines connecting pairs of samples indicate those samples which have very similar patterns of miRNA expression with average correlation above the threshold (>0.8).

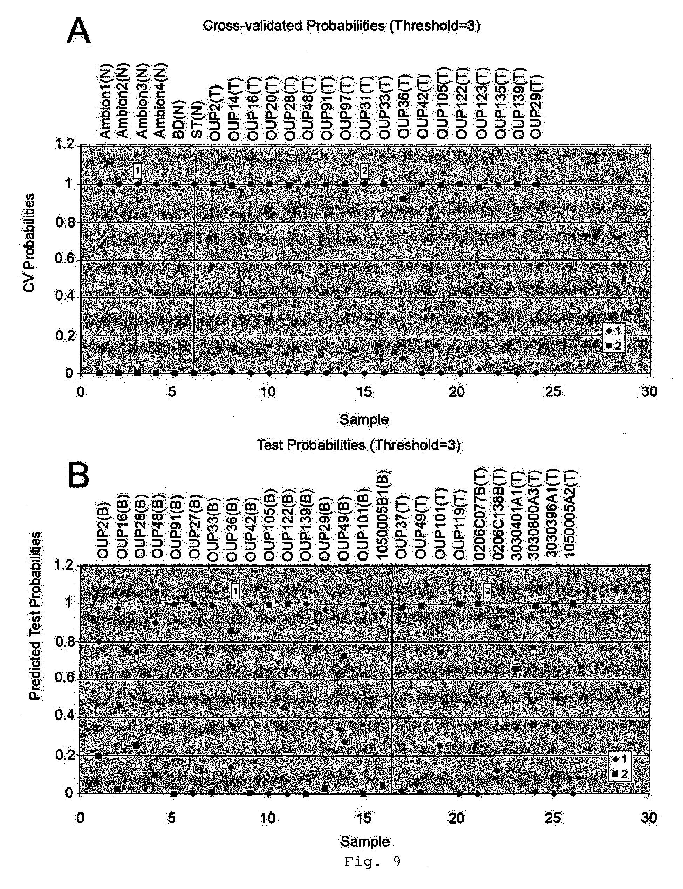

Figure 9. Estimated probabilities for the training and test data. All training data including 6 normal pancreas samples and 18 of the samples known to be pancreatic tumors are correctly classified (A). Eleven out of 15 adjacent benign samples and 10 samples known to be pancreatic tumors are correctly classified in the testing group (B). Samples are partitioned by the true class (A) and the predicted class (B).

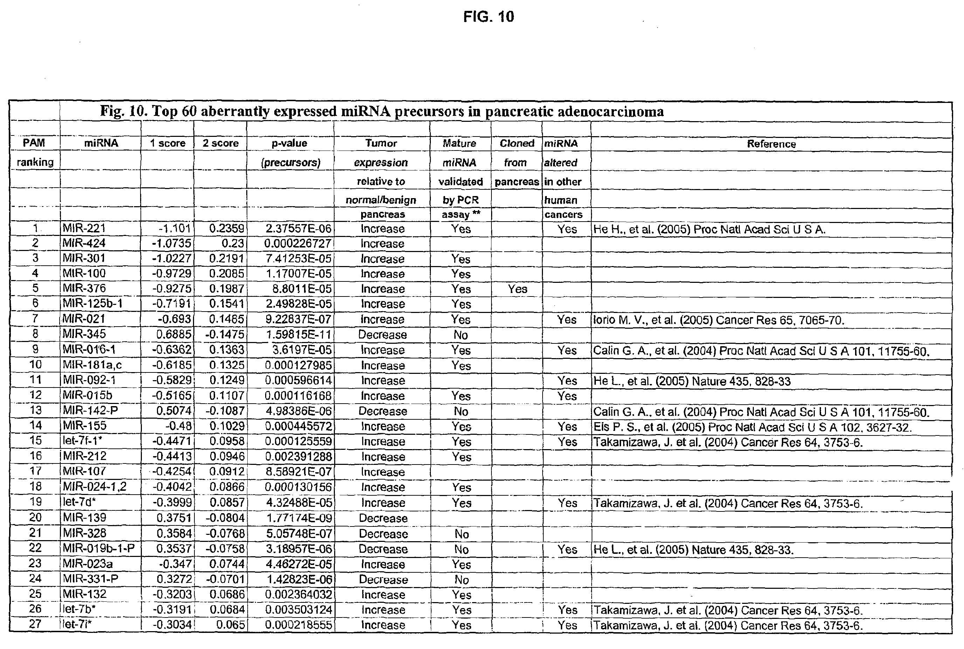

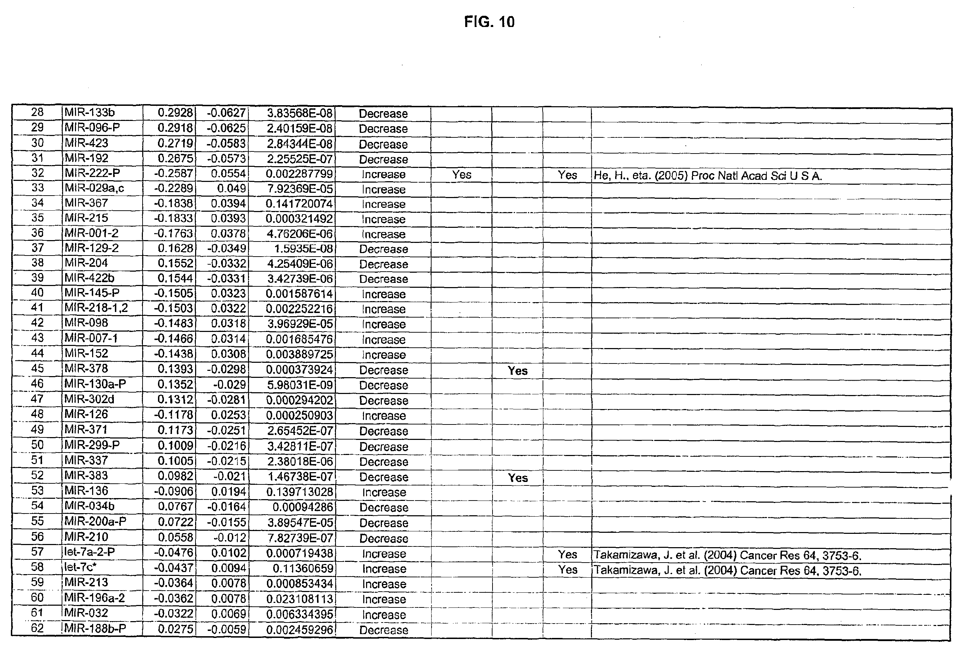

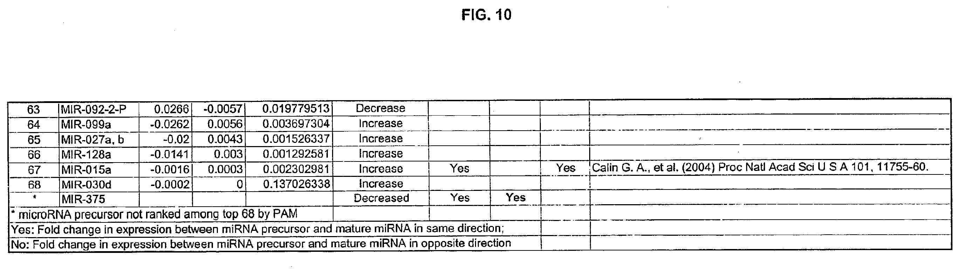

Figure 10. Top 69 aberrantly expressed miRNA precursors in pancreatic adenocarcinoma.

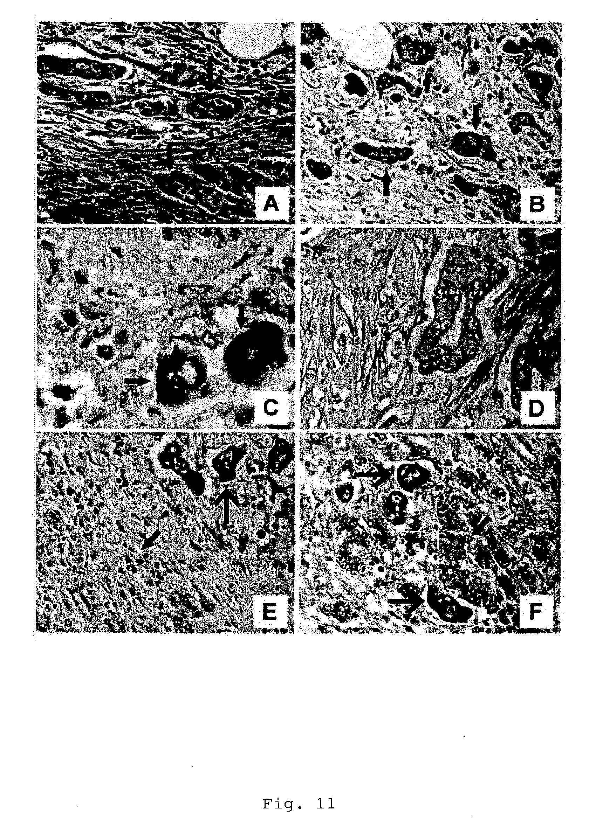

Figure 11. Histologic and molecular analyses of pancreatic cancer for microRNA expression. Panel A (400X) depicts the hematoxylin and eosin analysis of a pancreatic adenocarcinoma. The normal pancreatic glands (small arrow) are being invaded by the poorly formed glands of the carcinoma (large arrow). Serial section analysis of miR-221 after in situ amplification of the corresponding cDNA showed that many of the tumor cells contained the target sequence; note the cytoplasmic localization (arrows, panel B - 400X and at higher magnification, panel C - 1000X; the signal is blue due to NBT/BCIP with negative cells counterstained with fast red). The signal was lost with either omission of the primers or substitution with HPV-specific primers (panel D, 400X). The adjacent serial section also showed many of the tumor cells expressed miR-376a after in situ amplification of the cDNA (E, F). Panel E (400X) shows the positive tumor cells (large arrow) and the negative stromal cells in the areas of desmoplasia (small arrow) while panel F (400X) depicts the positive tumor cells (large arrow) adjacent to the negative benign pancreatic gland acini (small arrow).

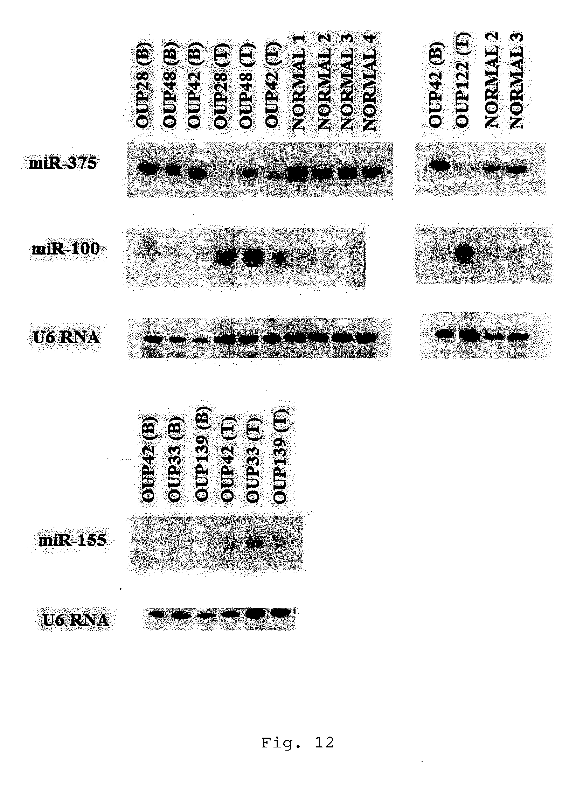

Figure 12. miRNA expression by Northern blotting. The expression of miR-100, - 375, -155 and U6 RNA was determined in tissue specimens of pancreatic cancer (T), adjacent benign tissue (B) or normal pancreas (N). Blots were stripped and re-probed.

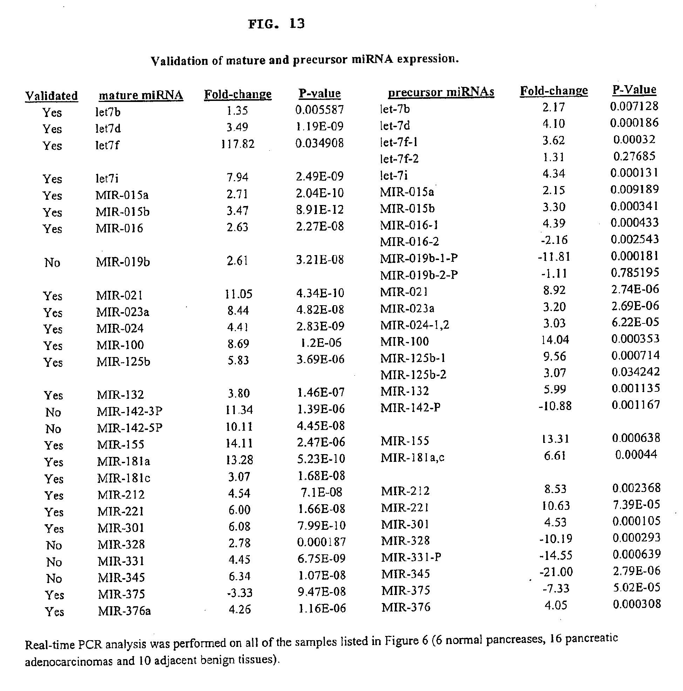

Figure 13. Validation of mature and precursor miRNA expression.

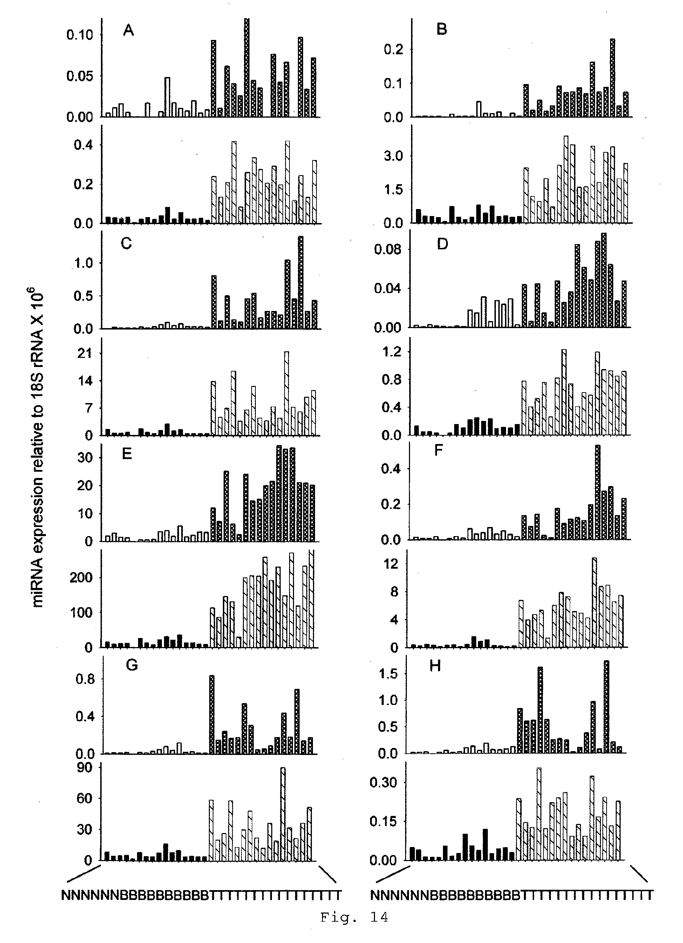

Figure 14. Validation of precursor and mature miRNA levels. The expression of eight miRNAs was validated in six normal pancreas specimens, ten adjacent benign tissues and sixteen pancreatic adenocarcinomas. The relative expression of the miRNA precursors (open bars) was determined using a real-time PCR assay to the miRNA precursors while the relative expression of the mature miRNA (closed bars) was determined using a real-time PCR assay to the mature miRNAs. The mean differences in miRNA expression between the normal pancreas (black) and tumors (red) was significant P < 0.01 (student's t-test). A, let-7i; B, miR-221; C, miR-100; D, miR-301; E, miR-21; F, miR-181a,c (precursor) & miR-181a (mature); G, miR-125b-1 (precursor) & miR-125b (mature); H, miR-212.

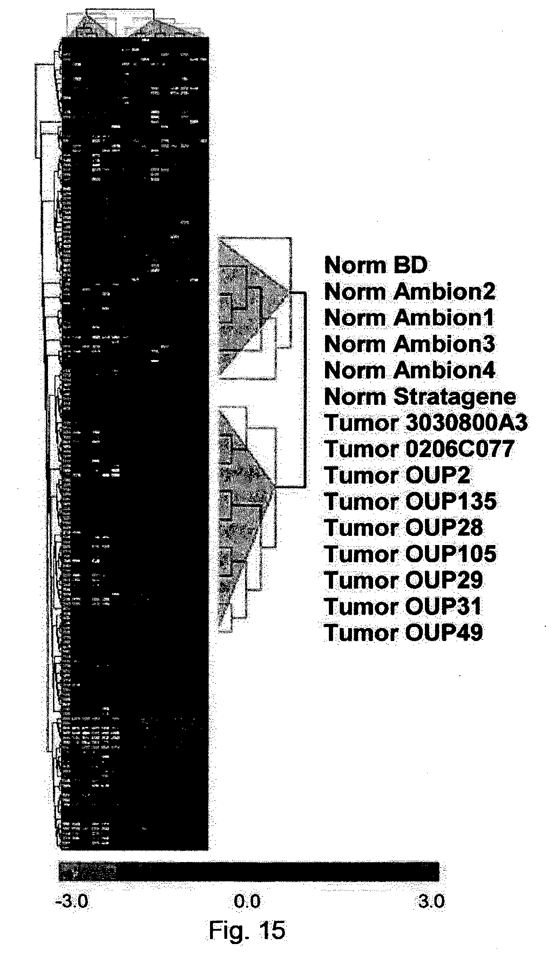

Figure. 15. Heatmap of mature miRNA expression in pancreatic samples. A. The relative expression of 184 mature miRNAs profiled using real-time PCR in nine pancreatic cancer tumors and in pancreas from six donors without pancreatic disease. Data are presented as Δ CT. Unsupervised hierarchical clustering was performed on a subset of the entire data set; data are unfiltered. A median expression value equal to one was designated black; red increased expression; green, reduced expression; grey, undetectable expression. B. Dendrogram representing the results of hierarchical clustering analysis of the mature miRNA precursor expression pattern in 15 samples.

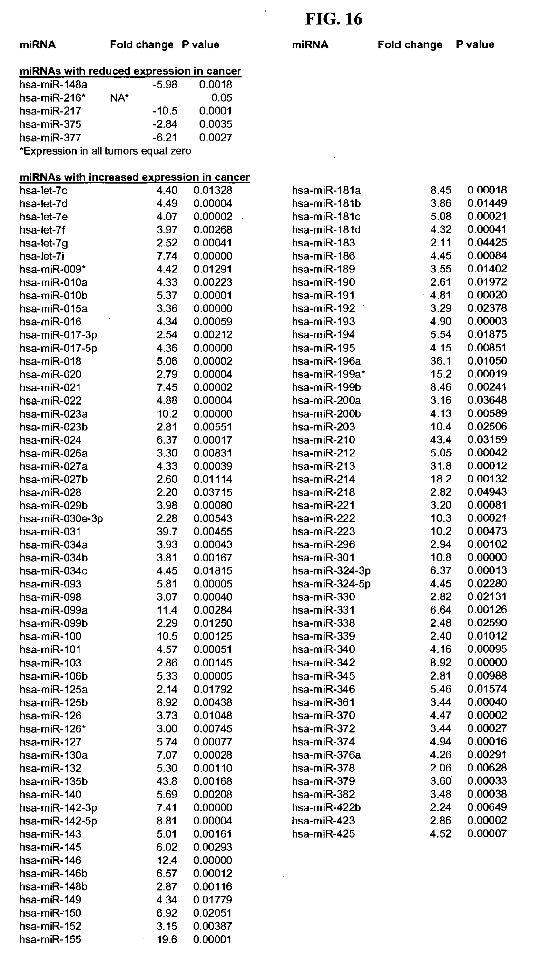

Figure. 16. Aberrantly expressed mature miRNAs in pancreatic adenocarcinoma.

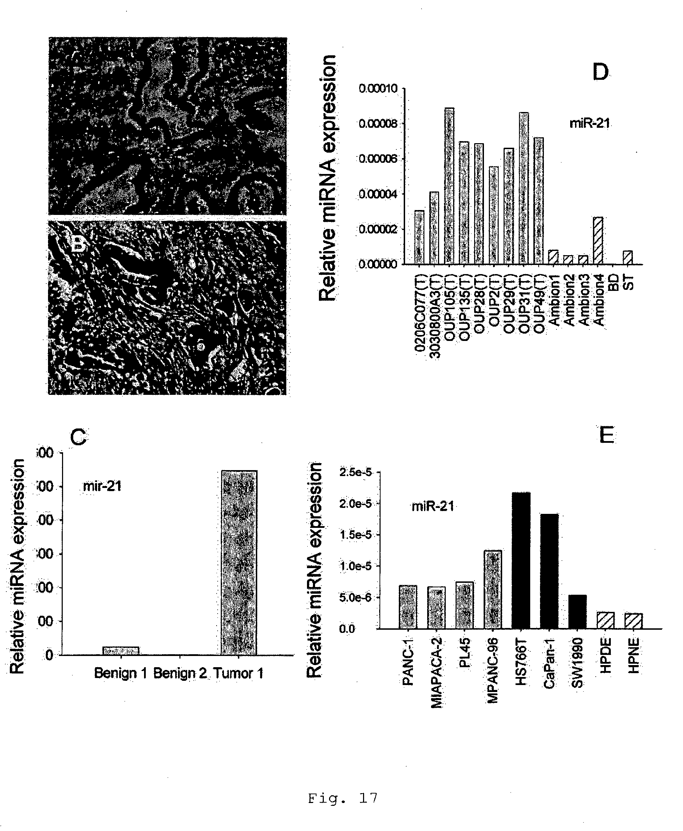

Figure. 17. In situ RT-PCR assay applied to a section of normal pancreas (A) and pancreas cancer (B), demonstrating that increased expression of miR-21 is localized to the tumor. Mature miR-21 was substantially increased in the microdissected tumor tissue compared to normal pancreas ducts (C). The mature miR-21 expression was increased in the tumors (D). The mature miR-21 expression was also increased in all seven pancreatic cancer cell lines compared to the normal pancreas cell lines (E).

### DETAILED DESCRIPTION

The present invention is based, in part, on the identification of particular microRNA gene products whose expression is altered in biological samples obtained from subjects with pancreatic adenocarcinoma (hereinafter "pancreatic cancer") relative to certain control samples. The present invention encompasses methods of diagnosing whether a subject has, or is at risk for developing, pancreatic cancer. Also disclosed are methods of screening subjects who are thought to be at risk for developing pancreatic cancer, method of treating pancreatic cancer by inhibiting the progression of pancreatic cancer, and pharmaceutical compounds that can be used for such treatments. Also provided are methods of determining the efficacy of therapeutic regimens for inhibiting pancreatic cancer, and methods of identifying an anti-pancreatic cancer agent. Also disclosed are various kits suitable for carrying out the above mentioned methods.

The invention will now be described with reference to more detailed examples. The examples illustrate how a person skilled in the art can make and use the invention, and are described here to provide enablement and best mode of the invention without imposing any limitations that are not recited in the claims.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations that may vary depending upon the desired properties sought to be obtained by the present invention. At the very least, and not as an attempt to limit the application of the doctrine of equivalents to the scope of the claims, each numerical parameter should be construed in light of the number of significant digits and ordinary rounding approaches.

Notwithstanding that the numerical ranges and parameters setting forth the broad scope of the invention are approximations, the numerical values set forth in the specific examples are reported as precisely as possible. Any numerical value, however, inherently contains certain errors necessarily resulting from the standard deviation found in their respective testing measurements. Every numerical range given throughout this specification will include every narrower numerical range that falls within such broader numerical range, as if such narrower numerical ranges were all expressly written herein.

The following introduction is useful for understanding the terms used in this description. Without being bound to the following theory and with reference to Fig. 1, it is believed that microRNAs (or "miRNAs") are encoded by miR genes that are transcribed into single or clustered miRNA precursors.

As used herein interchangeably, a "miR gene product," "microRNA," or "miRNA" refers to the unprocessed or processed RNA transcript from an *miR* gene. The unprocessed miR gene transcript is also called an "miRNA precursor." These miRNA precursors are converted to mature forms of miRNAs through a stepwise processing, as depicted in Fig. 1. It is believed that the processing first generates (A) a large primary precursor, or a "pri-miRNA," that is then processed by the nuclear enzyme Drosha to produce (B) a putative precursor, or a "pre-miRNA." The pre-miRNA usually has 50-90 nucleotides (nt), particularly 60-80 nt. The terms "miRNA precursors" or "unprocessed" miRNA used herein are inclusive of both the pri-miRNA and the pre-miRNA and refer to molecules A and/or B in Fig. 1. An active 19-25 nt mature miRNA is processed from the pre-miRNA by the ribonuclease Dicer. The underlined portion of the pre-miRNA sequence in Fig. 1 represents the sequence of the mature miRNA, which is also called the "processed" miR gene transcript, and is depicted as molecule C in Fig. 1.

The active or mature miRNA molecule can be obtained from the miRNA precursor through natural processing routes (*e.g*., using intact cells or cell lysates) or by synthetic processing routes (*e.g*., using isolated processing enzymes, such as isolated Dicer, Argonaut, or RNAase III). It is understood that the active 19-25 nucleotide RNA molecule can also be produced directly by biological or chemical synthesis, without having been processed from the miR precursor.

Still referring to Fig. 1, both the pri-miRNA and pre-miRNA molecules have a characteristic "hairpin sequence," which is an oligonucleotide sequence having a first half which is at least partially complementary to a second half thereof, thereby causing the halves to fold onto themselves, forming a "hairpin structure." The hairpin structure is typically made of a "stem" part, which consists of the complementary or partially complementary sequences, and a "loop" part, which is a region located between the two complementary strands of the stem, as depicted in Fig. 1.

As used herein, the term "miR gene expression" refers to the production of miR gene products from an miR gene, including processing of the miR precursor into a mature miRNA gene product.

The level of the target miR gene product is measured in a biological sample obtained from a subject. For example, a biological sample can be removed from a subject suspected of having pancreatic cancer. Such a biological sample can include a tissue or cell biopsy obtained from a region of the pancreas suspected to be precancerous or cancerous. Alternatively, a biological sample can include pancreatic juice extracted after stimulation of the pancreas. In another example, a blood sample can be removed from the subject. The use of pancreatic juice samples is known in the art, for example, as described in Fukushima, N., et al., (2003), Cancer Biol Ther. 2:78-83; Rosty, C. et al. (2002), Hematol Oncol Clin North Am. 16:37-52; Matsubayashi, H., et al., (2005), Clinical Cancer Research 11:573-583; and Gronborg, M., et al., (2004) Journal of Proteome Research, 3 (5), 1042-1055 .
A corresponding control sample can be obtained from unaffected pancreatic tissues of the subject, from a normal subject or population of normal subjects. The control sample is then processed along with the test sample from the subject, so that the levels of miR gene product produced from a given miR gene in the subject's sample can be compared to the corresponding miR gene product levels from the control sample.

As used herein, the term "subject" includes any animal whose biological sample contains a miR gene product. The animal can be a mammal and can include pet animals, such as dogs and cats; farm animals, such as cows, horses and sheep; laboratory animals, such as rats, mice and rabbits; and primates, such as monkeys and humans. In one embodiment, the mammal is human or mouse.

An alteration (*i.e*., an increase or decrease) in the level of a miR gene product in the sample obtained from the subject, relative to the level of a corresponding miR gene product in a control sample, is indicative of the presence of pancreatic cancer in the subject. In some embodiments, the level of the target miR gene product in the test sample is greater than the level of the corresponding miR gene product in the control sample (i.e., expression of the miR gene product is "up-regulated" or the miR gene product is "over-expressed"). As used herein, expression of an miR gene product is "up-regulated" when the amount of miR gene product in a test sample from a subject is greater than the amount of the same gene product in a control sample.

In some embodiments, the up-regulated miR gene products include one or more of the following: let-7a-2-P, let-7b, let-7c, let-7d, let-7f-1, let-7i, MIR-001-2, MIR-007-1, MIR-015a, MIR-015b, MIR-016-1, MIR-019b-1-P, MIR-021, MIR-023a, MIR-024-1,2, MIR-027a, b, MIR-029a,c, MIR-030d, MIR-032, MIR-092-1, MIR-098, MIR-099a, MIR-100, MIR-107, MIR-125b-1, MIR-126, MIR-128a, MIR-132, MIR-136, MIR-142-P, MIR-145-P, MIR-152, MIR-155, MIR-181a,c, MIR-196a-2, MIR-212, MIR-213, MIR-215, MIR-218-1,2, MIR-221, MIR-222-P, MIR-301, MIR-328, MIR-331-P, MIR-345, MIR-367, MIR-376, MIR-424,

In other embodiments, the level of the target miR gene product in the test sample is less than the level of the corresponding miR gene product in the control sample (i.e., expression of the miR gene product is "down-regulated" or the miR gene product is "under-expressed"). As used herein, expression of an miR gene is "down-regulated" when the amount of miR gene product in a test sample from a subject is less than the amount of the same gene product in a control sample. The relative miR gene expression in the control samples can be determined with respect to one or more RNA expression standards. The standards can comprise, for example, the average level of miR gene expression previously obtained for a population of normal controls.

In some embodiments, the down-regulated miR gene products include one or more of the following: MIR-092-2-P, MIR-096-P, MIR-129-2, MIR-133b, MIR-139, MIR-188b-P, MIR-204, MIR-299-P, MIR-337, MIR-371, MIR-383, MIR-375.

Since more than one miR gene products is associated with pancreatic cancer, it is understood that pancreatic cancer can be diagnosed by evaluating any one of the listed miR gene products, or by evaluating any combination of the listed miR gene products that when profiled, are diagnostic of pancreatic cancer. In some examples, a miR gene product that is uniquely associated with pancreatic adenocarcinoma is evaluated.

A change in levels of miR gene products associated with pancreatic cancer can be detected prior to, or in the early stages of, the development of transformed or neoplastic phenotypes in cells of a subject. The invention therefore also provides a method for screening a subject who is at risk of developing pancreatic cancer, comprising evaluating the level of at least one miR gene product, or a combination of miR gene products, associated with pancreatic cancer in a biological sample obtained form the subject's pancreas. Accordingly, an alteration in the level of the miR gene product, or combination of miR gene products, in the biological sample as compared to the level of a corresponding miR gene product in a control sample, is indicative of the subject being at risk for developing pancreatic cancer. The biological sample used for such screening can include pancreatic tissue that is either normal or suspected to be precancerous. Subjects with a change in the level of one or more miR gene products associated with pancreatic cancer are candidates for further monitoring and testing. Such further testing can comprise histological examination of tissue samples, or other techniques within the skill in the art.

The term "target nucleotide sequence" or "target nucleotide" as used herein, refers to the polynucleotide sequence that is sought to be detected. Target nucleotide sequence is intended to include DNA (e.g., cDNA or genomic DNA), RNA, analogs of the DNA or RNA generated using nucleotide analogs, and derivatives, fragments and homologs thereof.

The level of a miR gene product in a sample can be measured using any technique that is suitable for detecting RNA expression levels in a biological sample. Suitable techniques for determining RNA expression levels in biological sample include amplification-based and hybridization-based assays.

Amplification-based assays use a nucleic acid sequence of the miR gene product, or the *miR* gene, as a template in an amplification reaction (for example polymerase chain reaction or PCR). The relative number of miR gene transcripts can also be determined by reverse transcription of miR gene transcripts, followed by amplification of the reverse-transcribed transcripts by polymerase chain reaction (RT-PCR). The levels of miR gene transcripts can be quantified in comparison with an internal standard, for example, the level of mRNA from a "housekeeping" gene present in the same sample. A suitable "housekeeping" gene for use as an internal standard includes, e.g., 18S rRNA, myosin or glyceraldehyde-3-phosphate dehydrogenase (G3PDH). In a quantitative amplification, the amount of amplification product will be proportional to the amount of template in the original sample. Methods of real-time quantitative PCR or RT-PCR using TaqMan probes are well known in the art and are described in for example, Heid et al. 1996, Real time quantitative PCR, Genome Res., 10:986-994; and Gibson et al., 1996, A novel method for real time quantitative RT-PCR, Genome Res. 10:995-1001. A quantitative real-time RT-PCR method that can determine the expression level of the transcripts of all known miR genes correlated with a cancer is described in Jiang, J., et al. (2005), Nucleic Acids Res. 33, 5394-5403; Schmittgen T. D., et al. (2004), Nucleic Acids Res. 32, E43; and U.S. Provisional Application Ser. No. 60/656,109, filed February 24, 2005 .
Other examples of amplification-based assays for detection of miRNAs are well known in the art, see for example the description in US PAT Appl. No. 2006/0078924.

Hybridization-based assays can also be used to detect the level of miR gene products in a sample. These assays, including for example Northern blot analysis, in-situ hybridization, solution hybridization, and RNAse protection assay (Ma YJ, et al. (1996) RNase protection assay, Methods, 10:273-8) are well known to those of skill in the art.

As used herein, the term "hybridization" refers to the complementary base-pairing interaction of one nucleic acid with another nucleic acid that results in formation of a duplex, triplex, or other higher-ordered structure, and is used herein interchangeably with "annealing." Typically, the primary interaction is base specific, e.g., A/T and G/C, by Watson/Crick and Hoogsteen-type hydrogen bonding. Base-stacking and hydrophobic interactions can also contribute to duplex stability. Conditions for hybridizing detector probes and primers to complementary and substantially complementary target sequences are well known, e.g., as described in, e.g., Nucleic Acid Hybridization, A Practical Approach, B. Hames and S. Higgins, eds., IRL Press, Washington, D.C. (1985). In general, whether such annealing takes place is influenced by, among other things, the length of the polynucleotides and the complementary, the pH, the temperature, the presence of mono- and divalent cations, the proportion of G and C nucleotides in the hybridizing region, the viscosity of the medium, and the presence of denaturants. Such variables influence the time required for hybridization. Thus, the preferred annealing conditions will depend upon the particular application. Such conditions, however, can be routinely determined by the person of ordinary skill in the art without undue experimentation. It will be appreciated that complementarity need not be perfect; there can be a small number of base pair mismatches that will minimally interfere with hybridization between the target sequence and the single stranded nucleic acids of the present teachings. However, if the number of base pair mismatches is so great that no hybridization can occur under minimally stringent conditions then the sequence is generally not a complementary target sequence. Thus, complementarity herein is meant that the probes or primers are sufficiently complementary to the target sequence to hybridize under the selected reaction conditions to achieve the ends of the present teachings.

A suitable technique for determining the level of RNA transcripts of a particular gene in a biological sample is Northern blotting. For example, total cellular RNA can be purified from cells by homogenization in the presence of nucleic acid extraction buffer, followed by centrifugation. Nucleic acids are precipitated, and DNA is removed by treatment with DNase and precipitation. The RNA molecules are then separated by gel electrophoresis on agarose gels according to standard techniques, and transferred to nitrocellulose filters. The RNA is then immobilized on the filters by heating. Detection and quantification of specific RNA is accomplished using appropriately labeled DNA or RNA probes complementary to the RNA in question. See, for example, Molecular Cloning: A Laboratory Manual, J. Sambrook et al., eds., 2nd edition, Cold Spring Harbor Laboratory Press, 1989, Chapter 7.

Suitable probes for Northern blot hybridization of a given miR gene product can be produced from the nucleic acid sequences provided in Fig. 2 or published sequences of known miRNA species that are available, for example on the miRNA registry at (http://www.sanger.ac.uk/Software/Rfam/mirna/index.shtml) (Griffiths-Jones, S., The microRNA Registry. Nucleic Acids Res, 2004. 32(1): p. D109-11.). Methods for preparation of labeled DNA and RNA probes, and the conditions for hybridization thereof to target nucleotide sequences, are known in the art and are described, for example, in Molecular Cloning: A Laboratory Manual, J. Sambrook et al., eds., 2nd edition, Cold Spring Harbor Laboratory Press, 1989, Chapters 10 and 11 .

In addition to Northern and other RNA hybridization techniques, determining the levels of RNA transcripts can be accomplished using the technique of in situ hybridization. This technique requires fewer cells than the Northern blotting technique, and involves depositing whole cells onto a microscope cover slip or slide and probing the nucleic acid content of the cell with a solution containing radioactive or otherwise labeled nucleic acid (e.g., cDNA or RNA) probes. This technique is particularly well-suited for analyzing tissue biopsy samples from subjects. The practice of the in situ hybridization technique is described in more detail in U.S. Pat. No. 5,427,916 .
Suitable probes for in situ hybridization of a given miR gene product can be produced from the nucleic acid sequences provided in Fig. 2, as described above.

A suitable technique for simultaneously measuring the expression level of multiple miR gene products in a sample is a high-throughput, microarray-based method. Such a technique may be used to, for example, determine the expression level of the transcripts of all known miR genes correlated with cancer. Such a method involves constructing an oligolibrary, in microchip format (i.e., a microarray), that contains a set of probe oligonucleotides that are specific for a set of miR genes. Using such a microarray, the expression level of multiple microRNAs in a biological sample is determined by reverse transcribing the RNAs to generate a set of target oligonucleotides, and hybridizing them to probe oligonucleotides on the microarray to generate a hybridization, or expression, profile. The hybridization profile of the test sample can then be compared to that of a control sample to determine which microRNAs have an altered expression level in pancreatic cancer or precancerous cells. In one example, the oligolibrary contains probes corresponding to all known miRs from the human genome. The microarray may be expanded to include additional miRNAs as they are discovered. The array can contain two different oligonucletode probes for each miRNA, one containing the active sequence of the mature miR and the other being specific for the miR precursor. The array may also contain controls for hybridization stringency conditions. An example of a microarray technique for detecting miRNAs is described in US PAT Appl No. 2005/0277139 .

The level of miR gene products can also be determined by using an assay that detects the copy number of the miR gene that encodes the miR gene product. The presence of an miR gene that has undergone amplification in tumors is evaluated by determining the copy number of the miR genes, i.e., the number of DNA sequences in a cell encoding the miR gene products. Generally, a normal diploid cell has two copies of a given autosomal gene. The copy number can be increased, however, by gene amplification or duplication, for example, in cancer cells, or reduced by deletion. Methods of evaluating the copy number of a particular gene are well known in the art, and include, inter alia, hybridization and amplification based assays.

Any of a number of hybridization based assays can be used to detect the copy number of an miR gene in the cells of a biological sample. One such method is Southern blot analysis (see Ausubel, et al., Eds., Current Protocols in Molecular Biology, Greene Publishing and Wiley-Interscience, New York, 1995; Sambrook et al., Molecular Cloning, A Laboratory Manual 2d Ed.), where the genomic DNA is typically fragmented, separated electrophoretically, transferred to a membrane, and subsequently hybridized to an miR gene specific probe. Comparison of the intensity of the hybridization signal from the probe for the target region with a signal from a control probe from a region of normal nonamplified, single-copied genomic DNA in the same genome provides an estimate of the relative miR gene copy number, corresponding to the specific probe used. An increased signal compared to control represents the presence of amplification.

A methodology for determining the copy number of the miR gene in a sample is in situ hybridization, for example, fluorescence in situ hybridization (FISH) (see Angerer, 1987 Meth. Enzymol., 152: 649). Generally, in situ hybridization comprises the following major steps: (1) fixation of tissue or biological structure to be analyzed; (2) prehybridization treatment of the biological structure to increase accessibility of target DNA, and to reduce nonspecific binding; (3) hybridization of the mixture of nucleic acids to the nucleic acid in the biological structure or tissue; (4) post-hybridization washes to remove nucleic acid fragments not bound in the hybridization, and (5) detection of the hybridized nucleic acid fragments. The probes used in such applications are typically labeled, for example, with radioisotopes or fluorescent reporters. Suitable probes are sufficiently long, for example, from about 50, 100, or 200 nucleotides to about 1000 or more nucleotides, to enable specific hybridization with the target nucleic acid(s) under stringent conditions.

Another alternative methodology for determining number of DNA copies is comparative genomic hybridization (CGH). In comparative genomic hybridization methods, a "test" collection of nucleic acids is labeled with a first label, while a second collection (for example, from a normal cell or tissue) is labeled with a second label. The ratio of hybridization of the nucleic acids is determined by the ratio of the first and second labels binding to each fiber in an array. Differences in the ratio of the signals from the two labels, for example, due to gene amplification in the test collection, is detected and the ratio provides a measure of the miR gene copy number, corresponding to the specific probe used. A cytogenetic representation of DNA copy-number variation can be generated by CGH, which provides fluorescence ratios along the length of chromosomes from differentially labeled test and reference genomic DNAs.

Hybridization protocols suitable for use with the methods of the invention are described, for example, in Albertson (1984) EMBO J. 3:1227-1234; Pinkel (1988) Proc. Natl. Acad. Sci. USA, 85:9138-9142; EPO Pub. No. 430:402; Methods in Molecular Biology, Vol. 33: In Situ Hybridization Protocols, Choo, ed., Humana Press, Totowa, N.J. (1994).

Amplification-based assays also can be used to measure the copy number of the miR gene. In such assays, the corresponding miR nucleic acid sequences act as a template in an amplification reaction (for example, PCR). In a quantitative amplification, the amount of amplification product will be proportional to the amount of template in the original sample. Comparison to appropriate controls provides a measure of the copy number of the miR gene, corresponding to the specific probe used, according to the principles discussed above. Methods of real-time quantitative PCR using TaqMan probes are well known in the art. Detailed protocols for real-time quantitative PCR are provided, for example, in: Heid et al., 1996, Real time quantitative PCR. Genome Res., 10:986-994.

A TaqMan-based assay also can be used to quantify miR polynucleotides. TaqMan based assays use a fluorogenic oligonucleotide probe that contains a 5' fluorescent dye and a 3' quenching agent. The probe hybridizes to a PCR product, but cannot itself be extended due to a blocking agent at the 3' end. When the PCR product is amplified in subsequent cycles, the 5' nuclease activity of the polymerase, for example, AmpliTaq, results in the cleavage of the TaqMan probe. This cleavage separates the 5' fluorescent dye and the 3' quenching agent, thereby resulting in an increase in fluorescence as a function of amplification (see, for example, http://www2.perkin-elmer.com).

Other examples of suitable amplification methods include, but are not limited to, ligase chain reaction (LCR) (see, Wu and Wallace, Genomics, 4: 560, 1989; Landegren et al., Science, 241: 1077, 1988; and Barringer et al., Gene, 89:117, 1990), transcription amplification (Kwoh et al., Proc. Natl. Acad. Sci. USA, 86:1173, 1989), self-sustained sequence replication (Guatelli et al., Proc Nat Acad Sci, USA 87:1874, 1990), dot PCR, and linker adapter PCR,.

One powerful method for determining DNA copy numbers uses microarray-based platforms. Microarray technology may be used because it offers high resolution. For example, the traditional CGH generally has a 20 Mb limited mapping resolution; whereas in microarray-based CGH, the fluorescence ratios of the differentially labeled test and reference genomic DNAs provide a locus-by-locus measure of DNA copy-number variation, thereby achieving increased mapping resolution. Details of various microarray methods can be found in the literature. See, for example, U.S. Pat. No. 6,232,068; Pollack et al., Nat. Genet., 23(1):41-6, (1999), and others.

As used herein, "probe oligonucleotide" refers to an oligonucleotide that is capable of hybridizing to a target oligonucleotide. "Target oligonucleotide" refers to a molecule or sequence to be detected (e.g., via hybridization). By "miR-specific probe oligonucleotide" or "probe oligonucleotide specific for an miR" is meant a probe oligonucleotide that has a sequence selected to hybridize to a specific miR gene product, or to a reverse transcript of the specific miR gene product.

An "expression profile" or "hybridization profile" of a particular sample is essentially a fingerprint of the state of the sample; while two states may have any particular gene similarly expressed, the evaluation of a number of genes simultaneously allows the generation of a gene expression profile that is unique to the state of the cell. That is, normal tissue may be distinguished from pancreatic cancer tissue, pancreatitis tissue or "benign tissue" obtained from a non-cancerous part of a subject's pancreas adjacent the cancerous tissue. By comparing expression profiles of pancreatic tissue in different states, information regarding which genes are important (including both up- and down-regulation of genes) in each of these states is obtained. The identification of sequences that are differentially expressed in pancreatic cancer tissue or normal pancreatic tissue, as well as differential expression resulting in different prognostic outcomes, allows the use of this information in a number of ways. For example, a particular treatment regime may be evaluated (e.g., to determine whether a chemotherapeutic drug acts to improve the long-term prognosis in a particular patient). Similarly, diagnosis may be done or confirmed by comparing patient samples with the known expression profiles. Furthermore, these gene expression profiles (or individual genes) allow screening of drug candidates that suppress the pancreatic cancer expression profile or convert a poor prognosis profile to a better prognosis profile.

Accordingly, the target nucleotide sequence of the miR gene product to be detected can be: (a) a portion of, or the entire sequence of the mature miRNA; (b) a portion of, or the entire hairpin sequence of the miRNA precursor; or (c) a portion of or the entire sequence of the pri-miRNA; (d) the complement of sequences (a)-(c); or (f) a sequence that is substantially identical to the sequences (a)-(d). (See Fig. 1). A substantially identical nucleic acid may have greater than 80%, 85%, 90%, 95%, 97%, 98% or 99% sequence identity to the target sequence.

In one example of the invention, the level of miR gene products is detected by profiling miRNA precursors on biopsy specimens of human pancreatic tissue or pancreatic cells. The sequences of 201 profiled miR gene products are provided in Fig. 2. All nucleic acid sequences herein are given in the 5' to 3' direction. In addition, genes are represented by italics, and gene products are represented by normal type; e.g., *mir-17* is the gene and miR-17 is the gene product. The primers used to amplify the human miRNAs precursors are shown in Fig. 4.

In another example, the level of miR gene products is detected by profiling mature miRNAs on biopsy specimens of human pancreatic cancer or benign tissue. The sequences of the profiled mature miR gene products are provided in Figs. 13 and 16.

In another example, expression of the active, mature miRNA is evaluated using Northern blotting.

In another example, reverse transcription in situ PCR is used to localize three of the top differentially expressed miRNAs to pancreatic tumor cells.

Also contemplated is a kit for diagnosing pancreatic cancer in a subject suspected of having, or being at risk for developing, pancreatic cancer. Such a kit can include: (a) a means for measuring the level of at least one miR gene product in a biological sample derived from the subject's pancreas, and (b) a means for comparing the level of the miR gene product in the biological sample to the level of a corresponding miR gene product in a control sample. Accordingly, a detected difference between the level of the miR gene product in the biological sample as compared with the level of the corresponding miR gene product in the control sample is indicative of the subject either having, or being at risk for developing, pancreatic cancer.

In addition to diagnosing whether a subject has, or is at risk for developing, pancreatic cancer, the present disclosure contemplates methods for treating subjects who have altered expression of pancreatic cancer-specific miR gene products. Without wishing to be bound by any one theory, it is believed that alterations in the level of one or more miR gene products in cells can result in the deregulation of one or more intended targets for these miRs, which can lead to the formation of cancer. Therefore, altering the level of the cancer-specific miR gene product (e.g., by decreasing the level of a miR gene product that is up-regulated in cancer cells, and/or by increasing the level of a miR gene product that is down-regulated in cancer cells) may successfully treat the pancreatic cancer.

Accordingly, the present disclosure encompasses methods of treating pancreatic cancer in a subject, wherein at least one pancreatic cancer-specific miR gene product is de-regulated (e.g., down-regulated, up-regulated) in the cancer cells of the subject. When the miR gene product is down-regulated in the pancreatic cancer cells, the method comprises administering an effective amount of the miR gene product such that progression of cancer in the subject is inhibited. When the miR gene product is up-regulated in the cancer cells, the method comprises administering to the subject an effective amount of at least one compound for inhibiting expression of the up-regulated miR gene, referred to herein as miR gene expression inhibition compounds, such that progression of cancer in the subject is inhibited.

The terms "inhibiting the progression" of cancer or "treating" cancer mean stopping or slowing cancer formation, development, or growth and elimination or reduction of cancer symptoms, including invasion and/or metastasis. Such treatment includes causing further differentiation of cancer cells.

As used herein, an "effective amount" of an isolated miR gene product is an amount sufficient to inhibit progression of cancer in a subject suffering from pancreatic cancer. One skilled in the art can readily determine an effective amount of an miR gene product to be administered to a given subject, by taking into account factors, such as the size and weight of the subject; the extent of disease penetration; the age, health and sex of the subject; the route of administration; and whether the administration is regional or systemic.

For example, an effective amount of an isolated miR gene product can be based on the approximate weight of a tumor mass to be treated. The approximate weight of a tumor mass can be determined by calculating the approximate volume of the mass, wherein one cubic centimeter of volume is roughly equivalent to one gram. An effective amount of the isolated miR gene product based on the weight of a tumor mass can be in the range of about 10-500 micrograms/gram of tumor mass. In certain embodiments, the tumor mass can be at least about 10 micrograms/gram of tumor mass, at least about 60 micrograms/gram of tumor mass or at least about 100 micrograms/gram of tumor mass.

An effective amount of an isolated miR gene product can also be based on the approximate or estimated body weight of a subject to be treated. Such effective amounts can be administered parenterally or enterally, as described herein.

One skilled in the art can also readily determine an appropriate dosage regimen for the administration of an isolated miR gene product to a given subject. For example, an miR gene product can be administered to the subject once (e.g., as a single injection or deposition). Alternatively, an miR gene product can be administered once or twice daily to a subject for a period of days to several months, particularly from about three to about twenty-eight days, more particularly from about seven to about ten days. In a particular dosage regimen, an miR gene product is administered once a day for seven days. Where a dosage regimen comprises multiple administrations, it is understood that the effective amount of the miR gene product administered to the subject can comprise the total amount of gene product administered over the entire dosage regimen.

As used herein, an "isolated" miR gene product is one which is synthesized, or altered or removed from the natural state through human intervention. For example, a synthetic miR gene product, or an miR gene product partially or completely separated from the coexisting materials of its natural state, is considered to be "isolated." An isolated miR gene product can exist in substantially-purified form, or can exist in a cell into which the miR gene product has been delivered.

The isolated gene products can be oligonucleotides comprising the functional mature miR gene product, oligonucleotides comprising the short hairpin of miRNA precursors containing the looped portion of the hairpin, duplex miRNA precursors lacking the hairpin, or vectors expressing such molecules. Thus, an miR gene product which is deliberately delivered to, or expressed in, a cell is considered an "isolated" miR gene product. An miR gene product produced inside a cell from an miR precursor molecule is also considered to be an "isolated" molecule.

In some embodiments, the isolated miR gene products include one or more of the following: MIR-092-2-P, MIR-096-P, MIR-129-2, MIR-133b, MIR-139, MIR-188b-P, MIR-204, MIR-299-P, MIR-337, MIR-371, MIR-383, MIR-375.

Isolated miR gene products can be obtained using a number of standard techniques. For example, the miR gene products can be chemically synthesized or recombinantly produced using methods known in the art. In one embodiment, miR gene products are chemically synthesized using appropriately protected ribonucleoside phosphoramidites and a conventional DNA/RNA synthesizer. Commercial suppliers of synthetic RNA molecules or synthesis reagents include, e.g., Proligo (Hamburg, Germany), Dharmacon Research (Lafayette, CO, U.S.A.), Pierce Chemical (part of Perbio Science, Rockford, IL, U.S.A.), Glen Research (Sterling, VA, U.S.A.), ChemGenes (Ashland, MA, U.S.A.) and Cruachem (Glasgow, UK).

An isolated miR gene product may be administered as naked RNA, in conjunction with a delivery agent. Alternatively, the miR gene product can be expressed from a recombinant circular or linear DNA plasmid using any suitable promoter. Suitable promoters for expressing RNA from a plasmid include, e.g., the U6 or H1 RNA pol III promoter sequences, or the cytomegalovirus promoters. Selection of other suitable promoters is within the skill in the art. The recombinant plasmids of the invention can also comprise inducible or regulatable promoters for expression of the miR gene products in cancer cells.

The miR gene products that are expressed from recombinant plasmids can be isolated from cultured cell expression systems by standard techniques. The miR gene products which are expressed from recombinant plasmids can also be delivered to, and expressed directly in, the cancer cells. The use of recombinant plasmids to deliver the miR gene products to cancer cells is discussed in more detail below.

The miR gene products can be expressed from a separate recombinant plasmid, or they can be expressed from the same recombinant plasmid. In one embodiment, the miR gene products are expressed as RNA precursor molecules from a single plasmid, and the precursor molecules are processed into the functional mature miR gene product by a suitable processing system, including, but not limited to, processing systems extant within a cancer cell. Other suitable processing systems include, e.g., the in vitro Drosophila cell lysate system (e.g., as described in U.S. Published Patent Application No. 2002/0086356 to Tuschl et al. ) and the E. coli RNAse III system (e.g., as described in U.S. Published Patent Application No. 2004/0014113 to Yang et al. ) .

Selection of plasmids suitable for expressing the miR gene products, methods for inserting nucleic acid sequences into the plasmid to express the gene products, and methods of delivering the recombinant plasmid to the cells of interest are within the skill in the art. See, for example, Zeng et al. (2002), Molecular Cell 9:1327-1333; Tuschl (2002), Nat. Biotechnol, 20:446-448; Brummelkamp et al. (2002), Science 296:550-553; Miyagishi et al. (2002), Nat. Biotechnol. 20:497-500; Paddison et al. (2002), Genes Dev. 16:948-958; Lee et al. (2002), Nat. Biotechnol. 20:500-505; and Paul et al. (2002), Nat. Biotechnol. 20:505-508.

In one embodiment, a plasmid expressing the miR gene products comprises a sequence encoding a miR precursor RNA under the control of the CMV intermediate-early promoter. As used herein, "under the control" of a promoter means that the nucleic acid sequences encoding the miR gene product are operatively linked to the promoter, so that the promoter can initiate transcription of the miR gene product coding sequences.

The miR gene products can also be expressed from recombinant viral vectors. It is contemplated that the miR gene products can be expressed from two separate recombinant viral vectors, or from the same viral vector. The RNA expressed from the recombinant viral vectors can either be isolated from cultured cell expression systems by standard techniques, or can be expressed directly in cancer cells. The use of recombinant viral vectors to deliver the miR gene products to cancer cells is discussed in more detail below.

The recombinant viral vectors of the invention comprise sequences encoding the miR gene products and any suitable promoter for expressing the RNA sequences. Suitable promoters include, for example, the U6 or H1 RNA pol III promoter sequences, or the cytomegalovirus promoters. Selection of other suitable promoters is within the skill in the art. The recombinant viral vectors of the invention can also comprise inducible or regulatable promoters for expression of the miR gene products in a cancer cell.

Any viral vector capable of accepting the coding sequences for the miR gene products can be used; for example, vectors derived from adenovirus (AV); adeno-associated virus (AAV); retroviruses (e.g., lentiviruses (LV), Rhabdoviruses, murine leukemia virus); herpes virus, and the like. The tropism of the viral vectors can be modified by pseudotyping the vectors with envelope proteins or other surface antigens from other viruses, or by substituting different viral capsid proteins, as appropriate.

For example, the vectors of the invention can be pseudotyped with surface proteins from vesicular stomatitis virus (VSV), rabies, Ebola, Mokola, and the like. AAV vectors of the invention can be made to target different cells by engineering the vectors to express different capsid protein serotypes. For example, an AAV vector expressing a serotype 2 capsid on a serotype 2 genome is called AAV 2/2. This serotype 2 capsid gene in the AAV 2/2 vector can be replaced by a serotype 5 capsid gene to produce an AAV 2/5 vector. Techniques for constructing AAV vectors that express different capsid protein serotypes are within the skill in the art; see, e.g., Rabinowitz, J.E., et al. (2002), J. Virol. 76:791-801.

Selection of recombinant viral vectors suitable for use in the invention, methods for inserting nucleic acid sequences for expressing RNA into the vector, methods of delivering the viral vector to the cells of interest, and recovery of the expressed RNA products are within the skill in the art. See, for example, Dornburg (1995), Gene Therap. 2:301-310; Eglitis (1988), Biotechniques 6:608-614; Miller (1990), Hum. Gene Therap. 1:5-14; and Anderson (1998), Nature 392:25-30 .

Suitable viral vectors are those derived from AV and AAV. A suitable AV vector for expressing the miR gene products, a method for constructing the recombinant AV vector, and a method for delivering the vector into target cells, are described in Xia et al. (2002), Nat. Biotech. 20:1006-1010 .
Suitable AAV vectors for expressing the miR gene products, methods for constructing the recombinant AAV vector, and methods for delivering the vectors into target cells are described in Samulski et al. (1987), J. Virol. 61:3096-3101; Fisher et al. (1996), J. Virol., 70:520-532; Samulski et al. (1989), J. Virol. 63:3822-3826; U.S. Pat. No. 5,252,479; U.S. Pat. No. 5,139,941; International Patent Application No. WO 94/13788; and International Patent Application No. WO 93/24641 .
In one embodiment, the miR gene products are expressed from a single recombinant AAV vector comprising the CMV intermediate early promoter.

In one embodiment, a recombinant AAV viral vector of the invention comprises a nucleic acid sequence encoding an miR precursor RNA in operable connection with a polyT termination sequence under the control of a human U6 RNA promoter. As used herein, "in operable connection with a polyT termination sequence" means that the nucleic acid sequences encoding the sense or antisense strands are immediately adjacent to the polyT termination signal in the 5' direction. During transcription of the miR sequences from the vector, the polyT termination signals act to terminate transcription.

Another method of altering miRNA expression levels in pancreatic cancer is through epigenetic events such as hyper- or hypo- methylation of the promoters of miRNA genes. Hyper methylation of the promoter will result in reduced expression of the miRNA gene and hypomethylation of the promoter will result in increased expression of the miRNA gene. (Jones PA, Baylin SB. The fundamental role of epigenetic events in cancer. Nat Rev Genet. 3:415-28 (2005)). Therefore, another method of inhibiting the progression of cancer is the administration of a compound that causes hypo-methylation of the promoter region of a down-regulated miR gene product.

In other embodiments of the treatment methods of the invention, an effective amount of at least one compound which inhibits miR expression can also be administered to the subject. As used herein, "inhibiting miR expression" means that the production of the active, mature form of miR gene product after treatment is less than the amount produced prior to treatment. One skilled in the art can readily determine whether miR expression has been inhibited in a cancer cell, using for example the techniques for determining miR transcript level discussed above for the diagnostic method. Inhibition can occur at the level of gene expression (i.e., by inhibiting transcription of a miR gene encoding the miR gene product) or at the level of processing (e.g., by inhibiting processing of a miR precursor into a mature, active miR).

As used herein, an "effective amount" of a compound that inhibits miR expression is an amount sufficient to inhibit progression of cancer in a subject suffering from pancreatic cancer. One skilled in the art can readily determine an effective amount of an miR expression-inhibiting compound to be administered to a given subject, by taking into account factors, such as the size and weight of the subject; the extent of disease penetration; the age, health and sex of the subject; the route of administration; and whether the administration is regional or systemic.

For example, an effective amount of the expression-inhibiting compound can be based on the approximate weight of a tumor mass to be treated. The approximate weight of a tumor mass can be determined by calculating the approximate volume of the mass, wherein one cubic centimeter of volume is roughly equivalent to one gram. An effective amount based on the weight of a tumor mass can be between about 10-500 micrograms/gram of tumor mass, at least about 10 micrograms/gram of tumor mass, at least about 60 micrograms/gram of tumor mass, and at least about 100 micrograms/gram of tumor mass.

An effective amount of a compound that inhibits miR expression can also be based on the approximate or estimated body weight of a subject to be treated. Such effective amounts are administered parenterally or enterally, among others, as described herein. For example, an effective amount of the expression-inhibiting compound administered to a subject can range from about 5̃ -3000 micrograms/kg of body weight, from about 700 - 1000 micrograms/kg of body weight, or it can be greater than about 1000 micrograms/kg of body weight.

One skilled in the art can also readily determine an appropriate dosage regimen for administering a compound that inhibits miR expression to a given subject. For example, an expression-inhibiting compound can be administered to the subject once (e.g., as a single injection or deposition). Alternatively, an expression-inhibiting compound can be administered once or twice daily to a subject for a period of from about a few days to a few months, from about three to about twenty-eight days, or from about seven to about ten days. In a particular dosage regimen, an expression-inhibiting compound is administered once a day for seven days. Where a dosage regimen comprises multiple administrations, it is understood that the effective amount of the expression-inhibiting compound administered to the subject can comprise the total amount of compound administered over the entire dosage regimen.

In some embodiments, the miR gene products whose levels can be reduced include one or more of the following: let-7a-2-P, let-7b, let-7c, let-7d, let-7f-1, let-7i, MIR-001-2, MIR-007-1, MIR-015a, MIR-015b, MIR-016-1, MIR-019b-1-P, MIR-021, MIR-023a, MIR-024-1,2, MIR-027a, b, MIR-029a,c, MIR-030d, MIR-032, MIR-092-1, MIR-098, MIR-099a, MIR-100, MIR-107, MIR-125b-1, MIR-126, MIR-128a, MIR-132, MIR-136, MIR-142-P, MIR-145-P, MIR-152, MIR-155, MIR-181a,c, MIR-196a-2, MIR-212, MIR-213, MIR-215, MIR-218-1,2, MIR-221, MIR-222-P, MIR-301, MIR-328, MIR-331-P, MIR-345, MIR-367, MIR-376, MIR-424.

Suitable compounds for inhibiting miR gene expression include double-stranded RNA (such as short- or small-interfering RNA or "siRNA"), antisense nucleic acids, enzymatic RNA molecules such as ribozymes, or molecules capable of forming a triple helix with the miR gene. Another class of inhibitor compound can cause hyper-methylation of the miR gene product promoter, resulting in reduced expression of the miR gene. Each of these compounds can be targeted to a given miR gene product to destroy, induce the destruction of, or otherwise reduce the level of the target miR gene product.

For example, expression of a given miR gene can be inhibited by inducing RNA interference of the miR gene with an isolated double-stranded RNA ("dsRNA") molecule which has at least 90%, for example at least 95%, at least 98%, at least 99% or 100%, sequence homology with at least a portion of the miR gene product. In a particular embodiment, the dsRNA molecule is a "short or small interfering RNA" or "siRNA."

siRNA useful in the present methods comprise short double-stranded RNA from about 17 nucleotides to about 29 nucleotides in length, or from about 19 to about 25 nucleotides in length. The siRNA comprise a sense RNA strand and a complementary antisense RNA strand annealed together by standard Watson-Crick base-pairing interactions (hereinafter "base-paired"). The sense strand comprises a nucleic acid sequence which is substantially identical to a nucleic acid sequence contained within the target miR gene product.

As used herein, a nucleic acid sequence in an siRNA which is "substantially identical" to a target sequence contained within the target mRNA is a nucleic acid sequence that is identical to the target sequence, or that differs from the target sequence by one or two nucleotides. The sense and antisense strands of the siRNA can comprise two complementary, single-stranded RNA molecules, or can comprise a single molecule in which two complementary portions are base-paired and are covalently linked by a single-stranded "hairpin" area.

The siRNA can also be altered RNA that differs from naturally-occurring RNA by the addition, deletion, substitution and/or alteration of one or more nucleotides. Such alterations can include addition of non-nucleotide material, such as to the end(s) of the siRNA or to one or more internal nucleotides of the siRNA, or modifications that make the siRNA resistant to nuclease digestion, or the substitution of one or more nucleotides in the siRNA with deoxyribo-nucleotides.

The siRNA can also be engineered to contain certain "drug like" properties. Such modifications include chemical modifications for stability and cholesterol conjugation for delivery. Such modifications impart better pharmacological properties to the siRNA and using such modifications, pharmacologically active siRNAs can achieve broad biodistribution and efficient silencing of miRNAs in most tissues in vivo.

One or both strands of the siRNA can also comprise a 3' overhang. As used herein, a "3' overhang" refers to at least one unpaired nucleotide extending from the 3'-end of a duplexed RNA strand. Thus, in certain embodiments, the siRNA comprises at least one 3' overhang of from 1 to about 6 nucleotides (which includes ribonucleotides or deoxyribonucleotides) in length, from 1 to about 5 nucleotides in length, from 1 to about 4 nucleotides in length, or from about 2 to about 4 nucleotides in length. In one embodiment, the 3' overhang is present on both strands of the siRNA, and is 2 nucleotides in length. For example, each strand of the siRNA can comprise 3' overhangs of dithymidylic acid ("TT") or diuridylic acid ("uu").

The siRNA can be produced chemically or biologically, or can be expressed from a recombinant plasmid or viral vector, as described above for the isolated miR gene products. Exemplary methods for producing and testing dsRNA or siRNA molecules are described in U.S. Published Patent Application No. 2002/0173478 to Gewirtz and in U.S. Published Patent Application No. 2004/0018176 to Reich et al..
Methods for synthesizing and validating a therapeutically effective siRNA engineered to silence miRNAs in vivo is described in Krutzfeldt J, et al. (2005), Silencing of microRNAs in vivo with 'antagomirs,' Nature 438(7068):685-9 .

Expression of a given miR gene can also be inhibited by an antisense nucleic acid. As used herein, an "antisense nucleic acid" refers to a nucleic acid molecule that binds to target RNA by means of RNA-RNA or RNA-DNA or RNA-peptide nucleic acid interactions, which alters the activity of the target RNA. Antisense nucleic acids suitable for use in the present methods are single-stranded nucleic acids (e.g., RNA, DNA, RNA-DNA chimeras, PNA) that generally comprise a nucleic acid sequence complementary to a contiguous nucleic acid sequence in an miR gene product. The antisense nucleic acid can comprise a nucleic acid sequence that is 50-100% complementary, 75-100% complementary, or 95-100% complementary to a contiguous nucleic acid sequence in an miR gene product. Without wishing to be bound by any theory, it is believed that the antisense nucleic acids activate RNase H or another cellular nuclease that digests the miR gene product/antisense nucleic acid duplex.

For example, in eukaryotes, RNA polymerase catalyzes the transcription of a structural gene to produce mRNA. A DNA molecule can be designed to contain an RNA polymerase template in which the RNA transcript has a sequence that is complementary to that of a preferred mRNA. The RNA transcript is termed an "antisense RNA". Antisense RNA molecules can inhibit mRNA expression (for example, Rylova et al., Cancer Res, 62(3):801-8, 2002; Shim et al., Int. J. Cancer, 94(1):6-15, 2001).

Alternatively, with respect to a first nucleic acid molecule, a second DNA molecule or a second chimeric nucleic acid molecule that is created with a sequence, which is a complementary sequence or homologous to the complementary sequence of the first molecule or portions thereof, is referred to as the "antisense DNA or DNA decoy or decoy molecule" of the first molecule. The term "decoy molecule" also includes a nucleic molecule, which may be single or double stranded, that comprises DNA or PNA (peptide nucleic acid) (Mischiati et al., Int. J. Mol. Med., 9(6):633-9, 2002), and that contains a sequence of a protein binding site, such as a binding site for a regulatory protein or a binding site for a transcription factor. Applications of antisense nucleic acid molecules, including antisense DNA and decoy DNA molecules are known in the art, for example, Morishita et al, Ann. N Y Acad. Sci., 947:294-301, 2001; Andratschke et al., Anticancer Res, 21:(5)3541-3550, 2001 .

Antisense nucleic acids can also contain modifications to the nucleic acid backbone or to the sugar and base moieties (or their equivalent) to enhance target specificity, nuclease resistance, delivery or other properties related to efficacy of the molecule. Such modifications include cholesterol moieties, duplex intercalators, such as acridine, or one or more nuclease-resistant groups.

Antisense nucleic acids can be produced chemically or biologically, or can be expressed from a recombinant plasmid or viral vector, as described above for the isolated miR gene products. Exemplary methods for producing and testing are within the skill in the art; see, e.g., Stein and Cheng (1993), Science 261:1004 and U.S. Pat. No. 5,849,902 to Woolf et al..

Expression of a given miR gene can also be inhibited by an enzymatic nucleic acid. As used herein, an "enzymatic nucleic acid" refers to a nucleic acid comprising a substrate binding region that has complementarity to a contiguous nucleic acid sequence of an miR gene product, and which is able to specifically cleave the miR gene product. The enzymatic nucleic acid substrate binding region can be, for example, 50-100% complementary, 75-100% complementary, or 95-100% complementary to a contiguous nucleic acid sequence in an miR gene product. The enzymatic nucleic acids can also comprise modifications at the base, sugar, and/or phosphate groups. An exemplary enzymatic nucleic acid for use in the present methods is a ribozyme.

Ribozymes are enzymatic RNA molecules capable of catalyzing the specific cleavage of RNA. A review is provided in Rossi, Current Biology, 4:469-471 (1994). The mechanism of ribozyme action involves sequence-specific hybridization of the ribozyme molecule to complementary target RNA, followed by an endonucleolytic cleavage. A composition of ribozyme molecules must include one or more sequences complementary to the target gene mRNA, and must include a well-known catalytic sequence responsible for mRNA cleavage (U.S. Pat. No. 5,093,246).

Specific ribozyme cleavage sites within any potential RNA target are initially identified by scanning the molecule of interest for ribozyme cleavage sites which include the following sequences, GUA, GUU and GUC. Once identified, short RNA sequences of between 15 and 20 ribonucleotides corresponding to the region of the miR gene containing the cleavage site can be evaluated for predicted structural features, for example, secondary structure, that can render an oligonucleotide sequence unsuitable. The suitability of candidate sequences also can be evaluated by testing their accessibility to hybridization with complementary oligonucleotides, using ribonuclease protection assays.

The enzymatic nucleic acids can be produced chemically or biologically, or can be expressed from a recombinant plasmid or viral vector, as described above for the isolated miR gene products. Exemplary methods for producing and testing dsRNA or siRNA molecules are described in Werner and Uhlenbeck (1995), Nucl. Acids Res. 23:2092-96; Hammann et al. (1999), Antisense and Nucleic Acid Drug Dev. 9:25-31; and U.S. Pat. No. 4,987,071 to Cech et al ..

Triple helix forming molecules can be used in reducing the level of a target miR gene activity. Nucleic acid molecules that can associate together in a triple-stranded conformation (triple helix) and that thereby can be used to inhibit translation of a target gene, should be single helices composed of deoxynucleotides. The base composition of these oligonucleotides must be designed to promote triple helix formation via Hoogsteen base pairing rules, which generally require sizeable stretches of either purines or pyrimidines on one strand of a duplex. Nucleotide sequences can be pyrimidine-based, which will result in TAT and CGC triplets across the three associated strands of the resulting triple helix. The pyrimidine-rich molecules provide bases complementary to a purine-rich region of a single strand of the duplex in a parallel orientation to that strand. In addition, nucleic acid molecules can be chosen that are purine-rich, for example, those that contain a stretch of G residues. These molecules will form a triple helix with a DNA duplex that is rich in GC pairs, in which the majority of the purine residues are located on a single strand of the targeted duplex, resulting in GGC triplets across the three strands in the triplex. Alternatively, the potential sequences that can be targeted for triple helix formation can be increased by creating a so-called "switchback" nucleic acid molecule. Switchback molecules are synthesized in an alternating 5'-3', 3'-5' manner, such that they base pair with first one strand of a duplex and then the other, eliminating the necessity for a sizeable stretch of either purines or pyrimidines on one strand of a duplex.

Another method of altering miRNA expression levels in pancreatic cancer is through epigenetic events such as hyper- or hypo- methylation of the promoters of miRNA genes. Hyper methylation of the promoter will result in reduced expression of the miRNA gene and hypomethylation of the promoter will result in increased expression of the miRNA gene. (Jones PA, Baylin SB. The fundamental role of epigenetic events in cancer. Nat Rev Genet. 3:415-28 (2005)).

Administration of at least one miR gene product, or at least one compound for inhibiting miR expression, will inhibit the progression of pancreatic cancer in a subject. Inhibition of cancer cell proliferation can be inferred if the number of such cells in the subject remains constant or decreases after administration of the miR gene products or miR gene expression-inhibiting compounds. An inhibition of cancer cell proliferation can also be inferred if the absolute number of such cells increases, but the rate of tumor growth decreases.

The number of cancer cells in a subject's body can be determined by direct measurement, or by estimation from the size of primary or metastatic tumor masses. For example, the number of cancer cells in a subject can be measured by immunohistological methods, flow cytometry, or other techniques designed to detect characteristic surface markers of cancer cells.

The size of a tumor mass can be ascertained by direct visual observation, or by diagnostic imaging methods, such as X-ray, magnetic resonance imaging, ultrasound, and scintigraphy. Diagnostic imaging methods used to ascertain size of the tumor mass can be employed with or without contrast agents, as is known in the art. The size of a tumor mass can also be ascertained by physical means, such as palpation of the tissue mass or measurement of the tissue mass with a measuring instrument, such as a caliper.

The miR gene products or miR gene expression-inhibiting compounds can be administered to a subject by any means suitable for delivering these compounds to cancer cells of the subject. For example, the miR gene products or miR expression inhibiting compounds can be administered by methods suitable to transfect cells of the subject with these compounds, or with nucleic acids comprising sequences encoding these compounds. In one embodiment, the cells are transfected with a plasmid or viral vector comprising sequences encoding at least one miR gene product or miR gene expression inhibiting compound.

Transfection methods for eukaryotic cells are well known in the art, and include direct injection of the nucleic acid into the nucleus or pronucleus of a cell; electroporation; liposome transfer or transfer mediated by lipophilic materials; receptor mediated nucleic acid delivery, bioballistic or particle acceleration; calcium phosphate precipitation, and transfection mediated by viral vectors.

For example, cells can be transfected with a liposomal transfer compound, e.g., DOTAP (N-[1-(2,3-dioleoyloxy)propyl]-N,N,N-trimethyl-ammo- nium methylsulfate, Boehringer-Mannheim) or an equivalent, such as LIPOFECTIN. The amount of nucleic acid used is not critical to the practice of the invention; acceptable results may be achieved with 0.1-100 micrograms of nucleic acid/10.sup.5 cells. For example, a ratio of about 0.5 micrograms of plasmid vector in 3 micrograms of DOTAP per 10.sup.5 cells can be used.

In one embodiment, pancreatic cancer cells are isolated from the subject, transfected with nucleic acids encoding the down-regulated miR gene product, and reintroduced into the subject.

An miR gene product or miR gene expression inhibiting compound can also be administered to a subject by any suitable enteral or parenteral administration route. Suitable enteral administration routes for the present methods include, e.g., oral, rectal, or intranasal delivery. Suitable parenteral administration routes include, e.g., intravascular administration (e.g., intravenous bolus injection, intravenous infusion, intra-arterial bolus injection, intra-arterial infusion and catheter instillation into the vasculature); peri- and intra-tissue injection (e.g., peri-tumoral and intra-tumoral injection, intra-retinal injection, or subretinal injection); subcutaneous injection or deposition, including subcutaneous infusion (such as by osmotic pumps); direct application to the tissue of interest, for example by a catheter or other placement device (e.g., a retinal pellet or a suppository or an implant comprising a porous, non-porous, or gelatinous material); and inhalation. Suitable administration routes are injection, infusion and direct injection into the tumor.

In the present methods, an miR gene product or miR gene product expression inhibiting compound can be administered to the subject either as naked RNA, in combination with a delivery reagent, or as a nucleic acid (e.g., a recombinant plasmid or viral vector) comprising sequences that express the miR gene product or expression inhibiting compound. Suitable delivery reagents include, e.g., the Mirus Transit TKO lipophilic reagent; lipofectin; lipofectamine; cellfectin; polycations (e.g., polylysine), and liposomes.

Recombinant plasmids and viral vectors comprising sequences that express the miR gene products or miR gene expression inhibiting compounds, and techniques for delivering such plasmids and vectors to cancer cells, are discussed herein.

In one embodiment, liposomes are used to deliver an miR gene product or miR gene expression-inhibiting compound (or nucleic acids comprising sequences encoding them) to a subject. Liposomes can also increase the blood half-life of the gene products or nucleic acids. Suitable liposomes for use in the invention can be formed from standard vesicle-forming lipids, which generally include neutral or negatively charged phospholipids and a sterol, such as cholesterol. The selection of lipids is generally guided by consideration of factors, such as the desired liposome size and half-life of the liposomes in the blood stream. A variety of methods are known for preparing liposomes, for example, as described in Szoka et al. (1980), Ann. Rev. Biophys. Bioeng. 9:467; and U.S. Pat. Nos. 4,235,871, 4,501,728, 4,837,028, and 5,019,369 .

The liposomes for use in the present methods can comprise a ligand molecule that targets the liposome to cancer cells. Ligands which bind to receptors prevalent in cancer cells, such as monoclonal antibodies that bind to tumor cell antigens, are suitable.

The liposomes for use in the present methods can also be modified so as to avoid clearance by the mononuclear macrophage system ("MMS") and reticuloendothelial system ("RES"). Such modified liposomes have opsonization-inhibition moieties on the surface or incorporated into the liposome structure. In some embodiments, a liposome of the invention can comprise both opsonization-inhibition moieties and a ligand.

Opsonization-inhibiting moieties for use in preparing the liposomes of the invention are typically large hydrophilic polymers that are bound to the liposome membrane. As used herein, an opsonization inhibiting moiety is "bound" to a liposome membrane when it is chemically or physically attached to the membrane, e.g., by the intercalation of a lipid-soluble anchor into the membrane itself, or by binding directly to active groups of membrane lipids. These opsonization-inhibiting hydrophilic polymers form a protective surface layer that significantly decreases the uptake of the liposomes by the MMS and RES; e.g., as described in U.S. Pat. No. 4,920,016 .

Opsonization inhibiting moieties suitable for modifying liposomes include watersoluble polymers with a number-average molecular weight from about 500 to about 40,000 daltons, or from about 2,000 to about 20,000 daltons. Such polymers include polyethylene glycol (PEG) or polypropylene glycol (PPG) derivatives; e.g., methoxy PEG or PPG, and PEG or PPG stearate; synthetic polymers, such as polyacrylamide or poly N-vinyl pyrrolidone; linear, branched, or dendrimeric polyamidoamines; polyacrylic acids; polyalcohols, e.g., polyvinylalcohol and polyxylitol to which carboxylic or amino groups are chemically linked, as well as gangliosides, such as ganglioside GM1. Copolymers of PEG, methoxy PEG, or methoxy PPG, or derivatives thereof, are also suitable. In addition, the opsonization inhibiting polymer can be a block copolymer of PEG and either a polyamino acid, polysaccharide, polyamidoamine, polyethyleneamine, or polynucleotide. The opsonization inhibiting polymers can also be natural polysaccharides containing amino acids or carboxylic acids, e.g., galacturonic acid, glucuronic acid, mannuronic acid, hyaluronic acid, pectic acid, neuraminic acid, alginic acid, carrageenan; aminated polysaccharides or oligosaccharides (linear or branched); or carboxylated polysaccharides or oligosaccharides, e.g., reacted with derivatives of carbonic acids with resultant linking of carboxylic groups. The opsonization-inhibiting moiety can be a PEG, PPG, or derivatives thereof. Liposomes modified with PEG or PEG-derivatives are sometimes called "PEGylated liposomes."

The opsonization inhibiting moiety can be bound to the liposome membrane by any one of numerous well-known techniques. For example, an N-hydroxysuccinimide ester of PEG can be bound to a phosphatidyl-ethanolamine lipid-soluble anchor, and then bound to a membrane. Similarly, a dextran polymer can be derivatized with a stearylamine lipid-soluble anchor via reductive amination using Na(CN)BH3 and a solvent mixture, such as tetrahydrofuran and water in a 30:12 ratio at 60 oC.

Liposomes modified with opsonization-inhibition moieties remain in the circulation much longer than unmodified liposomes. For this reason, such liposomes are sometimes called "stealth" liposomes. Stealth liposomes are known to accumulate in tissues fed by porous or "leaky" microvasculature. Thus, tissue characterized by such microvasculature defects, for example solid tumors, will efficiently accumulate these liposomes; see Gabizon, et al. (1988), Proc. Natl. Acad. Sci., U.S.A., 18:6949-53. In addition, the reduced uptake by the RES lowers the toxicity of stealth liposomes by preventing significant accumulation of the liposomes in the liver and spleen. Thus, liposomes that are modified with opsonization-inhibition moieties are well suited to deliver the miR gene products or miR gene expression inhibition compounds (or nucleic acids comprising sequences encoding them) to tumor cells.

The miR gene products or miR gene expression inhibition compounds can be formulated as pharmaceutical compositions, sometimes called "medicaments," prior to administering them to a subject, according to techniques known in the art. Accordingly, the invention encompasses pharmaceutical compositions for treating pancreatic cancer. In one embodiment, the pharmaceutical compositions comprise at least one isolated miR gene product and a pharmaceutically-acceptable carrier. In a particular embodiment, the miR gene product corresponds to a miR gene product that has a decreased level of expression in pancreatic cancer cells relative to suitable control cells.

In other embodiments, the pharmaceutical compositions of the invention comprise at least one miR expression inhibition compound. In a particular embodiment, the at least one miR gene expression inhibition compound is specific for a miR gene whose expression is greater in pancreatic cancer cells than control cells.

Pharmaceutical compositions of the present invention are characterized as being at least sterile and pyrogen-free. As used herein, pharmaceutical "compositions" or "formulations" include formulations for human and veterinary use. Methods for preparing pharmaceutical compositions of the invention are within the skill in the art, for example as described in Remington's Pharmaceutical Science, 17th ed., Mack Publishing Company, Easton, Pa. (1985) .

The present pharmaceutical formulations comprise at least one miR gene product or miR gene expression inhibition compound (or at least one nucleic acid comprising sequences encoding them) (e.g., 0.1 to 90% by weight), or a physiologically acceptable salt thereof, mixed with a pharmaceutically-acceptable carrier. The pharmaceutical formulations of the invention can also comprise at least one miR gene product or miR gene expression inhibition compound (or at least one nucleic acid comprising sequences encoding them) which are encapsulated by liposomes and a pharmaceutically-acceptable carrier.

Especially suitable pharmaceutically-acceptable carriers are water, buffered water, normal saline, 0.4% saline, 0.3% glycine, hyaluronic acid and the like.

The pharmaceutical compositions of the invention can comprise at least one miR gene product or miR gene expression inhibition compound (or at least one nucleic acid comprising sequences encoding them) which is resistant to degradation by nucleases. One skilled in the art can readily synthesize nucleic acids which are nuclease resistant, for example by incorporating one or more ribonucleotides that are modified at the 2'-position into the miR gene products. Suitable 2'-modified ribonucleotides include those modified at the 2'-position with fluoro, amino, alkyl, alkoxy, and O-allyl.

Pharmaceutical compositions of the invention can also comprise conventional pharmaceutical excipients and/or additives. Suitable pharmaceutical excipients include stabilizers, antioxidants, osmolality adjusting agents, buffers, and pH adjusting agents. Suitable additives include, e.g., physiologically biocompatible buffers (e.g., tromethamine hydrochloride), additions of chelants (such as, for example, DTPA or DTPA-bisamide) or calcium chelate complexes (such as, for example, calcium DTPA, CaNaDTPA-bisamide), or, optionally, additions of calcium or sodium salts (for example, calcium chloride, calcium ascorbate, calcium gluconate or calcium lactate). Pharmaceutical compositions of the invention can be packaged for use in liquid form, or can be lyophilized.

For solid pharmaceutical compositions of the invention, conventional nontoxic solid pharmaceutically-acceptable carriers can be used; for example, pharmaceutical grades of mannitol, lactose, starch, magnesium stearate, sodium saccharin, talcum, cellulose, glucose, sucrose, magnesium carbonate, and the like.

For example, a solid pharmaceutical composition for oral administration can comprise any of the carriers and excipients listed above and 10-95%, preferably 25%-75%, of the at least one miR gene product or miR gene expression inhibition compound (or at least one nucleic acid comprising sequences encoding them). A pharmaceutical composition for aerosol (inhalational) administration can comprise 0.01-20% by weight, preferably 1%-10% by weight, of the at least one miR gene product or miR gene expression inhibition compound (or at least one nucleic acid comprising sequences encoding them) encapsulated in a liposome as described above, and a propellant. A carrier can also be included as desired; e.g., lecithin for intranasal delivery.

The invention also contemplates a method for determining the efficacy of a therapeutic regimen inhibiting progression of pancreatic cancer in a subject. The method includes: (a) obtaining a first test sample from the subject's pancreas that contains cancer cells with an up-regulated miR gene product relative to control cells; (b) administering the therapeutic regimen to the subject; (d) obtaining a second test sample from the subject's pancreas after a time period; and (e) comparing the levels of the up-regulated miR gene product in the first and the second test samples. A lower level of the up-regulated miR gene product in the second test sample as compared to the first test sample indicates that the therapeutic regimen is effective in inhibiting progression of pancreatic cancer in the subject.

Another method for determining the efficacy of a therapeutic regimen inhibiting progression of pancreatic cancer in a subject, includes: (a) obtaining a first test sample from the subject's pancreas that contains cancer cells with a down-regulated miR gene product relative to control cells; (b) administering the therapeutic regimen to the subject; (d) obtaining a second test sample from the subject's pancreas after a time period; and (e) comparing the levels of the down-regulated miR gene product in the first and the second test samples. In this case, a higher level of the down-regulated miR gene product in the second test sample as compared to the first test sample indicates that the therapeutic regimen is effective in inhibiting progression of pancreatic cancer in the subject.

The invention also encompasses a method of identifying an anti-pancreatic cancer agent. The method includes: (a) determining the expression level of at least one miR gene product which is over-expressed in a biological sample containing pancreatic cancer cells, thereby generating data for a pre-test expression level of said miR gene product; (b) contacting the biological sample with a test agent; (c) determining the expression level of the miR gene product in the biological sample after step (b), thereby generating data for a post-test expression level; and (d) comparing the post-test expression level to the pre-test expression level of said miR gene product. A decrease in the post-test expression level of the over-expressed miR gene product is indicative that the test agent has anti-pancreatic cancer properties.

In another embodiment, the method of identifying an anti-pancreatic cancer agent, includes: (a) determining the expression level of at least one miR gene product which is under-expressed in a biological sample containing pancreatic cancer cells, thereby generating data for a pre-test expression level of said miR gene product; (b) contacting the biological sample with a test agent; (c) determining the expression level of the miR gene product in the biological sample after step (b), thereby generating data for a post-test expression level; and (d) comparing the post-test expression level to the pre-test expression level of said miR gene product, wherein an increase in the post-test expression level of the under-expressed miR gene product is indicative that the test agent has anti-pancreatic cancer properties.

Suitable agents include, but are not limited to drugs (e.g., small molecules, peptides), and biological macromolecules (e.g., proteins, nucleic acids). The agent can be produced recombinantly, synthetically, or it may be isolated (i.e., purified) from a natural source. Various methods for providing such agents to a cell (e.g., transfection) are well known in the art, and several of such methods are described hereinabove. Methods for detecting the expression of at least one miR gene product (e.g., Northern blotting, in situ hybridization, RT-PCR, expression profiling) are also well known in the art. Several of these methods are also described hereinabove.

The invention will now be illustrated by the following non-limiting examples.

### EXAMPLE 1:

### Identification of a microRNA expression signature that discriminates pancreatic cancer from normal or pancreatitis tissue

A real-time, quantitative PCR assay was used to profile the expression of over 200 miRNA precursors in clinical specimens of human pancreatic cancer (adenocarcinoma), paired benign tissue, normal pancreas, chronic pancreatitis and pancreatic cancer cell lines. A unique miRNA signature was identified that distinguished pancreatic cancer from normal and benign pancreas. The methods used for the real-time quantitative RT-PCR are described in detail in Jiang, J., et al. (2005), Nucleic Acids Res 33:5394-5403; Schmittgen T. D., et al. (2004), Nucleic Acids Res 32:E43; and U.S. Provisional Application Ser. No. 60/656,109, filed February 24, 2005 .
The experimental procedure and the results were also reported in Lee et al., (2006) Int. J. Cancer 120:1046-1054 .

### MATERIALS AND METHODS

*Tissue procurement.* The tissue samples analyzed in this study were derived from patients undergoing a surgical procedure to remove a portion of the pancreas at the University of Oklahoma Health Sciences Center and The Ohio State University. The collection of samples conformed to the policies and practices of the facility's Institutional Review Board. Upon removal of the surgical specimen, research personnel immediately transported the tissue to the surgical pathology lab. Pathology faculty performed a gross analysis of the specimen and selected cancerous appearing pancreatic tissue and normal appearing pancreatic tissue for research. Each sample was placed in a cryovial and flash frozen in liquid nitrogen and stored at -150° C until analysis. Subsequent pathologic analysis by the institutes providing the surgical specimens confirmed the histopathology of the samples taken for research. A second level of quality control was performed on the adjacent benign tissues by the laboratory who performed the RNA analysis. Histological slides were prepared from the section of the frozen tissue directly adjacent to tissue from which RNA was isolated. These slides were examined by one of us (W.L.F.) to determine if the benign tissues contained any pancreatic tumor cells. Benign tissue that contained residual tumor was not included in the study. The clinical data on the specimens are listed in Supplemental Table 1.

*Cell lines.* The following pancreatic tumor cell lines were purchased from American Type Tissue Collection (Manassas, VA). Panc-1, HS766T, MIA PaCa-2, HPAF-II, BxPC-3, Mpanc-96, PL45, Panc03.27 and Panc10.05. Cell lines were cultured in RPMI 1640 medium with 10% FBS or other optimized complete medium using standard conditions.

*miRNA precursor expression profiling.* Total RNA was isolated from the cell lines or tissues in 1 ml of Trizol (Invitrogen, Carslbad, CA). Frozen tissue (∼10 mg) were first pulverized in a stainless steel mortar and pestle. Total RNA from normal pancreases were purchased from Ambion (Austin, TX), BD Biosciences (Mountain View, CA) and Stratagene (La Jolla, CA). All donors of the normal tissue died from complications other than pancreatic diseases (Fig. 3). RNA integrity was evaluated using the Agilent 2100 Bioanalyzer (Agilent Technologies, Palo Alto, CA). RNA integrity number (RIN) was determined using the RIN algorithm of the Agilent 2100 expert software, according to the protocol in Imbeaud, S., et al. (2005), Nucleic Acids Res 33, e56. RNA samples with a RIN ≥ 4 were included in the study (Fig. 3). RNA was briefly treated with RNase-free DNAase I and cDNA was synthesized from 1 µg of total RNA using gene specific primers to 222 miRNA precursors plus 18S rRNA. The expression of 222 miRNA precursors was profiled using a real-time quantitative PCR assay. Duplicate PCRs were performed for each miRNA precursor gene in each sample of cDNA. The mean C_{T} was determined from the duplicate PCRs. Relative gene expression was calculated as 2-(C_{TmiRNA} -C_{T18S rRNA}). Relative gene expression was multiplied by 10⁶ to simplify the presentation of the data. The raw relative miRNA precursor expression data is presented in Fig. 4.

*Statistics.* The ΔC_{T} Data for 201 gene expression values (relative to control gene expression) were mean-centered and analyzed using the following strategy. The expression patterns of unfiltered data were assessed using unsupervised hierarchical clustering of samples and unsupervised hierarchical clustering of genes based on average linkage and Euclidian distance (Eisen MB, et al. (1998), Proc Natl Acad Sci USA 95:14863-8; Saeed AI, et al. (2003), Biotechniques 34:374-8). To determine genes that are differentially expressed between groups of samples, the data were filtered on significance of differences using multi-group permutations-based ANOVA test (Welch approximation) with p < 0.01 (10,000 random permutations) and multiple testing correction (Westfall-Young step-down correction with maxT). To compare the expression patterns of differentially expressed genes, the filtered data were analyzed using hierarchical clustering of samples and hierarchical clustering of genes based on average linkage and Euclidian distance.

Additional cluster analysis of filtered data was done using an expression terrain map (Kim, et al. (2001), Science, 293:2087-92). Terrain maps provide a three-dimensional overview of the major clusters inherent in the data. Samples were first mapped into a two-dimensional grid in which the placement of each element is influenced by a number of nearest neighbors based on Euclidian distance that was calculated using miRNA precursor expression data. The third dimension is determined by the density of points over the two-dimensional grid and its' value is projected as a surface; higher peaks indicate larger numbers of very similar elements. The average correlation between each pair of samples was also calculated. Each pair of samples with an average correlation above the threshold (0.8) is indicated on the expression terrain map by a line connecting the two samples. This allows visualizing subsets of samples with a highly correlated pattern of miRNA expression. Peaks representing samples from a particular cluster are labeled with color coded spheres on top of each peak.

A supervised machine learning algorithm was applied for classification of samples based on unfiltered miRNA expression data. The predictive scores for each miRNA were calculated based on 2 class comparison (normal vs. tumor) of expression data using the prediction analysis of micro-arrays algorithm (PAM) (Tibshirani R, et al. (2002), Proc Natl Acad Sci USA 99:6567-72) based on training, take-one-out cross validation and testing procedures. The division of the samples into training and test sets is done using the commonly accepted approach of randomly splitting data into training and test sets (usually 2/3 for train, 1/3 for test). For this study, tumor samples were randomly split into ∼75% for training and 25% for testing. Since the number of normal cases was small (N = 6), normal cases were included in the training set only. The small number of chronic pancreatitis samples (N = 4) were not included in the PAM analysis. While, two levels of quality control were performed to eliminate adjacent benign tissue that contained any tumor cells, the RNA was extracted from whole tissue rather than microdissected tissue. Thus, we could not completely rule out the possibility that some tumor cells contaminated the adjacent benign, nor do we know if early premalignant changes have occurred in these benign samples that are obtained from tissue adjacent to tumor. Therefore benign samples were excluded from the training set in order to train the classifier on true normal and tumor cases. Benign cases were used in the test set

### RESULTS

*Validation of internal control gene:* Pancreatic tissue is rich in ribonuclease and care must be taken during RNA isolation to reduce the possibility of autolysis. To validate the integrity of the RNA isolated from the pancreatic tissue, ∼100 ng of each RNA sample was assayed using the Agilent 2100 Bioanalyzer. Fifty-two tissues had a RIN ≥ 4 (median 7.6, range 4.3-9.6, Fig. 3).

miRNA precursor expression in each of the samples was determined using real-time PCR and normalized to an internal control gene. Since equivalent amounts of total RNA was added to each RT reaction, 18S rRNA was validated as the internal control by comparing the mean expression among the various samples (i.e. tumor, normal and benign). There was no statistically significant difference in the mean 18S rRNA expression between the tumor samples or the normal pancreas (p = 0.116, Fig. 5). The 18S rRNA expression in the tumor and normal tissue determined here are in agreement with those previously reported in pancreatic tissue (Rubie C, et al., (2005) Mol Cell Probes 19:101-9). Since there was no significant difference in the 18S rRNA expression between the tumor, normal and benign groups, 18S rRNA was selected as the internal control gene in the study.

*miRNA precursor profiling in pancreatic tissues:* The expression of 201 miRNA precursors, representing the 222 miRNAs discovered as of April, 2005 was profiled in 28 tumors, 15 adjacent benign tissues, four chronic pancreatitis specimens, six normal pancreas tissues and nine pancreatic cancer cell lines. Unsupervised hierarchical clustering of samples was performed on the entire set of unfiltered data for the tumor, normal pancreas, pancreatitis and pancreatic cancer cell lines. The heatmap demonstrates that unfiltered expression data of only 201 miRNA precursors sufficiently sorts the samples into clusters of normal pancreas, tumor, chronic pancreatitis and cell lines (Fig. 6).

The miRNA expression of 15 adjacent benign tissues from the identical pancreatic cancer patients was included along with the normal pancreases, pancreatitis, pancreatic tumors and cancer cell lines. The miRNA precursor expression data was filtered using multi-group ANOVA. Statistical filtering of data is conventionally considered to be a required step of the expression data preprocessing because gene expression data are inherently noisy. Hierarchical clustering of samples and genes was performed on the resulting 112 miRNAs. Hierarchical clustering of filtered data allowed us to identify major groups of miRNAs that have different patterns of expression in the resulting four clusters of samples (Fig. 7). One cluster contained only cell lines. Another cluster contained all 6 normal pancreases and 9 of 15 benign tissues. A third cluster contained the 4 chronic pancreatitis specimens and 1 benign tissue. Finally, a large cluster contained 28 of 28 tumors and 5 benign tissues (Fig. 7).

The filtered data were also analyzed using a different clustering technique known as expression terrain maps. The terrain map separated the expression data into 5 main groups of samples (Fig. 8). Like the hierarchical clustering, the terrain map separates groups of samples from the normal pancreas, the cell lines, pancreatic adenocarcinomas and chronic pancreatitis. This analysis showed that each of the clusters of samples occupies distinct regions on the expression terrain map thus providing additional evidence that each of the 4 groups of samples has distinct patterns of miRNA expression and suggests the possibility of finding subsets of microRNAs that discriminate normal and tumor samples. Most of the adjacent benign tissue grouped in between the normal and tumor, while those benign samples that clustered with the tumor also grouped with the tumor on the terrain map (Fig. 8). As an additional feature of terrain map, the average correlations of all miRNA expression values were calculated between each pair of samples. Those correlations above the threshold of 0.8 are shown as lines connecting pairs of samples with similar patterns of miRNA expression. This allows visualizing subsets of samples with a highly correlated pattern of miRNA expression. The samples within each of the 4 main groups are connected (i.e. average correlation >0.8) but there were no between the groups connections, except for two pancreatitis samples that correlated with some of the tumor samples (Fig. 8).

Comparing the gene expression profile among different pancreas tissues has been used to eliminate the stroma and cell proliferation (e.g. cell line) contributions and identify genes expressed in pancreatic tumors. An attempt to perform such analysis on the miRNA expression data in Fig. 7 was unsuccessful due to the uniformly high expression in the cancer cell lines and the uniformly low expression in the pancreatitis samples. However, a number of possible tumor-related miRNAs were identified that were increased in the cell lines and/or tumors but not in the normal or pancreatitis (Fig.7).

*Data analysis by Prediction Analysis of Micro-arrays algorithm (PAM):* The PAM classification algorithm was used to determine if the miRNA expression data could predict which class the samples fit (tumor or normal) and to determine the most important, differentially expressed miRNAs related to pancreatic adenocarcinoma. The unfiltered data on 201 pre-miRNAs were analyzed by the PAM algorithm according to the method described in Tibshirani R, et al. (2002), Proc Natl Acad Sci USA 99, 6567-72. The three genes with more than 75% of missing data were eliminated from the analysis in order to prevent possible artifacts of the imputation algorithm. PAM training and cross-validation were conducted using the 6 normal pancreas and 18 pancreatic tumors. PAM has correctly classified 100% of the normal and tumor samples (Fig. 9A). PAM testing was conducted on 10 tumor samples and 15 adjacent benign samples. PAM has correctly classified 100% of the tested tumor samples and 11 of 15 of the tested benign samples (Fig. 9B). The 68 top ranked differentially expressed miRNAs in pancreatic adenocarcinoma as selected by PAM are listed in Fig. 10. The 20 top ranked differentially expressed miRNAs in pancreatic adenocarcinoma as selected by PAM are listed in Table 1.

**Table 1- Top 20 aberrantly expressed miRNA precursors in pancreatic adenocarcinoma**

| Rank | Name | p-value (*t*-test) | Fold change | Chromosome location |
|---|---|---|---|---|
| 1 | miR-221 | 5.66E-05 | 26.2 | Xp11.3 |
| 2 | miR-424 | 3.62E-08 | 56.3 | Xq26.2 |
| 3 | miR-301 | 1.11E-05 | 34.2 | 17q23.2 |
| 4 | miR-100 | 4.40E-06 | 36.9 | 11q24.1 |
| 5 | miR-376a | 7.00E-04 | 7.79 | 14q32.31 |
| 6 | miR-125b-1 | 1.00E-04 | 23.2 | 11q24.1 |
| 7 | miR-021 | 2.00E-04 | 15.7 | 17q23.2 |
| 8 | miR-345 | 1.44E-15 | -14.5 | 14q32.2 |
| 9 | miR-016-1 | 3.73E-04 | 14.3 | 13q14.2 |
| 10 | miR-181a,c | 8.31E-04 | 18.6 | 9q33.3, 19p13.13 |
| 11 | miR-092-1 | 3.40E-03 | 19.6 | 13q31.3 |
| 12 | miR-015b | 4.00E-04 | 8.55 | 3q25.33 |
| 13 | miR-142-P | 3.63E-07 | -15.4 | 17q23.2 |
| 14 | miR-155 | 1.51E-03 | 14.0 | 21q21 |
| 15 | let-7f-1 | 4.00E-04 | 10.9 | 9q22.32 |
| 16 | miR-212 | 2.00E-04 | 22.2 | 17p13.3 |
| 17 | miR-107 | 3.86E-05 | 8.20 | 10q23.31 |
| 18 | miR-024-1,2 | 9.12E-08 | 8.17 | 9q22.32, 19p13 |
| 19 | let-7d | 7.06E-04 | 8.38 | 9q22.32 |
| 20 | miR-139 | 6.79E-11 | -7.91 | 11q13.4 |

### DISCUSSION

Reported here are the results of the first detailed miRNA expression profiling study in pancreatic ductal adenocarcinoma. Expression profiling identified a large number of miRNAs that are aberrantly expressed in pancreatic ductal adenocarcinoma.

miRNAs are believed to function primarily as negative regulators of gene expression following binding to conserved sequences within the 3' untranslated region of target mRNAs. While the biological roles of miRNA are under intense investigation, they are believed to define and maintain cellular fate in a manner similar to transcription factors by regulating developmental timing and differentiation. Since alterations in developmental pathways play a critical role in pancreatic cancer development, alterations in miRNA expression may be an important contributor to the development of pancreatic adenocarcinoma.

miRNA expression profiling correctly identified 28 of 28 tissues as tumor (Fig. 9). All 6 normal pancreases were correctly predicted and 11 of 15 adjacent benign tissues were classified as normal tissue (Fig. 9). The data presented here shows that miRNA expression profiling can generate a unique molecular signature for a given cancer.

The three factors that are likely driving the differences in gene expression between tumor and normal tissue are the normal acini, stroma and tumor cells. We confirmed by RT in situ PCR that 3 of the top differentially expressed miRNAs that were identified in the screen are localized to the tumor cells (Fig. 11). Some of the benign tissues failed to cluster with the normal pancreas (Fig. 7). While 2 levels of quality control were used to reduce the possibility of contaminating tumor cells, it is possible that some tumor cells were present in the benign tissue since the RNA was isolated from whole tissue and not microdissected tissue. Another possibility is that premalignant changes have already occurred in some of the benign tissues as those samples are obtained from tissue adjacent to tumor. Supporting this concept is the fact that most of the benign samples (and chronic pancreatitis as well) lie in between the normal pancreas and tumor on the expression terrain map (Fig. 8). This observation shows that miRNA expression profiling can detect premalignant alterations that have occurred in these benign tissues.

Some of the differentially expressed miRNAs in pancreatic cancer were aberrantly expressed in other cancers. These include miR-155, which was increased in the present study and in diffuse large B-cell lymphoma; miR-21 was increased here and in glioblastomas, breast cancer and papillary thyroid cancer; miR-221 was increased in pancreatic cancer, in glioblastoma and in thyroid cancer. miR-221 is located ∼700 bp from miR-222 on the X chromosome; both miR-221 and miR-222 are predicted to bind to and regulate kit. miR-222 precursor was not among the top 20 differentially expressed miRNAs, however subsequent analysis of mature miR-222 by PCR showed that miR-222 was increased in pancreas cancer at levels that were similar to miR-221 (data not shown). Thus, deregulation of the miRNAs mentioned above may be unique to cancer in general. miRNAs differentially expressed in other cancers were not deregulated to the same degree in pancreatic cancer. The let-7 family, decreased in lung cancer, was increased here. Expression of the miR-17-92 polycistron (encoding miR-17, -18, -19a, -19b-1 and -92-1) was increased in lymphoma and colorectal cancer but was not significantly altered in pancreatic cancer. We report deregulation of a number of miRNAs in pancreatic cancer such as miR-376a, miR-212, miR-301 and miR-181 a that have not been reported in any other cancers to our knowledge. Also of interest is the fact that most of the deregulated miRNAs reported here show increased expression in the tumors compared to the normal pancreas. A few miRNAs had reduced expression in pancreatic cancer, including miR-375 and miR-139 (Table 1, Figs. 12-13). miR-375 was cloned from pancreas and is believed to be islet cell specific (Poy MN, et al. (2004) Nature, 432:226-30).

### EXAMPLE 2

### Northern blot of mature miRNA expression in pancreatic cancer tissue

The real-time PCR assay used in EXAMPLE 1 quantifies the miRNA precursors and not the active, mature miRNA. Northern blotting was performed on the identical RNA used in the real-time PCR analysis to validate if the mature miRNA correlated with the precursor miRNA.

Expression levels of mature miR-100, mir-375 and miR-155 from three normal pancreases and 4 pairs of tumor/adjacent benign tissue paralleled the miRNA precursor levels by PCR (Fig. 12). miR-100 and miR-155 were among the top 20 differentially expressed miRNAs (Table 1). miR-375 was validated by Northern blotting because it was one of the few miRNAs with decreased expression in pancreas cancer. While miR-375 was not among the top 20 miRNAs (Table 1), the expression of both precursor and mature miR-375 was significantly decreased in pancreas cancer by real-time PCR (p < 1 × 10⁻⁵). miRNA expression in the paired benign and tumor tissue was consistently increased or decreased in all cases, demonstrating that the differences in miRNA expression between tumor and benign are due to differences in individual patient's tissues and not due to differences in the mean expression of the group.

*Northern blotting.* Northern blotting was performed as previously described in Lau NC, et al. (2001), Science 294:858-62 and Schmittgen TD, et al. (2004), Nucleic Acids Res 32:E43. DNA oligonucleotides of the reverse compliment to the mature miRNA were used as probes. Blots were successfully stripped and reprobed up to three times.

### EXAMPLE 3

### Real-time PCR of mature miRNA in pancreatic cancer tissue

The real-time PCR data described in EXAMPLE 1 was validated using a commercially available real-time PCR assay to amplify and quantify the mature miRNA. Mature miRNA expression was validated for the top 28 aberrantly expressed miRNAs from PAM. cDNA from the following tissues were assayed: 6 normal pancreases, 16 pancreatic adenocarcinomas and 10 adjacent benign tissues that were predicted as normal from PAM.

The mature miRNA expression highly correlated with the miRNA precursor (Figs. 13 & 14). To our knowledge, this is the initial presentation of both precursor and mature miRNA expression determined by sensitive, real-time PCR assays. Of interest is that while the relative expression values for these mature miRNAs spanned 3-logs (from 0.1 to 100), the trend in differential expression between the tumor and normal tissues remained (Fig. 14). This suggests that miRNAs function in tumor and normal pancreas at different expression levels, yet the differential expression is maintained between cancer and normal tissue.

*Real-time PCR of mature miRNA.* Assays to quantify the mature miRNA (i.e. TaqMan® microRNA Assays, Applied Biosystems Foster City, CA) were conducted as described in Chen, C., et al. (2005), Nucleic Acids Res 33:e179, with one modification. A 5X cocktail containing 28 different antisense looped RT primers was prepared by concentrating the 2X stock solutions in a Speed Vac. One hundred nanograms of total RNA was heated for 5 mins at 80° C and then incubated for 5 mins at 60° C with 10 µM of the 18S rRNA antisense primer followed by cooling to room temperature. Three microliters of the 5X looped primer mix was then added and the cDNA was made. This allowed for the creation of a library of 28 miRNA cDNAs plus the 18S rRNA internal control. Real-time PCR (10 µl total reaction) was performed as described using 1 µl of a 1:50 dilution of cDNA. Duplicate PCRs were performed for each mature miRNA gene in each sample of cDNA. The mean C_{T} was determined from the duplicate PCRs. Gene expression was calculated relative to 18S rRNA as described above and multiplied by 10⁶ to simplify data presentation.

### EXAMPLE 4

### RT in situ PCR of miRNA precursors in pancreatic tumor cells

The cell-type miRNA expression was studied using RT in situ PCR. miR-221, miR-376a and miR-301 were selected for the in situ PCR since they were among the top differentially expressed miRNAs (Table 1) and had increased expression in the tumor and cell lines compared to the normal pancreas and pancreatitis (Fig. 7). We were particularly interested in the cell type expression of miR-376a since it was cloned from pancreas cells in Poy M. N., et al. (2004), Nature 432, 226-30. Our results showed that miR-221 and miR-376a were localized to the tumor cells and not to the benign pancreatic acini or stromal cells (Fig. 11) or benign ducts (not shown). miR-301 was also localized to tumor (data not shown).

*RT in situ PCR.* The RT in situ PCR protocol was performed as previously described in Nuovo GJ, et al. (1999), Proc Natl Acad Sci USA 96, 12754-9. Briefly, optimal protease digestion time was determined using nonspecific incorporation of the reporter nucleotide digoxigenin dUTP. Optimal protease digestion was followed by overnight incubation in RNase-free DNase (10 U per sample, Boehringer Mannheim, Indianapolis, IN) and one step RT/PCR using the rT^{th} system and digoxigenin dUTP. The chromogen is nitroblue tetrazolium and bromochloroindolyl phosphate (NBT/BCIP) with nuclear fast red as the counterstain. The primer sequences to the precursors of miR-221, miR-301 and miR-376a were the same as those used for the profiling in EXAMPLE 1. The negative controls included omission of the primers and substitution with irrelevant (human papillomavirus specific) primers, as this virus does not infect pancreatic tissue. RT in situ PCR was performed on the archived, formalin fixed paraffin embedded sample 1050005A2(T) (Fig. 3).

### EXAMPLE 5

### Identification of pancreas specific and enriched miRNAs

To identify miRNAs that are either specific to normal pancreas tissue or are enriched in normal pancreas tissue, the expression of 184 mature miRNAs was profiled using real-time PCR in 22 normal human tissues. These tissues included adipose, bladder, brain, cervix, colon, esophagus, heart, kidney, liver, lung, skeletal muscle, ovary, pancreas, placenta, prostate, spleen, small intestine, trachea, thymus, thyroid, testes and normal B cells. Pancreas-specific miRNAs were defined as those miRNAs with high expression in pancreas and little to undetectable expression in the other 21 normal tissues. Pancreas-enriched miRNAs were defined as those miRNAs with 10-fold or greater expression in the pancreas tissue compared to the mean of the other 21 tissues. Pancreas-enriched miRNAs may be present in some of the other tissues. The pancreas-specific miRNAs include miR-216 and miR-217. The pancreas-enriched miRNAs include miR-7, miR-148a and miR-375.

**Table 2. Pancreas-specific and pancreas-enriched miRNAs**

| Pancreas-specific miRNAs | Expression, pancreas | Mean expression, remaining tissues | Fold-change | Other tissues with significant expression |
|---|---|---|---|---|
| miR-216 | 3.60 E-07 | 0 | NA | None |
| miR-217 | 6.64 E-08 | 6.67 E-10 | 99.5 | None |

| Pancreas-enriched miRNAs | | | | |
|---|---|---|---|---|
| miR-7 | 3.06 E-06 | 2.69 E-07 | 11.3 | Brain, thyroid |
| miR-148a | 3.61 E-05 | 2.15 E-06 | 16.7 | |
| miR-375 | 0.0001588 | 2.92 E-06 | 54.3 | |

### EXAMPLE 6

### Profiling of mature miRNA in pancreas tissues

To identify miRNAs that are differentially expressed in pancreatic cancer, the expression of 184 mature miRNAs was profiled using real-time PCR in 9 pancreatic cancer tumors and in pancreas from 6 donors without pancreatic disease. Mature miRNA was profiled using a commercially available real-time PCR assay (TaqMan® microRNA Assays, Applied Biosystems Foster City, CA). The assay was conducted as described in Example 3. Real-time PCR data was presented as a heatmap and was analyzed using unsupervised hierarchical clustering (Figure 15). Some miRNAs had undetectable expression in all 15 samples, these miRNAs were not included in the heatmap. A large number of mature miRNA had increased expression in the pancreas tumors compared to the normal pancreas. The heatmap also demonstrates that the mature miRNA expression profile was able to distinguish the pancreas tumors from normal since they both appear as separate branches of the dendrogram. The fold change in mature miRNA expression in the tumor compared to normal and the p value are shown in Fig. 16.

### EXAMPLE 7

### Expression of miR-21 is increased in pancreas cancer cells

Several approaches were used to demonstrate that expression of mature miR-21 was localized to pancreatic adenocarcinoma cells and not acini, stroma or other noncancerous cells of the pancreas. (Fig. 17) In situ RT-PCR was applied to a section of normal pancreas (A) and pancreas cancer (B) and demonstrated that increased expression of miR-21 is localized to the tumor. Pancreatic ductal adenocarcinoma and normal (noncancerous) pancreatic ducts were isolated from several specimens using laser microdissection. RNA was isolated from the microdissected tissue and the mature miR-21 as well as the 18S rRNA internal control was quantified by real-time PCR. Mature miR-21 was substantially increased in the microdissected tumor tissue compared to normal pancreas ducts (C). Mature miR-21 was assayed in 9 specimens of pancreas tumors and in 6 normal pancreas specimens using the commercially available real-time PCR assay for mature miRNA as described previously. The mature miR-21 expression was increased in the tumors (D). The mature miR-21 expression was quantified by real-time PCR in two normal pancreas epithelial cell lines and in 7 human pancreatic cancer cells lines including three that were derived from metastatic cancer and four that were derived from primary pancreas cancer. The mature miR-21 expression was increased in all seven pancreatic cancer cell lines compared to the normal pancreas cell lines (E).

## Claims

1. A method of diagnosing whether a subject has, or is at risk of developing, pancreatic adenocarcinoma, comprising measuring the level of an miR gene product in a biological sample derived from the subject's pancreas, wherein an increase in the level of the miR gene product in the biological sample as compared to the level of a corresponding miR gene product in a control sample of normal pancreatic tissue is indicative of the subject either having, or being at risk for developing, pancreatic adenocarcinoma, wherein the miR gene product is a precursor or mature form of MIR-135b (SEQ ID NO: 101).

2. The method of claim 1, which comprises measuring the level of a combination of miR gene products listed in Fig. 16 in a biological sample derived from the subject's pancreas, wherein a decrease in the level of miR gene product in the biological sample as compared to the level of a corresponding miR gene product in a control sample of normal pancreatic tissue is indicative of the subject either having, or being at risk for developing, pancreatic adenocarcinoma in the case of miR-148a, miR-216*, miR-217, miR-375 and miR-377; and wherein an increase in the level of miR gene product in the biological sample as compared to the level of a corresponding miR gene product in a control sample of normal pancreatic tissue is indicative of the subject either having, or being at risk for developing, pancreatic adenocarcinoma in the case of let-7c, let-7d, let-7e, let-7f, let-7g, let-7i, miR-009*, miR-010a, miR-010b, miR-015a, miR-016, miR-017-3p, miR-017-5p, miR-018, miR-020, miR-021, miR-022, miR-023a, miR-023b, miR-024, miR-026a, miR-027a, miR-027b, miR-028, miR-029b, miR-030e-3p, miR-031, miR-034a, miR-034b, miR-034c, miR-093, miR-098, miR-099a, miR-099b, miR-100, miR-101, miR-103, miR-106b, miR-125a, miR-125b, miR-126, miR-126*, miR-127, miR-130a, miR-132, miR-140, miR-142-3p, miR-142-5p, miR-143, miR-145, miR-146, miR-146b, miR-148b, miR-149, miR-150, miR-152, miR-155, miR-181a, miR-181b, miR-181c, miR-181d, miR-183, miR-186, miR-189, miR-190, miR-191, miR-192, miR-193, miR-194, miR-195, miR-196a, miR-199a*, miR-199b, miR-200a, miR-200b, miR-203, miR-210, miR-212, miR-213, miR-214, miR-218, miR-221, miR-222, miR-223, miR-296, miR-301, miR-324-3p, miR-324-5p, miR-330, miR-331, miR-338, miR-339, miR-340, miR-342, miR-345, miR-346, miR-361, miR-370, miR-372, miR-374, miR-376a, miR-378, miR-379, miR-382, miR-422b, miR-423, and miR-425,
and wherein the combination includes a precursor or mature form of MIR-135b.

3. The method of claim 1, which further comprises measuring the level of at least one additional miR gene product in a biological sample derived from the subject's pancreas, wherein an increase in the level of the miR gene product in the biological sample as compared to the level of a corresponding miR gene product in a control sample of normal pancreatic tissue is indicative of the subject either having, or being at risk for developing, pancreatic adenocarcinoma; and wherein the at least one additional miR gene product is selected from the group consisting of: MIR-021 (SEQ ID NO: 32), MIR-221 (SEQ ID NO: 172), MIR-301 (SEQ ID NO: 178), MIR-212 (SEQ ID NO: 161), MIR-100 (SEQ ID NO: 69), let-7i (SEQ ID NO: 11), MIR-222-P (SEQ ID NO: 173), MIR-155 (SEQ ID NO: 124), MIR-023a (SEQ ID NO: 34), MIR-181a (SEQ ID NO: 125), MIR-376 (SEQ ID NO: 210), let-7a-2-P (SEQ ID NO: 2), let-7b (SEQ ID NO: 4), let-7c (SEQ ID NO: 5), let-7d (SEQ ID NO: 6), let-7f-1 (SEQ ID NO: 8), let-7i (SEQ ID NO: 11), MIR-001-2 (SEQ ID NO: 13), MIR-007-1 (SEQ ID NO: 14), MIR-015a (SEQ ID NO: 22), MIR-015b (SEQ ID NO: 23), MIR-016-1 (SEQ ID NO: 24), MIR-019b-1-P (SEQ ID NO: 29), MIR-024-1,2 (SEQ ID NO: 36, 37), MIR-027a, b (SEQ ID NO: 42, 43), MIR-029a,c (SEQ ID NO: 45, 46), MIR-030d (SEQ ID NO: 53), MIR-032 (SEQ ID NO: 56), MIR-092-1 (SEQ ID NO: 61), MIR-098 (SEQ ID NO: 66), MIR-099a (SEQ ID NO: 67), MIR-107 (SEQ ID NO: 78), MIR-125b-1 (SEQ ID NO: 85), MIR-126 (SEQ ID NO: 87), MIR-128a (SEQ ID NO: 89), MIR-132 (SEQ ID NO: 94), MIR-136 (SEQ ID NO: 102), MIR-142-P (SEQ ID NO: 109), MIR-145-P (SEQ ID NO: 112), MIR-152 (SEQ ID NO: 120), MIR-181c (SEQ ID NO: 126), MIR-196a-2 (SEQ ID NO: 144), MIR-213 (SEQ ID NO: 162), MIR-215 (SEQ ID NO: 164), MIR-218-1,2 (SEQ ID NO: 167, 168), MIR-328 (SEQ ID NO: 189), MIR-331-P (SEQ ID NO: 191), MIR-345 (SEQ ID NO: 198), MIR-367 (SEQ ID NO: 201), and MIR-424 (SEQ ID NO: 221) or any combination thereof.

4. The method of claim 3, wherein the at least one additional miR gene product is selected from the group consisting of let-7b, let-7c, let-7d, let-7f-1, let-7i, MIR-007-1, MIR-015a, MIR-015b, MIR016-1, MIR-021, MIR-023a, MIR-024-1,2, MIR-027a, b, MIR-030d, MIR-098, MIR-099a, MIR-100, MIR-125b-1, MIR-126, MIR-132, MIR-142-P, MIR-145-P, MIR-152, MIR-155, MIR-181a,c, MIR-196a-2, MIR-212, MIR-213, MIR-218-1,2, MIR-221, MIR-222-P, MIR301, MIR-331-P, MIR-345, MIR-376, or any combination thereof.

5. The method of claim 3, wherein the at least one additional miR gene product is selected from the group consisting of: let-7b, let-7d, let-7f, let-7i, MIR-015a,, MIR-015b, MIR-016-1, MIR-021,MIR-023a, MIR-024, MIR-100, MIR-125b-1, MIR-132, MIR-155, MIR-181a, MIR-181c, MIR-212, MIR-221, MIR-301, MIR376a, or any combination thereof.

6. The method of claim 3, wherein the at least one additional miR gene product is selected from the group consisting of: let-7a-2-P, let-7b, let-7c, let-7d, let-7f-1, let-7i, MIR-015b, MIR-019b-1-P, MIR-098, MIR-128a, MIR-136, MIR-152, MIR-155, MIR-181a, MIR-196a-2, MIR-212, MIR-215, MIR-218-1, MIR-218-2, MIR-301, MIR-328, MIR-331-P, MIR-424, or any combination thereof.

7. The method of claim 3, wherein the at least one additional miR gene product is MIR-021 (SEQ ID NO: 32), MIR-024, MIR-155, MIR-196a-2, MIR-210, MIR-223 or any combination thereof.

8. The method of claim 3, wherein the at least one additional miR gene product is MIR-021 (SEQ ID NO: 32).

9. The method of claim 1, which further comprises measuring the level of at least one additional miR gene product in a biological sample derived from the subject's pancreas, wherein a decrease in the level of the miR gene product in the biological sample as compared to the level of a corresponding miR gene product in a control sample of normal pancreatic tissue is indicative of the subject either having, or being at risk for developing, pancreatic adenocarcinoma; and wherein the at least one additional miR gene product is selected from the group consisting of: MIR-139 (SEQ ID NO: 106), MIR-337 (SEQ ID NO: 193), MIR-133b (SEQ ID NO: 97), MIR-096-P (SEQ ID NO: 65), MIR-371 (SEQ ID NO: 205), MIR-383 (SEQ ID NO: 217), MIR-092-2-P (SEQ ID NO: 62), MIR-204 (SEQ ID NO: 155), MIR-299-P (SEQ ID NO: 177), MIR-375 (SEQ ID NO: 209), MIR-188b-P (SEQ ID NO: 135), and MIR-129-2 (SEQ ID NO: 91), or any combination thereof.

10. The method of claim 9, wherein the at least one additional miR gene product is MIR-096-P.

11. The method of claim 9, wherein the at least one additional miR gene product is MIR-375.

12. The method of any preceding claim, wherein at least one of the plurality of miR gene products is mature miRNA.

13. The method of any preceding claim, wherein the biological sample comprises pancreatic tissue, pancreatic tumor, pancreatic cells or pancreatic juice.

## Patentansprüche

1. Verfahren zur Diagnose, ob ein Individuum ein Pankreasadenokarzinom hat oder ob das Risiko besteht, dass es ein solches entwickelt, welches das Messen des Gehalts eines miR-Genprodukts in einer biologischen Probe, die von der Pankreas des Individuums stammt, umfasst, wobei eine Erhöhung des Gehalts des miR-Genprodukts in der biologischen Probe im Vergleich zum Wert eines entsprechenden miR-Genprodukts in einer Kontrollprobe von normalem Pankreasgewebe darauf hinweist, dass das Individuum entweder ein Pankreasadenokarzinom hat oder das Risiko besteht, dass es ein solches entwickelt, wobei das miR-Genprodukt ein Vorläufer oder eine reife Form von MIR-135b (Seq.-ID Nr. 101) ist.

2. Verfahren nach Anspruch 1, welches das Messen des Gehalts einer Kombination aus miR-Genprodukten, die in Fig. 16 aufgelistet sind, in einer biologischen Probe, die von der Pankreas des Individuums stammt, umfasst, wobei eine Verringerung des Gehalts des miR-Genprodukts in der biologischen Probe im Vergleich zum Wert eines entsprechenden miR-Genprodukts in einer Kontrollprobe von normalem Pankreasgewebe im Falle von miR-148a, miR-216*, miR217, miR-375 und miR-377 darauf hinweist, dass das Individuum entweder ein Pankreasadenokarzinom hat oder das Risiko besteht, dass es ein solches entwickelt; und wobei eine Erhöhung des Gehalts des miR-Genprodukts in der biologischen Probe im Vergleich zum Wert eines entsprechenden miR-Genprodukts in einer Kontrollprobe von normalem Pankreasgewebe im Falle von let-7c, let-7d, let-7e, let-7f, let-7g, let-7i, miR-009*, miR-010a, miR-010b, miR-015a, miR-016, miR-017-3p, miR-017-5p, miR-018, miR-020, miR-021, miR-022, miR-023a, miR-023b, miR-024, miR-026a, miR-027a, miR-027b, miR-028, miR-029b, miR-030e-3p, miR-031, miR-034a, miR-034b, miR-034c, miR-093, miR-098, miR-099a, miR-099b, miR-100, miR-101, miR-103, miR-106b, miR-125a, miR-125b, miR-126, miR-126*, miR-127, miR-130a, miR-132, miR-140, miR-142-3p, miR-142-5p, miR-143, miR-145, miR-146, miR-146b, miR-148b, miR-149, miR-150, miR-152, miR-155, miR-181a, miR-181b, miR-181c, miR-181d, miR-183, miR-186, miR-189, miR-190, miR-191, miR-192, miR-193, miR-194, miR-195, miR-196a, miR-199a*, miR-199b, miR-200a, miR-200b, miR-203, miR-210, miR-212, miR-213, miR-214, miR-218, miR-221, miR-222, miR-223, miR-296, miR-301, miR-324-3p, miR-324-5p, miR-330, miR-331, miR-338, miR-339, miR-340, miR-342, miR-345, miR-346, miR-361, miR-370, miR-372, miR-374, miR-376a, miR-378, miR-379, miR-382, miR-422b, miR-423 und miR-425 darauf hinweist, dass das Individuum entweder ein Pankreasadenokarzinom hat oder das Risiko besteht, dass es ein solches entwickelt,
und wobei die Kombination einen Vorläufer oder eine reife Form von MIR-135b umfasst.

3. Verfahren nach Anspruch 1, das weiters das Messen zumindest eines weiteren miR-Genprodukts in einer biologischen Probe, die von der Pankreas des Individuums stammt, umfasst, wobei eine Erhöhung des Gehalts des miR-Genprodukts in der biologischen Probe im Vergleich zum Wert eines entsprechenden miR-Genprodukts in einer Kontrollprobe von normalem Pankreasgewebe darauf hinweist, dass das Individuum entweder ein Pankreasadenokarzinom hat oder das Risiko besteht, dass es ein solches entwickelt; und wobei das zumindest eine weitere miR-Genprodukt aus der aus MIR-021 (Seq.-ID Nr. 32), MIR-221 (Seq.-ID Nr. 172), MIR-301 (Seq.-ID Nr. 178), MIR-212 (Seq.-ID Nr. 161), MIR-100 (Seq.-ID Nr. 69), let-7i (Seq.-ID Nr. 11), MIR-222-P (Seq.-ID Nr. 173), MIR-155 (Seq.-ID Nr. 124), MIR-023a (Seq.-ID Nr. 34), MIR-181a (Seq.-ID Nr. 125), MIR-376 (Seq.-ID Nr. 210), let-7a-2-P (Seq.-ID Nr. 2), let-7b (Seq.-ID Nr. 4), let-7c (Seq.-ID Nr. 5), let-7d (Seq.-ID Nr. 6), let-7f-1 (Seq.-ID Nr. 8), let-7i (Seq.-ID Nr. 11), MIR-001-2 (Seq.-ID Nr. 13), MIR-007-1 (Seq.-ID Nr. 14), MIR-015a (Seq.-ID Nr. 22), MIR-015b (Seq.-ID Nr. 23), MIR-016-1 (Seq.-ID Nr. 24), MIR-019b-1-P (Seq.-ID Nr. 29), MIR-024-1,2 (Seq.-ID Nr. 36, 37), MIR-027a,b (Seq.-ID Nr. 42, 43), MIR-029a,c (Seq.-ID Nr. 45, 46), MIR-030d (Seq.-ID Nr. 53), MIR-032 (Seq.-ID Nr. 56), MIR-092-1 (Seq.-ID Nr. 61), MIR-098 (Seq.-ID Nr. 66), MIR-099a (Seq.-ID Nr. 67), MIR-107 (Seq.-ID Nr. 78), MIR-125b-1 (Seq.-ID Nr. 85), MIR-126 (Seq.-ID Nr. 87), MIR-128a (Seq.-ID Nr. 89), MIR-132 (Seq.-ID Nr. 94), MIR-136 (Seq.-ID Nr. 102), MIR-142-P (Seq.-ID Nr. 109), MrR-145-P (Seq.-ID Nr. 112), MIR-152 (Seq.-ID Nr. 120), MIR-181c (Seq.-ID Nr. 126), MIR-196a-2 (Seq.-ID Nr. 144), MIR-213 (Seq.-ID Nr. 162), MIR-215 (Seq.-ID Nr. 164), MIR-218-1,2 (Seq.-ID Nr. 167, 168), MIR-328 (Seq.-ID Nr. 189), MIR-331-P (Seq.-ID Nr. 191), MIR-345 (Seq.-ID Nr. 198), MIR-367 (Seq.-ID Nr. 201) und MIR-424 (Seq.-ID Nr. 221) bestehenden Gruppe oder einer beliebigen Kombination davon ausgewählt ist.

4. Verfahren nach Anspruch 3, wobei das zumindest eine weitere miR-Genprodukt aus der aus let-7b, let-7c, let-7d, let-7f-1, let-7i, MIR-007-1, MIR-015a, MIR-015b, MIR016-1, MIR-021, MIR-023a, MIR-024-1,2, MIR-027a,b, MIR-030d, MIR-098, MIR-099a, MIR-100, MIR-125b-1, MIR-126, MIR-132, MIR-142-P, MIR-145-P, MIR-152, MIR-155, MIR-181a,c, MIR-196a-2, MIR-212, MIR-213, MIR-218-1,2, MIR-221, MIR-222-P, MIR301, MIR-331-P, MIR-345, MIR-376 bestehenden Gruppe oder einer beliebigen Kombination davon ausgewählt ist.

5. Verfahren nach Anspruch 3, wobei das zumindest eine weitere miR-Genprodukt aus der aus let-7b, let-7d, let-7f, let-7i, MIR-015a, MIR-015b, MIR-016-1, MIR-021, MIR-023a, MIR-024, MIR-100, MIR-125b-1, MIR-132, MIR-155, MIR-181a, MIR-181C, MIR-212, MIR-221, MIR-301, MIR376a bestehenden Gruppe oder einer beliebigen Kombination davon ausgewählt ist.

6. Verfahren nach Anspruch 3, wobei das zumindest eine weitere miR-Genprodukt aus der aus let-7a-2-P, let-7b, let-7c, let-7d, let-7f-1, let-7i, MIR-015b, MIR-019b-1-P, MIR-098, MIR-128a, MIR-136, MIR-152, MIR-155, MIR-181a, MIR-196a-2, MIR-212, MIR-215, MIR-218-1, MIR-218-2, MIR-301, MIR-328, MIR-331-P, MIR-424 bestehenden Gruppe oder einer beliebigen Kombination davon ausgewählt ist.

7. Verfahren nach Anspruch 3, wobei das zumindest eine weitere miR-Genprodukt MIR-021 (Seq.-ID Nr. 32), MIR-024, MIR-155, MIR-196a-2, MIR-210, MIR-223 oder eine beliebige Kombination davon ist.

8. Verfahren nach Anspruch 3, wobei das zumindest eine weitere miR-Genprodukt MIR-021 (Seq.-ID Nr. 32) ist.

9. Verfahren nach Anspruch 1, das weiters das Messen zumindest eines weiteren miR-Genprodukts in einer biologischen Probe, die von der Pankreas des Individuums stammt, umfasst, wobei eine Verringerung des Gehalts des miR-Genprodukts in der biologischen Probe im Vergleich zum Wert eines entsprechenden miR-Genprodukts in einer Kontrollprobe von normalem Pankreasgewebe darauf hinweist, dass das Individuum entweder ein Pankreasadenokarzinom hat oder das Risiko besteht, dass es ein solches entwickelt; und wobei das zumindest eine weitere miR-Genprodukt aus der aus MIR-139 (Seq.-ID Nr. 106), MIR-337 (Seq.-ID Nr. 193), MIR-133b (Seq.-ID Nr. 97), MIR-096-P (Seq.-ID Nr. 65), MIR-371 (Seq.-ID Nr. 205), MIR-383 (Seq.-ID Nr. 217), MIR-092-2-P (Seq.-ID Nr. 62), MIR-204 (Seq.-ID Nr. 155), MIR-299-P (Seq.-ID Nr. 177), MIR-375 (Seq.-ID Nr. 209), MIR-188b-P (Seq.-ID Nr. 135) und MIR-129-2 (Seq.-ID Nr. 91) bestehenden Gruppe oder einer beliebigen Kombination davon ausgewählt ist.

10. Verfahren nach Anspruch 9, wobei das zumindest eine weitere miR-Genprodukt MIR-096-P ist.

11. Verfahren nach Anspruch 9, wobei das zumindest eine weitere miR-Genprodukt MIR-375 ist.

12. Verfahren nach einem der vorangegangenen Ansprüche, wobei zumindest eines der Vielzahl von miR-Genprodukten reife miRNA ist.

13. Verfahren nach einem der vorangegangenen Ansprüche, wobei die biologische Probe Pankreasgewebe, Pankreastumor, Pankreaszellen oder Pankreassaft umfasst.

## Revendications

1. Procédé pour diagnostiquer si un sujet présente, ou est exposé à un risque de développer, un adénocarcinome pancréatique, qui comprend la mesure du taux d'un produit du gène miR dans un échantillon biologique dérivé du pancréas du sujet, où une augmentation du taux du produit du gène miR dans l'échantillon biologique par comparaison au taux d'un produit du gène miR correspondant dans un échantillon de contrôle de tissu pancréatique normal indique que le sujet présente, ou est exposé à un risque de développer, un adénocarcinome pancréatique, où le produit du gène miR est une forme précurseur ou mature de MIR-135b (SEQ ID NO: 101).

2. Procédé selon la revendication 1, qui comprend la mesure du taux d'une combinaison de produits du gène miR listés sur la Figure 16 dans un échantillon biologique dérivé du pancréas du sujet, où une réduction du taux du produit du gène miR dans l'échantillon biologique par comparaison au taux d'un produit du gène miR correspondant dans un échantillon de contrôle de tissu pancréatique normal indique que le sujet présente, ou est exposé à un risque de développer, un adénocarcinome pancréatique dans le cas de miR-148a, miR-216*, miR-217, miR-375 et miR-377 ; et où une augmentation du taux du produit du gène miR dans l'échantillon biologique par comparaison au taux d'un produit du gène miR correspondant dans un échantillon de contrôle de tissu pancréatique normal indique que le sujet présente, ou est exposé à un risque de développer, un adénocarcinome pancréatique dans le cas de let-7c, let-7d, let-7e, let-7f, let-7g, let-7i, miR-009*, miR-010a, miR-010b, miR-015a, miR-016, miR-017-3p, miR-017-5p, miR-018, miR-20, miR-021, miR-022, miR-023a, miR-023b, miR-024, miR-026a, miR-027a, miR-027b, miR-028, miR-029b, miR-030e-3p, miR-031, miR-034a, miR-034b, miR-034c, miR-093, miR-098, miR-099a, miR-099b, miR-100, miR-101, miR-103, miR-106b, miR-125a, miR-125b, miR-126, miR-126*, miR-127, miR-130a, miR-132, miR-140, miR-142-3p, miR-142-5p, miR-143, miR-145, miR-146, miR-146b, miR-148b, miR-149, miR-150, miR-152, miR-155, miR-181a, miR-181b, miR-181c, miR-181d, miR-183, miR-186, miR-189, miR-190, miR-191, miR-192, miR-193, miR-194, miR-195, miR-196a, miR-199a*, miR-199b, miR-200a, miR-200b, miR-203, miR-210, miR-212, miR-213, miR-214, miR-218, miR-221, miR-222, miR-223, miR-296, miR-301, miR-324-3p, miR-324-5p, miR-330, miR-331, miR-338, miR-339, miR-340, miR-342, miR-345, miR-346, miR-361, miR-370, miR-372, miR-374, miR-376a, miR-378, miR-379, miR-382, miR-422b, miR-423, et miR-425,
et où la combinaison comprend une forme précurseur ou mature de MIR-135b.

3. Procédé selon la revendication 1, qui comprend en outre la mesure du taux d'au moins un produit du gène miR supplémentaire dans un échantillon biologique dérivé du pancréas du sujet, où une augmentation du taux du produit du gène miR dans l'échantillon biologique par comparaison au taux d'un produit du gène miR correspondant dans un échantillon de contrôle de tissu pancréatique normal indique que le sujet présente, ou est exposé à un risque de développer, un adénocarcinome pancréatique ; et où le au moins un produit du gène miR supplémentaire est sélectionné dans le groupe consistant en : MIR-021 (SEQ ID NO: 32), MIR-221 (SEQ ID NO: 172), MIR-301 (SEQ ID NO: 178), MIR-212 (SEQ ID NO: 161), MIR-100 (SEQ ID NO: 69), let-7i (SEQ ID NO: 11), MIR-222-P (SEQ ID NO: 173), MIR-155 (SEQ ID NO: 124), MIR-023a (SEQ ID NO: 34), MIR-181a (SEQ ID NO: 125), MIR-376 (SEQ ID NO: 210), let-7a-2-P (SEQ ID NO: 2), let-7b (SEQ ID NO: 4), let-7c (SEQ ID NO: 5), let-7d (SEQ ID NO: 6), let-7f-1 (SEQ ID NO: 8), let-7i (SEQ ID NO: 11), MIR-001-2 (SEQ ID NO: 13), MIR-007-1 (SEQ ID NO: 14), MIR-015a (SEQ ID NO: 22), MIR-015b (SEQ ID NO: 23), MIR-016-1 (SEQ ID NO: 24), MIR-019b-1-P (SEQ ID NO: 29), MIR-024-1,2 (SEQ ID NO: 36, 37), MIR-027a,b (SEQ ID NO: 42, 43), MIR-029a,c (SEQ ID NO: 45, 46), MIR-030d (SEQ ID NO: 53), MIR-032 (SEQ ID NO: 56), MIR-092-1 (SEQ ID NO: 61), MIR-098 (SEQ ID NO: 66), MIR-099a (SEQ ID NO: 67), MIR-107 (SEQ ID NO: 78), MIR-125b-1 (SEQ ID NO: 85), MIR-126 (SEQ ID NO: 87), MIR-128a (SEQ ID NO: 89), MIR-132 (SEQ ID NO: 94), MIR-136 (SEQ ID NO: 102), MIR-142-P (SEQ ID NO: 109), MIR-145-P (SEQ ID NO: 112), MIR-152 (SEQ ID NO: 120), MIR-181c (SEQ ID NO: 126), MIR-196a-2 (SEQ ID NO: 144), MIR-213 (SEQ ID NO: 162), MIR-215 (SEQ ID NO: 164), MIR-218-1,2 (SEQ ID NO: 167, 168), MIR-328 (SEQ ID NO: 189), MIR-331-P (SEQ ID NO: 191), MIR-345 (SEQ ID NO: 198), MIR-367 (SEQ ID NO: 201), et MIR-424 (SEQ ID NO: 221) ou une quelconque combinaison de ceux-ci.

4. Procédé selon la revendication 3, dans lequel le au moins un produit du gène miR supplémentaire est sélectionné dans le groupe consistant en let-7b, let-7c, let-7d, let-7f-1, let-7i, MIR-007-1, MIR-015a, MIR-015b, MIR-016-1, MIR-021, MIR-023a, MIR-024-1,2, MIR-027a,b, MIR-030d, MIR-098, MIR-099a, MIR-100, MIR-125b-1, MIR-126, MIR-132, MIR-142-P, MIR-145-P, MIR-152, MIR-155, MIR-181a,c, MIR-196a-2, MIR-212, MIR-213, MIR-218-1,2, MIR-221, MIR-222-P, MIR-301, MIR-331-P, MIR-345, MIR-376, ou une quelconque combinaison de ceux-ci.

5. Procédé selon la revendication 3, dans lequel le au moins un produit du gène miR supplémentaire est sélectionné dans le groupe consistant en : let-7b, let-7d, let-7f, let-7i, MIR-015a, MIR-015b, MIR-016-1, MIR-021, MIR-023a, MIR-024, MIR-100, MIR-125b-1, MIR-132, MIR-155, MIR-181a, MIR-181c, MIR-212, MIR-221, MIR-301, MIR-376a, ou une quelconque combinaison de ceux-ci.

6. Procédé selon la revendication 3, dans lequel le au moins un produit du gène miR supplémentaire est sélectionné dans le groupe consistant en : let-7a-2-P, let-7b, let-7c, let-7d, let-7f-1, let-7i, MIR-015b, MIR-019b-1-P, MIR-098, MIR-128a, MIR-136, MIR-152, MIR-155, MIR-181a, MIR-196a-2, MIR-212, MIR-215, MIR-218-1, MIR-218-2, MIR-301, MIR-328, MIR-331-P, MIR-424, ou une quelconque combinaison de ceux-ci.

7. Procédé selon la revendication 3, dans lequel le au moins un produit du gène miR supplémentaire est MIR-021 (SEQ ID NO: 32), MIR-024, MIR-155, MIR-196a-2, MIR-210, MIR-223 ou une quelconque combinaison de ceux-ci.

8. Procédé selon la revendication 3, dans lequel le au moins un produit du gène miR supplémentaire est MIR-021 (SEQ ID NO: 32).

9. Procédé selon la revendication 1, qui comprend en outre la mesure du taux d'au moins un produit du gène miR supplémentaire dans un échantillon biologique dérivé du pancréas du sujet, où une réduction du taux du produit du gène miR dans l'échantillon biologique par comparaison au taux d'un produit du gène miR correspondant dans un échantillon de contrôle de tissu pancréatique normal indique que le sujet présente, ou est exposé à un risque de développer, un adénocarcinome pancréatique ; et où le au moins un produit du gène miR supplémentaire est sélectionné dans le groupe consistant en : MIR-139 (SEQ ID NO: 106), MIR-337 (SEQ ID NO: 193), MIR-133b (SEQ ID NO: 97), MIR-096-P (SEQ ID NO: 65), MIR-371 (SEQ ID NO: 205), MIR-383 (SEQ ID NO: 217), MIR-092-2-P (SEQ ID NO: 62), MIR-204 (SEQ ID NO: 155), MIR-299-P (SEQ ID NO: 177), MIR-375 (SEQ ID NO: 209), MIR-188b-P (SEQ ID NO: 135), et MIR-129-2 (SEQ ID NO: 91), ou une quelconque combinaison de ceux-ci.

10. Procédé selon la revendication 9, dans lequel le au moins un produit du gène miR supplémentaire est MIR-096-P.

11. Procédé selon la revendication 9, dans lequel le au moins un produit du gène miR supplémentaire est MIR-375.

12. Procédé selon l'une quelconque des revendications précédentes, dans lequel au moins un de la pluralité de produits du gène miR est un ARNmi mature.

13. Procédé selon l'une quelconque des revendications précédentes, dans lequel l'échantillon biologique comprend un tissu pancréatique, une tumeur pancréatique, des cellules pancréatiques ou du jus pancréatique.


### SEQUENCE LISTING

<110> THE OHIO STATE UNIVERSITY
<120> MICRORNA EXPRESSION PROFILE ASSOCIATED WITH PANCREATIC CANCER
<130> CMD/FP6825251
<140> Divisional application of: EP 07757822.7
   <141> 2007-03-02
<150> 07757822.7
   <151> 2007-03-02
<150> PCT/US2007/063208
   <151> 2007-03-02
<150> 60/778,271
   <151> 2006-03-02
<160> 662
<170> PatentIn version 3.5
<210> 1
   <211> 80
   <212> DNA
   <213> Homo sapiens
<400> 1
   tgggatgagg tagtaggttg tatagtttta gggtcacacc caccactggg agataactat 60
   acaatctact gtctttccta 80
<210> 2
   <211> 72
   <212> DNA
   <213> Homo sapiens
<400> 2
   aggttgaggt agtaggttgt atagtttaga attacatcaa gggagataac tgtacagcct 60
   cctagctttc ct 72
<210> 3
   <211> 74
   <212> DNA
   <213> Homo sapiens
<400> 3
   gggtgaggta gtaggttgta tagtttgggg ctctgccctg ctatgggata actatacaat 60
   ctactgtctt tcct 74
<210> 4
   <211> 83
   <212> DNA
   <213> Homo sapiens
<400> 4
   cggggtgagg tagtaggttg tgtggtttca gggcagtgat gttgcccctc ggaagataac 60
   tatacaacct actgccttcc ctg 83
<210> 5
   <211> 84
   <212> DNA
   <213> Homo sapiens
<400> 5
   gcatccgggt tgaggtagta ggttgtatgg tttagagtta caccctggga gttaactgta 60
   caaccttcta gctttccttg gagc 84
<210> 6
   <211> 87
   <212> DNA
   <213> Homo sapiens
<400> 6
   cctaggaaga ggtagtaggt tgcatagttt tagggcaggg attttgccca caaggaggta 60
   actatacgac ctgctgcctt tcttagg 87
<210> 7
   <211> 79
   <212> DNA
   <213> Homo sapiens
<400> 7
   cccgggctga ggtaggaggt tgtatagttg aggaggacac ccaaggagat cactatacgg 60
   cctcctagct ttccccagg 79
<210> 8
   <211> 87
   <212> DNA
   <213> Homo sapiens
<400> 8
   tcagagtgag gtagtagatt gtatagttgt ggggtagtga ttttaccctg ttcaggagat 60
   aactatacaa tctattgcct tccctga 87
<210> 9
   <211> 83
   <212> DNA
   <213> Homo sapiens
<400> 9
   tgtgggatga ggtagtagat tgtatagttt tagggtcata ccccatcttg gagataacta 60
   tacagtctac tgtctttccc acg 83
<210> 10
   <211> 84
   <212> DNA
   <213> Homo sapiens
<400> 10
   aggctgaggt agtagtttgt acagtttgag ggtctatgat accacccggt acaggagata 60
   actgtacagg ccactgcctt gcca 84
<210> 11
   <211> 84
   <212> DNA
   <213> Homo sapiens
<400> 11
   ctggctgagg tagtagtttg tgctgttggt cgggttgtga cattgcccgc tgtggagata 60
   actgcgcaag ctactgcctt gcta 84
<210> 12
   <211> 71
   <212> DNA
   <213> Homo sapiens
<400> 12
   tgggaaacat acttctttat atgcccatat ggacctgcta agctatggaa tgtaaagaag 60
   tatgtatctc a 71
<210> 13
   <211> 85
   <212> DNA
   <213> Homo sapiens
<400> 13
   acctactcag agtacatact tctttatgta cccatatgaa catacaatgc tatggaatgt 60
   aaagaagtat gtatttttgg taggc 85
<210> 14
   <211> 110
   <212> DNA
   <213> Homo sapiens
<400> 14
   ttggatgttg gcctagttct gtgtggaaga ctagtgattt tgttgttttt agataactaa 60
   atcgacaaca aatcacagtc tgccatatgg cacaggccat gcctctacag 110
<210> 15
   <211> 110
   <212> DNA
   <213> Homo sapiens
<400> 15
   ctggatacag agtggaccgg ctggccccat ctggaagact agtgattttg ttgttgtctt 60
   actgcgctca acaacaaatc ccagtctacc taatggtgcc agccatcgca 110
<210> 16
   <211> 110
   <212> DNA
   <213> Homo sapiens
<400> 16
   agattagagt ggctgtggtc tagtgctgtg tggaagacta gtgattttgt tgttctgatg 60
   tactacgaca acaagtcaca gccggcctca tagcgcagac tcccttcgac 110
<210> 17
   <211> 89
   <212> DNA
   <213> Homo sapiens
<400> 17
   cggggttggt tgttatcttt ggttatctag ctgtatgagt ggtgtggagt cttcataaag 60
   ctagataacc gaaagtaaaa ataacccca 89
<210> 18
   <211> 87
   <212> DNA
   <213> Homo sapiens
<400> 18
   ggaagcgagt tgttatcttt ggttatctag ctgtatgagt gtattggtct tcataaagct 60
   agataaccga aagtaaaaac tccttca 87
<210> 19
   <211> 90
   <212> DNA
   <213> Homo sapiens
<400> 19
   ggaggcccgt ttctctcttt ggttatctag ctgtatgagt gccacagagc cgtcataaag 60
   ctagataacc gaaagtagaa atgattctca 90
<210> 20
   <211> 110
   <212> DNA
   <213> Homo sapiens
<400> 20
   gatctgtctg tcttctgtat ataccctgta gatccgaatt tgtgtaagga attttgtggt 60
   cacaaattcg tatctagggg aatatgtagt tgacataaac actccgctct 110
<210> 21
   <211> 110
   <212> DNA
   <213> Homo sapiens
<400> 21
   ccagaggttg taacgttgtc tatatatacc ctgtagaacc gaatttgtgt ggtatccgta 60
   tagtcacaga ttcgattcta ggggaatata tggtcgatgc aaaaacttca 110
<210> 22
   <211> 83
   <212> DNA
   <213> Homo sapiens
<400> 22
   ccttggagta aagtagcagc acataatggt ttgtggattt tgaaaaggtg caggccatat 60
   tgtgctgcct caaaaataca agg 83
<210> 23
   <211> 98
   <212> DNA
   <213> Homo sapiens
<400> 23
   ttgaggcctt aaagtactgt agcagcacat catggtttac atgctacagt caagatgcga 60
   atcattattt gctgctctag aaatttaagg aaattcat 98
<210> 24
   <211> 89
   <212> DNA
   <213> Homo sapiens
<400> 24
   gtcagcagtg ccttagcagc acgtaaatat tggcgttaag attctaaaat tatctccagt 60
   attaactgtg ctgctgaagt aaggttgac 89
<210> 25
   <211> 81
   <212> DNA
   <213> Homo sapiens
<400> 25
   gttccactct agcagcacgt aaatattggc gtagtgaaat atatattaaa caccaatatt 60
   actgtgctgc tttagtgtga c 81
<210> 26
   <211> 84
   <212> DNA
   <213> Homo sapiens
<400> 26
   gtcagaataa tgtcaaagtg cttacagtgc aggtagtgat atgtgcatct actgcagtga 60
   aggcacttgt agcattatgg tgac 84
<210> 27
   <211> 71
   <212> DNA
   <213> Homo sapiens
<400> 27
   tgttctaagg tgcatctagt gcagatagtg aagtagatta gcatctactg ccctaagtgc 60
   tccttctggc a 71
<210> 28
   <211> 82
   <212> DNA
   <213> Homo sapiens
<400> 28
   gcagtcctct gttagttttg catagttgca ctacaagaag aatgtagttg tgcaaatcta 60
   tgcaaaactg atggtggcct gc 82
<210> 29
   <211> 87
   <212> DNA
   <213> Homo sapiens
<400> 29
   cactgttcta tggttagttt tgcaggtttg catccagctg tgtgatattc tgctgtgcaa 60
   atccatgcaa aactgactgt ggtagtg 87
<210> 30
   <211> 96
   <212> DNA
   <213> Homo sapiens
<400> 30
   acattgctac ttacaattag ttttgcaggt ttgcatttca gcgtatatat gtatatgtgg 60
   ctgtgcaaat ccatgcaaaa ctgattgtga taatgt 96
<210> 31
   <211> 71
   <212> DNA
   <213> Homo sapiens
<400> 31
   gtagcactaa agtgcttata gtgcaggtag tgtttagtta tctactgcat tatgagcact 60
   taaagtactg c 71
<210> 32
   <211> 72
   <212> DNA
   <213> Homo sapiens
<400> 32
   tgtcgggtag cttatcagac tgatgttgac tgttgaatct catggcaaca ccagtcgatg 60
   ggctgtctga ca 72
<210> 33
   <211> 85
   <212> DNA
   <213> Homo sapiens
<400> 33
   ggctgagccg cagtagttct tcagtggcaa gctttatgtc ctgacccagc taaagctgcc 60
   agttgaagaa ctgttgccct ctgcc 85
<210> 34
   <211> 73
   <212> DNA
   <213> Homo sapiens
<400> 34
   ggccggctgg ggttcctggg gatgggattt gcttcctgtc acaaatcaca ttgccaggga 60
   tttccaaccg acc 73
<210> 35
   <211> 97
   <212> DNA
   <213> Homo sapiens
<400> 35
   ctcaggtgct ctggctgctt gggttcctgg catgctgatt tgtgacttaa gattaaaatc 60
   acattgccag ggattaccac gcaaccacga ccttggc 97
<210> 36
   <211> 68
   <212> DNA
   <213> Homo sapiens
<400> 36
   ctccggtgcc tactgagctg atatcagttc tcattttaca cactggctca gttcagcagg 60
   aacaggag 68
<210> 37
   <211> 73
   <212> DNA
   <213> Homo sapiens
<400> 37
   ctctgcctcc cgtgcctact gagctgaaac acagttggtt tgtgtacact ggctcagttc 60
   agcaggaaca ggg 73
<210> 38
   <211> 84
   <212> DNA
   <213> Homo sapiens
<400> 38
   ggccagtgtt gagaggcgga gacttgggca attgctggac gctgccctgg gcattgcact 60
   tgtctcggtc tgacagtgcc ggcc 84
<210> 39
   <211> 77
   <212> DNA
   <213> Homo sapiens
<400> 39
   gtggcctcgt tcaagtaatc caggataggc tgtgcaggtc ccaatgggcc tattcttggt 60
   tacttgcacg gggacgc 77
<210> 40
   <211> 84
   <212> DNA
   <213> Homo sapiens
<400> 40
   ggctgtggct ggattcaagt aatccaggat aggctgtttc catctgtgag gcctattctt 60
   gattacttgt ttctggaggc agct 84
<210> 41
   <211> 77
   <212> DNA
   <213> Homo sapiens
<400> 41
   ccgggaccca gttcaagtaa ttcaggatag gttgtgtgct gtccagcctg ttctccatta 60
   cttggctcgg ggaccgg 77
<210> 42
   <211> 78
   <212> DNA
   <213> Homo sapiens
<400> 42
   ctgaggagca gggcttagct gcttgtgagc agggtccaca ccaagtcgtg ttcacagtgg 60
   ctaagttccg ccccccag 78
<210> 43
   <211> 97
   <212> DNA
   <213> Homo sapiens
<400> 43
   acctctctaa caaggtgcag agcttagctg attggtgaac agtgattggt ttccgctttg 60
   ttcacagtgg ctaagttctg cacctgaaga gaaggtg 97
<210> 44
   <211> 86
   <212> DNA
   <213> Homo sapiens
<400> 44
   ggtccttgcc ctcaaggagc tcacagtcta ttgagttacc tttctgactt tcccactaga 60
   ttgtgagctc ctggagggca ggcact 86
<210> 45
   <211> 64
   <212> DNA
   <213> Homo sapiens
<400> 45
   atgactgatt tcttttggtg ttcagagtca atataatttt ctagcaccat ctgaaatcgg 60
   ttat 64
<210> 46
   <211> 88
   <212> DNA
   <213> Homo sapiens
<400> 46
   atctcttaca caggctgacc gatttctcct ggtgttcaga gtctgttttt gtctagcacc 60
   atttgaaatc ggttatgatg taggggga 88
<210> 47
   <211> 81
   <212> DNA
   <213> Homo sapiens
<400> 47
   cttcaggaag ctggtttcat atggtggttt agatttaaat agtgattgtc tagcaccatt 60
   tgaaatcagt gttcttgggg g 81
<210> 48
   <211> 81
   <212> DNA
   <213> Homo sapiens
<400> 48
   cttctggaag ctggtttcac atggtggctt agatttttcc atctttgtat ctagcaccat 60
   ttgaaatcag tgttttagga g 81
<210> 49
   <211> 71
   <212> DNA
   <213> Homo sapiens
<400> 49
   gcgactgtaa acatcctcga ctggaagctg tgaagccaca gatgggcttt cagtcggatg 60
   tttgcagctg c 71
<210> 50
   <211> 88
   <212> DNA
   <213> Homo sapiens
<400> 50
   accaagtttc agttcatgta aacatcctac actcagctgt aatacatgga ttggctggga 60
   ggtggatgtt tacttcagct gacttgga 88
<210> 51
   <211> 89
   <212> DNA
   <213> Homo sapiens
<400> 51
   accatgctgt agtgtgtgta aacatcctac actctcagct gtgagctcaa ggtggctggg 60
   agagggttgt ttactccttc tgccatgga 89
<210> 52
   <211> 72
   <212> DNA
   <213> Homo sapiens
<400> 52
   agatactgta aacatcctac actctcagct gtggaaagta agaaagctgg gagaaggctg 60
   tttactcttt ct 72
<210> 53
   <211> 70
   <212> DNA
   <213> Homo sapiens
<400> 53
   gttgttgtaa acatccccga ctggaagctg taagacacag ctaagctttc agtcagatgt 60
   ttgctgctac 70
<210> 54
   <211> 92
   <212> DNA
   <213> Homo sapiens
<400> 54
   gggcagtctt tgctactgta aacatccttg actggaagct gtaaggtgtt cagaggagct 60
   ttcagtcgga tgtttacagc ggcaggctgc ca 92
<210> 55
   <211> 71
   <212> DNA
   <213> Homo sapiens
<400> 55
   ggagaggagg caagatgctg gcatagctgt tgaactggga acctgctatg ccaacatatt 60
   gccatctttc c 71
<210> 56
   <211> 70
   <212> DNA
   <213> Homo sapiens
<400> 56
   ggagatattg cacattacta agttgcatgt tgtcacggcc tcaatgcaat ttagtgtgtg 60
   tgatattttc 70
<210> 57
   <211> 69
   <212> DNA
   <213> Homo sapiens
<400> 57
   ctgtggtgca ttgtagttgc attgcatgtt ctggtggtac ccatgcaatg tttccacagt 60
   gcatcacag 69
<210> 58
   <211> 110
   <212> DNA
   <213> Homo sapiens
<400> 58
   ggccagctgt gagtgtttct ttggcagtgt cttagctggt tgttgtgagc aatagtaagg 60
   aagcaatcag caagtatact gccctagaag tgctgcacgt tgtggggccc 110
<210> 59
   <211> 84
   <212> DNA
   <213> Homo sapiens
<400> 59
   gtgctcggtt tgtaggcagt gtcattagct gattgtactg tggtggttac aatcactaac 60
   tccactgcca tcaaaacaag gcac 84
<210> 60
   <211> 77
   <212> DNA
   <213> Homo sapiens
<400> 60
   agtctagtta ctaggcagtg tagttagctg attgctaata gtaccaatca ctaaccacac 60
   ggccaggtaa aaagatt 77
<210> 61
   <211> 78
   <212> DNA
   <213> Homo sapiens
<400> 61
   ctttctacac aggttgggat cggttgcaat gctgtgtttc tgtatggtat tgcacttgtc 60
   ccggcctgtt gagtttgg 78
<210> 62
   <211> 75
   <212> DNA
   <213> Homo sapiens
<400> 62
   tcatccctgg gtggggattt gttgcattac ttgtgttcta tataaagtat tgcacttgtc 60
   ccggcctgtg gaaga 75
<210> 63
   <211> 80
   <212> DNA
   <213> Homo sapiens
<400> 63
   ctgggggctc caaagtgctg ttcgtgcagg tagtgtgatt acccaaccta ctgctgagct 60
   agcacttccc gagcccccgg 80
<210> 64
   <211> 81
   <212> DNA
   <213> Homo sapiens
<400> 64
   aacacagtgg gcactcaata aatgtctgtt gaattgaaat gcgttacatt caacgggtat 60
   ttattgagca cccactctgt g 81
<210> 65
   <211> 78
   <212> DNA
   <213> Homo sapiens
<400> 65
   tggccgattt tggcactagc acatttttgc ttgtgtctct ccgctctgag caatcatgtg 60
   cagtgccaat atgggaaa 78
<210> 66
   <211> 119
   <212> DNA
   <213> Homo sapiens
<400> 66
   aggattctgc tcatgccagg gtgaggtagt aagttgtatt gttgtggggt agggatatta 60
   ggccccaatt agaagataac tatacaactt actactttcc ctggtgtgtg gcatattca 119
<210> 67
   <211> 81
   <212> DNA
   <213> Homo sapiens
<400> 67
   cccattggca taaacccgta gatccgatct tgtggtgaag tggaccgcac aagctcgctt 60
   ctatgggtct gtgtcagtgt g 81
<210> 68
   <211> 70
   <212> DNA
   <213> Homo sapiens
<400> 68
   ggcacccacc cgtagaaccg accttgcggg gccttcgccg cacacaagct cgtgtctgtg 60
   ggtccgtgtc 70
<210> 69
   <211> 80
   <212> DNA
   <213> Homo sapiens
<400> 69
   cctgttgcca caaacccgta gatccgaact tgtggtatta gtccgcacaa gcttgtatct 60
   ataggtatgt gtctgttagg 80
<210> 70
   <211> 75
   <212> DNA
   <213> Homo sapiens
<400> 70
   tgccctggct cagttatcac agtgctgatg ctgtctattc taaaggtaca gtactgtgat 60
   aactgaagga tggca 75
<210> 71
   <211> 79
   <212> DNA
   <213> Homo sapiens
<400> 71
   actgtccttt ttcggttatc atggtaccga tgctgtatat ctgaaaggta cagtactgtg 60
   ataactgaag aatggtggt 79
<210> 72
   <211> 78
   <212> DNA
   <213> Homo sapiens
<400> 72
   tactgccctc ggcttcttta cagtgctgcc ttgttgcata tggatcaagc agcattgtac 60
   agggctatga aggcattg 78
<210> 73
   <211> 78
   <212> DNA
   <213> Homo sapiens
<400> 73
   ttgtgctttc agcttcttta cagtgctgcc ttgtagcatt caggtcaagc agcattgtac 60
   agggctatga aagaacca 78
<210> 74
   <211> 81
   <212> DNA
   <213> Homo sapiens
<400> 74
   tgtgcatcgt ggtcaaatgc tcagactcct gtggtggctg ctcatgcacc acggatgttt 60
   gagcatgtgc tacggtgtct a 81
<210> 75
   <211> 81
   <212> DNA
   <213> Homo sapiens
<400> 75
   tgtgcatcgt ggtcaaatgc tcagactcct gtggtggctg cttatgcacc acggatgttt 60
   gagcatgtgc tatggtgtct a 81
<210> 76
   <211> 81
   <212> DNA
   <213> Homo sapiens
<400> 76
   ccttggccat gtaaaagtgc ttacagtgca ggtagctttt tgagatctac tgcaatgtaa 60
   gcacttctta cattaccatg g 81
<210> 77
   <211> 82
   <212> DNA
   <213> Homo sapiens
<400> 77
   cctgccgggg ctaaagtgct gacagtgcag atagtggtcc tctccgtgct accgcactgt 60
   gggtacttgc tgctccagca gg 82
<210> 78
   <211> 81
   <212> DNA
   <213> Homo sapiens
<400> 78
   ctctctgctt tcagcttctt tacagtgttg ccttgtggca tggagttcaa gcagcattgt 60
   acagggctat caaagcacag a 81
<210> 79
   <211> 92
   <212> DNA
   <213> Homo sapiens
<400> 79
   acactgcaag aacaataagg atttttaggg gcattatgac tgagtcagaa aacacagctg 60
   cccctgaaag tccctcattt ttcttgctgt cc 92
<210> 80
   <211> 85
   <212> DNA
   <213> Homo sapiens
<400> 80
   ccttagcaga gctgtggagt gtgacaatgg tgtttgtgtc taaactatca aacgccatta 60
   tcacactaaa tagctactgc taggc 85
<210> 81
   <211> 85
   <212> DNA
   <213> Homo sapiens
<400> 81
   aggcctctct ctccgtgttc acagcggacc ttgatttaaa tgtccataca attaaggcac 60
   gcggtgaatg ccaagaatgg ggctg 85
<210> 82
   <211> 109
   <212> DNA
   <213> Homo sapiens
<400> 82
   atcaagatta gaggctctgc tctccgtgtt cacagcggac cttgatttaa tgtcatacaa 60
   ttaaggcacg cggtgaatgc caagagcgga gcctacggct gcacttgaa 109
<210> 83
   <211> 87
   <212> DNA
   <213> Homo sapiens
<400> 83
   tgagggcccc tctgcgtgtt cacagcggac cttgatttaa tgtctataca attaaggcac 60
   gcggtgaatg ccaagagagg cgcctcc 87
<210> 84
   <211> 86
   <212> DNA
   <213> Homo sapiens
<400> 84
   tgccagtctc taggtccctg agacccttta acctgtgagg acatccaggg tcacaggtga 60
   ggttcttggg agcctggcgt ctggcc 86
<210> 85
   <211> 88
   <212> DNA
   <213> Homo sapiens
<400> 85
   tgcgctcctc tcagtccctg agaccctaac ttgtgatgtt taccgtttaa atccacgggt 60
   taggctcttg ggagctgcga gtcgtgct 88
<210> 86
   <211> 89
   <212> DNA
   <213> Homo sapiens
<400> 86
   accagacttt tcctagtccc tgagacccta acttgtgagg tattttagta acatcacaag 60
   tcaggctctt gggacctagg cggagggga 89
<210> 87
   <211> 85
   <212> DNA
   <213> Homo sapiens
<400> 87
   cgctggcgac gggacattat tacttttggt acgcgctgtg acacttcaaa ctcgtaccgt 60
   gagtaataat gcgccgtcca cggca 85
<210> 88
   <211> 97
   <212> DNA
   <213> Homo sapiens
<400> 88
   tgtgatcact gtctccagcc tgctgaagct cagagggctc tgattcagaa agatcatcgg 60
   atccgtctga gcttggctgg tcggaagtct catcatc 97
<210> 89
   <211> 82
   <212> DNA
   <213> Homo sapiens
<400> 89
   tgagctgttg gattcggggc cgtagcactg tctgagaggt ttacatttct cacagtgaac 60
   cggtctcttt ttcagctgct tc 82
<210> 90
   <211> 84
   <212> DNA
   <213> Homo sapiens
<400> 90
   tgtgcagtgg gaaggggggc cgatacactg tacgagagtg agtagcaggt ctcacagtga 60
   accggtctct ttccctactg tgtc 84
<210> 91
   <211> 90
   <212> DNA
   <213> Homo sapiens
<400> 91
   tgcccttcgc gaatcttttt gcggtctggg cttgctgtac ataactcaat agccggaagc 60
   ccttacccca aaaagcattt gcggagggcg 90
<210> 92
   <211> 89
   <212> DNA
   <213> Homo sapiens
<400> 92
   tgctgctggc cagagctctt ttcacattgt gctactgtct gcacctgtca ctagcagtgc 60
   aatgttaaaa gggcattggc cgtgtagtg 89
<210> 93
   <211> 82
   <212> DNA
   <213> Homo sapiens
<400> 93
   ggcctgcccg acactctttc cctgttgcac tactataggc cgctgggaag cagtgcaatg 60
   atgaaagggc atcggtcagg tc 82
<210> 94
   <211> 101
   <212> DNA
   <213> Homo sapiens
<400> 94
   ccgcccccgc gtctccaggg caaccgtggc tttcgattgt tactgtggga actggaggta 60
   acagtctaca gccatggtcg ccccgcagca cgcccacgcg c 101
<210> 95
   <211> 88
   <212> DNA
   <213> Homo sapiens
<400> 95
   acaatgcttt gctagagctg gtaaaatgga accaaatcgc ctcttcaatg gatttggtcc 60
   ccttcaacca gctgtagcta tgcattga 88
<210> 96
   <211> 102
   <212> DNA
   <213> Homo sapiens
<400> 96
   gggagccaaa tgctttgcta gagctggtaa aatggaacca aatcgactgt ccaatggatt 60
   tggtcccctt caaccagctg tagctgtgca ttgatggcgc cg 102
<210> 97
   <211> 119
   <212> DNA
   <213> Homo sapiens
<400> 97
   cctcagaaga aagatgcccc ctgctctggc tggtcaaacg gaaccaagtc cgtcttcctg 60
   agaggtttgg tccccttcaa ccagctacag cagggctggc aatgcccagt ccttggaga 119
<210> 98
   <211> 73
   <212> DNA
   <213> Homo sapiens
<400> 98
   cagggtgtgt gactggttga ccagaggggc atgcactgtg ttcaccctgt gggccaccta 60
   gtcaccaacc ctc 73
<210> 99
   <211> 90
   <212> DNA
   <213> Homo sapiens
<400> 99
   aggcctcgct gttctctatg gctttttatt cctatgtgat tctactgctc actcatatag 60
   ggattggagc cgtggcgcac ggcggggaca 90
<210> 100
   <211> 100
   <212> DNA
   <213> Homo sapiens
<400> 100
   agataaattc actctagtgc tttatggctt tttattccta tgtgatagta ataaagtctc 60
   atgtagggat ggaagccatg aaatacattg tgaaaaatca 100
<210> 101
   <211> 97
   <212> DNA
   <213> Homo sapiens
<400> 101
   cactctgctg tggcctatgg cttttcattc ctatgtgatt gctgtcccaa actcatgtag 60
   ggctaaaagc catgggctac agtgaggggc gagctcc 97
<210> 102
   <211> 82
   <212> DNA
   <213> Homo sapiens
<400> 102
   tgagccctcg gaggactcca tttgttttga tgatggattc ttatgctcca tcatcgtctc 60
   aaatgagtct tcagagggtt ct 82
<210> 103
   <211> 102
   <212> DNA
   <213> Homo sapiens
<400> 103
   ggtcctctga ctctcttcgg tgacgggtat tcttgggtgg ataatacgga ttacgttgtt 60
   attgcttaag aatacgcgta gtcgaggaga gtaccagcgg ca 102
<210> 104
   <211> 99
   <212> DNA
   <213> Homo sapiens
<400> 104
   ccctggcatg gtgtggtggg gcagctggtg ttgtgaatca ggccgttgcc aatcagagaa 60
   cggctacttc acaacaccag ggccacacca cactacagg 99
<210> 105
   <211> 84
   <212> DNA
   <213> Homo sapiens
<400> 105
   cgttgctgca gctggtgttg tgaatcaggc cgacgagcag cgcatcctct tacccggcta 60
   tttcacgaca ccagggttgc atca 84
<210> 106
   <211> 68
   <212> DNA
   <213> Homo sapiens
<400> 106
   gtgtattcta cagtgcacgt gtctccagtg tggctcggag gctggagacg cggccctgtt 60
   ggagtaac 68
<210> 107
   <211> 100
   <212> DNA
   <213> Homo sapiens
<400> 107
   tgtgtctctc tctgtgtcct gccagtggtt ttaccctatg gtaggttacg tcatgctgtt 60
   ctaccacagg gtagaaccac ggacaggata ccggggcacc 100
<210> 108
   <211> 95
   <212> DNA
   <213> Homo sapiens
<400> 108
   cggccggccc tgggtccatc ttccagtaca gtgttggatg gtctaattgt gaagctccta 60
   acactgtctg gtaaagatgg ctcccgggtg ggttc 95
<210> 109
   <211> 87
   <212> DNA
   <213> Homo sapiens
<400> 109
   gacagtgcag tcacccataa agtagaaagc actactaaca gcactggagg gtgtagtgtt 60
   tcctacttta tggatgagtg tactgtg 87
<210> 110
   <211> 106
   <212> DNA
   <213> Homo sapiens
<400> 110
   gcgcagcgcc ctgtctccca gcctgaggtg cagtgctgca tctctggtca gttgggagtc 60
   tgagatgaag cactgtagct caggaagaga gaagttgttc tgcagc 106
<210> 111
   <211> 86
   <212> DNA
   <213> Homo sapiens
<400> 111
   tggggccctg gctgggatat catcatatac tgtaagtttg cgatgagaca ctacagtata 60
   gatgatgtac tagtccgggc accccc 86
<210> 112
   <211> 88
   <212> DNA
   <213> Homo sapiens
<400> 112
   caccttgtcc tcacggtcca gttttcccag gaatccctta gatgctaaga tggggattcc 60
   tggaaatact gttcttgagg tcatggtt 88
<210> 113
   <211> 99
   <212> DNA
   <213> Homo sapiens
<400> 113
   ccgatgtgta tcctcagctt tgagaactga attccatggg ttgtgtcagt gtcagacctc 60
   tgaaattcag ttcttcagct gggatatctc tgtcatcgt 99
<210> 114
   <211> 72
   <212> DNA
   <213> Homo sapiens
<400> 114
   aatctaaaga caacatttct gcacacacac cagactatgg aagccagtgt gtggaaatgc 60
   ttctgctaga tt 72
<210> 115
   <211> 68
   <212> DNA
   <213> Homo sapiens
<400> 115
   gaggcaaagt tctgagacac tccgactctg agtatgatag aagtcagtgc actacagaac 60
   tttgtctc 68
<210> 116
   <211> 99
   <212> DNA
   <213> Homo sapiens
<400> 116
   caagcacgat tagcatttga ggtgaagttc tgttatacac tcaggctgtg gctctctgaa 60
   agtcagtgca tcacagaact ttgtctcgaa agctttcta 99
<210> 117
   <211> 89
   <212> DNA
   <213> Homo sapiens
<400> 117
   gccggcgccc gagctctggc tccgtgtctt cactcccgtg cttgtccgag gagggaggga 60
   gggacggggg ctgtgctggg gcagctgga 89
<210> 118
   <211> 84
   <212> DNA
   <213> Homo sapiens
<400> 118
   ctccccatgg ccctgtctcc caacccttgt accagtgctg ggctcagacc ctggtacagg 60
   cctgggggac agggacctgg ggac 84
<210> 119
   <211> 90
   <212> DNA
   <213> Homo sapiens
<400> 119
   tttcctgccc tcgaggagct cacagtctag tatgtctcat cccctactag actgaagctc 60
   cttgaggaca gggatggtca tactcacctc 90
<210> 120
   <211> 87
   <212> DNA
   <213> Homo sapiens
<400> 120
   tgtccccccc ggcccaggtt ctgtgataca ctccgactcg ggctctggag cagtcagtgc 60
   atgacagaac ttgggcccgg aaggacc 87
<210> 121
   <211> 90
   <212> DNA
   <213> Homo sapiens
<400> 121
   ctcacagctg ccagtgtcat ttttgtgatc tgcagctagt attctcactc cagttgcata 60
   gtcacaaaag tgatcattgg caggtgtggc 90
<210> 122
   <211> 87
   <212> DNA
   <213> Homo sapiens
<400> 122
   agcggtggcc agtgtcattt ttgtgatgtt gcagctagta atatgagccc agttgcatag 60
   tcacaaaagt gatcattgga aactgtg 87
<210> 123
   <211> 84
   <212> DNA
   <213> Homo sapiens
<400> 123
   gtggtacttg aagataggtt atccgtgttg ccttcgcttt atttgtgacg aatcatacac 60
   ggttgaccta tttttcagta ccaa 84
<210> 124
   <211> 65
   <212> DNA
   <213> Homo sapiens
<400> 124
   ctgttaatgc taatcgtgat aggggttttt gcctccaact gactcctaca tattagcatt 60
   aacag 65
<210> 125
   <211> 110
   <212> DNA
   <213> Homo sapiens
<400> 125
   agaagggcta tcaggccagc cttcagagga ctccaaggaa cattcaacgc tgtcggtgag 60
   tttgggattt gaaaaaacca ctgaccgttg actgtacctt ggggtcctta 110
<210> 126
   <211> 110
   <212> DNA
   <213> Homo sapiens
<400> 126
   cggaaaattt gccaagggtt tgggggaaca ttcaacctgt cggtgagttt gggcagctca 60
   ggcaaaccat cgaccgttga gtggaccctg aggcctggaa ttgccatcct 110
<210> 127
   <211> 110
   <212> DNA
   <213> Homo sapiens
<400> 127
   cctgtgcaga gattattttt taaaaggtca caatcaacat tcattgctgt cggtgggttg 60
   aactgtgtgg acaagctcac tgaacaatga atgcaactgt ggccccgctt 110
<210> 128
   <211> 89
   <212> DNA
   <213> Homo sapiens
<400> 128
   ctgatggctg cactcaacat tcattgctgt cggtgggttt gagtctgaat caactcactg 60
   atcaatgaat gcaaactgcg gaccaaaca 89
<210> 129
   <211> 110
   <212> DNA
   <213> Homo sapiens
<400> 129
   gagctgcttg cctccccccg tttttggcaa tggtagaact cacactggtg aggtaacagg 60
   atccggtggt tctagacttg ccaactatgg ggcgaggact cagccggcac 110
<210> 130
   <211> 110
   <212> DNA
   <213> Homo sapiens
<400> 130
   ccgcagagtg tgactcctgt tctgtgtatg gcactggtag aattcactgt gaacagtctc 60
   agtcagtgaa ttaccgaagg gccataaaca gagcagagac agatccacga 110
<210> 131
   <211> 84
   <212> DNA
   <213> Homo sapiens
<400> 131
   ccagtcacgt ccccttatca cttttccagc ccagctttgt gactgtaagt gttggacgga 60
   gaactgataa gggtaggtga ttga 84
<210> 132
   <211> 82
   <212> DNA
   <213> Homo sapiens
<400> 132
   agggggcgag ggattggaga gaaaggcagt tcctgatggt cccctcccca ggggctggct 60
   ttcctctggt ccttccctcc ca 82
<210> 133
   <211> 86
   <212> DNA
   <213> Homo sapiens
<400> 133
   tgcttgtaac tttccaaaga attctccttt tgggctttct ggttttattt taagcccaaa 60
   ggtgaatttt ttgggaagtt tgagct 86
<210> 134
   <211> 109
   <212> DNA
   <213> Homo sapiens
<400> 134
   ggtcgggctc accatgacac agtgtgagac ctcgggctac aacacaggac ccgggcgctg 60
   ctctgacccc tcgtgtcttg tgttgcagcc ggagggacgc aggtccgca 109
<210> 135
   <211> 86
   <212> DNA
   <213> Homo sapiens
<400> 135
   tgctccctct ctcacatccc ttgcatggtg gagggtgagc tttctgaaaa cccctcccac 60
   atgcagggtt tgcaggatgg cgagcc 86
<210> 136
   <211> 85
   <212> DNA
   <213> Homo sapiens
<400> 136
   tgcaggcctc tgtgtgatat gtttgatata ttaggttgtt atttaatcca actatatatc 60
   aaacatattc ctacagtgtc ttgcc 85
<210> 137
   <211> 92
   <212> DNA
   <213> Homo sapiens
<400> 137
   cggctggaca gcgggcaacg gaatcccaaa agcagctgtt gtctccagag cattccagct 60
   gcgcttggat ttcgtcccct gctctcctgc ct 92
<210> 138
   <211> 110
   <212> DNA
   <213> Homo sapiens
<400> 138
   gccgagaccg agtgcacagg gctctgacct atgaattgac agccagtgct ctcgtctccc 60
   ctctggctgc caattccata ggtcacaggt atgttcgcct caatgccagc 110
<210> 139
   <211> 88
   <212> DNA
   <213> Homo sapiens
<400> 139
   cgaggatggg agctgagggc tgggtctttg cgggcgagat gagggtgtcg gatcaactgg 60
   cctacaaagt cccagttctc ggcccccg 88
<210> 140
   <211> 85
   <212> DNA
   <213> Homo sapiens
<400> 140
   atggtgttat caagtgtaac agcaactcca tgtggactgt gtaccaattt ccagtggaga 60
   tgctgttact tttgatggtt accaa 85
<210> 141
   <211> 85
   <212> DNA
   <213> Homo sapiens
<400> 141
   tggttcccgc cccctgtaac agcaactcca tgtggaagtg cccactggtt ccagtggggc 60
   tgctgttatc tggggcgagg gccag 85
<210> 142
   <211> 87
   <212> DNA
   <213> Homo sapiens
<400> 142
   agcttccctg gctctagcag cacagaaata ttggcacagg gaagcgagtc tgccaatatt 60
   ggctgtgctg ctccaggcag ggtggtg 87
<210> 143
   <211> 70
   <212> DNA
   <213> Homo sapiens
<400> 143
   gtgaattagg tagtttcatg ttgttgggcc tgggtttctg aacacaacaa cattaaacca 60
   cccgattcac 70
<210> 144
   <211> 110
   <212> DNA
   <213> Homo sapiens
<400> 144
   tgctcgctca gctgatctgt ggcttaggta gtttcatgtt gttgggattg agttttgaac 60
   tcggcaacaa gaaactgcct gagttacatc agtcggtttt cgtcgagggc 110
<210> 145
   <211> 84
   <212> DNA
   <213> Homo sapiens
<400> 145
   actggtcggt gatttaggta gtttcctgtt gttgggatcc acctttctct cgacagcacg 60
   acactgcctt cattacttca gttg 84
<210> 146
   <211> 75
   <212> DNA
   <213> Homo sapiens
<400> 146
   ggctgtgccg ggtagagagg gcagtgggag gtaagagctc ttcacccttc accaccttct 60
   ccacccagca tggcc 75
<210> 147
   <211> 62
   <212> DNA
   <213> Homo sapiens
<400> 147
   tcattggtcc agaggggaga taggttcctg tgatttttcc ttcttctcta tagaataaat 60
   ga 62
<210> 148
   <211> 71
   <212> DNA
   <213> Homo sapiens
<400> 148
   gccaacccag tgttcagact acctgttcag gaggctctca atgtgtacag tagtctgcac 60
   attggttagg c 71
<210> 149
   <211> 110
   <212> DNA
   <213> Homo sapiens
<400> 149
   aggaagcttc tggagatcct gctccgtcgc cccagtgttc agactacctg ttcaggacaa 60
   tgccgttgta cagtagtctg cacattggtt agactgggca agggagagca 110
<210> 150
   <211> 110
   <212> DNA
   <213> Homo sapiens
<400> 150
   ccagaggaca cctccactcc gtctacccag tgtttagact atctgttcag gactcccaaa 60
   ttgtacagta gtctgcacat tggttaggct gggctgggtt agaccctcgg 110
<210> 151
   <211> 90
   <212> DNA
   <213> Homo sapiens
<400> 151
   ccgggcccct gtgagcatct taccggacag tgctggattt cccagcttga ctctaacact 60
   gtctggtaac gatgttcaaa ggtgacccgc 90
<210> 152
   <211> 95
   <212> DNA
   <213> Homo sapiens
<400> 152
   ccagctcggg cagccgtggc catcttactg ggcagcattg gatggagtca ggtctctaat 60
   actgcctggt aatgatgacg gcggagccct gcacg 95
<210> 153
   <211> 68
   <212> DNA
   <213> Homo sapiens
<400> 153
   ccctcgtctt acccagcagt gtttgggtgc ggttgggagt ctctaatact gccgggtaat 60
   gatggagg 68
<210> 154
   <211> 110
   <212> DNA
   <213> Homo sapiens
<400> 154
   gtgttgggga ctcgcgcgct gggtccagtg gttcttaaca gttcaacagt tctgtagcgc 60
   aattgtgaaa tgtttaggac cactagaccc ggcgggcgcg gcgacagcga 110
<210> 155
   <211> 110
   <212> DNA
   <213> Homo sapiens
<400> 155
   ggctacagtc tttcttcatg tgactcgtgg acttcccttt gtcatcctat gcctgagaat 60
   atatgaagga ggctgggaag gcaaagggac gttcaattgt catcactggc 110
<210> 156
   <211> 110
   <212> DNA
   <213> Homo sapiens
<400> 156
   aaagatcctc agacaatcca tgtgcttctc ttgtccttca ttccaccgga gtctgtctca 60
   tacccaacca gatttcagtg gagtgaagtt caggaggcat ggagctgaca 110
<210> 157
   <211> 86
   <212> DNA
   <213> Homo sapiens
<400> 157
   tgcttcccga ggccacatgc ttctttatat ccccatatgg attactttgc tatggaatgt 60
   aaggaagtgt gtggtttcgg caagtg 86
<210> 158
   <211> 71
   <212> DNA
   <213> Homo sapiens
<400> 158
   tgacgggcga gcttttggcc cgggttatac ctgatgctca cgtataagac gagcaaaaag 60
   cttgttggtc a 71
<210> 159
   <211> 110
   <212> DNA
   <213> Homo sapiens
<400> 159
   acccggcagt gcctccaggc gcagggcagc ccctgcccac cgcacactgc gctgccccag 60
   acccactgtg cgtgtgacag cggctgatct gtgcctgggc agcgcgaccc 110
<210> 160
   <211> 110
   <212> DNA
   <213> Homo sapiens
<400> 160
   tcacctggcc atgtgacttg tgggcttccc tttgtcatcc ttcgcctagg gctctgagca 60
   gggcagggac agcaaagggg tgctcagttg tcacttccca cagcacggag 110
<210> 161
   <211> 110
   <212> DNA
   <213> Homo sapiens
<400> 161
   cggggcaccc cgcccggaca gcgcgccggc accttggctc tagactgctt actgcccggg 60
   ccgccctcag taacagtctc cagtcacggc caccgacgcc tggccccgcc 110
<210> 162
   <211> 110
   <212> DNA
   <213> Homo sapiens
<400> 162
   tgagttttga ggttgcttca gtgaacattc aacgctgtcg gtgagtttgg aattaaaatc 60
   aaaaccatcg accgttgatt gtaccctatg gctaaccatc atctactcca 110
<210> 163
   <211> 110
   <212> DNA
   <213> Homo sapiens
<400> 163
   ggcctggctg gacagagttg tcatgtgtct gcctgtctac acttgctgtg cagaacatcc 60
   gctcacctgt acagcaggca cagacaggca gtcacatgac aacccagcct 110
<210> 164
   <211> 110
   <212> DNA
   <213> Homo sapiens
<400> 164
   atcattcaga aatggtatac aggaaaatga cctatgaatt gacagacaat atagctgagt 60
   ttgtctgtca tttctttagg ccaatattct gtatgactgt gctacttcaa 110
<210> 165
   <211> 110
   <212> DNA
   <213> Homo sapiens
<400> 165
   gatggctgtg agttggctta atctcagctg gcaactgtga gatgttcata caatccctca 60
   cagtggtctc tgggattatg ctaaacagag caatttccta gccctcacga 110
<210> 166
   <211> 110
   <212> DNA
   <213> Homo sapiens
<400> 166
   agtataatta ttacatagtt tttgatgtcg cagatactgc atcaggaact gattggataa 60
   gaatcagtca ccatcagttc ctaatgcatt gccttcagca tctaaacaag 110
<210> 167
   <211> 110
   <212> DNA
   <213> Homo sapiens
<400> 167
   gtgataatgt agcgagattt tctgttgtgc ttgatctaac catgtggttg cgaggtatga 60
   gtaaaacatg gttccgtcaa gcaccatgga acgtcacgca gctttctaca 110
<210> 168
   <211> 110
   <212> DNA
   <213> Homo sapiens
<400> 168
   gaccagtcgc tgcggggctt tcctttgtgc ttgatctaac catgtggtgg aacgatggaa 60
   acggaacatg gttctgtcaa gcaccgcgga aagcaccgtg ctctcctgca 110
<210> 169
   <211> 110
   <212> DNA
   <213> Homo sapiens
<400> 169
   ccgccccggg ccgcggctcc tgattgtcca aacgcaattc tcgagtctat ggctccggcc 60
   gagagttgag tctggacgtc ccgagccgcc gcccccaaac ctcgagcggg 110
<210> 170
   <211> 97
   <212> DNA
   <213> Homo sapiens
<400> 170
   actcaggggc ttcgccactg attgtccaaa cgcaattctt gtacgagtct gcggccaacc 60
   gagaattgtg gctggacatc tgtggctgag ctccggg 97
<210> 171
   <211> 110
   <212> DNA
   <213> Homo sapiens
<400> 171
   gacagtgtgg cattgtaggg ctccacaccg tatctgacac tttgggcgag ggcaccatgc 60
   tgaaggtgtt catgatgcgg tctgggaact cctcacggat cttactgatg 110
<210> 172
   <211> 110
   <212> DNA
   <213> Homo sapiens
<400> 172
   tgaacatcca ggtctggggc atgaacctgg catacaatgt agatttctgt gttcgttagg 60
   caacagctac attgtctgct gggtttcagg ctacctggaa acatgttctc 110
<210> 173
   <211> 110
   <212> DNA
   <213> Homo sapiens
<400> 173
   gctgctggaa ggtgtaggta ccctcaatgg ctcagtagcc agtgtagatc ctgtctttcg 60
   taatcagcag ctacatctgg ctactgggtc tctgatggca tcttctagct 110
<210> 174
   <211> 110
   <212> DNA
   <213> Homo sapiens
<400> 174
   cctggcctcc tgcagtgcca cgctccgtgt atttgacaag ctgagttgga cactccatgt 60
   ggtagagtgt cagtttgtca aataccccaa gtgcggcaca tgcttaccag 110
<210> 175
   <211> 81
   <212> DNA
   <213> Homo sapiens
<400> 175
   gggctttcaa gtcactagtg gttccgttta gtagatgatt gtgcattgtt tcaaaatggt 60
   gccctagtga ctacaaagcc c 81
<210> 176
   <211> 80
   <212> DNA
   <213> Homo sapiens
<400> 176
   aggacccttc cagagggccc cccctcaatc ctgttgtgcc taattcagag ggttgggtgg 60
   aggctctcct gaagggctct 80
<210> 177
   <211> 63
   <212> DNA
   <213> Homo sapiens
<400> 177
   aagaaatggt ttaccgtccc acatacattt tgaatatgta tgtgggatgg taaaccgctt 60
   ctt 63
<210> 178
   <211> 86
   <212> DNA
   <213> Homo sapiens
<400> 178
   actgctaacg aatgctctga ctttattgca ctactgtact ttacagctag cagtgcaata 60
   gtattgtcaa agcatctgaa agcagg 86
<210> 179
   <211> 69
   <212> DNA
   <213> Homo sapiens
<400> 179
   ccaccactta aacgtggatg tacttgcttt gaaactaaag aagtaagtgc ttccatgttt 60
   tggtgatgg 69
<210> 180
   <211> 73
   <212> DNA
   <213> Homo sapiens
<400> 180
   gctcccttca actttaacat ggaagtgctt tctgtgactt taaaagtaag tgcttccatg 60
   ttttagtagg agt 73
<210> 181
   <211> 68
   <212> DNA
   <213> Homo sapiens
<400> 181
   cctttgcttt aacatggggg tacctgctgt gtgaaacaaa agtaagtgct tccatgtttc 60
   agtggagg 68
<210> 182
   <211> 68
   <212> DNA
   <213> Homo sapiens
<400> 182
   cctctacttt aacatggagg cacttgctgt gacatgacaa aaataagtgc ttccatgttt 60
   gagtgtgg 68
<210> 183
   <211> 82
   <212> DNA
   <213> Homo sapiens
<400> 183
   gcttcgctcc cctccgcctt ctcttcccgg ttcttcccgg agtcgggaaa agctgggttg 60
   agagggcgaa aaaggatgag gt 82
<210> 184
   <211> 59
   <212> DNA
   <213> Homo sapiens
<400> 184
   ttggcctcct aagccaggga ttgtgggttc gagtccccac cggggtaaag aaaggccga 59
<210> 185
   <211> 86
   <212> DNA
   <213> Homo sapiens
<400> 185
   ttggtacttg gagagaggtg gtccgtggcg cgttcgcttt atttatggcg cacattacac 60
   ggtcgacctc tttgcagtat ctaatc 86
<210> 186
   <211> 83
   <212> DNA
   <213> Homo sapiens
<400> 186
   ctgactatgc ctccccgcat cccctagggc attggtgtaa agctggagac ccactgcccc 60
   aggtgctgct gggggttgta gtc 83
<210> 187
   <211> 98
   <212> DNA
   <213> Homo sapiens
<400> 187
   atacagtgct tggttcctag taggtgtcca gtaagtgttt gtgacataat ttgtttattg 60
   aggacctcct atcaatcaag cactgtgcta ggctctgg 98
<210> 188
   <211> 95
   <212> DNA
   <213> Homo sapiens
<400> 188
   ctcatctgtc tgttgggctg gaggcagggc ctttgtgaag gcgggtggtg ctcagatcgc 60
   ctctgggccc ttcctccagc cccgaggcgg attca 95
<210> 189
   <211> 75
   <212> DNA
   <213> Homo sapiens
<400> 189
   tggagtgggg gggcaggagg ggctcaggga gaaagtgcat acagcccctg gccctctctg 60
   cccttccgtc ccctg 75
<210> 190
   <211> 94
   <212> DNA
   <213> Homo sapiens
<400> 190
   ctttggcgat cactgcctct ctgggcctgt gtcttaggct ctgcaagatc aaccgagcaa 60
   agcacacggc ctgcagagag gcagcgctct gccc 94
<210> 191
   <211> 94
   <212> DNA
   <213> Homo sapiens
<400> 191
   gagtttggtt ttgtttgggt ttgttctagg tatggtccca gggatcccag atcaaaccag 60
   gcccctgggc ctatcctaga accaacctaa gctc 94
<210> 192
   <211> 94
   <212> DNA
   <213> Homo sapiens
<400> 192
   tgttttgagc gggggtcaag agcaataacg aaaaatgttt gtcataaacc gtttttcatt 60
   attgctcctg acctcctctc atttgctata ttca 94
<210> 193
   <211> 93
   <212> DNA
   <213> Homo sapiens
<400> 193
   gtagtcagta gttggggggt gggaacggct tcatacagga gttgatgcac agttatccag 60
   ctcctatatg atgcctttct tcatcccctt caa 93
<210> 194
   <211> 67
   <212> DNA
   <213> Homo sapiens
<400> 194
   tctccaacaa tatcctggtg ctgagtgatg actcaggcga ctccagcatc agtgattttg 60
   ttgaaga 67
<210> 195
   <211> 94
   <212> DNA
   <213> Homo sapiens
<400> 195
   cggggcggcc gctctccctg tcctccagga gctcacgtgt gcctgcctgt gagcgcctcg 60
   acgacagagc cggcgcctgc cccagtgtct gcgc 94
<210> 196
   <211> 95
   <212> DNA
   <213> Homo sapiens
<400> 196
   ttgtacctgg tgtgattata aagcaatgag actgattgtc atatgtcgtt tgtgggatcc 60
   gtctcagtta ctttatagcc atacctggta tctta 95
<210> 197
   <211> 99
   <212> DNA
   <213> Homo sapiens
<400> 197
   gaaactgggc tcaaggtgag gggtgctatc tgtgattgag ggacatggtt aatggaattg 60
   tctcacacag aaatcgcacc cgtcaccttg gcctactta 99
<210> 198
   <211> 98
   <212> DNA
   <213> Homo sapiens
<400> 198
   acccaaaccc taggtctgct gactcctagt ccagggctcg tgatggctgg tgggccctga 60
   acgaggggtc tggaggcctg ggtttgaata tcgacagc 98
<210> 199
   <211> 86
   <212> DNA
   <213> Homo sapiens
<400> 199
   gtctgtctgc ccgcatgcct gcctctctgt tgctctgaag gaggcagggg ctgggcctgc 60
   agctgcctgg gcagagcggc tcctgc 86
<210> 200
   <211> 72
   <212> DNA
   <213> Homo sapiens
<400> 200
   ggagcttatc agaatctcca ggggtacttt ataatttcaa aaagtccccc aggtgtgatt 60
   ctgatttgct tc 72
<210> 201
   <211> 68
   <212> DNA
   <213> Homo sapiens
<400> 201
   ccattactgt tgctaatatg caactctgtt gaatataaat tggaattgca ctttagcaat 60
   ggtgatgg 68
<210> 202
   <211> 66
   <212> DNA
   <213> Homo sapiens
<400> 202
   aaaaggtgga tattccttct atgtttatgt tatttatggt taaacataga ggaaattcca 60
   cgtttt 66
<210> 203
   <211> 70
   <212> DNA
   <213> Homo sapiens
<400> 203
   ttgaagggag atcgaccgtg ttatattcgc tttattgact tcgaataata catggttgat 60
   cttttctcag 70
<210> 204
   <211> 75
   <212> DNA
   <213> Homo sapiens
<400> 204
   agacagagaa gccaggtcac gtctctgcag ttacacagct cacgagtgcc tgctggggtg 60
   gaacctggtc tgtct 75
<210> 205
   <211> 67
   <212> DNA
   <213> Homo sapiens
<400> 205
   gtggcactca aactgtgggg gcactttctg ctctctggtg aaagtgccgc catcttttga 60
   gtgttac 67
<210> 206
   <211> 67
   <212> DNA
   <213> Homo sapiens
<400> 206
   gtgggcctca aatgtggagc actattctga tgtccaagtg gaaagtgctg cgacatttga 60
   gcgtcac 67
<210> 207
   <211> 69
   <212> DNA
   <213> Homo sapiens
<400> 207
   gggatactca aaatgggggc gctttccttt ttgtctgtac tgggaagtgc ttcgattttg 60
   gggtgtccc 69
<210> 208
   <211> 72
   <212> DNA
   <213> Homo sapiens
<400> 208
   tacatcggcc attataatac aacctgataa gtgttatagc acttatcaga ttgtattgta 60
   attgtctgtg ta 72
<210> 209
   <211> 64
   <212> DNA
   <213> Homo sapiens
<400> 209
   ccccgcgacg agcccctcgc acaaaccgga cctgagcgtt ttgttcgttc ggctcgcgtg 60
   aggc 64
<210> 210
   <211> 68
   <212> DNA
   <213> Homo sapiens
<400> 210
   taaaaggtag attctccttc tatgagtaca ttatttatga ttaatcatag aggaaaatcc 60
   acgttttc 68
<210> 211
   <211> 69
   <212> DNA
   <213> Homo sapiens
<400> 211
   ttgagcagag gttgcccttg gtgaattcgc tttatttatg ttgaatcaca caaaggcaac 60
   ttttgtttg 69
<210> 212
   <211> 66
   <212> DNA
   <213> Homo sapiens
<400> 212
   agggctcctg actccaggtc ctgtgtgtta cctagaaata gcactggact tggagtcaga 60
   aggcct 66
<210> 213
   <211> 67
   <212> DNA
   <213> Homo sapiens
<400> 213
   agagatggta gactatggaa cgtaggcgtt atgatttctg acctatgtaa catggtccac 60
   taactct 67
<210> 214
   <211> 61
   <212> DNA
   <213> Homo sapiens
<400> 214
   aagatggttg accatagaac atgcgctatc tctgtgtcgt atgtaatatg gtccacatct 60
   t 61
<210> 215
   <211> 75
   <212> DNA
   <213> Homo sapiens
<400> 215
   tacttaaagc gaggttgccc tttgtatatt cggtttattg acatggaata tacaagggca 60
   agctctctgt gagta 75
<210> 216
   <211> 76
   <212> DNA
   <213> Homo sapiens
<400> 216
   tacttgaaga gaagttgttc gtggtggatt cgctttactt atgacgaatc attcacggac 60
   aacacttttt tcagta 76
<210> 217
   <211> 73
   <212> DNA
   <213> Homo sapiens
<400> 217
   ctcctcagat cagaaggtga ttgtggcttt gggtggatat taatcagcca cagcactgcc 60
   tggtcagaaa gag 73
<210> 218
   <211> 88
   <212> DNA
   <213> Homo sapiens
<400> 218
   tgttaaatca ggaattttaa acaattccta gacaatatgt ataatgttca taagtcattc 60
   ctagaaattg ttcataatgc ctgtaaca 88
<210> 219
   <211> 68
   <212> DNA
   <213> Homo sapiens
<400> 219
   cagggctcct gactccaggt cctgtgtgtt acctagaaat agcactggac ttggagtcag 60
   aaggcctg 68
<210> 220
   <211> 94
   <212> DNA
   <213> Homo sapiens
<400> 220
   ataaaggaag ttaggctgag gggcagagag cgagactttt ctattttcca aaagctcggt 60
   ctgaggcccc tcagtcttgc ttcctaaccc gcgc 94
<210> 221
   <211> 98
   <212> DNA
   <213> Homo sapiens
<400> 221
   cgaggggata cagcagcaat tcatgttttg aagtgttcta aatggttcaa aacgtgaggc 60
   gctgctatac cccctcgtgg ggaaggtaga aggtgggg 98
<210> 222
   <211> 87
   <212> DNA
   <213> Homo sapiens
<400> 222
   gaaagcgctt tggaatgaca cgatcactcc cgttgagtgg gcacccgaga agccatcggg 60
   aatgtcgtgt ccgcccagtg ctctttc 87
<210> 223
   <211> 62
   <212> RNA
   <213> Homo sapiens
<400> 223
   uaaggugcau cuagugcaga uagugaagua gauuagcauc uacugcccua agugcuccuu 60
   cu 62
<210> 224
   <211> 22
   <212> RNA
   <213> Homo sapiens
<400> 224
   uaaggugcau cuagugcaga ua 22
<210> 225
   <211> 17
   <212> DNA
   <213> Artificial Sequence
<220>
   <223> Description of Artificial Sequence: Synthetic primer
<400> 225
   ctcgcttcgg cagcaca 17
<210> 226
   <211> 23
   <212> DNA
   <213> Artificial Sequence
<220>
   <223> Description of Artificial Sequence: Synthetic primer
<400> 226
   aaacatactt ctttatatgc cca 23
<210> 227
   <211> 25
   <212> DNA
   <213> Artificial Sequence
<220>
   <223> Description of Artificial Sequence: Synthetic primer
<400> 227
   acatacttct ttatgtaccc atatg 25
<210> 228
   <211> 23
   <212> DNA
   <213> Artificial Sequence
<220>
   <223> Description of Artificial Sequence: Synthetic primer
<400> 228
   tggaagacta gtgattttgt tgt 23
<210> 229
   <211> 23
   <212> DNA
   <213> Artificial Sequence
<220>
   <223> Description of Artificial Sequence: Synthetic primer
<400> 229
   tggaagacta gtgattttgt tgt 23
<210> 230
   <211> 23
   <212> DNA
   <213> Artificial Sequence
<220>
   <223> Description of Artificial Sequence: Synthetic primer
<400> 230
   tggaagacta gtgattttgt tgt 23
<210> 231
   <211> 18
   <212> DNA
   <213> Artificial Sequence
<220>
   <223> Description of Artificial Sequence: Synthetic primer
<400> 231
   ggaggctgcg tggaagag 18
<210> 232
   <211> 19
   <212> DNA
   <213> Artificial Sequence
<220>
   <223> Description of Artificial Sequence: Synthetic primer
<400> 232
   ctggagtctg gcaagagga 19
<210> 233
   <211> 17
   <212> DNA
   <213> Artificial Sequence
<220>
   <223> Description of Artificial Sequence: Synthetic primer
<400> 233
   cagcggcact ggctaag 17
<210> 234
   <211> 23
   <212> DNA
   <213> Artificial Sequence
<220>
   <223> Description of Artificial Sequence: Synthetic primer
<400> 234
   taccctgtag atccgaattt gtg 23
<210> 235
   <211> 21
   <212> DNA
   <213> Artificial Sequence
<220>
   <223> Description of Artificial Sequence: Synthetic primer
<400> 235
   cagcacataa tggtttgtgg a 21
<210> 236
   <211> 21
   <212> DNA
   <213> Artificial Sequence
<220>
   <223> Description of Artificial Sequence: Synthetic primer
<400> 236
   agcacatcat ggtttacatg c 21
<210> 237
   <211> 21
   <212> DNA
   <213> Artificial Sequence
<220>
   <223> Description of Artificial Sequence: Synthetic primer
<400> 237
   gcagcacgta aatattggcg t 21
<210> 238
   <211> 21
   <212> DNA
   <213> Artificial Sequence
<220>
   <223> Description of Artificial Sequence: Synthetic primer
<400> 238
   gcacgtaaat attggcgtag t 21
<210> 239
   <211> 23
   <212> DNA
   <213> Artificial Sequence
<220>
   <223> Description of Artificial Sequence: Synthetic primer
<400> 239
   gcaggaaaaa agagaacatc acc 23
<210> 240
   <211> 23
   <212> DNA
   <213> Artificial Sequence
<220>
   <223> Description of Artificial Sequence: Synthetic primer
<400> 240
   taaggtgcat ctagtgcaga tag 23
<210> 241
   <211> 20
   <212> DNA
   <213> Artificial Sequence
<220>
   <223> Description of Artificial Sequence: Synthetic primer
<400> 241
   ccaataattc aagccaagca 20
<210> 242
   <211> 17
   <212> DNA
   <213> Artificial Sequence
<220>
   <223> Description of Artificial Sequence: Synthetic primer
<400> 242
   cctgtcgccc aatcaaa 17
<210> 243
   <211> 21
   <212> DNA
   <213> Artificial Sequence
<220>
   <223> Description of Artificial Sequence: Synthetic primer
<400> 243
   ggcacttcca gtactcttgg a 21
<210> 244
   <211> 23
   <212> DNA
   <213> Artificial Sequence
<220>
   <223> Description of Artificial Sequence: Synthetic primer
<400> 244
   gcactaaagt gcttatagtg cag 23
<210> 245
   <211> 23
   <212> DNA
   <213> Artificial Sequence
<220>
   <223> Description of Artificial Sequence: Synthetic primer
<400> 245
   gcttatcaga ctgatgttga ctg 23
<210> 246
   <211> 17
   <212> DNA
   <213> Artificial Sequence
<220>
   <223> Description of Artificial Sequence: Synthetic primer
<400> 246
   agcaacatgc cctgctc 17
<210> 247
   <211> 17
   <212> DNA
   <213> Artificial Sequence
<220>
   <223> Description of Artificial Sequence: Synthetic primer
<400> 247
   ctggggttcc tggggat 17
<210> 248
   <211> 19
   <212> DNA
   <213> Artificial Sequence
<220>
   <223> Description of Artificial Sequence: Synthetic primer
<400> 248
   aagcccagtg tgtgcagac 19
<210> 249
   <211> 19
   <212> DNA
   <213> Artificial Sequence
<220>
   <223> Description of Artificial Sequence: Synthetic primer
<400> 249
   ctcccgtgcc tactgagct 19
<210> 250
   <211> 18
   <212> DNA
   <213> Artificial Sequence
<220>
   <223> Description of Artificial Sequence: Synthetic primer
<400> 250
   tgagaggcgg agacttgg 18
<210> 251
   <211> 24
   <212> DNA
   <213> Artificial Sequence
<220>
   <223> Description of Artificial Sequence: Synthetic primer
<400> 251
   ttcaagtaat ccaggatagg ctgt 24
<210> 252
   <211> 24
   <212> DNA
   <213> Artificial Sequence
<220>
   <223> Description of Artificial Sequence: Synthetic primer
<400> 252
   ttcaagtaat ccaggatagg ctgt 24
<210> 253
   <211> 18
   <212> DNA
   <213> Artificial Sequence
<220>
   <223> Description of Artificial Sequence: Synthetic primer
<400> 253
   gcagggctta gctgcttg 18
<210> 254
   <211> 25
   <212> DNA
   <213> Artificial Sequence
<220>
   <223> Description of Artificial Sequence: Synthetic primer
<400> 254
   ggagctcaca gtctattgag ttacc 25
<210> 255
   <211> 20
   <212> DNA
   <213> Artificial Sequence
<220>
   <223> Description of Artificial Sequence: Synthetic primer
<400> 255
   atgactgatt tcttttggtg 20
<210> 256
   <211> 20
   <212> DNA
   <213> Artificial Sequence
<220>
   <223> Description of Artificial Sequence: Synthetic primer
<400> 256
   tggtttcata tggtggttta 20
<210> 257
   <211> 23
   <212> DNA
   <213> Artificial Sequence
<220>
   <223> Description of Artificial Sequence: Synthetic primer
<400> 257
   gtaaacatcc tcgactggaa gct 23
<210> 258
   <211> 21
   <212> DNA
   <213> Artificial Sequence
<220>
   <223> Description of Artificial Sequence: Synthetic primer
<400> 258
   aggttaaccc aacagaaggc t 21
<210> 259
   <211> 17
   <212> DNA
   <213> Artificial Sequence
<220>
   <223> Description of Artificial Sequence: Synthetic primer
<400> 259
   cctcactgcg tctccgt 17
<210> 260
   <211> 24
   <212> DNA
   <213> Artificial Sequence
<220>
   <223> Description of Artificial Sequence: Synthetic primer
<400> 260
   catgtaaaca tcctacactc agct 24
<210> 261
   <211> 19
   <212> DNA
   <213> Artificial Sequence
<220>
   <223> Description of Artificial Sequence: Synthetic primer
<400> 261
   gtgaatgctg tgcctgttc 19
<210> 262
   <211> 24
   <212> DNA
   <213> Artificial Sequence
<220>
   <223> Description of Artificial Sequence: Synthetic primer
<400> 262
   tgtgtaaaca tcctacactc tcag 24
<210> 263
   <211> 18
   <212> DNA
   <213> Artificial Sequence
<220>
   <223> Description of Artificial Sequence: Synthetic primer
<400> 263
   cagtggtcag gggctgat 18
<210> 264
   <211> 19
   <212> DNA
   <213> Artificial Sequence
<220>
   <223> Description of Artificial Sequence: Synthetic primer
<400> 264
   gactgccaac cccatccta 19
<210> 265
   <211> 21
   <212> DNA
   <213> Artificial Sequence
<220>
   <223> Description of Artificial Sequence: Synthetic primer
<400> 265
   gttgttgtaa acatccccga c 21
<210> 266
   <211> 20
   <212> DNA
   <213> Artificial Sequence
<220>
   <223> Description of Artificial Sequence: Synthetic primer
<400> 266
   gctgaagatg atgactggca 20
<210> 267
   <211> 20
   <212> DNA
   <213> Artificial Sequence
<220>
   <223> Description of Artificial Sequence: Synthetic primer
<400> 267
   tgagtgtgtt ttccctccct 20
<210> 268
   <211> 23
   <212> DNA
   <213> Artificial Sequence
<220>
   <223> Description of Artificial Sequence: Synthetic primer
<400> 268
   gcacattact aagttgcatg ttg 23
<210> 269
   <211> 20
   <212> DNA
   <213> Artificial Sequence
<220>
   <223> Description of Artificial Sequence: Synthetic primer
<400> 269
   tgtggtgcat tgtagttgca 20
<210> 270
   <211> 21
   <212> DNA
   <213> Artificial Sequence
<220>
   <223> Description of Artificial Sequence: Synthetic primer
<400> 270
   tggcagtgtc ttagctggtt g 21
<210> 271
   <211> 23
   <212> DNA
   <213> Artificial Sequence
<220>
   <223> Description of Artificial Sequence: Synthetic primer
<400> 271
   gcagtgtcat tagctgattg tac 23
<210> 272
   <211> 24
   <212> DNA
   <213> Artificial Sequence
<220>
   <223> Description of Artificial Sequence: Synthetic primer
<400> 272
   gcagtgtagt tagctgattg ctaa 24
<210> 273
   <211> 20
   <212> DNA
   <213> Artificial Sequence
<220>
   <223> Description of Artificial Sequence: Synthetic primer
<400> 273
   tctacacagg ttgggatcgg 20
<210> 274
   <211> 20
   <212> DNA
   <213> Artificial Sequence
<220>
   <223> Description of Artificial Sequence: Synthetic primer
<400> 274
   atgcgtatct ccagcactca 20
<210> 275
   <211> 19
   <212> DNA
   <213> Artificial Sequence
<220>
   <223> Description of Artificial Sequence: Synthetic primer
<400> 275
   aagtgctgtt cgtgcaggt 19
<210> 276
   <211> 23
   <212> DNA
   <213> Artificial Sequence
<220>
   <223> Description of Artificial Sequence: Synthetic primer
<400> 276
   ggcactcaat aaatgtctgt tga 23
<210> 277
   <211> 17
   <212> DNA
   <213> Artificial Sequence
<220>
   <223> Description of Artificial Sequence: Synthetic primer
<400> 277
   agagagcccg caccagt 17
<210> 278
   <211> 24
   <212> DNA
   <213> Artificial Sequence
<220>
   <223> Description of Artificial Sequence: Synthetic primer
<400> 278
   ggtagtaagt tgtattgttg tggg 24
<210> 279
   <211> 22
   <212> DNA
   <213> Artificial Sequence
<220>
   <223> Description of Artificial Sequence: Synthetic primer
<400> 279
   taaacccgta gatccgatct tg 22
<210> 280
   <211> 18
   <212> DNA
   <213> Artificial Sequence
<220>
   <223> Description of Artificial Sequence: Synthetic primer
<400> 280
   cccacccgta gaaccgac 18
<210> 281
   <211> 20
   <212> DNA
   <213> Artificial Sequence
<220>
   <223> Description of Artificial Sequence: Synthetic primer
<400> 281
   aacccgtaga tccgaacttg 20
<210> 282
   <211> 20
   <212> DNA
   <213> Artificial Sequence
<220>
   <223> Description of Artificial Sequence: Synthetic primer
<400> 282
   gccctggctc agttatcaca 20
<210> 283
   <211> 20
   <212> DNA
   <213> Artificial Sequence
<220>
   <223> Description of Artificial Sequence: Synthetic primer
<400> 283
   ttttcggtta tcatggtacc 20
<210> 284
   <211> 20
   <212> DNA
   <213> Artificial Sequence
<220>
   <223> Description of Artificial Sequence: Synthetic primer
<400> 284
   gcttctttac agtgctgcct 20
<210> 285
   <211> 22
   <212> DNA
   <213> Artificial Sequence
<220>
   <223> Description of Artificial Sequence: Synthetic primer
<400> 285
   caaatgctca gactcctgtg gt 22
<210> 286
   <211> 20
   <212> DNA
   <213> Artificial Sequence
<220>
   <223> Description of Artificial Sequence: Synthetic primer
<400> 286
   ctgcatggat ctgtgaggac 20
<210> 287
   <211> 25
   <212> DNA
   <213> Artificial Sequence
<220>
   <223> Description of Artificial Sequence: Synthetic primer
<400> 287
   taaagtgctg acagtgcaga tagtg 25
<210> 288
   <211> 22
   <212> DNA
   <213> Artificial Sequence
<220>
   <223> Description of Artificial Sequence: Synthetic primer
<400> 288
   cagcttcttt acagtgttgc ct 22
<210> 289
   <211> 22
   <212> DNA
   <213> Artificial Sequence
<220>
   <223> Description of Artificial Sequence: Synthetic primer
<400> 289
   ggatttttag gggcattatg ac 22
<210> 290
   <211> 21
   <212> DNA
   <213> Artificial Sequence
<220>
   <223> Description of Artificial Sequence: Synthetic primer
<400> 290
   ggagtgtgac aatggtgttt g 21
<210> 291
   <211> 18
   <212> DNA
   <213> Artificial Sequence
<220>
   <223> Description of Artificial Sequence: Synthetic primer
<400> 291
   tccgtgttca cagcggac 18
<210> 292
   <211> 20
   <212> DNA
   <213> Artificial Sequence
<220>
   <223> Description of Artificial Sequence: Synthetic primer
<400> 292
   gtccctgaga ccctttaacc 20
<210> 293
   <211> 20
   <212> DNA
   <213> Artificial Sequence
<220>
   <223> Description of Artificial Sequence: Synthetic primer
<400> 293
   gtccctgaga ccctaacttg 20
<210> 294
   <211> 20
   <212> DNA
   <213> Artificial Sequence
<220>
   <223> Description of Artificial Sequence: Synthetic primer
<400> 294
   gtccctgaga ccctaacttg 20
<210> 295
   <211> 21
   <212> DNA
   <213> Artificial Sequence
<220>
   <223> Description of Artificial Sequence: Synthetic primer
<400> 295
   tattactttt ggtacgcgct g 21
<210> 296
   <211> 20
   <212> DNA
   <213> Artificial Sequence
<220>
   <223> Description of Artificial Sequence: Synthetic primer
<400> 296
   agcctgctga agctcagagg 20
<210> 297
   <211> 17
   <212> DNA
   <213> Artificial Sequence
<220>
   <223> Description of Artificial Sequence: Synthetic primer
<400> 297
   tggattcggg gccgtag 17
<210> 298
   <211> 16
   <212> DNA
   <213> Artificial Sequence
<220>
   <223> Description of Artificial Sequence: Synthetic primer
<400> 298
   ggaagggggg ccgata 16
<210> 299
   <211> 18
   <212> DNA
   <213> Artificial Sequence
<220>
   <223> Description of Artificial Sequence: Synthetic primer
<400> 299
   ctttttgcgg tctgggct 18
<210> 300
   <211> 16
   <212> DNA
   <213> Artificial Sequence
<220>
   <223> Description of Artificial Sequence: Synthetic primer
<400> 300
   tgagtgggcc agggac 16
<210> 301
   <211> 22
   <212> DNA
   <213> Artificial Sequence
<220>
   <223> Description of Artificial Sequence: Synthetic primer
<400> 301
   cctgttgcac tactataggc cg 22
<210> 302
   <211> 21
   <212> DNA
   <213> Artificial Sequence
<220>
   <223> Description of Artificial Sequence: Synthetic primer
<400> 302
   aaccgtggct ttcgattgtt a 21
<210> 303
   <211> 22
   <212> DNA
   <213> Artificial Sequence
<220>
   <223> Description of Artificial Sequence: Synthetic primer
<400> 303
   gagctggtaa aatggaacca aa 22
<210> 304
   <211> 19
   <212> DNA
   <213> Artificial Sequence
<220>
   <223> Description of Artificial Sequence: Synthetic primer
<400> 304
   ctggtcaaac ggaaccaag 19
<210> 305
   <211> 20
   <212> DNA
   <213> Artificial Sequence
<220>
   <223> Description of Artificial Sequence: Synthetic primer
<400> 305
   gtgactggtt gaccagaggg 20
<210> 306
   <211> 24
   <212> DNA
   <213> Artificial Sequence
<220>
   <223> Description of Artificial Sequence: Synthetic primer
<400> 306
   ctatggcttt ttattcctat gtga 24
<210> 307
   <211> 19
   <212> DNA
   <213> Artificial Sequence
<220>
   <223> Description of Artificial Sequence: Synthetic primer
<400> 307
   gcttctcgct tccctatga 19
<210> 308
   <211> 23
   <212> DNA
   <213> Artificial Sequence
<220>
   <223> Description of Artificial Sequence: Synthetic primer
<400> 308
   ggactccatt tgttttgatg atg 23
<210> 309
   <211> 19
   <212> DNA
   <213> Artificial Sequence
<220>
   <223> Description of Artificial Sequence: Synthetic primer
<400> 309
   gtgacgggta ttcttgggt 19
<210> 310
   <211> 20
   <212> DNA
   <213> Artificial Sequence
<220>
   <223> Description of Artificial Sequence: Synthetic primer
<400> 310
   cagctggtgt tgtgaatcag 20
<210> 311
   <211> 22
   <212> DNA
   <213> Artificial Sequence
<220>
   <223> Description of Artificial Sequence: Synthetic primer
<400> 311
   ttctacagtg cacgtgtctc ca 22
<210> 312
   <211> 23
   <212> DNA
   <213> Artificial Sequence
<220>
   <223> Description of Artificial Sequence: Synthetic primer
<400> 312
   cagtggtttt accctatggt agg 23
<210> 313
   <211> 23
   <212> DNA
   <213> Artificial Sequence
<220>
   <223> Description of Artificial Sequence: Synthetic primer
<400> 313
   gtccatcttc cagtacagtg ttg 23
<210> 314
   <211> 18
   <212> DNA
   <213> Artificial Sequence
<220>
   <223> Description of Artificial Sequence: Synthetic primer
<400> 314
   ttggagcagg agtcagga 18
<210> 315
   <211> 19
   <212> DNA
   <213> Artificial Sequence
<220>
   <223> Description of Artificial Sequence: Synthetic primer
<400> 315
   tgaggtgcag tgctgcatc 19
<210> 316
   <211> 22
   <212> DNA
   <213> Artificial Sequence
<220>
   <223> Description of Artificial Sequence: Synthetic primer
<400> 316
   gctgggatat catcatatac tg 22
<210> 317
   <211> 20
   <212> DNA
   <213> Artificial Sequence
<220>
   <223> Description of Artificial Sequence: Synthetic primer
<400> 317
   ggatgcagaa gagaactcca 20
<210> 318
   <211> 20
   <212> DNA
   <213> Artificial Sequence
<220>
   <223> Description of Artificial Sequence: Synthetic primer
<400> 318
   ttgagaactg aattccatgg 20
<210> 319
   <211> 23
   <212> DNA
   <213> Artificial Sequence
<220>
   <223> Description of Artificial Sequence: Synthetic primer
<400> 319
   ctaaagacaa catttctgca cac 23
<210> 320
   <211> 21
   <212> DNA
   <213> Artificial Sequence
<220>
   <223> Description of Artificial Sequence: Synthetic primer
<400> 320
   gaggaagaca gcacgtttgg t 21
<210> 321
   <211> 22
   <212> DNA
   <213> Artificial Sequence
<220>
   <223> Description of Artificial Sequence: Synthetic primer
<400> 321
   tctgtctaag tcacccaatc tc 22
<210> 322
   <211> 19
   <212> DNA
   <213> Artificial Sequence
<220>
   <223> Description of Artificial Sequence: Synthetic primer
<400> 322
   ctggctccgt gtcttcact 19
<210> 323
   <211> 21
   <212> DNA
   <213> Artificial Sequence
<220>
   <223> Description of Artificial Sequence: Synthetic primer
<400> 323
   gtctcccaac ccttgtacca g 21
<210> 324
   <211> 21
   <212> DNA
   <213> Artificial Sequence
<220>
   <223> Description of Artificial Sequence: Synthetic primer
<400> 324
   ctcgaggagc tcacagtcta g 21
<210> 325
   <211> 21
   <212> DNA
   <213> Artificial Sequence
<220>
   <223> Description of Artificial Sequence: Synthetic primer
<400> 325
   gcccaggttc tgtgatacac t 21
<210> 326
   <211> 23
   <212> DNA
   <213> Artificial Sequence
<220>
   <223> Description of Artificial Sequence: Synthetic primer
<400> 326
   catttttgtg atctgcagct agt 23
<210> 327
   <211> 20
   <212> DNA
   <213> Artificial Sequence
<220>
   <223> Description of Artificial Sequence: Synthetic primer
<400> 327
   taggttatcc gtgttgcctt 20
<210> 328
   <211> 22
   <212> DNA
   <213> Artificial Sequence
<220>
   <223> Description of Artificial Sequence: Synthetic primer
<400> 328
   gttaatgcta atcgtgatag gg 22
<210> 329
   <211> 19
   <212> DNA
   <213> Artificial Sequence
<220>
   <223> Description of Artificial Sequence: Synthetic primer
<400> 329
   aacattcaac gctgtcggt 19
<210> 330
   <211> 19
   <212> DNA
   <213> Artificial Sequence
<220>
   <223> Description of Artificial Sequence: Synthetic primer
<400> 330
   aacattcaac gctgtcggt 19
<210> 331
   <211> 21
   <212> DNA
   <213> Artificial Sequence
<220>
   <223> Description of Artificial Sequence: Synthetic primer
<400> 331
   tttggcaatg gtagaactca c 21
<210> 332
   <211> 22
   <212> DNA
   <213> Artificial Sequence
<220>
   <223> Description of Artificial Sequence: Synthetic primer
<400> 332
   gtatggcact ggtagaattc ac 22
<210> 333
   <211> 20
   <212> DNA
   <213> Artificial Sequence
<220>
   <223> Description of Artificial Sequence: Synthetic primer
<400> 333
   cttatcactt ttccagccca 20
<210> 334
   <211> 20
   <212> DNA
   <213> Artificial Sequence
<220>
   <223> Description of Artificial Sequence: Synthetic primer
<400> 334
   ggagagaaag gcagttcctg 20
<210> 335
   <211> 20
   <212> DNA
   <213> Artificial Sequence
<220>
   <223> Description of Artificial Sequence: Synthetic primer
<400> 335
   cacccatcat attcttccca 20
<210> 336
   <211> 19
   <212> DNA
   <213> Artificial Sequence
<220>
   <223> Description of Artificial Sequence: Synthetic primer
<400> 336
   ctcgggctac aacacagga 19
<210> 337
   <211> 20
   <212> DNA
   <213> Artificial Sequence
<220>
   <223> Description of Artificial Sequence: Synthetic primer
<400> 337
   ccatatgtcg tgccaagaga 20
<210> 338
   <211> 25
   <212> DNA
   <213> Artificial Sequence
<220>
   <223> Description of Artificial Sequence: Synthetic primer
<400> 338
   gtgatatgtt tgatatatta ggttg 25
<210> 339
   <211> 18
   <212> DNA
   <213> Artificial Sequence
<220>
   <223> Description of Artificial Sequence: Synthetic primer
<400> 339
   gcaacggaat cccaaaag 18
<210> 340
   <211> 21
   <212> DNA
   <213> Artificial Sequence
<220>
   <223> Description of Artificial Sequence: Synthetic primer
<400> 340
   ctgacctatg aattgacagc c 21
<210> 341
   <211> 17
   <212> DNA
   <213> Artificial Sequence
<220>
   <223> Description of Artificial Sequence: Synthetic primer
<400> 341
   gtctttgcgg gcgagat 17
<210> 342
   <211> 20
   <212> DNA
   <213> Artificial Sequence
<220>
   <223> Description of Artificial Sequence: Synthetic primer
<400> 342
   tgtaacagca actccatgtg 20
<210> 343
   <211> 19
   <212> DNA
   <213> Artificial Sequence
<220>
   <223> Description of Artificial Sequence: Synthetic primer
<400> 343
   ggagtctttg ttgcccaca 19
<210> 344
   <211> 22
   <212> DNA
   <213> Artificial Sequence
<220>
   <223> Description of Artificial Sequence: Synthetic primer
<400> 344
   taggtagttt catgttgttg gg 22
<210> 345
   <211> 22
   <212> DNA
   <213> Artificial Sequence
<220>
   <223> Description of Artificial Sequence: Synthetic primer
<400> 345
   taggtagttt catgttgttg gg 22
<210> 346
   <211> 22
   <212> DNA
   <213> Artificial Sequence
<220>
   <223> Description of Artificial Sequence: Synthetic primer
<400> 346
   taggtagttt catgttgttg gg 22
<210> 347
   <211> 18
   <212> DNA
   <213> Artificial Sequence
<220>
   <223> Description of Artificial Sequence: Synthetic primer
<400> 347
   ctgtgccggg tagagagg 18
<210> 348
   <211> 17
   <212> DNA
   <213> Artificial Sequence
<220>
   <223> Description of Artificial Sequence: Synthetic primer
<400> 348
   ggtccagagg ggagata 17
<210> 349
   <211> 18
   <212> DNA
   <213> Artificial Sequence
<220>
   <223> Description of Artificial Sequence: Synthetic primer
<400> 349
   gtggtggttt ccttggct 18
<210> 350
   <211> 19
   <212> DNA
   <213> Artificial Sequence
<220>
   <223> Description of Artificial Sequence: Synthetic primer
<400> 350
   ggaggctttt cctgaggac 19
<210> 351
   <211> 17
   <212> DNA
   <213> Artificial Sequence
<220>
   <223> Description of Artificial Sequence: Synthetic primer
<400> 351
   caccggatgg acagaca 17
<210> 352
   <211> 17
   <212> DNA
   <213> Artificial Sequence
<220>
   <223> Description of Artificial Sequence: Synthetic primer
<400> 352
   ttccacagca gcccctg 17
<210> 353
   <211> 20
   <212> DNA
   <213> Artificial Sequence
<220>
   <223> Description of Artificial Sequence: Synthetic primer
<400> 353
   catcttactg ggcagcattg 20
<210> 354
   <211> 20
   <212> DNA
   <213> Artificial Sequence
<220>
   <223> Description of Artificial Sequence: Synthetic primer
<400> 354
   ctcgtcttac ccagcagtgt 20
<210> 355
   <211> 22
   <212> DNA
   <213> Artificial Sequence
<220>
   <223> Description of Artificial Sequence: Synthetic primer
<400> 355
   tccagtggtt cttaacagtt ca 22
<210> 356
   <211> 20
   <212> DNA
   <213> Artificial Sequence
<220>
   <223> Description of Artificial Sequence: Synthetic primer
<400> 356
   ccctttgtca tcctatgcct 20
<210> 357
   <211> 18
   <212> DNA
   <213> Artificial Sequence
<220>
   <223> Description of Artificial Sequence: Synthetic primer
<400> 357
   ccttcattcc accggagt 18
<210> 358
   <211> 21
   <212> DNA
   <213> Artificial Sequence
<220>
   <223> Description of Artificial Sequence: Synthetic primer
<400> 358
   acatgcttct ttatatcccc a 21
<210> 359
   <211> 17
   <212> DNA
   <213> Artificial Sequence
<220>
   <223> Description of Artificial Sequence: Synthetic primer
<400> 359
   agcttttggc ccgggtt 17
<210> 360
   <211> 17
   <212> DNA
   <213> Artificial Sequence
<220>
   <223> Description of Artificial Sequence: Synthetic primer
<400> 360
   cctgcccacc gcacact 17
<210> 361
   <211> 19
   <212> DNA
   <213> Artificial Sequence
<220>
   <223> Description of Artificial Sequence: Synthetic primer
<400> 361
   cctttgtcat ccttcgcct 19
<210> 362
   <211> 21
   <212> DNA
   <213> Artificial Sequence
<220>
   <223> Description of Artificial Sequence: Synthetic primer
<400> 362
   caccttggct ctagactgct t 21
<210> 363
   <211> 20
   <212> DNA
   <213> Artificial Sequence
<220>
   <223> Description of Artificial Sequence: Synthetic primer
<400> 363
   gaacattcaa cgctgtcggt 20
<210> 364
   <211> 21
   <212> DNA
   <213> Artificial Sequence
<220>
   <223> Description of Artificial Sequence: Synthetic primer
<400> 364
   tctgcctgtc tacacttgct g 21
<210> 365
   <211> 24
   <212> DNA
   <213> Artificial Sequence
<220>
   <223> Description of Artificial Sequence: Synthetic primer
<400> 365
   atgacctatg aattgacaga caat 24
<210> 366
   <211> 20
   <212> DNA
   <213> Artificial Sequence
<220>
   <223> Description of Artificial Sequence: Synthetic primer
<400> 366
   tggcttaatc tcagctggca 20
<210> 367
   <211> 23
   <212> DNA
   <213> Artificial Sequence
<220>
   <223> Description of Artificial Sequence: Synthetic primer
<400> 367
   gatactgcat caggaactga ttg 23
<210> 368
   <211> 22
   <212> DNA
   <213> Artificial Sequence
<220>
   <223> Description of Artificial Sequence: Synthetic primer
<400> 368
   gtgcttgatc taaccatgtg gt 22
<210> 369
   <211> 20
   <212> DNA
   <213> Artificial Sequence
<220>
   <223> Description of Artificial Sequence: Synthetic primer
<400> 369
   tcctgattgt ccaaacgcaa 20
<210> 370
   <211> 21
   <212> DNA
   <213> Artificial Sequence
<220>
   <223> Description of Artificial Sequence: Synthetic primer
<400> 370
   ccacaccgta tctgacactt t 21
<210> 371
   <211> 24
   <212> DNA
   <213> Artificial Sequence
<220>
   <223> Description of Artificial Sequence: Synthetic primer
<400> 371
   cctggcatac aatgtagatt tctg 24
<210> 372
   <211> 18
   <212> DNA
   <213> Artificial Sequence
<220>
   <223> Description of Artificial Sequence: Synthetic primer
<400> 372
   ccccagaagg caaaggat 18
<210> 373
   <211> 22
   <212> DNA
   <213> Artificial Sequence
<220>
   <223> Description of Artificial Sequence: Synthetic primer
<400> 373
   ccgtgtattt gacaagctga gt 22
<210> 374
   <211> 23
   <212> DNA
   <213> Artificial Sequence
<220>
   <223> Description of Artificial Sequence: Synthetic primer
<400> 374
   ggctttcaag tcactagtgg ttc 23
<210> 375
   <211> 17
   <212> DNA
   <213> Artificial Sequence
<220>
   <223> Description of Artificial Sequence: Synthetic primer
<400> 375
   ccccccctca atcctgt 17
<210> 376
   <211> 17
   <212> DNA
   <213> Artificial Sequence
<220>
   <223> Description of Artificial Sequence: Synthetic primer
<400> 376
   accaccatcg tgcggta 17
<210> 377
   <211> 23
   <212> DNA
   <213> Artificial Sequence
<220>
   <223> Description of Artificial Sequence: Synthetic primer
<400> 377
   gctctgactt tattgcacta ctg 23
<210> 378
   <211> 23
   <212> DNA
   <213> Artificial Sequence
<220>
   <223> Description of Artificial Sequence: Synthetic primer
<400> 378
   ccacttaaac gtggatgtac ttg 23
<210> 379
   <211> 23
   <212> DNA
   <213> Artificial Sequence
<220>
   <223> Description of Artificial Sequence: Synthetic primer
<400> 379
   caactttaac atggaagtgc ttt 23
<210> 380
   <211> 20
   <212> DNA
   <213> Artificial Sequence
<220>
   <223> Description of Artificial Sequence: Synthetic primer
<400> 380
   gctttaacat gggggtacct 20
<210> 381
   <211> 23
   <212> DNA
   <213> Artificial Sequence
<220>
   <223> Description of Artificial Sequence: Synthetic primer
<400> 381
   tctactttaa catggaggca ctt 23
<210> 382
   <211> 17
   <212> DNA
   <213> Artificial Sequence
<220>
   <223> Description of Artificial Sequence: Synthetic primer
<400> 382
   cgccttctct tcccggt 17
<210> 383
   <211> 19
   <212> DNA
   <213> Artificial Sequence
<220>
   <223> Description of Artificial Sequence: Synthetic primer
<400> 383
   ctaagccagg gattgtggg 19
<210> 384
   <211> 17
   <212> DNA
   <213> Artificial Sequence
<220>
   <223> Description of Artificial Sequence: Synthetic primer
<400> 384
   agaggtggtc cgtggcg 17
<210> 385
   <211> 20
   <212> DNA
   <213> Artificial Sequence
<220>
   <223> Description of Artificial Sequence: Synthetic primer
<400> 385
   cattgctgtc tctcttcgca 20
<210> 386
   <211> 23
   <212> DNA
   <213> Artificial Sequence
<220>
   <223> Description of Artificial Sequence: Synthetic primer
<400> 386
   cctagtaggt gtccagtaag tgt 23
<210> 387
   <211> 18
   <212> DNA
   <213> Artificial Sequence
<220>
   <223> Description of Artificial Sequence: Synthetic primer
<400> 387
   cgggactccc atcaagaa 18
<210> 388
   <211> 19
   <212> DNA
   <213> Artificial Sequence
<220>
   <223> Description of Artificial Sequence: Synthetic primer
<400> 388
   gaggggctca gggagaaag 19
<210> 389
   <211> 20
   <212> DNA
   <213> Artificial Sequence
<220>
   <223> Description of Artificial Sequence: Synthetic primer
<400> 389
   ctgggcctgt gtcttaggct 20
<210> 390
   <211> 19
   <212> DNA
   <213> Artificial Sequence
<220>
   <223> Description of Artificial Sequence: Synthetic primer
<400> 390
   gagctgaaag cactcccaa 19
<210> 391
   <211> 23
   <212> DNA
   <213> Artificial Sequence
<220>
   <223> Description of Artificial Sequence: Synthetic primer
<400> 391
   gtcaagagca ataacgaaaa atg 23
<210> 392
   <211> 20
   <212> DNA
   <213> Artificial Sequence
<220>
   <223> Description of Artificial Sequence: Synthetic primer
<400> 392
   acggcttcat acaggagttg 20
<210> 393
   <211> 22
   <212> DNA
   <213> Artificial Sequence
<220>
   <223> Description of Artificial Sequence: Synthetic primer
<400> 393
   ccaacaatat cctggtgctg ag 22
<210> 394
   <211> 19
   <212> DNA
   <213> Artificial Sequence
<220>
   <223> Description of Artificial Sequence: Synthetic primer
<400> 394
   tccctgtcct ccaggagct 19
<210> 395
   <211> 23
   <212> DNA
   <213> Artificial Sequence
<220>
   <223> Description of Artificial Sequence: Synthetic primer
<400> 395
   ataaagcaat gagactgatt gtc 23
<210> 396
   <211> 21
   <212> DNA
   <213> Artificial Sequence
<220>
   <223> Description of Artificial Sequence: Synthetic primer
<400> 396
   gtgctatctg tgattgaggg a 21
<210> 397
   <211> 20
   <212> DNA
   <213> Artificial Sequence
<220>
   <223> Description of Artificial Sequence: Synthetic primer
<400> 397
   ctgactccta gtccagggct 20
<210> 398
   <211> 20
   <212> DNA
   <213> Artificial Sequence
<220>
   <223> Description of Artificial Sequence: Synthetic primer
<400> 398
   gcatgcctgc ctctctgttg 20
<210> 399
   <211> 23
   <212> DNA
   <213> Artificial Sequence
<220>
   <223> Description of Artificial Sequence: Synthetic primer
<400> 399
   cttatcagaa tctccagggg tac 23
<210> 400
   <211> 22
   <212> DNA
   <213> Artificial Sequence
<220>
   <223> Description of Artificial Sequence: Synthetic primer
<400> 400
   ctgttgctaa tatgcaactc tg 22
<210> 401
   <211> 25
   <212> DNA
   <213> Artificial Sequence
<220>
   <223> Description of Artificial Sequence: Synthetic primer
<400> 401
   ggtggatatt ccttctatgt ttatg 25
<210> 402
   <211> 22
   <212> DNA
   <213> Artificial Sequence
<220>
   <223> Description of Artificial Sequence: Synthetic primer
<400> 402
   ggagatcgac cgtgttatat tc 22
<210> 403
   <211> 21
   <212> DNA
   <213> Artificial Sequence
<220>
   <223> Description of Artificial Sequence: Synthetic primer
<400> 403
   ggtcacgtct ctgcagttac a 21
<210> 404
   <211> 19
   <212> DNA
   <213> Artificial Sequence
<220>
   <223> Description of Artificial Sequence: Synthetic primer
<400> 404
   tcaaactgtg ggggcactt 19
<210> 405
   <211> 22
   <212> DNA
   <213> Artificial Sequence
<220>
   <223> Description of Artificial Sequence: Synthetic primer
<400> 405
   gcctcaaatg tggagcacta tt 22
<210> 406
   <211> 18
   <212> DNA
   <213> Artificial Sequence
<220>
   <223> Description of Artificial Sequence: Synthetic primer
<400> 406
   ctcaaaatgg gggcgctt 18
<210> 407
   <211> 20
   <212> DNA
   <213> Artificial Sequence
<220>
   <223> Description of Artificial Sequence: Synthetic primer
<400> 407
   gccctcaagg agctcacagt 20
<210> 408
   <211> 16
   <212> DNA
   <213> Artificial Sequence
<220>
   <223> Description of Artificial Sequence: Synthetic primer
<400> 408
   acgagcccct cgcaca 16
<210> 409
   <211> 24
   <212> DNA
   <213> Artificial Sequence
<220>
   <223> Description of Artificial Sequence: Synthetic primer
<400> 409
   ggtagattct ccttctatga gtac 24
<210> 410
   <211> 18
   <212> DNA
   <213> Artificial Sequence
<220>
   <223> Description of Artificial Sequence: Synthetic primer
<400> 410
   gagcagaggt tgcccttg 18
<210> 411
   <211> 20
   <212> DNA
   <213> Artificial Sequence
<220>
   <223> Description of Artificial Sequence: Synthetic primer
<400> 411
   ctcctgactc caggtcctgt 20
<210> 412
   <211> 23
   <212> DNA
   <213> Artificial Sequence
<220>
   <223> Description of Artificial Sequence: Synthetic primer
<400> 412
   agagatggta gactatggaa cgt 23
<210> 413
   <211> 21
   <212> DNA
   <213> Artificial Sequence
<220>
   <223> Description of Artificial Sequence: Synthetic primer
<400> 413
   gatggttgac catagaacat g 21
<210> 414
   <211> 17
   <212> DNA
   <213> Artificial Sequence
<220>
   <223> Description of Artificial Sequence: Synthetic primer
<400> 414
   agcgaggttg ccctttg 17
<210> 415
   <211> 21
   <212> DNA
   <213> Artificial Sequence
<220>
   <223> Description of Artificial Sequence: Synthetic primer
<400> 415
   gaagttgttc gtggtggatt c 21
<210> 416
   <211> 23
   <212> DNA
   <213> Artificial Sequence
<220>
   <223> Description of Artificial Sequence: Synthetic primer
<400> 416
   cagatcagaa ggtgattgtg gct 23
<210> 417
   <211> 25
   <212> DNA
   <213> Artificial Sequence
<220>
   <223> Description of Artificial Sequence: Synthetic primer
<400> 417
   caattcctag acaatatgta taatg 25
<210> 418
   <211> 19
   <212> DNA
   <213> Artificial Sequence
<220>
   <223> Description of Artificial Sequence: Synthetic primer
<400> 418
   tcctgactcc aggtcctgt 19
<210> 419
   <211> 17
   <212> DNA
   <213> Artificial Sequence
<220>
   <223> Description of Artificial Sequence: Synthetic primer
<400> 419
   tgaggggcag agagcga 17
<210> 420
   <211> 22
   <212> DNA
   <213> Artificial Sequence
<220>
   <223> Description of Artificial Sequence: Synthetic primer
<400> 420
   agcagcaatt catgttttga ag 22
<210> 421
   <211> 21
   <212> DNA
   <213> Artificial Sequence
<220>
   <223> Description of Artificial Sequence: Synthetic primer
<400> 421
   atgacacgat cactcccgtt g 21
<210> 422
   <211> 25
   <212> DNA
   <213> Artificial Sequence
<220>
   <223> Description of Artificial Sequence: Synthetic primer
<400> 422
   aggtagtagg ttgtatagtt ttagg 25
<210> 423
   <211> 20
   <212> DNA
   <213> Artificial Sequence
<220>
   <223> Description of Artificial Sequence: Synthetic primer
<400> 423
   cctggatgtt ctcttcactg 20
<210> 424
   <211> 26
   <212> DNA
   <213> Artificial Sequence
<220>
   <223> Description of Artificial Sequence: Synthetic primer
<400> 424
   gaggtagtag gttgtatagt ttagaa 26
<210> 425
   <211> 20
   <212> DNA
   <213> Artificial Sequence
<220>
   <223> Description of Artificial Sequence: Synthetic primer
<400> 425
   ttccagccat tgtgactgca 20
<210> 426
   <211> 24
   <212> DNA
   <213> Artificial Sequence
<220>
   <223> Description of Artificial Sequence: Synthetic primer
<400> 426
   gaggtagtag gttgtatagt ttgg 24
<210> 427
   <211> 20
   <212> DNA
   <213> Artificial Sequence
<220>
   <223> Description of Artificial Sequence: Synthetic primer
<400> 427
   accaagaccg actgcccttt 20
<210> 428
   <211> 21
   <212> DNA
   <213> Artificial Sequence
<220>
   <223> Description of Artificial Sequence: Synthetic primer
<400> 428
   tgaggtagta ggttgtgtgg t 21
<210> 429
   <211> 16
   <212> DNA
   <213> Artificial Sequence
<220>
   <223> Description of Artificial Sequence: Synthetic primer
<400> 429
   cctcccgcag tgcaag 16
<210> 430
   <211> 23
   <212> DNA
   <213> Artificial Sequence
<220>
   <223> Description of Artificial Sequence: Synthetic primer
<400> 430
   ttgaggtagt aggttgtatg gtt 23
<210> 431
   <211> 20
   <212> DNA
   <213> Artificial Sequence
<220>
   <223> Description of Artificial Sequence: Synthetic primer
<400> 431
   ttggaggagc tgactgaaga 20
<210> 432
   <211> 21
   <212> DNA
   <213> Artificial Sequence
<220>
   <223> Description of Artificial Sequence: Synthetic primer
<400> 432
   aggttgcata gttttagggc a 21
<210> 433
   <211> 21
   <212> DNA
   <213> Artificial Sequence
<220>
   <223> Description of Artificial Sequence: Synthetic primer
<400> 433
   gccaagtaga agaccagcaa g 21
<210> 434
   <211> 24
   <212> DNA
   <213> Artificial Sequence
<220>
   <223> Description of Artificial Sequence: Synthetic primer
<400> 434
   gaggtaggag gttgtatagt tgag 24
<210> 435
   <211> 17
   <212> DNA
   <213> Artificial Sequence
<220>
   <223> Description of Artificial Sequence: Synthetic primer
<400> 435
   ctgtctgtct gtctgtc 17
<210> 436
   <211> 23
   <212> DNA
   <213> Artificial Sequence
<220>
   <223> Description of Artificial Sequence: Synthetic primer
<400> 436
   gattgtatag ttgtggggta gtg 23
<210> 437
   <211> 20
   <212> DNA
   <213> Artificial Sequence
<220>
   <223> Description of Artificial Sequence: Synthetic primer
<400> 437
   tgtactttcc attccagaag 20
<210> 438
   <211> 25
   <212> DNA
   <213> Artificial Sequence
<220>
   <223> Description of Artificial Sequence: Synthetic primer
<400> 438
   ggtagtagat tgtatagttt taggg 25
<210> 439
   <211> 20
   <212> DNA
   <213> Artificial Sequence
<220>
   <223> Description of Artificial Sequence: Synthetic primer
<400> 439
   tgaagatgga cactggtgct 20
<210> 440
   <211> 25
   <212> DNA
   <213> Artificial Sequence
<220>
   <223> Description of Artificial Sequence: Synthetic primer
<400> 440
   gtagtagttt gtacagtttg agggt 25
<210> 441
   <211> 18
   <212> DNA
   <213> Artificial Sequence
<220>
   <223> Description of Artificial Sequence: Synthetic primer
<400> 441
   agcgctccgt ttcctttt 18
<210> 442
   <211> 17
   <212> DNA
   <213> Artificial Sequence
<220>
   <223> Description of Artificial Sequence: Synthetic primer
<400> 442
   tgtgctgttg gtcgggt 17
<210> 443
   <211> 17
   <212> DNA
   <213> Artificial Sequence
<220>
   <223> Description of Artificial Sequence: Synthetic primer
<400> 443
   cgaggaagga cggagga 17
<210> 444
   <211> 20
   <212> DNA
   <213> Artificial Sequence
<220>
   <223> Description of Artificial Sequence: Synthetic primer
<400> 444
   aacgcttcac gaatttgcgt 20
<210> 445
   <211> 25
   <212> DNA
   <213> Artificial Sequence
<220>
   <223> Description of Artificial Sequence: Synthetic primer
<400> 445
   tacatacttc tttacattcc atagc 25
<210> 446
   <211> 25
   <212> DNA
   <213> Artificial Sequence
<220>
   <223> Description of Artificial Sequence: Synthetic primer
<400> 446
   tacatacttc tttacattcc atagc 25
<210> 447
   <211> 22
   <212> DNA
   <213> Artificial Sequence
<220>
   <223> Description of Artificial Sequence: Synthetic primer
<400> 447
   agactgtgat ttgttgtcga tt 22
<210> 448
   <211> 21
   <212> DNA
   <213> Artificial Sequence
<220>
   <223> Description of Artificial Sequence: Synthetic primer
<400> 448
   agactgggat ttgttgttga g 21
<210> 449
   <211> 20
   <212> DNA
   <213> Artificial Sequence
<220>
   <223> Description of Artificial Sequence: Synthetic primer
<400> 449
   ggctgtgact tgttgtcgta 20
<210> 450
   <211> 16
   <212> DNA
   <213> Artificial Sequence
<220>
   <223> Description of Artificial Sequence: Synthetic primer
<400> 450
   cgtgaggccg gctttc 16
<210> 451
   <211> 22
   <212> DNA
   <213> Artificial Sequence
<220>
   <223> Description of Artificial Sequence: Synthetic primer
<400> 451
   agtctttcat tctcacacgc tc 22
<210> 452
   <211> 17
   <212> DNA
   <213> Artificial Sequence
<220>
   <223> Description of Artificial Sequence: Synthetic primer
<400> 452
   gctcgcacgc agaagtt 17
<210> 453
   <211> 24
   <212> DNA
   <213> Artificial Sequence
<220>
   <223> Description of Artificial Sequence: Synthetic primer
<400> 453
   attcccctag atacgaattt gtga 24
<210> 454
   <211> 18
   <212> DNA
   <213> Artificial Sequence
<220>
   <223> Description of Artificial Sequence: Synthetic primer
<400> 454
   gcagcacaat atggcctg 18
<210> 455
   <211> 23
   <212> DNA
   <213> Artificial Sequence
<220>
   <223> Description of Artificial Sequence: Synthetic primer
<400> 455
   ctagagcagc aaataatgat tcg 23
<210> 456
   <211> 24
   <212> DNA
   <213> Artificial Sequence
<220>
   <223> Description of Artificial Sequence: Synthetic primer
<400> 456
   cagcagcaca gttaatactg gaga 24
<210> 457
   <211> 22
   <212> DNA
   <213> Artificial Sequence
<220>
   <223> Description of Artificial Sequence: Synthetic primer
<400> 457
   aagcagcaca gtaatattgg tg 22
<210> 458
   <211> 16
   <212> DNA
   <213> Artificial Sequence
<220>
   <223> Description of Artificial Sequence: Synthetic primer
<400> 458
   tggcttcccg aggcag 16
<210> 459
   <211> 20
   <212> DNA
   <213> Artificial Sequence
<220>
   <223> Description of Artificial Sequence: Synthetic primer
<400> 459
   gaaggagcac ttagggcagt 20
<210> 460
   <211> 21
   <212> DNA
   <213> Artificial Sequence
<220>
   <223> Description of Artificial Sequence: Synthetic primer
<400> 460
   caggcagatt ctacatcgac a 21
<210> 461
   <211> 21
   <212> DNA
   <213> Artificial Sequence
<220>
   <223> Description of Artificial Sequence: Synthetic primer
<400> 461
   caacctgtgt agaaaggggt t 21
<210> 462
   <211> 22
   <212> DNA
   <213> Artificial Sequence
<220>
   <223> Description of Artificial Sequence: Synthetic primer
<400> 462
   gtgtgttcac acagacgtag ga 22
<210> 463
   <211> 23
   <212> DNA
   <213> Artificial Sequence
<220>
   <223> Description of Artificial Sequence: Synthetic primer
<400> 463
   gtactttaag tgctcataat gca 23
<210> 464
   <211> 17
   <212> DNA
   <213> Artificial Sequence
<220>
   <223> Description of Artificial Sequence: Synthetic primer
<400> 464
   cagcccatcg actggtg 17
<210> 465
   <211> 21
   <212> DNA
   <213> Artificial Sequence
<220>
   <223> Description of Artificial Sequence: Synthetic primer
<400> 465
   tctgtcacct tccagatgat g 21
<210> 466
   <211> 20
   <212> DNA
   <213> Artificial Sequence
<220>
   <223> Description of Artificial Sequence: Synthetic primer
<400> 466
   tggtaatccc tggcaatgtg 20
<210> 467
   <211> 18
   <212> DNA
   <213> Artificial Sequence
<220>
   <223> Description of Artificial Sequence: Synthetic primer
<400> 467
   accacggttt ctggagga 18
<210> 468
   <211> 21
   <212> DNA
   <213> Artificial Sequence
<220>
   <223> Description of Artificial Sequence: Synthetic primer
<400> 468
   ccctgttcct gctgaactga g 21
<210> 469
   <211> 20
   <212> DNA
   <213> Artificial Sequence
<220>
   <223> Description of Artificial Sequence: Synthetic primer
<400> 469
   tcagaccgag acaagtgcaa 20
<210> 470
   <211> 22
   <212> DNA
   <213> Artificial Sequence
<220>
   <223> Description of Artificial Sequence: Synthetic primer
<400> 470
   tgcaagtaac caagaatagg cc 22
<210> 471
   <211> 21
   <212> DNA
   <213> Artificial Sequence
<220>
   <223> Description of Artificial Sequence: Synthetic primer
<400> 471
   caagtaatgg agaacaggct g 21
<210> 472
   <211> 19
   <212> DNA
   <213> Artificial Sequence
<220>
   <223> Description of Artificial Sequence: Synthetic primer
<400> 472
   ggcggaactt agccactgt 19
<210> 473
   <211> 20
   <212> DNA
   <213> Artificial Sequence
<220>
   <223> Description of Artificial Sequence: Synthetic primer
<400> 473
   cctccaggag ctcacaatct 20
<210> 474
   <211> 20
   <212> DNA
   <213> Artificial Sequence
<220>
   <223> Description of Artificial Sequence: Synthetic primer
<400> 474
   ataaccgatt tcagatggtg 20
<210> 475
   <211> 20
   <212> DNA
   <213> Artificial Sequence
<220>
   <223> Description of Artificial Sequence: Synthetic primer
<400> 475
   ataaccgatt tcagatggtg 20
<210> 476
   <211> 20
   <212> DNA
   <213> Artificial Sequence
<220>
   <223> Description of Artificial Sequence: Synthetic primer
<400> 476
   gctgcaaaca tccgactgaa 20
<210> 477
   <211> 18
   <212> DNA
   <213> Artificial Sequence
<220>
   <223> Description of Artificial Sequence: Synthetic primer
<400> 477
   ccttgaagtc cgaggcag 18
<210> 478
   <211> 17
   <212> DNA
   <213> Artificial Sequence
<220>
   <223> Description of Artificial Sequence: Synthetic primer
<400> 478
   cctgtgggca caaacct 17
<210> 479
   <211> 18
   <212> DNA
   <213> Artificial Sequence
<220>
   <223> Description of Artificial Sequence: Synthetic primer
<400> 479
   atccacctcc cagccaat 18
<210> 480
   <211> 23
   <212> DNA
   <213> Artificial Sequence
<220>
   <223> Description of Artificial Sequence: Synthetic primer
<400> 480
   gcctctgtat actattcttg cca 23
<210> 481
   <211> 20
   <212> DNA
   <213> Artificial Sequence
<220>
   <223> Description of Artificial Sequence: Synthetic primer
<400> 481
   gagtaaacaa ccctctccca 20
<210> 482
   <211> 21
   <212> DNA
   <213> Artificial Sequence
<220>
   <223> Description of Artificial Sequence: Synthetic primer
<400> 482
   ggagtggaga ctgttccttc t 21
<210> 483
   <211> 20
   <212> DNA
   <213> Artificial Sequence
<220>
   <223> Description of Artificial Sequence: Synthetic primer
<400> 483
   cctcagaaac aaacacggga 20
<210> 484
   <211> 22
   <212> DNA
   <213> Artificial Sequence
<220>
   <223> Description of Artificial Sequence: Synthetic primer
<400> 484
   gcagcaaaca tctgactgaa ag 22
<210> 485
   <211> 18
   <212> DNA
   <213> Artificial Sequence
<220>
   <223> Description of Artificial Sequence: Synthetic primer
<400> 485
   ctccactccg ggacagaa 18
<210> 486
   <211> 17
   <212> DNA
   <213> Artificial Sequence
<220>
   <223> Description of Artificial Sequence: Synthetic primer
<400> 486
   gccatggctg ctgtcag 17
<210> 487
   <211> 24
   <212> DNA
   <213> Artificial Sequence
<220>
   <223> Description of Artificial Sequence: Synthetic primer
<400> 487
   tatcacacac actaaattgc attg 24
<210> 488
   <211> 20
   <212> DNA
   <213> Artificial Sequence
<220>
   <223> Description of Artificial Sequence: Synthetic primer
<400> 488
   ctgtgatgca ctgtggaaac 20
<210> 489
   <211> 23
   <212> DNA
   <213> Artificial Sequence
<220>
   <223> Description of Artificial Sequence: Synthetic primer
<400> 489
   ggcagtatac ttgctgattg ctt 23
<210> 490
   <211> 21
   <212> DNA
   <213> Artificial Sequence
<220>
   <223> Description of Artificial Sequence: Synthetic primer
<400> 490
   gatggcagtg gagttagtga t 21
<210> 491
   <211> 18
   <212> DNA
   <213> Artificial Sequence
<220>
   <223> Description of Artificial Sequence: Synthetic primer
<400> 491
   cctggccgtg tggttagt 18
<210> 492
   <211> 21
   <212> DNA
   <213> Artificial Sequence
<220>
   <223> Description of Artificial Sequence: Synthetic primer
<400> 492
   cgggacaagt gcaataccat a 21
<210> 493
   <211> 17
   <212> DNA
   <213> Artificial Sequence
<220>
   <223> Description of Artificial Sequence: Synthetic primer
<400> 493
   ccacccgaca acagcaa 17
<210> 494
   <211> 19
   <212> DNA
   <213> Artificial Sequence
<220>
   <223> Description of Artificial Sequence: Synthetic primer
<400> 494
   ctcgggaagt gctagctca 19
<210> 495
   <211> 21
   <212> DNA
   <213> Artificial Sequence
<220>
   <223> Description of Artificial Sequence: Synthetic primer
<400> 495
   tgctcaataa atacccgttg a 21
<210> 496
   <211> 18
   <212> DNA
   <213> Artificial Sequence
<220>
   <223> Description of Artificial Sequence: Synthetic primer
<400> 496
   cttgaggagg agcaggct 18
<210> 497
   <211> 24
   <212> DNA
   <213> Artificial Sequence
<220>
   <223> Description of Artificial Sequence: Synthetic primer
<400> 497
   tatagttatc ttctaattgg ggcc 24
<210> 498
   <211> 19
   <212> DNA
   <213> Artificial Sequence
<220>
   <223> Description of Artificial Sequence: Synthetic primer
<400> 498
   ccacagacac gagcttgtg 19
<210> 499
   <211> 20
   <212> DNA
   <213> Artificial Sequence
<220>
   <223> Description of Artificial Sequence: Synthetic primer
<400> 499
   ccacagacac gagcttgtgt 20
<210> 500
   <211> 24
   <212> DNA
   <213> Artificial Sequence
<220>
   <223> Description of Artificial Sequence: Synthetic primer
<400> 500
   tacctataga tacaagcttg tgcg 24
<210> 501
   <211> 23
   <212> DNA
   <213> Artificial Sequence
<220>
   <223> Description of Artificial Sequence: Synthetic primer
<400> 501
   gccatccttc agttatcaca gta 23
<210> 502
   <211> 25
   <212> DNA
   <213> Artificial Sequence
<220>
   <223> Description of Artificial Sequence: Synthetic primer
<400> 502
   ccttcagtta tcacagtact gtacc 25
<210> 503
   <211> 21
   <212> DNA
   <213> Artificial Sequence
<220>
   <223> Description of Artificial Sequence: Synthetic primer
<400> 503
   ttcatagccc tgtacaatgc t 21
<210> 504
   <211> 20
   <212> DNA
   <213> Artificial Sequence
<220>
   <223> Description of Artificial Sequence: Synthetic primer
<400> 504
   gcacatgctc aaacatccgt 20
<210> 505
   <211> 20
   <212> DNA
   <213> Artificial Sequence
<220>
   <223> Description of Artificial Sequence: Synthetic primer
<400> 505
   agcagctcaa aagcatcaac 20
<210> 506
   <211> 19
   <212> DNA
   <213> Artificial Sequence
<220>
   <223> Description of Artificial Sequence: Synthetic primer
<400> 506
   caagtaccca cagtgcggt 19
<210> 507
   <211> 21
   <212> DNA
   <213> Artificial Sequence
<220>
   <223> Description of Artificial Sequence: Synthetic primer
<400> 507
   gatagccctg tacaatgctg c 21
<210> 508
   <211> 18
   <212> DNA
   <213> Artificial Sequence
<220>
   <223> Description of Artificial Sequence: Synthetic primer
<400> 508
   gagggacttt caggggca 18
<210> 509
   <211> 22
   <212> DNA
   <213> Artificial Sequence
<220>
   <223> Description of Artificial Sequence: Synthetic primer
<400> 509
   tttagtgtga taatggcgtt tg 22
<210> 510
   <211> 18
   <212> DNA
   <213> Artificial Sequence
<220>
   <223> Description of Artificial Sequence: Synthetic primer
<400> 510
   cattcaccgc gtgcctta 18
<210> 511
   <211> 19
   <212> DNA
   <213> Artificial Sequence
<220>
   <223> Description of Artificial Sequence: Synthetic primer
<400> 511
   aacctcacct gtgaccctg 19
<210> 512
   <211> 18
   <212> DNA
   <213> Artificial Sequence
<220>
   <223> Description of Artificial Sequence: Synthetic primer
<400> 512
   agcctaaccc gtggattt 18
<210> 513
   <211> 20
   <212> DNA
   <213> Artificial Sequence
<220>
   <223> Description of Artificial Sequence: Synthetic primer
<400> 513
   aagagcctga cttgtgatgt 20
<210> 514
   <211> 21
   <212> DNA
   <213> Artificial Sequence
<220>
   <223> Description of Artificial Sequence: Synthetic primer
<400> 514
   gcgcattatt actcacggta c 21
<210> 515
   <211> 19
   <212> DNA
   <213> Artificial Sequence
<220>
   <223> Description of Artificial Sequence: Synthetic primer
<400> 515
   gccaagctca gacggatcc 19
<210> 516
   <211> 22
   <212> DNA
   <213> Artificial Sequence
<220>
   <223> Description of Artificial Sequence: Synthetic primer
<400> 516
   aaagagaccg gttcactgtg ag 22
<210> 517
   <211> 22
   <212> DNA
   <213> Artificial Sequence
<220>
   <223> Description of Artificial Sequence: Synthetic primer
<400> 517
   aaagagaccg gttcactgtg ag 22
<210> 518
   <211> 19
   <212> DNA
   <213> Artificial Sequence
<220>
   <223> Description of Artificial Sequence: Synthetic primer
<400> 518
   gctttttggg gtaagggct 19
<210> 519
   <211> 17
   <212> DNA
   <213> Artificial Sequence
<220>
   <223> Description of Artificial Sequence: Synthetic primer
<400> 519
   gcaatgctga ggaggca 17
<210> 520
   <211> 21
   <212> DNA
   <213> Artificial Sequence
<220>
   <223> Description of Artificial Sequence: Synthetic primer
<400> 520
   tgccctttta acattgcact g 21
<210> 521
   <211> 23
   <212> DNA
   <213> Artificial Sequence
<220>
   <223> Description of Artificial Sequence: Synthetic primer
<400> 521
   cgaccatggc tgtagactgt tac 23
<210> 522
   <211> 19
   <212> DNA
   <213> Artificial Sequence
<220>
   <223> Description of Artificial Sequence: Synthetic primer
<400> 522
   acagctggtt gaaggggac 19
<210> 523
   <211> 19
   <212> DNA
   <213> Artificial Sequence
<220>
   <223> Description of Artificial Sequence: Synthetic primer
<400> 523
   acagctggtt gaaggggac 19
<210> 524
   <211> 19
   <212> DNA
   <213> Artificial Sequence
<220>
   <223> Description of Artificial Sequence: Synthetic primer
<400> 524
   ggtgactagg tggcccaca 19
<210> 525
   <211> 17
   <212> DNA
   <213> Artificial Sequence
<220>
   <223> Description of Artificial Sequence: Synthetic primer
<400> 525
   cacggctcca atcccta 17
<210> 526
   <211> 16
   <212> DNA
   <213> Artificial Sequence
<220>
   <223> Description of Artificial Sequence: Synthetic primer
<400> 526
   tccgaacctg gtccca 16
<210> 527
   <211> 23
   <212> DNA
   <213> Artificial Sequence
<220>
   <223> Description of Artificial Sequence: Synthetic primer
<400> 527
   agactcattt gagacgatga tgg 23
<210> 528
   <211> 22
   <212> DNA
   <213> Artificial Sequence
<220>
   <223> Description of Artificial Sequence: Synthetic primer
<400> 528
   gactacgcgt attcttaagc aa 22
<210> 529
   <211> 20
   <212> DNA
   <213> Artificial Sequence
<220>
   <223> Description of Artificial Sequence: Synthetic primer
<400> 529
   accctggtgt cgtgaaatag 20
<210> 530
   <211> 17
   <212> DNA
   <213> Artificial Sequence
<220>
   <223> Description of Artificial Sequence: Synthetic primer
<400> 530
   tactccaaca gggccgc 17
<210> 531
   <211> 21
   <212> DNA
   <213> Artificial Sequence
<220>
   <223> Description of Artificial Sequence: Synthetic primer
<400> 531
   cgtggttcta ccctgtggta g 21
<210> 532
   <211> 22
   <212> DNA
   <213> Artificial Sequence
<220>
   <223> Description of Artificial Sequence: Synthetic primer
<400> 532
   agccatcttt accagacagt gt 22
<210> 533
   <211> 16
   <212> DNA
   <213> Artificial Sequence
<220>
   <223> Description of Artificial Sequence: Synthetic primer
<400> 533
   cgccgaggaa gatggt 16
<210> 534
   <211> 24
   <212> DNA
   <213> Artificial Sequence
<220>
   <223> Description of Artificial Sequence: Synthetic primer
<400> 534
   gctacagtgc ttcatctcag actc 24
<210> 535
   <211> 24
   <212> DNA
   <213> Artificial Sequence
<220>
   <223> Description of Artificial Sequence: Synthetic primer
<400> 535
   cggactagta catcatctat actg 24
<210> 536
   <211> 18
   <212> DNA
   <213> Artificial Sequence
<220>
   <223> Description of Artificial Sequence: Synthetic primer
<400> 536
   cctcatcctg tgagccag 18
<210> 537
   <211> 22
   <212> DNA
   <213> Artificial Sequence
<220>
   <223> Description of Artificial Sequence: Synthetic primer
<400> 537
   gctgaagaac tgaatttcag ag 22
<210> 538
   <211> 21
   <212> DNA
   <213> Artificial Sequence
<220>
   <223> Description of Artificial Sequence: Synthetic primer
<400> 538
   atctagcaga agcatttcca c 21
<210> 539
   <211> 16
   <212> DNA
   <213> Artificial Sequence
<220>
   <223> Description of Artificial Sequence: Synthetic primer
<400> 539
   aaaggcgcag cgacgt 16
<210> 540
   <211> 20
   <212> DNA
   <213> Artificial Sequence
<220>
   <223> Description of Artificial Sequence: Synthetic primer
<400> 540
   tctattcttc cctcccactc 20
<210> 541
   <211> 16
   <212> DNA
   <213> Artificial Sequence
<220>
   <223> Description of Artificial Sequence: Synthetic primer
<400> 541
   agcacagccc ccgtcc 16
<210> 542
   <211> 18
   <212> DNA
   <213> Artificial Sequence
<220>
   <223> Description of Artificial Sequence: Synthetic primer
<400> 542
   tgtcccccag gcctgtac 18
<210> 543
   <211> 20
   <212> DNA
   <213> Artificial Sequence
<220>
   <223> Description of Artificial Sequence: Synthetic primer
<400> 543
   gtcctcaagg agcttcagtc 20
<210> 544
   <211> 19
   <212> DNA
   <213> Artificial Sequence
<220>
   <223> Description of Artificial Sequence: Synthetic primer
<400> 544
   cccaagttct gtcatgcac 19
<210> 545
   <211> 23
   <212> DNA
   <213> Artificial Sequence
<220>
   <223> Description of Artificial Sequence: Synthetic primer
<400> 545
   tcacttttgt gactatgcaa ctg 23
<210> 546
   <211> 23
   <212> DNA
   <213> Artificial Sequence
<220>
   <223> Description of Artificial Sequence: Synthetic primer
<400> 546
   aataggtcaa ccgtgtatga ttc 23
<210> 547
   <211> 24
   <212> DNA
   <213> Artificial Sequence
<220>
   <223> Description of Artificial Sequence: Synthetic primer
<400> 547
   gctaatatgt aggagtcagt tgga 24
<210> 548
   <211> 20
   <212> DNA
   <213> Artificial Sequence
<220>
   <223> Description of Artificial Sequence: Synthetic primer
<400> 548
   cagtcaacgg tcagtggttt 20
<210> 549
   <211> 21
   <212> DNA
   <213> Artificial Sequence
<220>
   <223> Description of Artificial Sequence: Synthetic primer
<400> 549
   ttgcattcat tgttcagtga g 21
<210> 550
   <211> 21
   <212> DNA
   <213> Artificial Sequence
<220>
   <223> Description of Artificial Sequence: Synthetic primer
<400> 550
   gttggcaagt ctagaaccac c 21
<210> 551
   <211> 19
   <212> DNA
   <213> Artificial Sequence
<220>
   <223> Description of Artificial Sequence: Synthetic primer
<400> 551
   tatggccctt cggtaattc 19
<210> 552
   <211> 20
   <212> DNA
   <213> Artificial Sequence
<220>
   <223> Description of Artificial Sequence: Synthetic primer
<400> 552
   ccttatcagt tctccgtcca 20
<210> 553
   <211> 18
   <212> DNA
   <213> Artificial Sequence
<220>
   <223> Description of Artificial Sequence: Synthetic primer
<400> 553
   ggaccagagg aaagccag 18
<210> 554
   <211> 22
   <212> DNA
   <213> Artificial Sequence
<220>
   <223> Description of Artificial Sequence: Synthetic primer
<400> 554
   gacattcaca tgcttcaggt ag 22
<210> 555
   <211> 19
   <212> DNA
   <213> Artificial Sequence
<220>
   <223> Description of Artificial Sequence: Synthetic primer
<400> 555
   gctgcaacac aagacacga 19
<210> 556
   <211> 20
   <212> DNA
   <213> Artificial Sequence
<220>
   <223> Description of Artificial Sequence: Synthetic primer
<400> 556
   cacatgcaca agagagcaag 20
<210> 557
   <211> 24
   <212> DNA
   <213> Artificial Sequence
<220>
   <223> Description of Artificial Sequence: Synthetic primer
<400> 557
   ggaatatgtt tgatatatag ttgg 24
<210> 558
   <211> 17
   <212> DNA
   <213> Artificial Sequence
<220>
   <223> Description of Artificial Sequence: Synthetic primer
<400> 558
   gacgaaatcc aagcgca 17
<210> 559
   <211> 19
   <212> DNA
   <213> Artificial Sequence
<220>
   <223> Description of Artificial Sequence: Synthetic primer
<400> 559
   tgacctatgg aattggcag 19
<210> 560
   <211> 20
   <212> DNA
   <213> Artificial Sequence
<220>
   <223> Description of Artificial Sequence: Synthetic primer
<400> 560
   aactgggact ttgtaggcca 20
<210> 561
   <211> 20
   <212> DNA
   <213> Artificial Sequence
<220>
   <223> Description of Artificial Sequence: Synthetic primer
<400> 561
   taacagcatc tccactggaa 20
<210> 562
   <211> 16
   <212> DNA
   <213> Artificial Sequence
<220>
   <223> Description of Artificial Sequence: Synthetic primer
<400> 562
   ggctcagccc ctcctc 16
<210> 563
   <211> 19
   <212> DNA
   <213> Artificial Sequence
<220>
   <223> Description of Artificial Sequence: Synthetic primer
<400> 563
   atcgggtggt ttaatgttg 19
<210> 564
   <211> 20
   <212> DNA
   <213> Artificial Sequence
<220>
   <223> Description of Artificial Sequence: Synthetic primer
<400> 564
   cagtttcttg ttgccgagtt 20
<210> 565
   <211> 17
   <212> DNA
   <213> Artificial Sequence
<220>
   <223> Description of Artificial Sequence: Synthetic primer
<400> 565
   aggcagtgtc gtgctgt 17
<210> 566
   <211> 18
   <212> DNA
   <213> Artificial Sequence
<220>
   <223> Description of Artificial Sequence: Synthetic primer
<400> 566
   atgctgggtg gagaaggt 18
<210> 567
   <211> 24
   <212> DNA
   <213> Artificial Sequence
<220>
   <223> Description of Artificial Sequence: Synthetic primer
<400> 567
   tttattctat agagaagaag gaaa 24
<210> 568
   <211> 20
   <212> DNA
   <213> Artificial Sequence
<220>
   <223> Description of Artificial Sequence: Synthetic primer
<400> 568
   ggtggtggaa aatgacactc 20
<210> 569
   <211> 20
   <212> DNA
   <213> Artificial Sequence
<220>
   <223> Description of Artificial Sequence: Synthetic primer
<400> 569
   ccctagtgtg caaaacctgt 20
<210> 570
   <211> 17
   <212> DNA
   <213> Artificial Sequence
<220>
   <223> Description of Artificial Sequence: Synthetic primer
<400> 570
   cggtccagct ctccagt 17
<210> 571
   <211> 18
   <212> DNA
   <213> Artificial Sequence
<220>
   <223> Description of Artificial Sequence: Synthetic primer
<400> 571
   gatgtgcctc ggtggtgt 18
<210> 572
   <211> 24
   <212> DNA
   <213> Artificial Sequence
<220>
   <223> Description of Artificial Sequence: Synthetic primer
<400> 572
   gtcatcatta ccaggcagta ttag 24
<210> 573
   <211> 24
   <212> DNA
   <213> Artificial Sequence
<220>
   <223> Description of Artificial Sequence: Synthetic primer
<400> 573
   gtcatcatta ccaggcagta ttag 24
<210> 574
   <211> 23
   <212> DNA
   <213> Artificial Sequence
<220>
   <223> Description of Artificial Sequence: Synthetic primer
<400> 574
   ggtctagtgg tcctaaacat ttc 23
<210> 575
   <211> 17
   <212> DNA
   <213> Artificial Sequence
<220>
   <223> Description of Artificial Sequence: Synthetic primer
<400> 575
   gtccctttgc cttccca 17
<210> 576
   <211> 23
   <212> DNA
   <213> Artificial Sequence
<220>
   <223> Description of Artificial Sequence: Synthetic primer
<400> 576
   gaacttcact ccactgaaat ctg 23
<210> 577
   <211> 23
   <212> DNA
   <213> Artificial Sequence
<220>
   <223> Description of Artificial Sequence: Synthetic primer
<400> 577
   aaaccacaca cttccttaca ttc 23
<210> 578
   <211> 21
   <212> DNA
   <213> Artificial Sequence
<220>
   <223> Description of Artificial Sequence: Synthetic primer
<400> 578
   ccaacaagct ttttgctcgt c 21
<210> 579
   <211> 17
   <212> DNA
   <213> Artificial Sequence
<220>
   <223> Description of Artificial Sequence: Synthetic primer
<400> 579
   agccgctgtc acacgca 17
<210> 580
   <211> 17
   <212> DNA
   <213> Artificial Sequence
<220>
   <223> Description of Artificial Sequence: Synthetic primer
<400> 580
   cccctttgct gtccctg 17
<210> 581
   <211> 20
   <212> DNA
   <213> Artificial Sequence
<220>
   <223> Description of Artificial Sequence: Synthetic primer
<400> 581
   gccgtgactg gagactgtta 20
<210> 582
   <211> 21
   <212> DNA
   <213> Artificial Sequence
<220>
   <223> Description of Artificial Sequence: Synthetic primer
<400> 582
   gtacaatcaa cggtcgatgg t 21
<210> 583
   <211> 19
   <212> DNA
   <213> Artificial Sequence
<220>
   <223> Description of Artificial Sequence: Synthetic primer
<400> 583
   tgactgcctg tctgtgcct 19
<210> 584
   <211> 22
   <212> DNA
   <213> Artificial Sequence
<220>
   <223> Description of Artificial Sequence: Synthetic primer
<400> 584
   ttggcctaaa gaaatgacag ac 22
<210> 585
   <211> 21
   <212> DNA
   <213> Artificial Sequence
<220>
   <223> Description of Artificial Sequence: Synthetic primer
<400> 585
   tgagggctag gaaattgctc t 21
<210> 586
   <211> 21
   <212> DNA
   <213> Artificial Sequence
<220>
   <223> Description of Artificial Sequence: Synthetic primer
<400> 586
   ggcaatgcat taggaactga t 21
<210> 587
   <211> 20
   <212> DNA
   <213> Artificial Sequence
<220>
   <223> Description of Artificial Sequence: Synthetic primer
<400> 587
   gtgcttgacg gaaccatgtt 20
<210> 588
   <211> 22
   <212> DNA
   <213> Artificial Sequence
<220>
   <223> Description of Artificial Sequence: Synthetic primer
<400> 588
   gggacgtcca gactcaactc tc 22
<210> 589
   <211> 19
   <212> DNA
   <213> Artificial Sequence
<220>
   <223> Description of Artificial Sequence: Synthetic primer
<400> 589
   cagaccgcat catgaacac 19
<210> 590
   <211> 22
   <212> DNA
   <213> Artificial Sequence
<220>
   <223> Description of Artificial Sequence: Synthetic primer
<400> 590
   aaacccagca gacaatgtag ct 22
<210> 591
   <211> 22
   <212> DNA
   <213> Artificial Sequence
<220>
   <223> Description of Artificial Sequence: Synthetic primer
<400> 591
   ctctctcagg acactgaagc ag 22
<210> 592
   <211> 22
   <212> DNA
   <213> Artificial Sequence
<220>
   <223> Description of Artificial Sequence: Synthetic primer
<400> 592
   tggggtattt gacaaactga ca 22
<210> 593
   <211> 22
   <212> DNA
   <213> Artificial Sequence
<220>
   <223> Description of Artificial Sequence: Synthetic primer
<400> 593
   ctttgtagtc actagggcac ca 22
<210> 594
   <211> 18
   <212> DNA
   <213> Artificial Sequence
<220>
   <223> Description of Artificial Sequence: Synthetic primer
<400> 594
   ggagagcctc cacccaac 18
<210> 595
   <211> 17
   <212> DNA
   <213> Artificial Sequence
<220>
   <223> Description of Artificial Sequence: Synthetic primer
<400> 595
   gtttggatgg ctgggct 17
<210> 596
   <211> 23
   <212> DNA
   <213> Artificial Sequence
<220>
   <223> Description of Artificial Sequence: Synthetic primer
<400> 596
   gctttgacaa tactattgca ctg 23
<210> 597
   <211> 20
   <212> DNA
   <213> Artificial Sequence
<220>
   <223> Description of Artificial Sequence: Synthetic primer
<400> 597
   tcaccaaaac atggaagcac 20
<210> 598
   <211> 25
   <212> DNA
   <213> Artificial Sequence
<220>
   <223> Description of Artificial Sequence: Synthetic primer
<400> 598
   cctactaaaa catggaagca cttac 25
<210> 599
   <211> 20
   <212> DNA
   <213> Artificial Sequence
<220>
   <223> Description of Artificial Sequence: Synthetic primer
<400> 599
   tcaccaaaac atggaagcac 20
<210> 600
   <211> 20
   <212> DNA
   <213> Artificial Sequence
<220>
   <223> Description of Artificial Sequence: Synthetic primer
<400> 600
   tcaccaaaac atggaagcac 20
<210> 601
   <211> 17
   <212> DNA
   <213> Artificial Sequence
<220>
   <223> Description of Artificial Sequence: Synthetic primer
<400> 601
   ttcgccctct caaccca 17
<210> 602
   <211> 16
   <212> DNA
   <213> Artificial Sequence
<220>
   <223> Description of Artificial Sequence: Synthetic primer
<400> 602
   ctttaccccg ggtggg 16
<210> 603
   <211> 21
   <212> DNA
   <213> Artificial Sequence
<220>
   <223> Description of Artificial Sequence: Synthetic primer
<400> 603
   gaggtcgacc gtgtaatgtg c 21
<210> 604
   <211> 17
   <212> DNA
   <213> Artificial Sequence
<220>
   <223> Description of Artificial Sequence: Synthetic primer
<400> 604
   tggggctttc ttcccag 17
<210> 605
   <211> 22
   <212> DNA
   <213> Artificial Sequence
<220>
   <223> Description of Artificial Sequence: Synthetic primer
<400> 605
   gataggaggt cctcaataaa ca 22
<210> 606
   <211> 19
   <212> DNA
   <213> Artificial Sequence
<220>
   <223> Description of Artificial Sequence: Synthetic primer
<400> 606
   ctggaagctg aagctgcat 19
<210> 607
   <211> 17
   <212> DNA
   <213> Artificial Sequence
<220>
   <223> Description of Artificial Sequence: Synthetic primer
<400> 607
   ggacggaagg gcagaga 17
<210> 608
   <211> 17
   <212> DNA
   <213> Artificial Sequence
<220>
   <223> Description of Artificial Sequence: Synthetic primer
<400> 608
   tgcaggccgt gtgcttt 17
<210> 609
   <211> 21
   <212> DNA
   <213> Artificial Sequence
<220>
   <223> Description of Artificial Sequence: Synthetic primer
<400> 609
   cacactcttg atgttccagg a 21
<210> 610
   <211> 21
   <212> DNA
   <213> Artificial Sequence
<220>
   <223> Description of Artificial Sequence: Synthetic primer
<400> 610
   gaggtcagga gcaataatga a 21
<210> 611
   <211> 20
   <212> DNA
   <213> Artificial Sequence
<220>
   <223> Description of Artificial Sequence: Synthetic primer
<400> 611
   ggcatcatat aggagctgga 20
<210> 612
   <211> 22
   <212> DNA
   <213> Artificial Sequence
<220>
   <223> Description of Artificial Sequence: Synthetic primer
<400> 612
   caacaaaatc actgatgctg ga 22
<210> 613
   <211> 17
   <212> DNA
   <213> Artificial Sequence
<220>
   <223> Description of Artificial Sequence: Synthetic primer
<400> 613
   tctgtcgtcg aggcgct 17
<210> 614
   <211> 23
   <212> DNA
   <213> Artificial Sequence
<220>
   <223> Description of Artificial Sequence: Synthetic primer
<400> 614
   ggctataaag taactgagac gga 23
<210> 615
   <211> 17
   <212> DNA
   <213> Artificial Sequence
<220>
   <223> Description of Artificial Sequence: Synthetic primer
<400> 615
   cgggtgcgat ttctgtg 17
<210> 616
   <211> 18
   <212> DNA
   <213> Artificial Sequence
<220>
   <223> Description of Artificial Sequence: Synthetic primer
<400> 616
   ctccagaccc ctcgttca 18
<210> 617
   <211> 16
   <212> DNA
   <213> Artificial Sequence
<220>
   <223> Description of Artificial Sequence: Synthetic primer
<400> 617
   tgcccaggca gctgca 16
<210> 618
   <211> 20
   <212> DNA
   <213> Artificial Sequence
<220>
   <223> Description of Artificial Sequence: Synthetic primer
<400> 618
   gcaaatcaga atcacacctg 20
<210> 619
   <211> 20
   <212> DNA
   <213> Artificial Sequence
<220>
   <223> Description of Artificial Sequence: Synthetic primer
<400> 619
   caccattgct aaagtgcaat 20
<210> 620
   <211> 22
   <212> DNA
   <213> Artificial Sequence
<220>
   <223> Description of Artificial Sequence: Synthetic primer
<400> 620
   aacgtggaat ttcctctatg tt 22
<210> 621
   <211> 25
   <212> DNA
   <213> Artificial Sequence
<220>
   <223> Description of Artificial Sequence: Synthetic primer
<400> 621
   gaaaagatca accatgtatt attcg 25
<210> 622
   <211> 17
   <212> DNA
   <213> Artificial Sequence
<220>
   <223> Description of Artificial Sequence: Synthetic primer
<400> 622
   accaggttcc accccag 17
<210> 623
   <211> 19
   <212> DNA
   <213> Artificial Sequence
<220>
   <223> Description of Artificial Sequence: Synthetic primer
<400> 623
   acactcaaaa gatggcggc 19
<210> 624
   <211> 20
   <212> DNA
   <213> Artificial Sequence
<220>
   <223> Description of Artificial Sequence: Synthetic primer
<400> 624
   tcaaatgtcg cagcactttc 20
<210> 625
   <211> 20
   <212> DNA
   <213> Artificial Sequence
<220>
   <223> Description of Artificial Sequence: Synthetic primer
<400> 625
   caccccaaaa tcgaagcact 20
<210> 626
   <211> 20
   <212> DNA
   <213> Artificial Sequence
<220>
   <223> Description of Artificial Sequence: Synthetic primer
<400> 626
   caccccctgg gaagaaattt 20
<210> 627
   <211> 17
   <212> DNA
   <213> Artificial Sequence
<220>
   <223> Description of Artificial Sequence: Synthetic primer
<400> 627
   cctcacgcga gccgaac 17
<210> 628
   <211> 19
   <212> DNA
   <213> Artificial Sequence
<220>
   <223> Description of Artificial Sequence: Synthetic primer
<400> 628
   acgtggattt tcctctatg 19
<210> 629
   <211> 21
   <212> DNA
   <213> Artificial Sequence
<220>
   <223> Description of Artificial Sequence: Synthetic primer
<400> 629
   acaaaagttg cctttgtgtg a 21
<210> 630
   <211> 20
   <212> DNA
   <213> Artificial Sequence
<220>
   <223> Description of Artificial Sequence: Synthetic primer
<400> 630
   gccttctgac tccaagtcca 20
<210> 631
   <211> 23
   <212> DNA
   <213> Artificial Sequence
<220>
   <223> Description of Artificial Sequence: Synthetic primer
<400> 631
   gtggaccatg ttacataggt cag 23
<210> 632
   <211> 23
   <212> DNA
   <213> Artificial Sequence
<220>
   <223> Description of Artificial Sequence: Synthetic primer
<400> 632
   gatgtggacc atattacata cga 23
<210> 633
   <211> 22
   <212> DNA
   <213> Artificial Sequence
<220>
   <223> Description of Artificial Sequence: Synthetic primer
<400> 633
   acagagagct tgcccttgta ta 22
<210> 634
   <211> 22
   <212> DNA
   <213> Artificial Sequence
<220>
   <223> Description of Artificial Sequence: Synthetic primer
<400> 634
   aagtgttgtc cgtgaatgat tc 22
<210> 635
   <211> 20
   <212> DNA
   <213> Artificial Sequence
<220>
   <223> Description of Artificial Sequence: Synthetic primer
<400> 635
   ttctgaccag gcagtgctgt 20
<210> 636
   <211> 23
   <212> DNA
   <213> Artificial Sequence
<220>
   <223> Description of Artificial Sequence: Synthetic primer
<400> 636
   gtcattccta gaaattgttc ata 23
<210> 637
   <211> 19
   <212> DNA
   <213> Artificial Sequence
<220>
   <223> Description of Artificial Sequence: Synthetic primer
<400> 637
   ggccttctga ctccaagtc 19
<210> 638
   <211> 17
   <212> DNA
   <213> Artificial Sequence
<220>
   <223> Description of Artificial Sequence: Synthetic primer
<400> 638
   gaggggcctc agaccga 17
<210> 639
   <211> 17
   <212> DNA
   <213> Artificial Sequence
<220>
   <223> Description of Artificial Sequence: Synthetic primer
<400> 639
   gcagcgcctc acgtttt 17
<210> 640
   <211> 17
   <212> DNA
   <213> Artificial Sequence
<220>
   <223> Description of Artificial Sequence: Synthetic primer
<400> 640
   gggcggacac gacattc 17
<210> 641
   <211> 25
   <212> DNA
   <213> Artificial Sequence
<220>
   <223> Description of Artificial Sequence: Synthetic primer
<400> 641
   taggaaagac agtagattgt atagt 25
<210> 642
   <211> 20
   <212> DNA
   <213> Artificial Sequence
<220>
   <223> Description of Artificial Sequence: Synthetic primer
<400> 642
   gcctggatgc agacttttct 20
<210> 643
   <211> 19
   <212> DNA
   <213> Artificial Sequence
<220>
   <223> Description of Artificial Sequence: Synthetic primer
<400> 643
   aaagctagga ggctgtaca 19
<210> 644
   <211> 20
   <212> DNA
   <213> Artificial Sequence
<220>
   <223> Description of Artificial Sequence: Synthetic primer
<400> 644
   ctcaccatgt tgtttagtgc 20
<210> 645
   <211> 26
   <212> DNA
   <213> Artificial Sequence
<220>
   <223> Description of Artificial Sequence: Synthetic primer
<400> 645
   ggaaagacag tagattgtat agttat 26
<210> 646
   <211> 20
   <212> DNA
   <213> Artificial Sequence
<220>
   <223> Description of Artificial Sequence: Synthetic primer
<400> 646
   ctctgtccac cgcagatatt 20
<210> 647
   <211> 21
   <212> DNA
   <213> Artificial Sequence
<220>
   <223> Description of Artificial Sequence: Synthetic primer
<400> 647
   ggaaggcagt aggttgtata g 21
<210> 648
   <211> 18
   <212> DNA
   <213> Artificial Sequence
<220>
   <223> Description of Artificial Sequence: Synthetic primer
<400> 648
   catggggtcg tgtcactg 18
<210> 649
   <211> 22
   <212> DNA
   <213> Artificial Sequence
<220>
   <223> Description of Artificial Sequence: Synthetic primer
<400> 649
   ggaaagctag aaggttgtac ag 22
<210> 650
   <211> 19
   <212> DNA
   <213> Artificial Sequence
<220>
   <223> Description of Artificial Sequence: Synthetic primer
<400> 650
   aagaattcct cgacggctc 19
<210> 651
   <211> 20
   <212> DNA
   <213> Artificial Sequence
<220>
   <223> Description of Artificial Sequence: Synthetic primer
<400> 651
   aaggcagcag gtcgtatagt 20
<210> 652
   <211> 21
   <212> DNA
   <213> Artificial Sequence
<220>
   <223> Description of Artificial Sequence: Synthetic primer
<400> 652
   caaggaaaca ggttatcggt g 21
<210> 653
   <211> 21
   <212> DNA
   <213> Artificial Sequence
<220>
   <223> Description of Artificial Sequence: Synthetic primer
<400> 653
   gaaagctagg aggccgtata g 21
<210> 654
   <211> 19
   <212> DNA
   <213> Artificial Sequence
<220>
   <223> Description of Artificial Sequence: Synthetic primer
<400> 654
   agaaaagagc ccggctctt 19
<210> 655
   <211> 22
   <212> DNA
   <213> Artificial Sequence
<220>
   <223> Description of Artificial Sequence: Synthetic primer
<400> 655
   gggaaggcaa tagattgtat ag 22
<210> 656
   <211> 20
   <212> DNA
   <213> Artificial Sequence
<220>
   <223> Description of Artificial Sequence: Synthetic primer
<400> 656
   taatgcagca agtctactcc 20
<210> 657
   <211> 24
   <212> DNA
   <213> Artificial Sequence
<220>
   <223> Description of Artificial Sequence: Synthetic primer
<400> 657
   gggaaagaca gtagactgta tagt 24
<210> 658
   <211> 20
   <212> DNA
   <213> Artificial Sequence
<220>
   <223> Description of Artificial Sequence: Synthetic primer
<400> 658
   cagtcggaga agaagtgtac 20
<210> 659
   <211> 18
   <212> DNA
   <213> Artificial Sequence
<220>
   <223> Description of Artificial Sequence: Synthetic primer
<400> 659
   ggcagtggcc tgtacagt 18
<210> 660
   <211> 16
   <212> DNA
   <213> Artificial Sequence
<220>
   <223> Description of Artificial Sequence: Synthetic primer
<400> 660
   ccccacttgg cagctg 16
<210> 661
   <211> 18
   <212> DNA
   <213> Artificial Sequence
<220>
   <223> Description of Artificial Sequence: Synthetic primer
<400> 661
   gcagtagctt gcgcagtt 18
<210> 662
   <211> 18
   <212> DNA
   <213> Artificial Sequence
<220>
   <223> Description of Artificial Sequence: Synthetic primer
<400> 662
   gctgagcatc accagcac 18
139/139
1/139

### SEQUENCE LISTING

<110> THE OHIO STATE UNIVERSITY
<120> MICRORNA EXPRESSION PROFILE ASSOCIATED WITH PANCREATIC CANCER
<130> CMD/FP6825251
<140> Divisional application of: EP 07757822.7
   <141> 2007-03-02
<150> 07757822.7
   <151> 2007-03-02
<150> PCT/US2007/063208
   <151> 2007-03-02
<150> 60/778,271
   <151> 2006-03-02
<160> 662
<170> PatentIn version 3.5
<210> 1
   <211> 80
   <212> DNA
   <213> Homo sapiens
<400> 1
   tgggatgagg tagtaggttg tatagtttta gggtcacacc caccactggg agataactat        60
   acaatctact gtctttccta                                                    80
<210> 2
   <211> 72
   <212> DNA
   <213> Homo sapiens
<400> 2
   aggttgaggt agtaggttgt atagtttaga attacatcaa gggagataac tgtacagcct        60
   cctagctttc ct                                                            72
<210> 3
   <211> 74
   <212> DNA
   <213> Homo sapiens
<400> 3
   gggtgaggta gtaggttgta tagtttgggg ctctgccctg ctatgggata actatacaat        60
   ctactgtctt tcct                                                          74
<210> 4
   <211> 83
   <212> DNA
   <213> Homo sapiens
<400> 4
   cggggtgagg tagtaggttg tgtggtttca gggcagtgat gttgcccctc ggaagataac        60
   tatacaacct actgccttcc ctg                                                83
<210> 5
   <211> 84
   <212> DNA
   <213> Homo sapiens
<400> 5
   gcatccgggt tgaggtagta ggttgtatgg tttagagtta caccctggga gttaactgta 60
   caaccttcta gctttccttg gagc 84
<210> 6
   <211> 87
   <212> DNA
   <213> Homo sapiens
<400> 6
   cctaggaaga ggtagtaggt tgcatagttt tagggcaggg attttgccca caaggaggta 60
   actatacgac ctgctgcctt tcttagg 87
<210> 7
   <211> 79
   <212> DNA
   <213> Homo sapiens
<400> 7
   cccgggctga ggtaggaggt tgtatagttg aggaggacac ccaaggagat cactatacgg 60
   cctcctagct ttccccagg 79
<210> 8
   <211> 87
   <212> DNA
   <213> Homo sapiens
<400> 8
   tcagagtgag gtagtagatt gtatagttgt ggggtagtga ttttaccctg ttcaggagat 60
   aactatacaa tctattgcct tccctga 87
<210> 9
   <211> 83
   <212> DNA
   <213> Homo sapiens
<400> 9
   tgtgggatga ggtagtagat tgtatagttt tagggtcata ccccatcttg gagataacta 60
   tacagtctac tgtctttccc acg 83
<210> 10
   <211> 84
   <212> DNA
   <213> Homo sapiens
<400> 10
   aggctgaggt agtagtttgt acagtttgag ggtctatgat accacccggt acaggagata 60
   actgtacagg ccactgcctt gcca 84
<210> 11
   <211> 84
   <212> DNA
   <213> Homo sapiens
<400> 11
   ctggctgagg tagtagtttg tgctgttggt cgggttgtga cattgcccgc tgtggagata 60
   actgcgcaag ctactgcctt gcta 84
<210> 12
   <211> 71
   <212> DNA
   <213> Homo sapiens
<400> 12
   tgggaaacat acttctttat atgcccatat ggacctgcta agctatggaa tgtaaagaag 60
   tatgtatctc a 71
<210> 13
   <211> 85
   <212> DNA
   <213> Homo sapiens
<400> 13
   acctactcag agtacatact tctttatgta cccatatgaa catacaatgc tatggaatgt 60
   aaagaagtat gtatttttgg taggc 85
<210> 14
   <211> 110
   <212> DNA
   <213> Homo sapiens
<400> 14
   ttggatgttg gcctagttct gtgtggaaga ctagtgattt tgttgttttt agataactaa 60
   atcgacaaca aatcacagtc tgccatatgg cacaggccat gcctctacag 110
<210> 15
   <211> 110
   <212> DNA
   <213> Homo sapiens
<400> 15
   ctggatacag agtggaccgg ctggccccat ctggaagact agtgattttg ttgttgtctt 60
   actgcgctca acaacaaatc ccagtctacc taatggtgcc agccatcgca 110
<210> 16
   <211> 110
   <212> DNA
   <213> Homo sapiens
<400> 16
   agattagagt ggctgtggtc tagtgctgtg tggaagacta gtgattttgt tgttctgatg 60
   tactacgaca acaagtcaca gccggcctca tagcgcagac tcccttcgac 110
<210> 17
   <211> 89
   <212> DNA
   <213> Homo sapiens
<400> 17
   cggggttggt tgttatcttt ggttatctag ctgtatgagt ggtgtggagt cttcataaag 60
   ctagataacc gaaagtaaaa ataacccca 89
<210> 18
   <211> 87
   <212> DNA
   <213> Homo sapiens
<400> 18
   ggaagcgagt tgttatcttt ggttatctag ctgtatgagt gtattggtct tcataaagct 60
   agataaccga aagtaaaaac tccttca 87
<210> 19
   <211> 90
   <212> DNA
   <213> Homo sapiens
<400> 19
   ggaggcccgt ttctctcttt ggttatctag ctgtatgagt gccacagagc cgtcataaag 60
   ctagataacc gaaagtagaa atgattctca 90
<210> 20
   <211> 110
   <212> DNA
   <213> Homo sapiens
<400> 20
   gatctgtctg tcttctgtat ataccctgta gatccgaatt tgtgtaagga attttgtggt       60
   cacaaattcg tatctagggg aatatgtagt tgacataaac actccgctct                 110
<210> 21
   <211> 110
   <212> DNA
   <213> Homo sapiens
<400> 21
   ccagaggttg taacgttgtc tatatatacc ctgtagaacc gaatttgtgt ggtatccgta       60
   tagtcacaga ttcgattcta ggggaatata tggtcgatgc aaaaacttca                 110
<210> 22
   <211> 83
   <212> DNA
   <213> Homo sapiens
<400> 22
   ccttggagta aagtagcagc acataatggt ttgtggattt tgaaaaggtg caggccatat       60
   tgtgctgcct caaaaataca agg                                               83
<210> 23
   <211> 98
   <212> DNA
   <213> Homo sapiens
<400> 23
   ttgaggcctt aaagtactgt agcagcacat catggtttac atgctacagt caagatgcga       60
   atcattattt gctgctctag aaatttaagg aaattcat                               98
<210> 24
   <211> 89
   <212> DNA
   <213> Homo sapiens
<400> 24
   gtcagcagtg ccttagcagc acgtaaatat tggcgttaag attctaaaat tatctccagt 60
   attaactgtg ctgctgaagt aaggttgac 89
<210> 25
   <211> 81
   <212> DNA
   <213> Homo sapiens
<400> 25
   gttccactct agcagcacgt aaatattggc gtagtgaaat atatattaaa caccaatatt 60
   actgtgctgc tttagtgtga c 81
<210> 26
   <211> 84
   <212> DNA
   <213> Homo sapiens
<400> 26
   gtcagaataa tgtcaaagtg cttacagtgc aggtagtgat atgtgcatct actgcagtga 60
   aggcacttgt agcattatgg tgac 84
<210> 27
   <211> 71
   <212> DNA
   <213> Homo sapiens
<400> 27
   tgttctaagg tgcatctagt gcagatagtg aagtagatta gcatctactg ccctaagtgc 60
   tccttctggc a 71
<210> 28
   <211> 82
   <212> DNA
   <213> Homo sapiens
<400> 28
   gcagtcctct gttagttttg catagttgca ctacaagaag aatgtagttg tgcaaatcta 60
   tgcaaaactg atggtggcct gc 82
<210> 29
   <211> 87
   <212> DNA
   <213> Homo sapiens
<400> 29
   cactgttcta tggttagttt tgcaggtttg catccagctg tgtgatattc tgctgtgcaa 60
   atccatgcaa aactgactgt ggtagtg 87
<210> 30
   <211> 96
   <212> DNA
   <213> Homo sapiens
<400> 30
   acattgctac ttacaattag ttttgcaggt ttgcatttca gcgtatatat gtatatgtgg 60
   ctgtgcaaat ccatgcaaaa ctgattgtga taatgt 96
<210> 31
   <211> 71
   <212> DNA
   <213> Homo sapiens
<400> 31
   gtagcactaa agtgcttata gtgcaggtag tgtttagtta tctactgcat tatgagcact 60
   taaagtactg c 71
<210> 32
   <211> 72
   <212> DNA
   <213> Homo sapiens
<400> 32
   tgtcgggtag cttatcagac tgatgttgac tgttgaatct catggcaaca ccagtcgatg 60
   ggctgtctga ca 72
<210> 33
   <211> 85
   <212> DNA
   <213> Homo sapiens
<400> 33
   ggctgagccg cagtagttct tcagtggcaa gctttatgtc ctgacccagc taaagctgcc 60
   agttgaagaa ctgttgccct ctgcc 85
<210> 34
   <211> 73
   <212> DNA
   <213> Homo sapiens
<400> 34
   ggccggctgg ggttcctggg gatgggattt gcttcctgtc acaaatcaca ttgccaggga 60
   tttccaaccg acc 73
<210> 35
   <211> 97
   <212> DNA
   <213> Homo sapiens
<400> 35
   ctcaggtgct ctggctgctt gggttcctgg catgctgatt tgtgacttaa gattaaaatc 60
   acattgccag ggattaccac gcaaccacga ccttggc 97
<210> 36
   <211> 68
   <212> DNA
   <213> Homo sapiens
<400> 36
   ctccggtgcc tactgagctg atatcagttc tcattttaca cactggctca gttcagcagg 60
   aacaggag 68
<210> 37
   <211> 73
   <212> DNA
   <213> Homo sapiens
<400> 37
   ctctgcctcc cgtgcctact gagctgaaac acagttggtt tgtgtacact ggctcagttc 60
   agcaggaaca ggg 73
<210> 38
   <211> 84
   <212> DNA
   <213> Homo sapiens
<400> 38
   ggccagtgtt gagaggcgga gacttgggca attgctggac gctgccctgg gcattgcact 60
   tgtctcggtc tgacagtgcc ggcc 84
<210> 39
   <211> 77
   <212> DNA
   <213> Homo sapiens
<400> 39
   gtggcctcgt tcaagtaatc caggataggc tgtgcaggtc ccaatgggcc tattcttggt 60
   tacttgcacg gggacgc 77
<210> 40
   <211> 84
   <212> DNA
   <213> Homo sapiens
<400> 40
   ggctgtggct ggattcaagt aatccaggat aggctgtttc catctgtgag gcctattctt 60
   gattacttgt ttctggaggc agct 84
<210> 41
   <211> 77
   <212> DNA
   <213> Homo sapiens
<400> 41
   ccgggaccca gttcaagtaa ttcaggatag gttgtgtgct gtccagcctg ttctccatta 60
   cttggctcgg ggaccgg 77
<210> 42
   <211> 78
   <212> DNA
   <213> Homo sapiens
<400> 42
   ctgaggagca gggcttagct gcttgtgagc agggtccaca ccaagtcgtg ttcacagtgg 60
   ctaagttccg ccccccag 78
<210> 43
   <211> 97
   <212> DNA
   <213> Homo sapiens
<400> 43
   acctctctaa caaggtgcag agcttagctg attggtgaac agtgattggt ttccgctttg 60
   ttcacagtgg ctaagttctg cacctgaaga gaaggtg 97
<210> 44
   <211> 86
   <212> DNA
   <213> Homo sapiens
<400> 44
   ggtccttgcc ctcaaggagc tcacagtcta ttgagttacc tttctgactt tcccactaga 60
   ttgtgagctc ctggagggca ggcact 86
<210> 45
   <211> 64
   <212> DNA
   <213> Homo sapiens
<400> 45
   atgactgatt tcttttggtg ttcagagtca atataatttt ctagcaccat ctgaaatcgg 60
   ttat 64
<210> 46
   <211> 88
   <212> DNA
   <213> Homo sapiens
<400> 46
   atctcttaca caggctgacc gatttctcct ggtgttcaga gtctgttttt gtctagcacc 60
   atttgaaatc ggttatgatg taggggga 88
<210> 47
   <211> 81
   <212> DNA
   <213> Homo sapiens
<400> 47
   cttcaggaag ctggtttcat atggtggttt agatttaaat agtgattgtc tagcaccatt 60
   tgaaatcagt gttcttgggg g 81
<210> 48
   <211> 81
   <212> DNA
   <213> Homo sapiens
<400> 48
   cttctggaag ctggtttcac atggtggctt agatttttcc atctttgtat ctagcaccat 60
   ttgaaatcag tgttttagga g 81
<210> 49
   <211> 71
   <212> DNA
   <213> Homo sapiens
<400> 49
   gcgactgtaa acatcctcga ctggaagctg tgaagccaca gatgggcttt cagtcggatg 60
   tttgcagctg c 71
<210> 50
   <211> 88
   <212> DNA
   <213> Homo sapiens
<400> 50
   accaagtttc agttcatgta aacatcctac actcagctgt aatacatgga ttggctggga 60
   ggtggatgtt tacttcagct gacttgga 88
<210> 51
   <211> 89
   <212> DNA
   <213> Homo sapiens
<400> 51
   accatgctgt agtgtgtgta aacatcctac actctcagct gtgagctcaa ggtggctggg 60
   agagggttgt ttactccttc tgccatgga 89
<210> 52
   <211> 72
   <212> DNA
   <213> Homo sapiens
<400> 52
   agatactgta aacatcctac actctcagct gtggaaagta agaaagctgg gagaaggctg 60
   tttactcttt ct 72
<210> 53
   <211> 70
   <212> DNA
   <213> Homo sapiens
<400> 53
   gttgttgtaa acatccccga ctggaagctg taagacacag ctaagctttc agtcagatgt 60
   ttgctgctac 70
<210> 54
   <211> 92
   <212> DNA
   <213> Homo sapiens
<400> 54
   gggcagtctt tgctactgta aacatccttg actggaagct gtaaggtgtt cagaggagct 60
   ttcagtcgga tgtttacagc ggcaggctgc ca 92
<210> 55
   <211> 71
   <212> DNA
   <213> Homo sapiens
<400> 55
   ggagaggagg caagatgctg gcatagctgt tgaactggga acctgctatg ccaacatatt 60
   gccatctttc c 71
<210> 56
   <211> 70
   <212> DNA
   <213> Homo sapiens
<400> 56
   ggagatattg cacattacta agttgcatgt tgtcacggcc tcaatgcaat ttagtgtgtg 60
   tgatattttc 70
<210> 57
   <211> 69
   <212> DNA
   <213> Homo sapiens
<400> 57
   ctgtggtgca ttgtagttgc attgcatgtt ctggtggtac ccatgcaatg tttccacagt 60
   gcatcacag 69
<210> 58
   <211> 110
   <212> DNA
   <213> Homo sapiens
<400> 58
   ggccagctgt gagtgtttct ttggcagtgt cttagctggt tgttgtgagc aatagtaagg 60
   aagcaatcag caagtatact gccctagaag tgctgcacgt tgtggggccc 110
<210> 59
   <211> 84
   <212> DNA
   <213> Homo sapiens
<400> 59
   gtgctcggtt tgtaggcagt gtcattagct gattgtactg tggtggttac aatcactaac 60
   tccactgcca tcaaaacaag gcac 84
<210> 60
   <211> 77
   <212> DNA
   <213> Homo sapiens
<400> 60
   agtctagtta ctaggcagtg tagttagctg attgctaata gtaccaatca ctaaccacac 60
   ggccaggtaa aaagatt 77
<210> 61
   <211> 78
   <212> DNA
   <213> Homo sapiens
<400> 61
   ctttctacac aggttgggat cggttgcaat gctgtgtttc tgtatggtat tgcacttgtc 60
   ccggcctgtt gagtttgg 78
<210> 62
   <211> 75
   <212> DNA
   <213> Homo sapiens
<400> 62
   tcatccctgg gtggggattt gttgcattac ttgtgttcta tataaagtat tgcacttgtc 60
   ccggcctgtg gaaga 75
<210> 63
   <211> 80
   <212> DNA
   <213> Homo sapiens
<400> 63
   ctgggggctc caaagtgctg ttcgtgcagg tagtgtgatt acccaaccta ctgctgagct 60
   agcacttccc gagcccccgg 80
<210> 64
   <211> 81
   <212> DNA
   <213> Homo sapiens
<400> 64
   aacacagtgg gcactcaata aatgtctgtt gaattgaaat gcgttacatt caacgggtat 60
   ttattgagca cccactctgt g 81
<210> 65
   <211> 78
   <212> DNA
   <213> Homo sapiens
<400> 65
   tggccgattt tggcactagc acatttttgc ttgtgtctct ccgctctgag caatcatgtg 60
   cagtgccaat atgggaaa 78
<210> 66
   <211> 119
   <212> DNA
   <213> Homo sapiens
<400> 66
   aggattctgc tcatgccagg gtgaggtagt aagttgtatt gttgtggggt agggatatta 60
   ggccccaatt agaagataac tatacaactt actactttcc ctggtgtgtg gcatattca 119
<210> 67
   <211> 81
   <212> DNA
   <213> Homo sapiens
<400> 67
   cccattggca taaacccgta gatccgatct tgtggtgaag tggaccgcac aagctcgctt 60
   ctatgggtct gtgtcagtgt g 81
<210> 68
   <211> 70
   <212> DNA
   <213> Homo sapiens
<400> 68
   ggcacccacc cgtagaaccg accttgcggg gccttcgccg cacacaagct cgtgtctgtg 60
   ggtccgtgtc 70
<210> 69
   <211> 80
   <212> DNA
   <213> Homo sapiens
<400> 69
   cctgttgcca caaacccgta gatccgaact tgtggtatta gtccgcacaa gcttgtatct 60
   ataggtatgt gtctgttagg 80
<210> 70
   <211> 75
   <212> DNA
   <213> Homo sapiens
<400> 70
   tgccctggct cagttatcac agtgctgatg ctgtctattc taaaggtaca gtactgtgat 60
   aactgaagga tggca 75
<210> 71
   <211> 79
   <212> DNA
   <213> Homo sapiens
<400> 71
   actgtccttt ttcggttatc atggtaccga tgctgtatat ctgaaaggta cagtactgtg 60
   ataactgaag aatggtggt 79
<210> 72
   <211> 78
   <212> DNA
   <213> Homo sapiens
<400> 72
   tactgccctc ggcttcttta cagtgctgcc ttgttgcata tggatcaagc agcattgtac 60
   agggctatga aggcattg 78
<210> 73
   <211> 78
   <212> DNA
   <213> Homo sapiens
<400> 73
   ttgtgctttc agcttcttta cagtgctgcc ttgtagcatt caggtcaagc agcattgtac 60
   agggctatga aagaacca 78
<210> 74
   <211> 81
   <212> DNA
   <213> Homo sapiens
<400> 74
   tgtgcatcgt ggtcaaatgc tcagactcct gtggtggctg ctcatgcacc acggatgttt 60
   gagcatgtgc tacggtgtct a 81
<210> 75
   <211> 81
   <212> DNA
   <213> Homo sapiens
<400> 75
   tgtgcatcgt ggtcaaatgc tcagactcct gtggtggctg cttatgcacc acggatgttt 60
   gagcatgtgc tatggtgtct a 81
<210> 76
   <211> 81
   <212> DNA
   <213> Homo sapiens
<400> 76
   ccttggccat gtaaaagtgc ttacagtgca ggtagctttt tgagatctac tgcaatgtaa 60
   gcacttctta cattaccatg g 81
<210> 77
   <211> 82
   <212> DNA
   <213> Homo sapiens
<400> 77
   cctgccgggg ctaaagtgct gacagtgcag atagtggtcc tctccgtgct accgcactgt 60
   gggtacttgc tgctccagca gg 82
<210> 78
   <211> 81
   <212> DNA
   <213> Homo sapiens
<400> 78
   ctctctgctt tcagcttctt tacagtgttg ccttgtggca tggagttcaa gcagcattgt 60
   acagggctat caaagcacag a 81
<210> 79
   <211> 92
   <212> DNA
   <213> Homo sapiens
<400> 79
   acactgcaag aacaataagg atttttaggg gcattatgac tgagtcagaa aacacagctg 60
   cccctgaaag tccctcattt ttcttgctgt cc 92
<210> 80
   <211> 85
   <212> DNA
   <213> Homo sapiens
<400> 80
   ccttagcaga gctgtggagt gtgacaatgg tgtttgtgtc taaactatca aacgccatta 60
   tcacactaaa tagctactgc taggc 85
<210> 81
   <211> 85
   <212> DNA
   <213> Homo sapiens
<400> 81
   aggcctctct ctccgtgttc acagcggacc ttgatttaaa tgtccataca attaaggcac 60
   gcggtgaatg ccaagaatgg ggctg 85
<210> 82
   <211> 109
   <212> DNA
   <213> Homo sapiens
<400> 82
   atcaagatta gaggctctgc tctccgtgtt cacagcggac cttgatttaa tgtcatacaa 60
   ttaaggcacg cggtgaatgc caagagcgga gcctacggct gcacttgaa 109
<210> 83
   <211> 87
   <212> DNA
   <213> Homo sapiens
<400> 83
   tgagggcccc tctgcgtgtt cacagcggac cttgatttaa tgtctataca attaaggcac 60
   gcggtgaatg ccaagagagg cgcctcc 87
<210> 84
   <211> 86
   <212> DNA
   <213> Homo sapiens
<400> 84
   tgccagtctc taggtccctg agacccttta acctgtgagg acatccaggg tcacaggtga      60
   ggttcttggg agcctggcgt ctggcc 86
<210> 85
   <211> 88
   <212> DNA
   <213> Homo sapiens
<400> 85
   tgcgctcctc tcagtccctg agaccctaac ttgtgatgtt taccgtttaa atccacgggt 60
   taggctcttg ggagctgcga gtcgtgct 88
<210> 86
   <211> 89
   <212> DNA
   <213> Homo sapiens
<400> 86
   accagacttt tcctagtccc tgagacccta acttgtgagg tattttagta acatcacaag 60
   tcaggctctt gggacctagg cggagggga 89
<210> 87
   <211> 85
   <212> DNA
   <213> Homo sapiens
<400> 87
   cgctggcgac gggacattat tacttttggt acgcgctgtg acacttcaaa ctcgtaccgt 60
   gagtaataat gcgccgtcca cggca 85
<210> 88
   <211> 97
   <212> DNA
   <213> Homo sapiens
<400> 88
   tgtgatcact gtctccagcc tgctgaagct cagagggctc tgattcagaa agatcatcgg 60
   atccgtctga gcttggctgg tcggaagtct catcatc 97
<210> 89
   <211> 82
   <212> DNA
   <213> Homo sapiens
<400> 89
   tgagctgttg gattcggggc cgtagcactg tctgagaggt ttacatttct cacagtgaac 60
   cggtctcttt ttcagctgct tc 82
<210> 90
   <211> 84
   <212> DNA
   <213> Homo sapiens
<400> 90
   tgtgcagtgg gaaggggggc cgatacactg tacgagagtg agtagcaggt ctcacagtga 60
   accggtctct ttccctactg tgtc 84
<210> 91
   <211> 90
   <212> DNA
   <213> Homo sapiens
<400> 91
   tgcccttcgc gaatcttttt gcggtctggg cttgctgtac ataactcaat agccggaagc 60
   ccttacccca aaaagcattt gcggagggcg 90
<210> 92
   <211> 89
   <212> DNA
   <213> Homo sapiens
<400> 92
   tgctgctggc cagagctctt ttcacattgt gctactgtct gcacctgtca ctagcagtgc 60
   aatgttaaaa gggcattggc cgtgtagtg 89
<210> 93
   <211> 82
   <212> DNA
   <213> Homo sapiens
<400> 93
   ggcctgcccg acactctttc cctgttgcac tactataggc cgctgggaag cagtgcaatg 60
   atgaaagggc atcggtcagg tc 82
<210> 94
   <211> 101
   <212> DNA
   <213> Homo sapiens
<400> 94
   ccgcccccgc gtctccaggg caaccgtggc tttcgattgt tactgtggga actggaggta 60
   acagtctaca gccatggtcg ccccgcagca cgcccacgcg c 101
<210> 95
   <211> 88
   <212> DNA
   <213> Homo sapiens
<400> 95
   acaatgcttt gctagagctg gtaaaatgga accaaatcgc ctcttcaatg gatttggtcc 60
   ccttcaacca gctgtagcta tgcattga 88
<210> 96
   <211> 102
   <212> DNA
   <213> Homo sapiens
<400> 96
   gggagccaaa tgctttgcta gagctggtaa aatggaacca aatcgactgt ccaatggatt 60
   tggtcccctt caaccagctg tagctgtgca ttgatggcgc cg 102
<210> 97
   <211> 119
   <212> DNA
   <213> Homo sapiens
<400> 97
   cctcagaaga aagatgcccc ctgctctggc tggtcaaacg gaaccaagtc cgtcttcctg 60
   agaggtttgg tccccttcaa ccagctacag cagggctggc aatgcccagt ccttggaga 119
<210> 98
   <211> 73
   <212> DNA
   <213> Homo sapiens
<400> 98
   cagggtgtgt gactggttga ccagaggggc atgcactgtg ttcaccctgt gggccaccta 60
   gtcaccaacc ctc 73
<210> 99
   <211> 90
   <212> DNA
   <213> Homo sapiens
<400> 99
   aggcctcgct gttctctatg gctttttatt cctatgtgat tctactgctc actcatatag 60
   ggattggagc cgtggcgcac ggcggggaca 90
<210> 100
   <211> 100
   <212> DNA
   <213> Homo sapiens
<400> 100
   agataaattc actctagtgc tttatggctt tttattccta tgtgatagta ataaagtctc 60
   atgtagggat ggaagccatg aaatacattg tgaaaaatca 100
<210> 101
   <211> 97
   <212> DNA
   <213> Homo sapiens
<400> 101
   cactctgctg tggcctatgg cttttcattc ctatgtgatt gctgtcccaa actcatgtag 60
   ggctaaaagc catgggctac agtgaggggc gagctcc 97
<210> 102
   <211> 82
   <212> DNA
   <213> Homo sapiens
<400> 102
   tgagccctcg gaggactcca tttgttttga tgatggattc ttatgctcca tcatcgtctc 60
   aaatgagtct tcagagggtt ct 82
<210> 103
   <211> 102
   <212> DNA
   <213> Homo sapiens
<400> 103
   ggtcctctga ctctcttcgg tgacgggtat tcttgggtgg ataatacgga ttacgttgtt 60
   attgcttaag aatacgcgta gtcgaggaga gtaccagcgg ca 102
<210> 104
   <211> 99
   <212> DNA
   <213> Homo sapiens
<400> 104
   ccctggcatg gtgtggtggg gcagctggtg ttgtgaatca ggccgttgcc aatcagagaa 60
   cggctacttc acaacaccag ggccacacca cactacagg 99
<210> 105
   <211> 84
   <212> DNA
   <213> Homo sapiens
<400> 105
   cgttgctgca gctggtgttg tgaatcaggc cgacgagcag cgcatcctct tacccggcta 60
   tttcacgaca ccagggttgc atca 84
<210> 106
   <211> 68
   <212> DNA
   <213> Homo sapiens
<400> 106
   gtgtattcta cagtgcacgt gtctccagtg tggctcggag gctggagacg cggccctgtt 60
   ggagtaac 68
<210> 107
   <211> 100
   <212> DNA
   <213> Homo sapiens
<400> 107
   tgtgtctctc tctgtgtcct gccagtggtt ttaccctatg gtaggttacg tcatgctgtt 60
   ctaccacagg gtagaaccac ggacaggata ccggggcacc 100
<210> 108
   <211> 95
   <212> DNA
   <213> Homo sapiens
<400> 108
   cggccggccc tgggtccatc ttccagtaca gtgttggatg gtctaattgt gaagctccta 60
   acactgtctg gtaaagatgg ctcccgggtg ggttc 95
<210> 109
   <211> 87
   <212> DNA
   <213> Homo sapiens
<400> 109
   gacagtgcag tcacccataa agtagaaagc actactaaca gcactggagg gtgtagtgtt 60
   tcctacttta tggatgagtg tactgtg 87
<210> 110
   <211> 106
   <212> DNA
   <213> Homo sapiens
<400> 110
   gcgcagcgcc ctgtctccca gcctgaggtg cagtgctgca tctctggtca gttgggagtc 60
   tgagatgaag cactgtagct caggaagaga gaagttgttc tgcagc 106
<210> 111
   <211> 86
   <212> DNA
   <213> Homo sapiens
<400> 111
   tggggccctg gctgggatat catcatatac tgtaagtttg cgatgagaca ctacagtata 60
   gatgatgtac tagtccgggc accccc 86
<210> 112
   <211> 88
   <212> DNA
   <213> Homo sapiens
<400> 112
   caccttgtcc tcacggtcca gttttcccag gaatccctta gatgctaaga tggggattcc 60
   tggaaatact gttcttgagg tcatggtt 88
<210> 113
   <211> 99
   <212> DNA
   <213> Homo sapiens
<400> 113
   ccgatgtgta tcctcagctt tgagaactga attccatggg ttgtgtcagt gtcagacctc 60
   tgaaattcag ttcttcagct gggatatctc tgtcatcgt 99
<210> 114
   <211> 72
   <212> DNA
   <213> Homo sapiens
<400> 114
   aatctaaaga caacatttct gcacacacac cagactatgg aagccagtgt gtggaaatgc 60
   ttctgctaga tt 72
<210> 115
   <211> 68
   <212> DNA
   <213> Homo sapiens
<400> 115
   gaggcaaagt tctgagacac tccgactctg agtatgatag aagtcagtgc actacagaac 60
   tttgtctc 68
<210> 116
   <211> 99
   <212> DNA
   <213> Homo sapiens
<400> 116
   caagcacgat tagcatttga ggtgaagttc tgttatacac tcaggctgtg gctctctgaa    60
   agtcagtgca tcacagaact ttgtctcgaa agctttcta                           99
<210> 117
   <211> 89
   <212> DNA
   <213> Homo sapiens
<400> 117
   gccggcgccc gagctctggc tccgtgtctt cactcccgtg cttgtccgag gagggaggga    60
   gggacggggg ctgtgctggg gcagctgga                                      89
<210> 118
   <211> 84
   <212> DNA
   <213> Homo sapiens
<400> 118
   ctccccatgg ccctgtctcc caacccttgt accagtgctg ggctcagacc ctggtacagg    60
   cctgggggac agggacctgg ggac                                           84
<210> 119
   <211> 90
   <212> DNA
   <213> Homo sapiens
<400> 119
   tttcctgccc tcgaggagct cacagtctag tatgtctcat cccctactag actgaagctc    60
   cttgaggaca gggatggtca tactcacctc                                     90
<210> 120
   <211> 87
   <212> DNA
   <213> Homo sapiens
<400> 120
   tgtccccccc ggcccaggtt ctgtgataca ctccgactcg ggctctggag cagtcagtgc    60
   atgacagaac ttgggcccgg aaggacc                                        87
<210> 121
   <211> 90
   <212> DNA
   <213> Homo sapiens
<400> 121
   ctcacagctg ccagtgtcat ttttgtgatc tgcagctagt attctcactc cagttgcata 60
   gtcacaaaag tgatcattgg caggtgtggc 90
<210> 122
   <211> 87
   <212> DNA
   <213> Homo sapiens
<400> 122
   agcggtggcc agtgtcattt ttgtgatgtt gcagctagta atatgagccc agttgcatag 60
   tcacaaaagt gatcattgga aactgtg 87
<210> 123
   <211> 84
   <212> DNA
   <213> Homo sapiens
<400> 123
   gtggtacttg aagataggtt atccgtgttg ccttcgcttt atttgtgacg aatcatacac 60
   ggttgaccta tttttcagta ccaa 84
<210> 124
   <211> 65
   <212> DNA
   <213> Homo sapiens
<400> 124
   ctgttaatgc taatcgtgat aggggttttt gcctccaact gactcctaca tattagcatt 60
   aacag 65
<210> 125
   <211> 110
   <212> DNA
   <213> Homo sapiens
<400> 125
   agaagggcta tcaggccagc cttcagagga ctccaaggaa cattcaacgc tgtcggtgag 60
   tttgggattt gaaaaaacca ctgaccgttg actgtacctt ggggtcctta 110
<210> 126
   <211> 110
   <212> DNA
   <213> Homo sapiens
<400> 126
   cggaaaattt gccaagggtt tgggggaaca ttcaacctgt cggtgagttt gggcagctca 60
   ggcaaaccat cgaccgttga gtggaccctg aggcctggaa ttgccatcct 110
<210> 127
   <211> 110
   <212> DNA
   <213> Homo sapiens
<400> 127
   cctgtgcaga gattattttt taaaaggtca caatcaacat tcattgctgt cggtgggttg 60
   aactgtgtgg acaagctcac tgaacaatga atgcaactgt ggccccgctt 110
<210> 128
   <211> 89
   <212> DNA
   <213> Homo sapiens
<400> 128
   ctgatggctg cactcaacat tcattgctgt cggtgggttt gagtctgaat caactcactg 60
   atcaatgaat gcaaactgcg gaccaaaca 89
<210> 129
   <211> 110
   <212> DNA
   <213> Homo sapiens
<400> 129
   gagctgcttg cctccccccg tttttggcaa tggtagaact cacactggtg aggtaacagg 60
   atccggtggt tctagacttg ccaactatgg ggcgaggact cagccggcac 110
<210> 130
   <211> 110
   <212> DNA
   <213> Homo sapiens
<400> 130
   ccgcagagtg tgactcctgt tctgtgtatg gcactggtag aattcactgt gaacagtctc 60
   agtcagtgaa ttaccgaagg gccataaaca gagcagagac agatccacga 110
<210> 131
   <211> 84
   <212> DNA
   <213> Homo sapiens
<400> 131
   ccagtcacgt ccccttatca cttttccagc ccagctttgt gactgtaagt gttggacgga      60
   gaactgataa gggtaggtga ttga 84
<210> 132
   <211> 82
   <212> DNA
   <213> Homo sapiens
<400> 132
   agggggcgag ggattggaga gaaaggcagt tcctgatggt cccctcccca ggggctggct 60
   ttcctctggt ccttccctcc ca 82
<210> 133
   <211> 86
   <212> DNA
   <213> Homo sapiens
<400> 133
   tgcttgtaac tttccaaaga attctccttt tgggctttct ggttttattt taagcccaaa 60
   ggtgaatttt ttgggaagtt tgagct 86
<210> 134
   <211> 109
   <212> DNA
   <213> Homo sapiens
<400> 134
   ggtcgggctc accatgacac agtgtgagac ctcgggctac aacacaggac ccgggcgctg 60
   ctctgacccc tcgtgtcttg tgttgcagcc ggagggacgc aggtccgca 109
<210> 135
   <211> 86
   <212> DNA
   <213> Homo sapiens
<400> 135
   tgctccctct ctcacatccc ttgcatggtg gagggtgagc tttctgaaaa cccctcccac 60
   atgcagggtt tgcaggatgg cgagcc 86
<210> 136
   <211> 85
   <212> DNA
   <213> Homo sapiens
<400> 136
   tgcaggcctc tgtgtgatat gtttgatata ttaggttgtt atttaatcca actatatatc 60
   aaacatattc ctacagtgtc ttgcc 85
<210> 137
   <211> 92
   <212> DNA
   <213> Homo sapiens
<400> 137
   cggctggaca gcgggcaacg gaatcccaaa agcagctgtt gtctccagag cattccagct 60
   gcgcttggat ttcgtcccct gctctcctgc ct 92
<210> 138
   <211> 110
   <212> DNA
   <213> Homo sapiens
<400> 138
   gccgagaccg agtgcacagg gctctgacct atgaattgac agccagtgct ctcgtctccc 60
   ctctggctgc caattccata ggtcacaggt atgttcgcct caatgccagc 110
<210> 139
   <211> 88
   <212> DNA
   <213> Homo sapiens
<400> 139
   cgaggatggg agctgagggc tgggtctttg cgggcgagat gagggtgtcg gatcaactgg 60
   cctacaaagt cccagttctc ggcccccg 88
<210> 140
   <211> 85
   <212> DNA
   <213> Homo sapiens
<400> 140
   atggtgttat caagtgtaac agcaactcca tgtggactgt gtaccaattt ccagtggaga 60
   tgctgttact tttgatggtt accaa 85
<210> 141
   <211> 85
   <212> DNA
   <213> Homo sapiens
<400> 141
   tggttcccgc cccctgtaac agcaactcca tgtggaagtg cccactggtt ccagtggggc 60
   tgctgttatc tggggcgagg gccag 85
<210> 142
   <211> 87
   <212> DNA
   <213> Homo sapiens
<400> 142
   agcttccctg gctctagcag cacagaaata ttggcacagg gaagcgagtc tgccaatatt 60
   ggctgtgctg ctccaggcag ggtggtg 87
<210> 143
   <211> 70
   <212> DNA
   <213> Homo sapiens
<400> 143
   gtgaattagg tagtttcatg ttgttgggcc tgggtttctg aacacaacaa cattaaacca 60
   cccgattcac 70
<210> 144
   <211> 110
   <212> DNA
   <213> Homo sapiens
<400> 144
   tgctcgctca gctgatctgt ggcttaggta gtttcatgtt gttgggattg agttttgaac 60
   tcggcaacaa gaaactgcct gagttacatc agtcggtttt cgtcgagggc 110
<210> 145
   <211> 84
   <212> DNA
   <213> Homo sapiens
<400> 145
   actggtcggt gatttaggta gtttcctgtt gttgggatcc acctttctct cgacagcacg 60
   acactgcctt cattacttca gttg 84
<210> 146
   <211> 75
   <212> DNA
   <213> Homo sapiens
<400> 146
   ggctgtgccg ggtagagagg gcagtgggag gtaagagctc ttcacccttc accaccttct 60
   ccacccagca tggcc 75
<210> 147
   <211> 62
   <212> DNA
   <213> Homo sapiens
<400> 147
   tcattggtcc agaggggaga taggttcctg tgatttttcc ttcttctcta tagaataaat 60
   ga 62
<210> 148
   <211> 71
   <212> DNA
   <213> Homo sapiens
<400> 148
   gccaacccag tgttcagact acctgttcag gaggctctca atgtgtacag tagtctgcac 60
   attggttagg c 71
<210> 149
   <211> 110
   <212> DNA
   <213> Homo sapiens
<400> 149
   aggaagcttc tggagatcct gctccgtcgc cccagtgttc agactacctg ttcaggacaa 60
   tgccgttgta cagtagtctg cacattggtt agactgggca agggagagca 110
<210> 150
   <211> 110
   <212> DNA
   <213> Homo sapiens
<400> 150
   ccagaggaca cctccactcc gtctacccag tgtttagact atctgttcag gactcccaaa 60
   ttgtacagta gtctgcacat tggttaggct gggctgggtt agaccctcgg 110
<210> 151
   <211> 90
   <212> DNA
   <213> Homo sapiens
<400> 151
   ccgggcccct gtgagcatct taccggacag tgctggattt cccagcttga ctctaacact 60
   gtctggtaac gatgttcaaa ggtgacccgc 90
<210> 152
   <211> 95
   <212> DNA
   <213> Homo sapiens
<400> 152
   ccagctcggg cagccgtggc catcttactg ggcagcattg gatggagtca ggtctctaat 60
   actgcctggt aatgatgacg gcggagccct gcacg 95
<210> 153
   <211> 68
   <212> DNA
   <213> Homo sapiens
<400> 153
   ccctcgtctt acccagcagt gtttgggtgc ggttgggagt ctctaatact gccgggtaat 60
   gatggagg 68
<210> 154
   <211> 110
   <212> DNA
   <213> Homo sapiens
<400> 154
   gtgttgggga ctcgcgcgct gggtccagtg gttcttaaca gttcaacagt tctgtagcgc 60
   aattgtgaaa tgtttaggac cactagaccc ggcgggcgcg gcgacagcga 110
<210> 155
   <211> 110
   <212> DNA
   <213> Homo sapiens
<400> 155
   ggctacagtc tttcttcatg tgactcgtgg acttcccttt gtcatcctat gcctgagaat 60
   atatgaagga ggctgggaag gcaaagggac gttcaattgt catcactggc 110
<210> 156
   <211> 110
   <212> DNA
   <213> Homo sapiens
<400> 156
   aaagatcctc agacaatcca tgtgcttctc ttgtccttca ttccaccgga gtctgtctca 60
   tacccaacca gatttcagtg gagtgaagtt caggaggcat ggagctgaca 110
<210> 157
   <211> 86
   <212> DNA
   <213> Homo sapiens
<400> 157
   tgcttcccga ggccacatgc ttctttatat ccccatatgg attactttgc tatggaatgt 60
   aaggaagtgt gtggtttcgg caagtg 86
<210> 158
   <211> 71
   <212> DNA
   <213> Homo sapiens
<400> 158
   tgacgggcga gcttttggcc cgggttatac ctgatgctca cgtataagac gagcaaaaag 60
   cttgttggtc a 71
<210> 159
   <211> 110
   <212> DNA
   <213> Homo sapiens
<400> 159
   acccggcagt gcctccaggc gcagggcagc ccctgcccac cgcacactgc gctgccccag 60
   acccactgtg cgtgtgacag cggctgatct gtgcctgggc agcgcgaccc 110
<210> 160
   <211> 110
   <212> DNA
   <213> Homo sapiens
<400> 160
   tcacctggcc atgtgacttg tgggcttccc tttgtcatcc ttcgcctagg gctctgagca 60
   gggcagggac agcaaagggg tgctcagttg tcacttccca cagcacggag 110
<210> 161
   <211> 110
   <212> DNA
   <213> Homo sapiens
<400> 161
   cggggcaccc cgcccggaca gcgcgccggc accttggctc tagactgctt actgcccggg 60
   ccgccctcag taacagtctc cagtcacggc caccgacgcc tggccccgcc 110
<210> 162
   <211> 110
   <212> DNA
   <213> Homo sapiens
<400> 162
   tgagttttga ggttgcttca gtgaacattc aacgctgtcg gtgagtttgg aattaaaatc 60
   aaaaccatcg accgttgatt gtaccctatg gctaaccatc atctactcca 110
<210> 163
   <211> 110
   <212> DNA
   <213> Homo sapiens
<400> 163
   ggcctggctg gacagagttg tcatgtgtct gcctgtctac acttgctgtg cagaacatcc 60
   gctcacctgt acagcaggca cagacaggca gtcacatgac aacccagcct 110
<210> 164
   <211> 110
   <212> DNA
   <213> Homo sapiens
<400> 164
   atcattcaga aatggtatac aggaaaatga cctatgaatt gacagacaat atagctgagt 60
   ttgtctgtca tttctttagg ccaatattct gtatgactgt gctacttcaa 110
<210> 165
   <211> 110
   <212> DNA
   <213> Homo sapiens
<400> 165
   gatggctgtg agttggctta atctcagctg gcaactgtga gatgttcata caatccctca 60
   cagtggtctc tgggattatg ctaaacagag caatttccta gccctcacga 110
<210> 166
   <211> 110
   <212> DNA
   <213> Homo sapiens
<400> 166
   agtataatta ttacatagtt tttgatgtcg cagatactgc atcaggaact gattggataa 60
   gaatcagtca ccatcagttc ctaatgcatt gccttcagca tctaaacaag 110
<210> 167
   <211> 110
   <212> DNA
   <213> Homo sapiens
<400> 167
   gtgataatgt agcgagattt tctgttgtgc ttgatctaac catgtggttg cgaggtatga 60
   gtaaaacatg gttccgtcaa gcaccatgga acgtcacgca gctttctaca 110
<210> 168
   <211> 110
   <212> DNA
   <213> Homo sapiens
<400> 168
   gaccagtcgc tgcggggctt tcctttgtgc ttgatctaac catgtggtgg aacgatggaa 60
   acggaacatg gttctgtcaa gcaccgcgga aagcaccgtg ctctcctgca 110
<210> 169
   <211> 110
   <212> DNA
   <213> Homo sapiens
<400> 169
   ccgccccggg ccgcggctcc tgattgtcca aacgcaattc tcgagtctat ggctccggcc 60
   gagagttgag tctggacgtc ccgagccgcc gcccccaaac ctcgagcggg 110
<210> 170
   <211> 97
   <212> DNA
   <213> Homo sapiens
<400> 170
   actcaggggc ttcgccactg attgtccaaa cgcaattctt gtacgagtct gcggccaacc 60
   gagaattgtg gctggacatc tgtggctgag ctccggg 97
<210> 171
   <211> 110
   <212> DNA
   <213> Homo sapiens
<400> 171
   gacagtgtgg cattgtaggg ctccacaccg tatctgacac tttgggcgag ggcaccatgc 60
   tgaaggtgtt catgatgcgg tctgggaact cctcacggat cttactgatg 110
<210> 172
   <211> 110
   <212> DNA
   <213> Homo sapiens
<400> 172
   tgaacatcca ggtctggggc atgaacctgg catacaatgt agatttctgt gttcgttagg 60
   caacagctac attgtctgct gggtttcagg ctacctggaa acatgttctc 110
<210> 173
   <211> 110
   <212> DNA
   <213> Homo sapiens
<400> 173
   gctgctggaa ggtgtaggta ccctcaatgg ctcagtagcc agtgtagatc ctgtctttcg 60
   taatcagcag ctacatctgg ctactgggtc tctgatggca tcttctagct 110
<210> 174
   <211> 110
   <212> DNA
   <213> Homo sapiens
<400> 174
   cctggcctcc tgcagtgcca cgctccgtgt atttgacaag ctgagttgga cactccatgt 60
   ggtagagtgt cagtttgtca aataccccaa gtgcggcaca tgcttaccag 110
<210> 175
   <211> 81
   <212> DNA
   <213> Homo sapiens
<400> 175
   gggctttcaa gtcactagtg gttccgttta gtagatgatt gtgcattgtt tcaaaatggt 60
   gccctagtga ctacaaagcc c 81
<210> 176
   <211> 80
   <212> DNA
   <213> Homo sapiens
<400> 176
   aggacccttc cagagggccc cccctcaatc ctgttgtgcc taattcagag ggttgggtgg 60
   aggctctcct gaagggctct 80
<210> 177
   <211> 63
   <212> DNA
   <213> Homo sapiens
<400> 177
   aagaaatggt ttaccgtccc acatacattt tgaatatgta tgtgggatgg taaaccgctt 60
   ctt 63
<210> 178
   <211> 86
   <212> DNA
   <213> Homo sapiens
<400> 178
   actgctaacg aatgctctga ctttattgca ctactgtact ttacagctag cagtgcaata 60
   gtattgtcaa agcatctgaa agcagg 86
<210> 179
   <211> 69
   <212> DNA
   <213> Homo sapiens
<400> 179
   ccaccactta aacgtggatg tacttgcttt gaaactaaag aagtaagtgc ttccatgttt 60
   tggtgatgg 69
<210> 180
   <211> 73
   <212> DNA
   <213> Homo sapiens
<400> 180
   gctcccttca actttaacat ggaagtgctt tctgtgactt taaaagtaag tgcttccatg 60
   ttttagtagg agt 73
<210> 181
   <211> 68
   <212> DNA
   <213> Homo sapiens
<400> 181
   cctttgcttt aacatggggg tacctgctgt gtgaaacaaa agtaagtgct tccatgtttc 60
   agtggagg 68
<210> 182
   <211> 68
   <212> DNA
   <213> Homo sapiens
<400> 182
   cctctacttt aacatggagg cacttgctgt gacatgacaa aaataagtgc ttccatgttt 60
   gagtgtgg 68
<210> 183
   <211> 82
   <212> DNA
   <213> Homo sapiens
<400> 183
   gcttcgctcc cctccgcctt ctcttcccgg ttcttcccgg agtcgggaaa agctgggttg 60
   agagggcgaa aaaggatgag gt 82
<210> 184
   <211> 59
   <212> DNA
   <213> Homo sapiens
<400> 184
   ttggcctcct aagccaggga ttgtgggttc gagtccccac cggggtaaag aaaggccga 59
<210> 185
   <211> 86
   <212> DNA
   <213> Homo sapiens
<400> 185
   ttggtacttg gagagaggtg gtccgtggcg cgttcgcttt atttatggcg cacattacac 60
   ggtcgacctc tttgcagtat ctaatc 86
<210> 186
   <211> 83
   <212> DNA
   <213> Homo sapiens
<400> 186
   ctgactatgc ctccccgcat cccctagggc attggtgtaa agctggagac ccactgcccc 60
   aggtgctgct gggggttgta gtc 83
<210> 187
   <211> 98
   <212> DNA
   <213> Homo sapiens
<400> 187
   atacagtgct tggttcctag taggtgtcca gtaagtgttt gtgacataat ttgtttattg 60
   aggacctcct atcaatcaag cactgtgcta ggctctgg 98
<210> 188
   <211> 95
   <212> DNA
   <213> Homo sapiens
<400> 188
   ctcatctgtc tgttgggctg gaggcagggc ctttgtgaag gcgggtggtg ctcagatcgc 60
   ctctgggccc ttcctccagc cccgaggcgg attca 95
<210> 189
   <211> 75
   <212> DNA
   <213> Homo sapiens
<400> 189
   tggagtgggg gggcaggagg ggctcaggga gaaagtgcat acagcccctg gccctctctg 60
   cccttccgtc ccctg 75
<210> 190
   <211> 94
   <212> DNA
   <213> Homo sapiens
<400> 190
   ctttggcgat cactgcctct ctgggcctgt gtcttaggct ctgcaagatc aaccgagcaa 60
   agcacacggc ctgcagagag gcagcgctct gccc 94
<210> 191
   <211> 94
   <212> DNA
   <213> Homo sapiens
<400> 191
   gagtttggtt ttgtttgggt ttgttctagg tatggtccca gggatcccag atcaaaccag 60
   gcccctgggc ctatcctaga accaacctaa gctc 94
<210> 192
   <211> 94
   <212> DNA
   <213> Homo sapiens
<400> 192
   tgttttgagc gggggtcaag agcaataacg aaaaatgttt gtcataaacc gtttttcatt 60
   attgctcctg acctcctctc atttgctata ttca 94
<210> 193
   <211> 93
   <212> DNA
   <213> Homo sapiens
<400> 193
   gtagtcagta gttggggggt gggaacggct tcatacagga gttgatgcac agttatccag 60
   ctcctatatg atgcctttct tcatcccctt caa 93
<210> 194
   <211> 67
   <212> DNA
   <213> Homo sapiens
<400> 194
   tctccaacaa tatcctggtg ctgagtgatg actcaggcga ctccagcatc agtgattttg 60
   ttgaaga 67
<210> 195
   <211> 94
   <212> DNA
   <213> Homo sapiens
<400> 195
   cggggcggcc gctctccctg tcctccagga gctcacgtgt gcctgcctgt gagcgcctcg 60
   acgacagagc cggcgcctgc cccagtgtct gcgc 94
<210> 196
   <211> 95
   <212> DNA
   <213> Homo sapiens
<400> 196
   ttgtacctgg tgtgattata aagcaatgag actgattgtc atatgtcgtt tgtgggatcc 60
   gtctcagtta ctttatagcc atacctggta tctta 95
<210> 197
   <211> 99
   <212> DNA
   <213> Homo sapiens
<400> 197
   gaaactgggc tcaaggtgag gggtgctatc tgtgattgag ggacatggtt aatggaattg 60
   tctcacacag aaatcgcacc cgtcaccttg gcctactta 99
<210> 198
   <211> 98
   <212> DNA
   <213> Homo sapiens
<400> 198
   acccaaaccc taggtctgct gactcctagt ccagggctcg tgatggctgg tgggccctga 60
   acgaggggtc tggaggcctg ggtttgaata tcgacagc 98
<210> 199
   <211> 86
   <212> DNA
   <213> Homo sapiens
<400> 199
   gtctgtctgc ccgcatgcct gcctctctgt tgctctgaag gaggcagggg ctgggcctgc 60
   agctgcctgg gcagagcggc tcctgc 86
<210> 200
   <211> 72
   <212> DNA
   <213> Homo sapiens
<400> 200
   ggagcttatc agaatctcca ggggtacttt ataatttcaa aaagtccccc aggtgtgatt 60
   ctgatttgct tc 72
<210> 201
   <211> 68
   <212> DNA
   <213> Homo sapiens
<400> 201
   ccattactgt tgctaatatg caactctgtt gaatataaat tggaattgca ctttagcaat 60
   ggtgatgg 68
<210> 202
   <211> 66
   <212> DNA
   <213> Homo sapiens
<400> 202
   aaaaggtgga tattccttct atgtttatgt tatttatggt taaacataga ggaaattcca 60
   cgtttt 66
<210> 203
   <211> 70
   <212> DNA
   <213> Homo sapiens
<400> 203
   ttgaagggag atcgaccgtg ttatattcgc tttattgact tcgaataata catggttgat 60
   cttttctcag 70
<210> 204
   <211> 75
   <212> DNA
   <213> Homo sapiens
<400> 204
   agacagagaa gccaggtcac gtctctgcag ttacacagct cacgagtgcc tgctggggtg 60
   gaacctggtc tgtct 75
<210> 205
   <211> 67
   <212> DNA
   <213> Homo sapiens
<400> 205
   gtggcactca aactgtgggg gcactttctg ctctctggtg aaagtgccgc catcttttga 60
   gtgttac 67
<210> 206
   <211> 67
   <212> DNA
   <213> Homo sapiens
<400> 206
   gtgggcctca aatgtggagc actattctga tgtccaagtg gaaagtgctg cgacatttga 60
   gcgtcac 67
<210> 207
   <211> 69
   <212> DNA
   <213> Homo sapiens
<400> 207
   gggatactca aaatgggggc gctttccttt ttgtctgtac tgggaagtgc ttcgattttg 60
   gggtgtccc 69
<210> 208
   <211> 72
   <212> DNA
   <213> Homo sapiens
<400> 208
   tacatcggcc attataatac aacctgataa gtgttatagc acttatcaga ttgtattgta 60
   attgtctgtg ta 72
<210> 209
   <211> 64
   <212> DNA
   <213> Homo sapiens
<400> 209
   ccccgcgacg agcccctcgc acaaaccgga cctgagcgtt ttgttcgttc ggctcgcgtg 60
   aggc 64
<210> 210
   <211> 68
   <212> DNA
   <213> Homo sapiens
<400> 210
   taaaaggtag attctccttc tatgagtaca ttatttatga ttaatcatag aggaaaatcc 60
   acgttttc 68
<210> 211
   <211> 69
   <212> DNA
   <213> Homo sapiens
<400> 211
   ttgagcagag gttgcccttg gtgaattcgc tttatttatg ttgaatcaca caaaggcaac 60
   ttttgtttg 69
<210> 212
   <211> 66
   <212> DNA
   <213> Homo sapiens
<400> 212
   agggctcctg actccaggtc ctgtgtgtta cctagaaata gcactggact tggagtcaga 60
   aggcct 66
<210> 213
   <211> 67
   <212> DNA
   <213> Homo sapiens
<400> 213
   agagatggta gactatggaa cgtaggcgtt atgatttctg acctatgtaa catggtccac 60
   taactct 67
<210> 214
   <211> 61
   <212> DNA
   <213> Homo sapiens
<400> 214
   aagatggttg accatagaac atgcgctatc tctgtgtcgt atgtaatatg gtccacatct 60
   t 61
<210> 215
   <211> 75
   <212> DNA
   <213> Homo sapiens
<400> 215
   tacttaaagc gaggttgccc tttgtatatt cggtttattg acatggaata tacaagggca 60
   agctctctgt gagta 75
<210> 216
   <211> 76
   <212> DNA
   <213> Homo sapiens
<400> 216
   tacttgaaga gaagttgttc gtggtggatt cgctttactt atgacgaatc attcacggac 60
   aacacttttt tcagta 76
<210> 217
   <211> 73
   <212> DNA
   <213> Homo sapiens
<400> 217
   ctcctcagat cagaaggtga ttgtggcttt gggtggatat taatcagcca cagcactgcc 60
   tggtcagaaa gag 73
<210> 218
   <211> 88
   <212> DNA
   <213> Homo sapiens
<400> 218
   tgttaaatca ggaattttaa acaattccta gacaatatgt ataatgttca taagtcattc 60
   ctagaaattg ttcataatgc ctgtaaca 88
<210> 219
   <211> 68
   <212> DNA
   <213> Homo sapiens
<400> 219
   cagggctcct gactccaggt cctgtgtgtt acctagaaat agcactggac ttggagtcag 60
   aaggcctg 68
<210> 220
   <211> 94
   <212> DNA
   <213> Homo sapiens
<400> 220
   ataaaggaag ttaggctgag gggcagagag cgagactttt ctattttcca aaagctcggt 60
   ctgaggcccc tcagtcttgc ttcctaaccc gcgc 94
<210> 221
   <211> 98
   <212> DNA
   <213> Homo sapiens
<400> 221
   cgaggggata cagcagcaat tcatgttttg aagtgttcta aatggttcaa aacgtgaggc 60
   gctgctatac cccctcgtgg ggaaggtaga aggtgggg 98
<210> 222
   <211> 87
   <212> DNA
   <213> Homo sapiens
<400> 222
   gaaagcgctt tggaatgaca cgatcactcc cgttgagtgg gcacccgaga agccatcggg 60
   aatgtcgtgt ccgcccagtg ctctttc 87
<210> 223
   <211> 62
   <212> RNA
   <213> Homo sapiens
<400> 223
   uaaggugcau cuagugcaga uagugaagua gauuagcauc uacugcccua agugcuccuu 60
   cu 62
<210> 224
   <211> 22
   <212> RNA
   <213> Homo sapiens
<400> 224
   uaaggugcau cuagugcaga ua 22
<210> 225
   <211> 17
   <212> DNA
   <213> Artificial Sequence
<220>
   <223> Description of Artificial Sequence: Synthetic primer
<400> 225
   ctcgcttcgg cagcaca 17
<210> 226
   <211> 23
   <212> DNA
   <213> Artificial Sequence
<220>
   <223> Description of Artificial Sequence: Synthetic primer
<400> 226
   aaacatactt ctttatatgc cca 23
<210> 227
   <211> 25
   <212> DNA
   <213> Artificial Sequence
<220>
   <223> Description of Artificial Sequence: Synthetic primer
<400> 227
   acatacttct ttatgtaccc atatg 25
<210> 228
   <211> 23
   <212> DNA
   <213> Artificial Sequence
<220>
   <223> Description of Artificial Sequence: Synthetic primer
<400> 228
   tggaagacta gtgattttgt tgt 23
<210> 229
   <211> 23
   <212> DNA
   <213> Artificial Sequence
<220>
   <223> Description of Artificial Sequence: Synthetic primer
<400> 229
   tggaagacta gtgattttgt tgt 23
<210> 230
   <211> 23
   <212> DNA
   <213> Artificial Sequence
<220>
   <223> Description of Artificial Sequence: Synthetic primer
<400> 230
   tggaagacta gtgattttgt tgt 23
<210> 231
   <211> 18
   <212> DNA
   <213> Artificial Sequence
<220>
   <223> Description of Artificial Sequence: Synthetic primer
<400> 231
   ggaggctgcg tggaagag 18
<210> 232
   <211> 19
   <212> DNA
   <213> Artificial Sequence
<220>
   <223> Description of Artificial Sequence: Synthetic primer
<400> 232
   ctggagtctg gcaagagga 19
<210> 233
   <211> 17
   <212> DNA
   <213> Artificial Sequence
<220>
   <223> Description of Artificial Sequence: Synthetic primer
<400> 233
   cagcggcact ggctaag 17
<210> 234
   <211> 23
   <212> DNA
   <213> Artificial Sequence
<220>
   <223> Description of Artificial Sequence: Synthetic primer
<400> 234
   taccctgtag atccgaattt gtg 23
<210> 235
   <211> 21
   <212> DNA
   <213> Artificial Sequence
<220>
   <223> Description of Artificial Sequence: Synthetic primer
<400> 235
   cagcacataa tggtttgtgg a 21
<210> 236
   <211> 21
   <212> DNA
   <213> Artificial Sequence
<220>
   <223> Description of Artificial Sequence: Synthetic primer
<400> 236
   agcacatcat ggtttacatg c 21
<210> 237
   <211> 21
   <212> DNA
   <213> Artificial Sequence
<220>
   <223> Description of Artificial Sequence: Synthetic primer
<400> 237
   gcagcacgta aatattggcg t 21
<210> 238
   <211> 21
   <212> DNA
   <213> Artificial Sequence
<220>
   <223> Description of Artificial Sequence: Synthetic primer
<400> 238
   gcacgtaaat attggcgtag t 21
<210> 239
   <211> 23
   <212> DNA
   <213> Artificial Sequence
<220>
   <223> Description of Artificial Sequence: Synthetic primer
<400> 239
   gcaggaaaaa agagaacatc acc 23
<210> 240
   <211> 23
   <212> DNA
   <213> Artificial Sequence
<220>
   <223> Description of Artificial Sequence: Synthetic primer
<400> 240
   taaggtgcat ctagtgcaga tag 23
<210> 241
   <211> 20
   <212> DNA
   <213> Artificial Sequence
<220>
   <223> Description of Artificial Sequence: Synthetic primer
<400> 241
   ccaataattc aagccaagca 20
<210> 242
   <211> 17
   <212> DNA
   <213> Artificial Sequence
<220>
   <223> Description of Artificial Sequence: Synthetic primer
<400> 242
   cctgtcgccc aatcaaa 17
<210> 243
   <211> 21
   <212> DNA
   <213> Artificial Sequence
<220>
   <223> Description of Artificial Sequence: Synthetic primer
<400> 243
   ggcacttcca gtactcttgg a 21
<210> 244
   <211> 23
   <212> DNA
   <213> Artificial Sequence
<220>
   <223> Description of Artificial Sequence: Synthetic primer
<400> 244
   gcactaaagt gcttatagtg cag 23
<210> 245
   <211> 23
   <212> DNA
   <213> Artificial Sequence
<220>
   <223> Description of Artificial Sequence: Synthetic primer
<400> 245
   gcttatcaga ctgatgttga ctg 23
<210> 246
   <211> 17
   <212> DNA
   <213> Artificial Sequence
<220>
   <223> Description of Artificial Sequence: Synthetic primer
<400> 246
   agcaacatgc cctgctc 17
<210> 247
   <211> 17
   <212> DNA
   <213> Artificial Sequence
<220>
   <223> Description of Artificial Sequence: Synthetic primer
<400> 247
   ctggggttcc tggggat 17
<210> 248
   <211> 19
   <212> DNA
   <213> Artificial Sequence
<220>
   <223> Description of Artificial Sequence: Synthetic primer
<400> 248
   aagcccagtg tgtgcagac 19
<210> 249
   <211> 19
   <212> DNA
   <213> Artificial Sequence
<220>
   <223> Description of Artificial Sequence: Synthetic primer
<400> 249
   ctcccgtgcc tactgagct 19
<210> 250
   <211> 18
   <212> DNA
   <213> Artificial Sequence
<220>
   <223> Description of Artificial Sequence: Synthetic primer
<400> 250
   tgagaggcgg agacttgg 18
<210> 251
   <211> 24
   <212> DNA
   <213> Artificial Sequence
<220>
   <223> Description of Artificial Sequence: Synthetic primer
<400> 251
   ttcaagtaat ccaggatagg ctgt 24
<210> 252
   <211> 24
   <212> DNA
   <213> Artificial Sequence
<220>
   <223> Description of Artificial Sequence: Synthetic primer
<400> 252
   ttcaagtaat ccaggatagg ctgt 24
<210> 253
   <211> 18
   <212> DNA
   <213> Artificial Sequence
<220>
   <223> Description of Artificial Sequence: Synthetic primer
<400> 253
   gcagggctta gctgcttg 18
<210> 254
   <211> 25
   <212> DNA
   <213> Artificial Sequence
<220>
   <223> Description of Artificial Sequence: Synthetic primer
<400> 254
   ggagctcaca gtctattgag ttacc 25
<210> 255
   <211> 20
   <212> DNA
   <213> Artificial Sequence
<220>
   <223> Description of Artificial Sequence: Synthetic primer
<400> 255
   atgactgatt tcttttggtg 20
<210> 256
   <211> 20
   <212> DNA
   <213> Artificial Sequence
<220>
   <223> Description of Artificial Sequence: Synthetic primer
<400> 256
   tggtttcata tggtggttta 20
<210> 257
   <211> 23
   <212> DNA
   <213> Artificial Sequence
<220>
   <223> Description of Artificial Sequence: Synthetic primer
<400> 257
   gtaaacatcc tcgactggaa gct 23
<210> 258
   <211> 21
   <212> DNA
   <213> Artificial Sequence
<220>
   <223> Description of Artificial Sequence: Synthetic primer
<400> 258
   aggttaaccc aacagaaggc t 21
<210> 259
   <211> 17
   <212> DNA
   <213> Artificial Sequence
<220>
   <223> Description of Artificial Sequence: Synthetic primer
<400> 259
   cctcactgcg tctccgt 17
<210> 260
   <211> 24
   <212> DNA
   <213> Artificial Sequence
<220>
   <223> Description of Artificial Sequence: Synthetic primer
<400> 260
   catgtaaaca tcctacactc agct 24
<210> 261
   <211> 19
   <212> DNA
   <213> Artificial Sequence
<220>
   <223> Description of Artificial Sequence: Synthetic primer
<400> 261
   gtgaatgctg tgcctgttc 19
<210> 262
   <211> 24
   <212> DNA
   <213> Artificial Sequence
<220>
   <223> Description of Artificial Sequence: Synthetic primer
<400> 262
   tgtgtaaaca tcctacactc tcag 24
<210> 263
   <211> 18
   <212> DNA
   <213> Artificial Sequence
<220>
   <223> Description of Artificial Sequence: Synthetic primer
<400> 263
   cagtggtcag gggctgat 18
<210> 264
   <211> 19
   <212> DNA
   <213> Artificial Sequence
<220>
   <223> Description of Artificial Sequence: Synthetic primer
<400> 264
   gactgccaac cccatccta 19
<210> 265
   <211> 21
   <212> DNA
   <213> Artificial Sequence
<220>
   <223> Description of Artificial Sequence: Synthetic primer
<400> 265
   gttgttgtaa acatccccga c 21
<210> 266
   <211> 20
   <212> DNA
   <213> Artificial Sequence
<220>
   <223> Description of Artificial Sequence: Synthetic primer
<400> 266
   gctgaagatg atgactggca 20
<210> 267
   <211> 20
   <212> DNA
   <213> Artificial Sequence
<220>
   <223> Description of Artificial Sequence: Synthetic primer
<400> 267
   tgagtgtgtt ttccctccct 20
<210> 268
   <211> 23
   <212> DNA
   <213> Artificial Sequence
<220>
   <223> Description of Artificial Sequence: Synthetic primer
<400> 268
   gcacattact aagttgcatg ttg 23
<210> 269
   <211> 20
   <212> DNA
   <213> Artificial Sequence
<220>
   <223> Description of Artificial Sequence: Synthetic primer
<400> 269
   tgtggtgcat tgtagttgca 20
<210> 270
   <211> 21
   <212> DNA
   <213> Artificial Sequence
<220>
   <223> Description of Artificial Sequence: Synthetic primer
<400> 270
   tggcagtgtc ttagctggtt g 21
<210> 271
   <211> 23
   <212> DNA
   <213> Artificial Sequence
<220>
   <223> Description of Artificial Sequence: Synthetic primer
<400> 271
   gcagtgtcat tagctgattg tac 23
<210> 272
   <211> 24
   <212> DNA
   <213> Artificial Sequence
<220>
   <223> Description of Artificial Sequence: Synthetic primer
<400> 272
   gcagtgtagt tagctgattg ctaa 24
<210> 273
   <211> 20
   <212> DNA
   <213> Artificial Sequence
<220>
   <223> Description of Artificial Sequence: Synthetic primer
<400> 273
   tctacacagg ttgggatcgg 20
<210> 274
   <211> 20
   <212> DNA
   <213> Artificial Sequence
<220>
   <223> Description of Artificial Sequence: Synthetic primer
<400> 274
   atgcgtatct ccagcactca 20
<210> 275
   <211> 19
   <212> DNA
   <213> Artificial Sequence
<220>
   <223> Description of Artificial Sequence: Synthetic primer
<400> 275
   aagtgctgtt cgtgcaggt 19
<210> 276
   <211> 23
   <212> DNA
   <213> Artificial Sequence
<220>
   <223> Description of Artificial Sequence: Synthetic primer
<400> 276
   ggcactcaat aaatgtctgt tga 23
<210> 277
   <211> 17
   <212> DNA
   <213> Artificial Sequence
<220>
   <223> Description of Artificial Sequence: Synthetic primer
<400> 277
   agagagcccg caccagt 17
<210> 278
   <211> 24
   <212> DNA
   <213> Artificial Sequence
<220>
   <223> Description of Artificial Sequence: Synthetic primer
<400> 278
   ggtagtaagt tgtattgttg tggg 24
<210> 279
   <211> 22
   <212> DNA
   <213> Artificial Sequence
<220>
   <223> Description of Artificial Sequence: Synthetic primer
<400> 279
   taaacccgta gatccgatct tg 22
<210> 280
   <211> 18
   <212> DNA
   <213> Artificial Sequence
<220>
   <223> Description of Artificial Sequence: Synthetic primer
<400> 280
   cccacccgta gaaccgac 18
<210> 281
   <211> 20
   <212> DNA
   <213> Artificial Sequence
<220>
   <223> Description of Artificial Sequence: Synthetic primer
<400> 281
   aacccgtaga tccgaacttg 20
<210> 282
   <211> 20
   <212> DNA
   <213> Artificial Sequence
<220>
   <223> Description of Artificial Sequence: Synthetic primer
<400> 282
   gccctggctc agttatcaca 20
<210> 283
   <211> 20
   <212> DNA
   <213> Artificial Sequence
<220>
   <223> Description of Artificial Sequence: Synthetic primer
<400> 283
   ttttcggtta tcatggtacc 20
<210> 284
   <211> 20
   <212> DNA
   <213> Artificial Sequence
<220>
   <223> Description of Artificial Sequence: Synthetic primer
<400> 284
   gcttctttac agtgctgcct 20
<210> 285
   <211> 22
   <212> DNA
   <213> Artificial Sequence
<220>
   <223> Description of Artificial Sequence: Synthetic primer
<400> 285
   caaatgctca gactcctgtg gt 22
<210> 286
   <211> 20
   <212> DNA
   <213> Artificial Sequence
<220>
   <223> Description of Artificial Sequence: Synthetic primer
<400> 286
   ctgcatggat ctgtgaggac 20
<210> 287
   <211> 25
   <212> DNA
   <213> Artificial Sequence
<220>
   <223> Description of Artificial Sequence: Synthetic primer
<400> 287
   taaagtgctg acagtgcaga tagtg 25
<210> 288
   <211> 22
   <212> DNA
   <213> Artificial Sequence
<220>
   <223> Description of Artificial Sequence: Synthetic primer
<400> 288
   cagcttcttt acagtgttgc ct 22
<210> 289
   <211> 22
   <212> DNA
   <213> Artificial Sequence
<220>
   <223> Description of Artificial Sequence: Synthetic primer
<400> 289
   ggatttttag gggcattatg ac 22
<210> 290
   <211> 21
   <212> DNA
   <213> Artificial Sequence
<220>
   <223> Description of Artificial Sequence: Synthetic primer
<400> 290
   ggagtgtgac aatggtgttt g 21
<210> 291
   <211> 18
   <212> DNA
   <213> Artificial Sequence
<220>
   <223> Description of Artificial Sequence: Synthetic primer
<400> 291
   tccgtgttca cagcggac 18
<210> 292
   <211> 20
   <212> DNA
   <213> Artificial Sequence
<220>
   <223> Description of Artificial Sequence: Synthetic primer
<400> 292
   gtccctgaga ccctttaacc 20
<210> 293
   <211> 20
   <212> DNA
   <213> Artificial Sequence
<220>
   <223> Description of Artificial Sequence: Synthetic primer
<400> 293
   gtccctgaga ccctaacttg 20
<210> 294
   <211> 20
   <212> DNA
   <213> Artificial Sequence
<220>
   <223> Description of Artificial Sequence: Synthetic primer
<400> 294
   gtccctgaga ccctaacttg 20
<210> 295
   <211> 21
   <212> DNA
   <213> Artificial Sequence
<220>
   <223> Description of Artificial Sequence: Synthetic primer
<400> 295
   tattactttt ggtacgcgct g 21
<210> 296
   <211> 20
   <212> DNA
   <213> Artificial Sequence
<220>
   <223> Description of Artificial Sequence: Synthetic primer
<400> 296
   agcctgctga agctcagagg 20
<210> 297
   <211> 17
   <212> DNA
   <213> Artificial Sequence
<220>
   <223> Description of Artificial Sequence: Synthetic primer
<400> 297
   tggattcggg gccgtag 17
<210> 298
   <211> 16
   <212> DNA
   <213> Artificial Sequence
<220>
   <223> Description of Artificial Sequence: Synthetic primer
<400> 298
   ggaagggggg ccgata 16
<210> 299
   <211> 18
   <212> DNA
   <213> Artificial Sequence
<220>
   <223> Description of Artificial Sequence: Synthetic primer
<400> 299
   ctttttgcgg tctgggct 18
<210> 300
   <211> 16
   <212> DNA
   <213> Artificial Sequence
<220>
   <223> Description of Artificial Sequence: Synthetic primer
<400> 300
   tgagtgggcc agggac 16
<210> 301
   <211> 22
   <212> DNA
   <213> Artificial Sequence
<220>
   <223> Description of Artificial Sequence: Synthetic primer
<400> 301
   cctgttgcac tactataggc cg 22
<210> 302
   <211> 21
   <212> DNA
   <213> Artificial Sequence
<220>
   <223> Description of Artificial Sequence: Synthetic primer
<400> 302
   aaccgtggct ttcgattgtt a 21
<210> 303
   <211> 22
   <212> DNA
   <213> Artificial Sequence
<220>
   <223> Description of Artificial Sequence: Synthetic primer
<400> 303
   gagctggtaa aatggaacca aa 22
<210> 304
   <211> 19
   <212> DNA
   <213> Artificial Sequence
<220>
   <223> Description of Artificial Sequence: Synthetic primer
<400> 304
   ctggtcaaac ggaaccaag 19
<210> 305
   <211> 20
   <212> DNA
   <213> Artificial Sequence
<220>
   <223> Description of Artificial Sequence: Synthetic primer
<400> 305
   gtgactggtt gaccagaggg 20
<210> 306
   <211> 24
   <212> DNA
   <213> Artificial Sequence
<220>
   <223> Description of Artificial Sequence: Synthetic primer
<400> 306
   ctatggcttt ttattcctat gtga 24
<210> 307
   <211> 19
   <212> DNA
   <213> Artificial Sequence
<220>
   <223> Description of Artificial Sequence: Synthetic primer
<400> 307
   gcttctcgct tccctatga 19
<210> 308
   <211> 23
   <212> DNA
   <213> Artificial Sequence
<220>
   <223> Description of Artificial Sequence: Synthetic primer
<400> 308
   ggactccatt tgttttgatg atg 23
<210> 309
   <211> 19
   <212> DNA
   <213> Artificial Sequence
<220>
   <223> Description of Artificial Sequence: Synthetic primer
<400> 309
   gtgacgggta ttcttgggt 19
<210> 310
   <211> 20
   <212> DNA
   <213> Artificial Sequence
<220>
   <223> Description of Artificial Sequence: Synthetic primer
<400> 310
   cagctggtgt tgtgaatcag 20
<210> 311
   <211> 22
   <212> DNA
   <213> Artificial Sequence
<220>
   <223> Description of Artificial Sequence: Synthetic primer
<400> 311
   ttctacagtg cacgtgtctc ca 22
<210> 312
   <211> 23
   <212> DNA
   <213> Artificial Sequence
<220>
   <223> Description of Artificial Sequence: Synthetic primer
<400> 312
   cagtggtttt accctatggt agg 23
<210> 313
   <211> 23
   <212> DNA
   <213> Artificial Sequence
<220>
   <223> Description of Artificial Sequence: Synthetic primer
<400> 313
   gtccatcttc cagtacagtg ttg 23
<210> 314
   <211> 18
   <212> DNA
   <213> Artificial Sequence
<220>
   <223> Description of Artificial Sequence: Synthetic primer
<400> 314
   ttggagcagg agtcagga 18
<210> 315
   <211> 19
   <212> DNA
   <213> Artificial Sequence
<220>
   <223> Description of Artificial Sequence: Synthetic primer
<400> 315
   tgaggtgcag tgctgcatc 19
<210> 316
   <211> 22
   <212> DNA
   <213> Artificial Sequence
<220>
   <223> Description of Artificial Sequence: Synthetic primer
<400> 316
   gctgggatat catcatatac tg 22
<210> 317
   <211> 20
   <212> DNA
   <213> Artificial Sequence
<220>
   <223> Description of Artificial Sequence: Synthetic primer
<400> 317
   ggatgcagaa gagaactcca 20
<210> 318
   <211> 20
   <212> DNA
   <213> Artificial Sequence
<220>
   <223> Description of Artificial Sequence: Synthetic primer
<400> 318
   ttgagaactg aattccatgg 20
<210> 319
   <211> 23
   <212> DNA
   <213> Artificial Sequence
<220>
   <223> Description of Artificial Sequence: Synthetic primer
<400> 319
   ctaaagacaa catttctgca cac 23
<210> 320
   <211> 21
   <212> DNA
   <213> Artificial Sequence
<220>
   <223> Description of Artificial Sequence: Synthetic primer
<400> 320
   gaggaagaca gcacgtttgg t 21
<210> 321
   <211> 22
   <212> DNA
   <213> Artificial Sequence
<220>
   <223> Description of Artificial Sequence: Synthetic primer
<400> 321
   tctgtctaag tcacccaatc tc 22
<210> 322
   <211> 19
   <212> DNA
   <213> Artificial Sequence
<220>
   <223> Description of Artificial Sequence: Synthetic primer
<400> 322
   ctggctccgt gtcttcact 19
<210> 323
   <211> 21
   <212> DNA
   <213> Artificial Sequence
<220>
   <223> Description of Artificial Sequence: Synthetic primer
<400> 323
   gtctcccaac ccttgtacca g 21
<210> 324
   <211> 21
   <212> DNA
   <213> Artificial Sequence
<220>
   <223> Description of Artificial Sequence: Synthetic primer
<400> 324
   ctcgaggagc tcacagtcta g 21
<210> 325
   <211> 21
   <212> DNA
   <213> Artificial Sequence
<220>
   <223> Description of Artificial Sequence: Synthetic primer
<400> 325
   gcccaggttc tgtgatacac t 21
<210> 326
   <211> 23
   <212> DNA
   <213> Artificial Sequence
<220>
   <223> Description of Artificial Sequence: Synthetic primer
<400> 326
   catttttgtg atctgcagct agt 23
<210> 327
   <211> 20
   <212> DNA
   <213> Artificial Sequence
<220>
   <223> Description of Artificial Sequence: Synthetic primer
<400> 327
   taggttatcc gtgttgcctt 20
<210> 328
   <211> 22
   <212> DNA
   <213> Artificial Sequence
<220>
   <223> Description of Artificial Sequence: Synthetic primer
<400> 328
   gttaatgcta atcgtgatag gg 22
<210> 329
   <211> 19
   <212> DNA
   <213> Artificial Sequence
<220>
   <223> Description of Artificial Sequence: Synthetic primer
<400> 329
   aacattcaac gctgtcggt 19
<210> 330
   <211> 19
   <212> DNA
   <213> Artificial Sequence
<220>
   <223> Description of Artificial Sequence: Synthetic primer
<400> 330
   aacattcaac gctgtcggt 19
<210> 331
   <211> 21
   <212> DNA
   <213> Artificial Sequence
<220>
   <223> Description of Artificial Sequence: Synthetic primer
<400> 331
   tttggcaatg gtagaactca c 21
<210> 332
   <211> 22
   <212> DNA
   <213> Artificial Sequence
<220>
   <223> Description of Artificial Sequence: Synthetic primer
<400> 332
   gtatggcact ggtagaattc ac 22
<210> 333
   <211> 20
   <212> DNA
   <213> Artificial Sequence
<220>
   <223> Description of Artificial Sequence: Synthetic primer
<400> 333
   cttatcactt ttccagccca 20
<210> 334
   <211> 20
   <212> DNA
   <213> Artificial Sequence
<220>
   <223> Description of Artificial Sequence: Synthetic primer
<400> 334
   ggagagaaag gcagttcctg 20
<210> 335
   <211> 20
   <212> DNA
   <213> Artificial Sequence
<220>
   <223> Description of Artificial Sequence: Synthetic primer
<400> 335
   cacccatcat attcttccca 20
<210> 336
   <211> 19
   <212> DNA
   <213> Artificial Sequence
<220>
   <223> Description of Artificial Sequence: Synthetic primer
<400> 336
   ctcgggctac aacacagga 19
<210> 337
   <211> 20
   <212> DNA
   <213> Artificial Sequence
<220>
   <223> Description of Artificial Sequence: Synthetic primer
<400> 337
   ccatatgtcg tgccaagaga 20
<210> 338
   <211> 25
   <212> DNA
   <213> Artificial Sequence
<220>
   <223> Description of Artificial Sequence: Synthetic primer
<400> 338
   gtgatatgtt tgatatatta ggttg 25
<210> 339
   <211> 18
   <212> DNA
   <213> Artificial Sequence
<220>
   <223> Description of Artificial Sequence: Synthetic primer
<400> 339
   gcaacggaat cccaaaag 18
<210> 340
   <211> 21
   <212> DNA
   <213> Artificial Sequence
<220>
   <223> Description of Artificial Sequence: Synthetic primer
<400> 340
   ctgacctatg aattgacagc c 21
<210> 341
   <211> 17
   <212> DNA
   <213> Artificial Sequence
<220>
   <223> Description of Artificial Sequence: Synthetic primer
<400> 341
   gtctttgcgg gcgagat 17
<210> 342
   <211> 20
   <212> DNA
   <213> Artificial Sequence
<220>
   <223> Description of Artificial Sequence: Synthetic primer
<400> 342
   tgtaacagca actccatgtg 20
<210> 343
   <211> 19
   <212> DNA
   <213> Artificial Sequence
<220>
   <223> Description of Artificial Sequence: Synthetic primer
<400> 343
   ggagtctttg ttgcccaca 19
<210> 344
   <211> 22
   <212> DNA
   <213> Artificial Sequence
<220>
   <223> Description of Artificial Sequence: Synthetic primer
<400> 344
   taggtagttt catgttgttg gg 22
<210> 345
   <211> 22
   <212> DNA
   <213> Artificial Sequence
<220>
   <223> Description of Artificial Sequence: Synthetic primer
<400> 345
   taggtagttt catgttgttg gg 22
<210> 346
   <211> 22
   <212> DNA
   <213> Artificial Sequence
<220>
   <223> Description of Artificial Sequence: Synthetic primer
<400> 346
   taggtagttt catgttgttg gg 22
<210> 347
   <211> 18
   <212> DNA
   <213> Artificial Sequence
<220>
   <223> Description of Artificial Sequence: Synthetic primer
<400> 347
   ctgtgccggg tagagagg 18
<210> 348
   <211> 17
   <212> DNA
   <213> Artificial Sequence
<220>
   <223> Description of Artificial Sequence: Synthetic primer
<400> 348
   ggtccagagg ggagata 17
<210> 349
   <211> 18
   <212> DNA
   <213> Artificial Sequence
<220>
   <223> Description of Artificial Sequence: Synthetic primer
<400> 349
   gtggtggttt ccttggct 18
<210> 350
   <211> 19
   <212> DNA
   <213> Artificial Sequence
<220>
   <223> Description of Artificial Sequence: Synthetic primer
<400> 350
   ggaggctttt cctgaggac 19
<210> 351
   <211> 17
   <212> DNA
   <213> Artificial Sequence
<220>
   <223> Description of Artificial Sequence: Synthetic primer
<400> 351
   caccggatgg acagaca 17
<210> 352
   <211> 17
   <212> DNA
   <213> Artificial Sequence
<220>
   <223> Description of Artificial Sequence: Synthetic primer
<400> 352
   ttccacagca gcccctg 17
<210> 353
   <211> 20
   <212> DNA
   <213> Artificial Sequence
<220>
   <223> Description of Artificial Sequence: Synthetic primer
<400> 353
   catcttactg ggcagcattg 20
<210> 354
   <211> 20
   <212> DNA
   <213> Artificial Sequence
<220>
   <223> Description of Artificial Sequence: Synthetic primer
<400> 354
   ctcgtcttac ccagcagtgt 20
<210> 355
   <211> 22
   <212> DNA
   <213> Artificial Sequence
<220>
   <223> Description of Artificial Sequence: Synthetic primer
<400> 355
   tccagtggtt cttaacagtt ca 22
<210> 356
   <211> 20
   <212> DNA
   <213> Artificial Sequence
<220>
   <223> Description of Artificial Sequence: Synthetic primer
<400> 356
   ccctttgtca tcctatgcct 20
<210> 357
   <211> 18
   <212> DNA
   <213> Artificial Sequence
<220>
   <223> Description of Artificial Sequence: Synthetic primer
<400> 357
   ccttcattcc accggagt 18
<210> 358
   <211> 21
   <212> DNA
   <213> Artificial Sequence
<220>
   <223> Description of Artificial Sequence: Synthetic primer
<400> 358
   acatgcttct ttatatcccc a 21
<210> 359
   <211> 17
   <212> DNA
   <213> Artificial Sequence
<220>
   <223> Description of Artificial Sequence: Synthetic primer
<400> 359
   agcttttggc ccgggtt 17
<210> 360
   <211> 17
   <212> DNA
   <213> Artificial Sequence
<220>
   <223> Description of Artificial Sequence: Synthetic primer
<400> 360
   cctgcccacc gcacact 17
<210> 361
   <211> 19
   <212> DNA
   <213> Artificial Sequence
<220>
   <223> Description of Artificial Sequence: Synthetic primer
<400> 361
   cctttgtcat ccttcgcct 19
<210> 362
   <211> 21
   <212> DNA
   <213> Artificial Sequence
<220>
   <223> Description of Artificial Sequence: Synthetic primer
<400> 362
   caccttggct ctagactgct t 21
<210> 363
   <211> 20
   <212> DNA
   <213> Artificial Sequence
<220>
   <223> Description of Artificial Sequence: Synthetic primer
<400> 363
   gaacattcaa cgctgtcggt 20
<210> 364
   <211> 21
   <212> DNA
   <213> Artificial Sequence
<220>
   <223> Description of Artificial Sequence: Synthetic primer
<400> 364
   tctgcctgtc tacacttgct g 21
<210> 365
   <211> 24
   <212> DNA
   <213> Artificial Sequence
<220>
   <223> Description of Artificial Sequence: Synthetic primer
<400> 365
   atgacctatg aattgacaga caat 24
<210> 366
   <211> 20
   <212> DNA
   <213> Artificial Sequence
<220>
   <223> Description of Artificial Sequence: Synthetic primer
<400> 366
   tggcttaatc tcagctggca 20
<210> 367
   <211> 23
   <212> DNA
   <213> Artificial Sequence
<220>
   <223> Description of Artificial Sequence: Synthetic primer
<400> 367
   gatactgcat caggaactga ttg 23
<210> 368
   <211> 22
   <212> DNA
   <213> Artificial Sequence
<220>
   <223> Description of Artificial Sequence: Synthetic primer
<400> 368
   gtgcttgatc taaccatgtg gt 22
<210> 369
   <211> 20
   <212> DNA
   <213> Artificial Sequence
<220>
   <223> Description of Artificial Sequence: Synthetic primer
<400> 369
   tcctgattgt ccaaacgcaa 20
<210> 370
   <211> 21
   <212> DNA
   <213> Artificial Sequence
<220>
   <223> Description of Artificial Sequence: Synthetic primer
<400> 370
   ccacaccgta tctgacactt t 21
<210> 371
   <211> 24
   <212> DNA
   <213> Artificial Sequence
<220>
   <223> Description of Artificial Sequence: Synthetic primer
<400> 371
   cctggcatac aatgtagatt tctg 24
<210> 372
   <211> 18
   <212> DNA
   <213> Artificial Sequence
<220>
   <223> Description of Artificial Sequence: Synthetic primer
<400> 372
   ccccagaagg caaaggat 18
<210> 373
   <211> 22
   <212> DNA
   <213> Artificial Sequence
<220>
   <223> Description of Artificial Sequence: Synthetic primer
<400> 373
   ccgtgtattt gacaagctga gt 22
<210> 374
   <211> 23
   <212> DNA
   <213> Artificial Sequence
<220>
   <223> Description of Artificial Sequence: Synthetic primer
<400> 374
   ggctttcaag tcactagtgg ttc 23
<210> 375
   <211> 17
   <212> DNA
   <213> Artificial Sequence
<220>
   <223> Description of Artificial Sequence: Synthetic primer
<400> 375
   ccccccctca atcctgt 17
<210> 376
   <211> 17
   <212> DNA
   <213> Artificial Sequence
<220>
   <223> Description of Artificial Sequence: Synthetic primer
<400> 376
   accaccatcg tgcggta 17
<210> 377
   <211> 23
   <212> DNA
   <213> Artificial Sequence
<220>
   <223> Description of Artificial Sequence: Synthetic primer
<400> 377
   gctctgactt tattgcacta ctg 23
<210> 378
   <211> 23
   <212> DNA
   <213> Artificial Sequence
<220>
   <223> Description of Artificial Sequence: Synthetic primer
<400> 378
   ccacttaaac gtggatgtac ttg 23
<210> 379
   <211> 23
   <212> DNA
   <213> Artificial Sequence
<220>
   <223> Description of Artificial Sequence: Synthetic primer
<400> 379
   caactttaac atggaagtgc ttt 23
<210> 380
   <211> 20
   <212> DNA
   <213> Artificial Sequence
<220>
   <223> Description of Artificial Sequence: Synthetic primer
<400> 380
   gctttaacat gggggtacct 20
<210> 381
   <211> 23
   <212> DNA
   <213> Artificial Sequence
<220>
   <223> Description of Artificial Sequence: Synthetic primer
<400> 381
   tctactttaa catggaggca ctt 23
<210> 382
   <211> 17
   <212> DNA
   <213> Artificial Sequence
<220>
   <223> Description of Artificial Sequence: Synthetic primer
<400> 382
   cgccttctct tcccggt 17
<210> 383
   <211> 19
   <212> DNA
   <213> Artificial Sequence
<220>
   <223> Description of Artificial Sequence: Synthetic primer
<400> 383
   ctaagccagg gattgtggg 19
<210> 384
   <211> 17
   <212> DNA
   <213> Artificial Sequence
<220>
   <223> Description of Artificial Sequence: Synthetic primer
<400> 384
   agaggtggtc cgtggcg 17
<210> 385
   <211> 20
   <212> DNA
   <213> Artificial Sequence
<220>
   <223> Description of Artificial Sequence: Synthetic primer
<400> 385
   cattgctgtc tctcttcgca 20
<210> 386
   <211> 23
   <212> DNA
   <213> Artificial Sequence
<220>
   <223> Description of Artificial Sequence: Synthetic primer
<400> 386
   cctagtaggt gtccagtaag tgt 23
<210> 387
   <211> 18
   <212> DNA
   <213> Artificial Sequence
<220>
   <223> Description of Artificial Sequence: Synthetic primer
<400> 387
   cgggactccc atcaagaa 18
<210> 388
   <211> 19
   <212> DNA
   <213> Artificial Sequence
<220>
   <223> Description of Artificial Sequence: Synthetic primer
<400> 388
   gaggggctca gggagaaag 19

<210> 389
   <211> 20
   <212> DNA
   <213> Artificial Sequence
<220>
   <223> Description of Artificial Sequence: Synthetic primer
<400> 389
   ctgggcctgt gtcttaggct 20
<210> 390
   <211> 19
   <212> DNA
   <213> Artificial Sequence
<220>
   <223> Description of Artificial Sequence: Synthetic primer
<400> 390
   gagctgaaag cactcccaa 19
<210> 391
   <211> 23
   <212> DNA
   <213> Artificial Sequence
<220>
   <223> Description of Artificial Sequence: Synthetic primer
<400> 391
   gtcaagagca ataacgaaaa atg 23
<210> 392
   <211> 20
   <212> DNA
   <213> Artificial Sequence
<220>
   <223> Description of Artificial Sequence: Synthetic primer
<400> 392
   acggcttcat acaggagttg 20
<210> 393
   <211> 22
   <212> DNA
   <213> Artificial Sequence
<220>
   <223> Description of Artificial Sequence: Synthetic primer
<400> 393
   ccaacaatat cctggtgctg ag 22

<210> 394
   <211> 19
   <212> DNA
   <213> Artificial Sequence
<220>
   <223> Description of Artificial Sequence: Synthetic primer
<400> 394
   tccctgtcct ccaggagct 19
<210> 395
   <211> 23
   <212> DNA
   <213> Artificial Sequence
<220>
   <223> Description of Artificial Sequence: Synthetic primer
<400> 395
   ataaagcaat gagactgatt gtc 23
<210> 396
   <211> 21
   <212> DNA
   <213> Artificial Sequence
<220>
   <223> Description of Artificial Sequence: Synthetic primer
<400> 396
   gtgctatctg tgattgaggg a 21
<210> 397
   <211> 20
   <212> DNA
   <213> Artificial Sequence
<220>
   <223> Description of Artificial Sequence: Synthetic primer
<400> 397
   ctgactccta gtccagggct 20
<210> 398
   <211> 20
   <212> DNA
   <213> Artificial Sequence
<220>
   <223> Description of Artificial Sequence: Synthetic primer
<400> 398
   gcatgcctgc ctctctgttg 20
<210> 399
   <211> 23
   <212> DNA
   <213> Artificial Sequence
<220>
   <223> Description of Artificial Sequence: Synthetic primer
<400> 399
   cttatcagaa tctccagggg tac 23
<210> 400
   <211> 22
   <212> DNA
   <213> Artificial Sequence
<220>
   <223> Description of Artificial Sequence: Synthetic primer
<400> 400
   ctgttgctaa tatgcaactc tg 22
<210> 401
   <211> 25
   <212> DNA
   <213> Artificial Sequence
<220>
   <223> Description of Artificial Sequence: Synthetic primer
<400> 401
   ggtggatatt ccttctatgt ttatg 25
<210> 402
   <211> 22
   <212> DNA
   <213> Artificial Sequence
<220>
   <223> Description of Artificial Sequence: Synthetic primer
<400> 402
   ggagatcgac cgtgttatat tc 22
<210> 403
   <211> 21
   <212> DNA
   <213> Artificial Sequence
<220>
   <223> Description of Artificial Sequence: Synthetic primer
<400> 403
   ggtcacgtct ctgcagttac a 21
<210> 404
   <211> 19
   <212> DNA
   <213> Artificial Sequence
<220>
   <223> Description of Artificial Sequence: Synthetic primer
<400> 404
   tcaaactgtg ggggcactt 19
<210> 405
   <211> 22
   <212> DNA
   <213> Artificial Sequence
<220>
   <223> Description of Artificial Sequence: Synthetic primer
<400> 405
   gcctcaaatg tggagcacta tt 22
<210> 406
   <211> 18
   <212> DNA
   <213> Artificial Sequence
<220>
   <223> Description of Artificial Sequence: Synthetic primer
<400> 406
   ctcaaaatgg gggcgctt 18
<210> 407
   <211> 20
   <212> DNA
   <213> Artificial Sequence
<220>
   <223> Description of Artificial Sequence: Synthetic primer
<400> 407
   gccctcaagg agctcacagt 20
<210> 408
   <211> 16
   <212> DNA
   <213> Artificial Sequence
<220>
   <223> Description of Artificial Sequence: Synthetic primer
<400> 408
   acgagcccct cgcaca 16
<210> 409
   <211> 24
   <212> DNA
   <213> Artificial Sequence
<220>
   <223> Description of Artificial Sequence: Synthetic primer
<400> 409
   ggtagattct ccttctatga gtac 24
<210> 410
   <211> 18
   <212> DNA
   <213> Artificial Sequence
<220>
   <223> Description of Artificial Sequence: Synthetic primer
<400> 410
   gagcagaggt tgcccttg 18
<210> 411
   <211> 20
   <212> DNA
   <213> Artificial Sequence
<220>
   <223> Description of Artificial Sequence: Synthetic primer
<400> 411
   ctcctgactc caggtcctgt 20
<210> 412
   <211> 23
   <212> DNA
   <213> Artificial Sequence
<220>
   <223> Description of Artificial Sequence: Synthetic primer
<400> 412
   agagatggta gactatggaa cgt 23
<210> 413
   <211> 21
   <212> DNA
   <213> Artificial Sequence
<220>
   <223> Description of Artificial Sequence: Synthetic primer
<400> 413
   gatggttgac catagaacat g 21
<210> 414
   <211> 17
   <212> DNA
   <213> Artificial Sequence
<220>
   <223> Description of Artificial Sequence: Synthetic primer
<400> 414
   agcgaggttg ccctttg 17
<210> 415
   <211> 21
   <212> DNA
   <213> Artificial Sequence
<220>
   <223> Description of Artificial Sequence: Synthetic primer
<400> 415
   gaagttgttc gtggtggatt c 21
<210> 416
   <211> 23
   <212> DNA
   <213> Artificial Sequence
<220>
   <223> Description of Artificial Sequence: Synthetic primer
<400> 416
   cagatcagaa ggtgattgtg gct 23
<210> 417
   <211> 25
   <212> DNA
   <213> Artificial Sequence
<220>
   <223> Description of Artificial Sequence: Synthetic primer
<400> 417
   caattcctag acaatatgta taatg 25
<210> 418
   <211> 19
   <212> DNA
   <213> Artificial Sequence
<220>
   <223> Description of Artificial Sequence: Synthetic primer
<400> 418
   tcctgactcc aggtcctgt 19
<210> 419
   <211> 17
   <212> DNA
   <213> Artificial Sequence
<220>
   <223> Description of Artificial Sequence: Synthetic primer
<400> 419
   tgaggggcag agagcga 17
<210> 420
   <211> 22
   <212> DNA
   <213> Artificial Sequence
<220>
   <223> Description of Artificial Sequence: Synthetic primer
<400> 420
   agcagcaatt catgttttga ag 22
<210> 421
   <211> 21
   <212> DNA
   <213> Artificial Sequence
<220>
   <223> Description of Artificial Sequence: Synthetic primer
<400> 421
   atgacacgat cactcccgtt g 21
<210> 422
   <211> 25
   <212> DNA
   <213> Artificial Sequence
<220>
   <223> Description of Artificial Sequence: Synthetic primer
<400> 422
   aggtagtagg ttgtatagtt ttagg 25
<210> 423
   <211> 20
   <212> DNA
   <213> Artificial Sequence
<220>
   <223> Description of Artificial Sequence: Synthetic primer
<400> 423
   cctggatgtt ctcttcactg 20
<210> 424
   <211> 26
   <212> DNA
   <213> Artificial Sequence
<220>
   <223> Description of Artificial Sequence: Synthetic primer
<400> 424
   gaggtagtag gttgtatagt ttagaa 26
<210> 425
   <211> 20
   <212> DNA
   <213> Artificial Sequence
<220>
   <223> Description of Artificial Sequence: Synthetic primer
<400> 425
   ttccagccat tgtgactgca 20
<210> 426
   <211> 24
   <212> DNA
   <213> Artificial Sequence
<220>
   <223> Description of Artificial Sequence: Synthetic primer
<400> 426
   gaggtagtag gttgtatagt ttgg 24
<210> 427
   <211> 20
   <212> DNA
   <213> Artificial Sequence
<220>
   <223> Description of Artificial Sequence: Synthetic primer
<400> 427
   accaagaccg actgcccttt 20
<210> 428
   <211> 21
   <212> DNA
   <213> Artificial Sequence
<220>
   <223> Description of Artificial Sequence: Synthetic primer
<400> 428
   tgaggtagta ggttgtgtgg t 21
<210> 429
   <211> 16
   <212> DNA
   <213> Artificial Sequence
<220>
   <223> Description of Artificial Sequence: Synthetic primer
<400> 429
   cctcccgcag tgcaag 16
<210> 430
   <211> 23
   <212> DNA
   <213> Artificial Sequence
<220>
   <223> Description of Artificial Sequence: Synthetic primer
<400> 430
   ttgaggtagt aggttgtatg gtt 23
<210> 431
   <211> 20
   <212> DNA
   <213> Artificial Sequence
<220>
   <223> Description of Artificial Sequence: Synthetic primer
<400> 431
   ttggaggagc tgactgaaga 20
<210> 432
   <211> 21
   <212> DNA
   <213> Artificial Sequence
<220>
   <223> Description of Artificial Sequence: Synthetic primer
<400> 432
   aggttgcata gttttagggc a 21
<210> 433
   <211> 21
   <212> DNA
   <213> Artificial Sequence
<220>
   <223> Description of Artificial Sequence: Synthetic primer
<400> 433
   gccaagtaga agaccagcaa g 21
<210> 434
   <211> 24
   <212> DNA
   <213> Artificial Sequence
<220>
   <223> Description of Artificial Sequence: Synthetic primer
<400> 434
   gaggtaggag gttgtatagt tgag 24
<210> 435
   <211> 17
   <212> DNA
   <213> Artificial Sequence
<220>
   <223> Description of Artificial Sequence: Synthetic primer
<400> 435
   ctgtctgtct gtctgtc 17
<210> 436
   <211> 23
   <212> DNA
   <213> Artificial Sequence
<220>
   <223> Description of Artificial Sequence: Synthetic primer
<400> 436
   gattgtatag ttgtggggta gtg 23
<210> 437
   <211> 20
   <212> DNA
   <213> Artificial Sequence
<220>
   <223> Description of Artificial Sequence: Synthetic primer
<400> 437
   tgtactttcc attccagaag 20
<210> 438
   <211> 25
   <212> DNA
   <213> Artificial Sequence
<220>
   <223> Description of Artificial Sequence: Synthetic primer
<400> 438
   ggtagtagat tgtatagttt taggg 25
<210> 439
   <211> 20
   <212> DNA
   <213> Artificial Sequence
<220>
   <223> Description of Artificial Sequence: Synthetic primer
<400> 439
   tgaagatgga cactggtgct 20
<210> 440
   <211> 25
   <212> DNA
   <213> Artificial Sequence
<220>
   <223> Description of Artificial Sequence: Synthetic primer
<400> 440
   gtagtagttt gtacagtttg agggt 25
<210> 441
   <211> 18
   <212> DNA
   <213> Artificial Sequence
<220>
   <223> Description of Artificial Sequence: Synthetic primer
<400> 441
   agcgctccgt ttcctttt 18
<210> 442
   <211> 17
   <212> DNA
   <213> Artificial Sequence
<220>
   <223> Description of Artificial Sequence: Synthetic primer
<400> 442
   tgtgctgttg gtcgggt 17
<210> 443
   <211> 17
   <212> DNA
   <213> Artificial Sequence
<220>
   <223> Description of Artificial Sequence: Synthetic primer
<400> 443
   cgaggaagga cggagga 17
<210> 444
   <211> 20
   <212> DNA
   <213> Artificial Sequence
<220>
   <223> Description of Artificial Sequence: Synthetic primer
<400> 444
   aacgcttcac gaatttgcgt 20
<210> 445
   <211> 25
   <212> DNA
   <213> Artificial Sequence
<220>
   <223> Description of Artificial Sequence: Synthetic primer
<400> 445
   tacatacttc tttacattcc atagc 25
<210> 446
   <211> 25
   <212> DNA
   <213> Artificial Sequence
<220>
   <223> Description of Artificial Sequence: Synthetic primer
<400> 446
   tacatacttc tttacattcc atagc 25
<210> 447
   <211> 22
   <212> DNA
   <213> Artificial Sequence
<220>
   <223> Description of Artificial Sequence: Synthetic primer
<400> 447
   agactgtgat ttgttgtcga tt 22
<210> 448
   <211> 21
   <212> DNA
   <213> Artificial Sequence
<220>
   <223> Description of Artificial Sequence: Synthetic primer
<400> 448
   agactgggat ttgttgttga g 21
<210> 449
   <211> 20
   <212> DNA
   <213> Artificial Sequence
<220>
   <223> Description of Artificial Sequence: Synthetic primer
<400> 449
   ggctgtgact tgttgtcgta 20
<210> 450
   <211> 16
   <212> DNA
   <213> Artificial Sequence
<220>
   <223> Description of Artificial Sequence: Synthetic primer
<400> 450
   cgtgaggccg gctttc 16
<210> 451
   <211> 22
   <212> DNA
   <213> Artificial Sequence
<220>
   <223> Description of Artificial Sequence: Synthetic primer
<400> 451
   agtctttcat tctcacacgc tc 22
<210> 452
   <211> 17
   <212> DNA
   <213> Artificial Sequence
<220>
   <223> Description of Artificial Sequence: Synthetic primer
<400> 452
   gctcgcacgc agaagtt 17
<210> 453
   <211> 24
   <212> DNA
   <213> Artificial Sequence
<220>
   <223> Description of Artificial Sequence: Synthetic primer
<400> 453
   attcccctag atacgaattt gtga 24
<210> 454
   <211> 18
   <212> DNA
   <213> Artificial Sequence
<220>
   <223> Description of Artificial Sequence: Synthetic primer
<400> 454
   gcagcacaat atggcctg 18
<210> 455
   <211> 23
   <212> DNA
   <213> Artificial Sequence
<220>
   <223> Description of Artificial Sequence: Synthetic primer
<400> 455
   ctagagcagc aaataatgat tcg 23
<210> 456
   <211> 24
   <212> DNA
   <213> Artificial Sequence
<220>
   <223> Description of Artificial Sequence: Synthetic primer
<400> 456
   cagcagcaca gttaatactg gaga 24
<210> 457
   <211> 22
   <212> DNA
   <213> Artificial Sequence
<220>
   <223> Description of Artificial Sequence: Synthetic primer
<400> 457
   aagcagcaca gtaatattgg tg 22
<210> 458
   <211> 16
   <212> DNA
   <213> Artificial Sequence
<220>
   <223> Description of Artificial Sequence: Synthetic primer
<400> 458
   tggcttcccg aggcag 16
<210> 459
   <211> 20
   <212> DNA
   <213> Artificial Sequence
<220>
   <223> Description of Artificial Sequence: Synthetic primer
<400> 459
   gaaggagcac ttagggcagt 20
<210> 460
   <211> 21
   <212> DNA
   <213> Artificial Sequence
<220>
   <223> Description of Artificial Sequence: Synthetic primer
<400> 460
   caggcagatt ctacatcgac a 21
<210> 461
   <211> 21
   <212> DNA
   <213> Artificial Sequence
<220>
   <223> Description of Artificial Sequence: Synthetic primer
<400> 461
   caacctgtgt agaaaggggt t 21
<210> 462
   <211> 22
   <212> DNA
   <213> Artificial Sequence
<220>
   <223> Description of Artificial Sequence: Synthetic primer
<400> 462
   gtgtgttcac acagacgtag ga 22
<210> 463
   <211> 23
   <212> DNA
   <213> Artificial Sequence
<220>
   <223> Description of Artificial Sequence: Synthetic primer
<400> 463
   gtactttaag tgctcataat gca 23
<210> 464
   <211> 17
   <212> DNA
   <213> Artificial Sequence
<220>
   <223> Description of Artificial Sequence: Synthetic primer
<400> 464
   cagcccatcg actggtg 17
<210> 465
   <211> 21
   <212> DNA
   <213> Artificial Sequence
<220>
   <223> Description of Artificial Sequence: Synthetic primer
<400> 465
   tctgtcacct tccagatgat g 21
<210> 466
   <211> 20
   <212> DNA
   <213> Artificial Sequence
<220>
   <223> Description of Artificial Sequence: Synthetic primer
<400> 466
   tggtaatccc tggcaatgtg 20
<210> 467
   <211> 18
   <212> DNA
   <213> Artificial Sequence
<220>
   <223> Description of Artificial Sequence: Synthetic primer
<400> 467
   accacggttt ctggagga 18
<210> 468
   <211> 21
   <212> DNA
   <213> Artificial Sequence
<220>
   <223> Description of Artificial Sequence: Synthetic primer
<400> 468
   ccctgttcct gctgaactga g 21
<210> 469
   <211> 20
   <212> DNA
   <213> Artificial Sequence
<220>
   <223> Description of Artificial Sequence: Synthetic primer
<400> 469
   tcagaccgag acaagtgcaa 20
<210> 470
   <211> 22
   <212> DNA
   <213> Artificial Sequence
<220>
   <223> Description of Artificial Sequence: Synthetic primer
<400> 470
   tgcaagtaac caagaatagg cc 22
<210> 471
   <211> 21
   <212> DNA
   <213> Artificial Sequence
<220>
   <223> Description of Artificial Sequence: Synthetic primer
<400> 471
   caagtaatgg agaacaggct g 21
<210> 472
   <211> 19
   <212> DNA
   <213> Artificial Sequence
<220>
   <223> Description of Artificial Sequence: Synthetic primer
<400> 472
   ggcggaactt agccactgt 19
<210> 473
   <211> 20
   <212> DNA
   <213> Artificial Sequence
<220>
   <223> Description of Artificial Sequence: Synthetic primer
<400> 473
   cctccaggag ctcacaatct 20
<210> 474
   <211> 20
   <212> DNA
   <213> Artificial Sequence
<220>
   <223> Description of Artificial Sequence: Synthetic primer
<400> 474
   ataaccgatt tcagatggtg 20
<210> 475
   <211> 20
   <212> DNA
   <213> Artificial Sequence
<220>
   <223> Description of Artificial Sequence: Synthetic primer
<400> 475
   ataaccgatt tcagatggtg 20
<210> 476
   <211> 20
   <212> DNA
   <213> Artificial Sequence
<220>
   <223> Description of Artificial Sequence: Synthetic primer
<400> 476
   gctgcaaaca tccgactgaa 20
<210> 477
   <211> 18
   <212> DNA
   <213> Artificial Sequence
<220>
   <223> Description of Artificial Sequence: Synthetic primer
<400> 477
   ccttgaagtc cgaggcag 18
<210> 478
   <211> 17
   <212> DNA
   <213> Artificial Sequence
<220>
   <223> Description of Artificial Sequence: Synthetic primer
<400> 478
   cctgtgggca caaacct 17
<210> 479
   <211> 18
   <212> DNA
   <213> Artificial Sequence
<220>
   <223> Description of Artificial Sequence: Synthetic primer
<400> 479
   atccacctcc cagccaat 18
<210> 480
   <211> 23
   <212> DNA
   <213> Artificial Sequence
<220>
   <223> Description of Artificial Sequence: Synthetic primer
<400> 480
   gcctctgtat actattcttg cca 23
<210> 481
   <211> 20
   <212> DNA
   <213> Artificial Sequence
<220>
   <223> Description of Artificial Sequence: Synthetic primer
<400> 481
   gagtaaacaa ccctctccca 20
<210> 482
   <211> 21
   <212> DNA
   <213> Artificial Sequence
<220>
   <223> Description of Artificial Sequence: Synthetic primer
<400> 482
   ggagtggaga ctgttccttc t 21
<210> 483
   <211> 20
   <212> DNA
   <213> Artificial Sequence
<220>
   <223> Description of Artificial Sequence: Synthetic primer
<400> 483
   cctcagaaac aaacacggga 20
<210> 484
   <211> 22
   <212> DNA
   <213> Artificial Sequence
<220>
   <223> Description of Artificial Sequence: Synthetic primer
<400> 484
   gcagcaaaca tctgactgaa ag 22
<210> 485
   <211> 18
   <212> DNA
   <213> Artificial Sequence
<220>
   <223> Description of Artificial Sequence: Synthetic primer
<400> 485
   ctccactccg ggacagaa 18
<210> 486
   <211> 17
   <212> DNA
   <213> Artificial Sequence
<220>
   <223> Description of Artificial Sequence: Synthetic primer
<400> 486
   gccatggctg ctgtcag 17
<210> 487
   <211> 24
   <212> DNA
   <213> Artificial Sequence
<220>
   <223> Description of Artificial Sequence: Synthetic primer
<400> 487
   tatcacacac actaaattgc attg 24
<210> 488
   <211> 20
   <212> DNA
   <213> Artificial Sequence
<220>
   <223> Description of Artificial Sequence: Synthetic primer
<400> 488
   ctgtgatgca ctgtggaaac 20
<210> 489
   <211> 23
   <212> DNA
   <213> Artificial Sequence
<220>
   <223> Description of Artificial Sequence: Synthetic primer
<400> 489
   ggcagtatac ttgctgattg ctt 23
<210> 490
   <211> 21
   <212> DNA
   <213> Artificial Sequence
<220>
   <223> Description of Artificial Sequence: Synthetic primer
<400> 490
   gatggcagtg gagttagtga t 21
<210> 491
   <211> 18
   <212> DNA
   <213> Artificial Sequence
<220>
   <223> Description of Artificial Sequence: Synthetic primer
<400> 491
   cctggccgtg tggttagt 18
<210> 492
   <211> 21
   <212> DNA
   <213> Artificial Sequence
<220>
   <223> Description of Artificial Sequence: Synthetic primer
<400> 492
   cgggacaagt gcaataccat a 21
<210> 493
   <211> 17
   <212> DNA
   <213> Artificial Sequence
<220>
   <223> Description of Artificial Sequence: Synthetic primer
<400> 493
   ccacccgaca acagcaa 17
<210> 494
   <211> 19
   <212> DNA
   <213> Artificial Sequence
<220>
   <223> Description of Artificial Sequence: Synthetic primer
<400> 494
   ctcgggaagt gctagctca 19
<210> 495
   <211> 21
   <212> DNA
   <213> Artificial Sequence
<220>
   <223> Description of Artificial Sequence: Synthetic primer
<400> 495
   tgctcaataa atacccgttg a 21
<210> 496
   <211> 18
   <212> DNA
   <213> Artificial Sequence
<220>
   <223> Description of Artificial Sequence: Synthetic primer
<400> 496
   cttgaggagg agcaggct 18
<210> 497
   <211> 24
   <212> DNA
   <213> Artificial Sequence
<220>
   <223> Description of Artificial Sequence: Synthetic primer
<400> 497
   tatagttatc ttctaattgg ggcc 24
<210> 498
   <211> 19
   <212> DNA
   <213> Artificial Sequence
<220>
   <223> Description of Artificial Sequence: Synthetic primer
<400> 498
   ccacagacac gagcttgtg 19
<210> 499
   <211> 20
   <212> DNA
   <213> Artificial Sequence
<220>
   <223> Description of Artificial Sequence: Synthetic primer
<400> 499
   ccacagacac gagcttgtgt 20
<210> 500
   <211> 24
   <212> DNA
   <213> Artificial Sequence
<220>
   <223> Description of Artificial Sequence: Synthetic primer
<400> 500
   tacctataga tacaagcttg tgcg 24
<210> 501
   <211> 23
   <212> DNA
   <213> Artificial Sequence
<220>
   <223> Description of Artificial Sequence: Synthetic primer
<400> 501
   gccatccttc agttatcaca gta 23
<210> 502
   <211> 25
   <212> DNA
   <213> Artificial Sequence
<220>
   <223> Description of Artificial Sequence: Synthetic primer
<400> 502
   ccttcagtta tcacagtact gtacc 25
<210> 503
   <211> 21
   <212> DNA
   <213> Artificial Sequence
<220>
   <223> Description of Artificial Sequence: Synthetic primer
<400> 503
   ttcatagccc tgtacaatgc t 21
<210> 504
   <211> 20
   <212> DNA
   <213> Artificial Sequence
<220>
   <223> Description of Artificial Sequence: Synthetic primer
<400> 504
   gcacatgctc aaacatccgt 20
<210> 505
   <211> 20
   <212> DNA
   <213> Artificial Sequence
<220>
   <223> Description of Artificial Sequence: Synthetic primer
<400> 505
   agcagctcaa aagcatcaac 20
<210> 506
   <211> 19
   <212> DNA
   <213> Artificial Sequence
<220>
   <223> Description of Artificial Sequence: Synthetic primer
<400> 506
   caagtaccca cagtgcggt 19
<210> 507
   <211> 21
   <212> DNA
   <213> Artificial Sequence
<220>
   <223> Description of Artificial Sequence: Synthetic primer
<400> 507
   gatagccctg tacaatgctg c 21
<210> 508
   <211> 18
   <212> DNA
   <213> Artificial Sequence
<220>
   <223> Description of Artificial Sequence: Synthetic primer
<400> 508
   gagggacttt caggggca 18
<210> 509
   <211> 22
   <212> DNA
   <213> Artificial Sequence
<220>
   <223> Description of Artificial Sequence: Synthetic primer
<400> 509
   tttagtgtga taatggcgtt tg 22
<210> 510
   <211> 18
   <212> DNA
   <213> Artificial Sequence
<220>
   <223> Description of Artificial Sequence: Synthetic primer
<400> 510
   cattcaccgc gtgcctta 18
<210> 511
   <211> 19
   <212> DNA
   <213> Artificial Sequence
<220>
   <223> Description of Artificial Sequence: Synthetic primer
<400> 511
   aacctcacct gtgaccctg 19
<210> 512
   <211> 18
   <212> DNA
   <213> Artificial Sequence
<220>
   <223> Description of Artificial Sequence: Synthetic primer
<400> 512
   agcctaaccc gtggattt 18
<210> 513
   <211> 20
   <212> DNA
   <213> Artificial Sequence
<220>
   <223> Description of Artificial Sequence: Synthetic primer
<400> 513
   aagagcctga cttgtgatgt 20
<210> 514
   <211> 21
   <212> DNA
   <213> Artificial Sequence
<220>
   <223> Description of Artificial Sequence: Synthetic primer
<400> 514
   gcgcattatt actcacggta c 21
<210> 515
   <211> 19
   <212> DNA
   <213> Artificial Sequence
<220>
   <223> Description of Artificial Sequence: Synthetic primer
<400> 515
   gccaagctca gacggatcc 19
<210> 516
   <211> 22
   <212> DNA
   <213> Artificial Sequence
<220>
   <223> Description of Artificial Sequence: Synthetic primer
<400> 516
   aaagagaccg gttcactgtg ag 22
<210> 517
   <211> 22
   <212> DNA
   <213> Artificial Sequence
<220>
   <223> Description of Artificial Sequence: Synthetic primer
<400> 517
   aaagagaccg gttcactgtg ag 22
<210> 518
   <211> 19
   <212> DNA
   <213> Artificial Sequence
<220>
   <223> Description of Artificial Sequence: Synthetic primer
<400> 518
   gctttttggg gtaagggct 19
<210> 519
   <211> 17
   <212> DNA
   <213> Artificial Sequence
<220>
   <223> Description of Artificial Sequence: Synthetic primer
<400> 519
   gcaatgctga ggaggca 17
<210> 520
   <211> 21
   <212> DNA
   <213> Artificial Sequence
<220>
   <223> Description of Artificial Sequence: Synthetic primer
<400> 520
   tgccctttta acattgcact g 21
<210> 521
   <211> 23
   <212> DNA
   <213> Artificial Sequence
<220>
   <223> Description of Artificial Sequence: Synthetic primer
<400> 521
   cgaccatggc tgtagactgt tac 23
<210> 522
   <211> 19
   <212> DNA
   <213> Artificial Sequence
<220>
   <223> Description of Artificial Sequence: Synthetic primer
<400> 522
   acagctggtt gaaggggac 19
<210> 523
   <211> 19
   <212> DNA
   <213> Artificial Sequence
<220>
   <223> Description of Artificial Sequence: Synthetic primer
<400> 523
   acagctggtt gaaggggac 19
<210> 524
   <211> 19
   <212> DNA
   <213> Artificial Sequence
<220>
   <223> Description of Artificial Sequence: Synthetic primer
<400> 524
   ggtgactagg tggcccaca 19
<210> 525
   <211> 17
   <212> DNA
   <213> Artificial Sequence
<220>
   <223> Description of Artificial Sequence: Synthetic primer
<400> 525
   cacggctcca atcccta 17
<210> 526
   <211> 16
   <212> DNA
   <213> Artificial Sequence
<220>
   <223> Description of Artificial Sequence: Synthetic primer
<400> 526
   tccgaacctg gtccca 16
<210> 527
   <211> 23
   <212> DNA
   <213> Artificial Sequence
<220>
   <223> Description of Artificial Sequence: Synthetic primer
<400> 527
   agactcattt gagacgatga tgg 23
<210> 528
   <211> 22
   <212> DNA
   <213> Artificial Sequence
<220>
   <223> Description of Artificial Sequence: Synthetic primer
<400> 528
   gactacgcgt attcttaagc aa 22
<210> 529
   <211> 20
   <212> DNA
   <213> Artificial Sequence
<220>
   <223> Description of Artificial Sequence: Synthetic primer
<400> 529
   accctggtgt cgtgaaatag 20
<210> 530
   <211> 17
   <212> DNA
   <213> Artificial Sequence
<220>
   <223> Description of Artificial Sequence: Synthetic primer
<400> 530
   tactccaaca gggccgc 17
<210> 531
   <211> 21
   <212> DNA
   <213> Artificial Sequence
<220>
   <223> Description of Artificial Sequence: Synthetic primer
<400> 531
   cgtggttcta ccctgtggta g 21
<210> 532
   <211> 22
   <212> DNA
   <213> Artificial Sequence
<220>
   <223> Description of Artificial Sequence: Synthetic primer
<400> 532
   agccatcttt accagacagt gt 22
<210> 533
   <211> 16
   <212> DNA
   <213> Artificial Sequence
<220>
   <223> Description of Artificial Sequence: Synthetic primer
<400> 533
   cgccgaggaa gatggt 16
<210> 534
   <211> 24
   <212> DNA
   <213> Artificial Sequence
<220>
   <223> Description of Artificial Sequence: Synthetic primer
<400> 534
   gctacagtgc ttcatctcag actc 24
<210> 535
   <211> 24
   <212> DNA
   <213> Artificial Sequence
<220>
   <223> Description of Artificial Sequence: Synthetic primer
<400> 535
   cggactagta catcatctat actg 24
<210> 536
   <211> 18
   <212> DNA
   <213> Artificial Sequence
<220>
   <223> Description of Artificial Sequence: Synthetic primer
<400> 536
   cctcatcctg tgagccag 18
<210> 537
   <211> 22
   <212> DNA
   <213> Artificial Sequence
<220>
   <223> Description of Artificial Sequence: Synthetic primer
<400> 537
   gctgaagaac tgaatttcag ag 22
<210> 538
   <211> 21
   <212> DNA
   <213> Artificial Sequence
<220>
   <223> Description of Artificial Sequence: Synthetic primer
<400> 538
   atctagcaga agcatttcca c 21
<210> 539
   <211> 16
   <212> DNA
   <213> Artificial Sequence
<220>
   <223> Description of Artificial Sequence: Synthetic primer
<400> 539
   aaaggcgcag cgacgt 16
<210> 540
   <211> 20
   <212> DNA
   <213> Artificial Sequence
<220>
   <223> Description of Artificial Sequence: Synthetic primer
<400> 540
   tctattcttc cctcccactc 20
<210> 541
   <211> 16
   <212> DNA
   <213> Artificial Sequence
<220>
   <223> Description of Artificial Sequence: Synthetic primer
<400> 541
   agcacagccc ccgtcc 16
<210> 542
   <211> 18
   <212> DNA
   <213> Artificial Sequence
<220>
   <223> Description of Artificial Sequence: Synthetic primer
<400> 542
   tgtcccccag gcctgtac 18
<210> 543
   <211> 20
   <212> DNA
   <213> Artificial Sequence
<220>
   <223> Description of Artificial Sequence: Synthetic primer
<400> 543
   gtcctcaagg agcttcagtc 20
<210> 544
   <211> 19
   <212> DNA
   <213> Artificial Sequence
<220>
   <223> Description of Artificial Sequence: Synthetic primer
<400> 544
   cccaagttct gtcatgcac 19
<210> 545
   <211> 23
   <212> DNA
   <213> Artificial Sequence
<220>
   <223> Description of Artificial Sequence: Synthetic primer
<400> 545
   tcacttttgt gactatgcaa ctg 23
<210> 546
   <211> 23
   <212> DNA
   <213> Artificial Sequence
<220>
   <223> Description of Artificial Sequence: Synthetic primer
<400> 546
   aataggtcaa ccgtgtatga ttc 23
<210> 547
   <211> 24
   <212> DNA
   <213> Artificial Sequence
<220>
   <223> Description of Artificial Sequence: Synthetic primer
<400> 547
   gctaatatgt aggagtcagt tgga 24
<210> 548
   <211> 20
   <212> DNA
   <213> Artificial Sequence
<220>
   <223> Description of Artificial Sequence: Synthetic primer
<400> 548
   cagtcaacgg tcagtggttt 20
<210> 549
   <211> 21
   <212> DNA
   <213> Artificial Sequence
<220>
   <223> Description of Artificial Sequence: Synthetic primer
<400> 549
   ttgcattcat tgttcagtga g 21
<210> 550
   <211> 21
   <212> DNA
   <213> Artificial Sequence
<220>
   <223> Description of Artificial Sequence: Synthetic primer
<400> 550
   gttggcaagt ctagaaccac c 21
<210> 551
   <211> 19
   <212> DNA
   <213> Artificial Sequence
<220>
   <223> Description of Artificial Sequence: Synthetic primer
<400> 551
   tatggccctt cggtaattc 19
<210> 552
   <211> 20
   <212> DNA
   <213> Artificial Sequence
<220>
   <223> Description of Artificial Sequence: Synthetic primer
<400> 552
   ccttatcagt tctccgtcca 20
<210> 553
   <211> 18
   <212> DNA
   <213> Artificial Sequence
<220>
   <223> Description of Artificial Sequence: Synthetic primer
<400> 553
   ggaccagagg aaagccag 18
<210> 554
   <211> 22
   <212> DNA
   <213> Artificial Sequence
<220>
   <223> Description of Artificial Sequence: Synthetic primer
<400> 554
   gacattcaca tgcttcaggt ag 22
<210> 555
   <211> 19
   <212> DNA
   <213> Artificial Sequence
<220>
   <223> Description of Artificial Sequence: Synthetic primer
<400> 555
   gctgcaacac aagacacga 19
<210> 556
   <211> 20
   <212> DNA
   <213> Artificial Sequence
<220>
   <223> Description of Artificial Sequence: Synthetic primer
<400> 556
   cacatgcaca agagagcaag 20
<210> 557
   <211> 24
   <212> DNA
   <213> Artificial Sequence
<220>
   <223> Description of Artificial Sequence: Synthetic primer
<400> 557
   ggaatatgtt tgatatatag ttgg 24
<210> 558
   <211> 17
   <212> DNA
   <213> Artificial Sequence
<220>
   <223> Description of Artificial Sequence: Synthetic primer
<400> 558
   gacgaaatcc aagcgca 17
<210> 559
   <211> 19
   <212> DNA
   <213> Artificial Sequence
<220>
   <223> Description of Artificial Sequence: Synthetic primer
<400> 559
   tgacctatgg aattggcag 19
<210> 560
   <211> 20
   <212> DNA
   <213> Artificial Sequence
<220>
   <223> Description of Artificial Sequence: Synthetic primer
<400> 560
   aactgggact ttgtaggcca 20
<210> 561
   <211> 20
   <212> DNA
   <213> Artificial Sequence
<220>
   <223> Description of Artificial Sequence: Synthetic primer
<400> 561
   taacagcatc tccactggaa 20
<210> 562
   <211> 16
   <212> DNA
   <213> Artificial Sequence
<220>
   <223> Description of Artificial Sequence: Synthetic primer
<400> 562
   ggctcagccc ctcctc 16
<210> 563
   <211> 19
   <212> DNA
   <213> Artificial Sequence
<220>
   <223> Description of Artificial Sequence: Synthetic primer
<400> 563
   atcgggtggt ttaatgttg 19
<210> 564
   <211> 20
   <212> DNA
   <213> Artificial Sequence
<220>
   <223> Description of Artificial Sequence: Synthetic primer
<400> 564
   cagtttcttg ttgccgagtt 20
<210> 565
   <211> 17
   <212> DNA
   <213> Artificial Sequence
<220>
   <223> Description of Artificial Sequence: Synthetic primer
<400> 565
   aggcagtgtc gtgctgt 17
<210> 566
   <211> 18
   <212> DNA
   <213> Artificial Sequence
<220>
   <223> Description of Artificial Sequence: Synthetic primer
<400> 566
   atgctgggtg gagaaggt 18
<210> 567
   <211> 24
   <212> DNA
   <213> Artificial Sequence
<220>
   <223> Description of Artificial Sequence: Synthetic primer
<400> 567
   tttattctat agagaagaag gaaa 24
<210> 568
   <211> 20
   <212> DNA
   <213> Artificial Sequence
<220>
   <223> Description of Artificial Sequence: Synthetic primer
<400> 568
   ggtggtggaa aatgacactc 20
<210> 569
   <211> 20
   <212> DNA
   <213> Artificial Sequence
<220>
   <223> Description of Artificial Sequence: Synthetic primer
<400> 569
   ccctagtgtg caaaacctgt 20
<210> 570
   <211> 17
   <212> DNA
   <213> Artificial Sequence
<220>
   <223> Description of Artificial Sequence: Synthetic primer
<400> 570
   cggtccagct ctccagt 17
<210> 571
   <211> 18
   <212> DNA
   <213> Artificial Sequence
<220>
   <223> Description of Artificial Sequence: Synthetic primer
<400> 571
   gatgtgcctc ggtggtgt 18
<210> 572
   <211> 24
   <212> DNA
   <213> Artificial Sequence
<220>
   <223> Description of Artificial Sequence: Synthetic primer
<400> 572
   gtcatcatta ccaggcagta ttag 24
<210> 573
   <211> 24
   <212> DNA
   <213> Artificial Sequence
<220>
   <223> Description of Artificial Sequence: Synthetic primer
<400> 573
   gtcatcatta ccaggcagta ttag 24
<210> 574
   <211> 23
   <212> DNA
   <213> Artificial Sequence
<220>
   <223> Description of Artificial Sequence: Synthetic primer
<400> 574
   ggtctagtgg tcctaaacat ttc 23
<210> 575
   <211> 17
   <212> DNA
   <213> Artificial Sequence
<220>
   <223> Description of Artificial Sequence: Synthetic primer
<400> 575
   gtccctttgc cttccca 17
<210> 576
   <211> 23
   <212> DNA
   <213> Artificial Sequence
<220>
   <223> Description of Artificial Sequence: Synthetic primer
<400> 576
   gaacttcact ccactgaaat ctg 23
<210> 577
   <211> 23
   <212> DNA
   <213> Artificial Sequence
<220>
   <223> Description of Artificial Sequence: Synthetic primer
<400> 577
   aaaccacaca cttccttaca ttc 23
<210> 578
   <211> 21
   <212> DNA
   <213> Artificial Sequence
<220>
   <223> Description of Artificial Sequence: Synthetic primer
<400> 578
   ccaacaagct ttttgctcgt c 21
<210> 579
   <211> 17
   <212> DNA
   <213> Artificial Sequence
<220>
   <223> Description of Artificial Sequence: Synthetic primer
<400> 579
   agccgctgtc acacgca 17
<210> 580
   <211> 17
   <212> DNA
   <213> Artificial Sequence
<220>
   <223> Description of Artificial Sequence: Synthetic primer
<400> 580
   cccctttgct gtccctg 17
<210> 581
   <211> 20
   <212> DNA
   <213> Artificial Sequence
<220>
   <223> Description of Artificial Sequence: Synthetic primer
<400> 581
   gccgtgactg gagactgtta 20
<210> 582
   <211> 21
   <212> DNA
   <213> Artificial Sequence
<220>
   <223> Description of Artificial Sequence: Synthetic primer
<400> 582
   gtacaatcaa cggtcgatgg t 21
<210> 583
   <211> 19
   <212> DNA
   <213> Artificial Sequence
<220>
   <223> Description of Artificial Sequence: Synthetic primer
<400> 583
   tgactgcctg tctgtgcct 19
<210> 584
   <211> 22
   <212> DNA
   <213> Artificial Sequence
<220>
   <223> Description of Artificial Sequence: Synthetic primer
<400> 584
   ttggcctaaa gaaatgacag ac 22
<210> 585
   <211> 21
   <212> DNA
   <213> Artificial Sequence
<220>
   <223> Description of Artificial Sequence: Synthetic primer
<400> 585
   tgagggctag gaaattgctc t 21
<210> 586
   <211> 21
   <212> DNA
   <213> Artificial Sequence
<220>
   <223> Description of Artificial Sequence: Synthetic primer
<400> 586
   ggcaatgcat taggaactga t 21
<210> 587
   <211> 20
   <212> DNA
   <213> Artificial Sequence
<220>
   <223> Description of Artificial Sequence: Synthetic primer
<400> 587
   gtgcttgacg gaaccatgtt 20
<210> 588
   <211> 22
   <212> DNA
   <213> Artificial Sequence
<220>
   <223> Description of Artificial Sequence: Synthetic primer
<400> 588
   gggacgtcca gactcaactc tc 22
<210> 589
   <211> 19
   <212> DNA
   <213> Artificial Sequence
<220>
   <223> Description of Artificial Sequence: Synthetic primer
<400> 589
   cagaccgcat catgaacac 19
<210> 590
   <211> 22
   <212> DNA
   <213> Artificial Sequence
<220>
   <223> Description of Artificial Sequence: Synthetic primer
<400> 590
   aaacccagca gacaatgtag ct 22
<210> 591
   <211> 22
   <212> DNA
   <213> Artificial Sequence
<220>
   <223> Description of Artificial Sequence: Synthetic primer
<400> 591
   ctctctcagg acactgaagc ag 22
<210> 592
   <211> 22
   <212> DNA
   <213> Artificial Sequence
<220>
   <223> Description of Artificial Sequence: Synthetic primer
<400> 592
   tggggtattt gacaaactga ca 22
<210> 593
   <211> 22
   <212> DNA
   <213> Artificial Sequence
<220>
   <223> Description of Artificial Sequence: Synthetic primer
<400> 593
   ctttgtagtc actagggcac ca 22
<210> 594
   <211> 18
   <212> DNA
   <213> Artificial Sequence
<220>
   <223> Description of Artificial Sequence: Synthetic primer
<400> 594
   ggagagcctc cacccaac 18
<210> 595
   <211> 17
   <212> DNA
   <213> Artificial Sequence
<220>
   <223> Description of Artificial Sequence: Synthetic primer
<400> 595
   gtttggatgg ctgggct 17
<210> 596
   <211> 23
   <212> DNA
   <213> Artificial Sequence
<220>
   <223> Description of Artificial Sequence: Synthetic primer
<400> 596
   gctttgacaa tactattgca ctg 23
<210> 597
   <211> 20
   <212> DNA
   <213> Artificial Sequence
<220>
   <223> Description of Artificial Sequence: Synthetic primer
<400> 597
   tcaccaaaac atggaagcac 20
<210> 598
   <211> 25
   <212> DNA
   <213> Artificial Sequence
<220>
   <223> Description of Artificial Sequence: Synthetic primer
<400> 598
   cctactaaaa catggaagca cttac 25
<210> 599
   <211> 20
   <212> DNA
   <213> Artificial Sequence
<220>
   <223> Description of Artificial Sequence: Synthetic primer
<400> 599
   tcaccaaaac atggaagcac 20
<210> 600
   <211> 20
   <212> DNA
   <213> Artificial Sequence
<220>
   <223> Description of Artificial Sequence: Synthetic primer
<400> 600
   tcaccaaaac atggaagcac 20
<210> 601
   <211> 17
   <212> DNA
   <213> Artificial Sequence
<220>
   <223> Description of Artificial Sequence: Synthetic primer
<400> 601
   ttcgccctct caaccca 17
<210> 602
   <211> 16
   <212> DNA
   <213> Artificial Sequence
<220>
   <223> Description of Artificial Sequence: Synthetic primer
<400> 602
   ctttaccccg ggtggg 16
<210> 603
   <211> 21
   <212> DNA
   <213> Artificial Sequence
<220>
   <223> Description of Artificial Sequence: Synthetic primer
<400> 603
   gaggtcgacc gtgtaatgtg c 21
<210> 604
   <211> 17
   <212> DNA
   <213> Artificial Sequence
<220>
   <223> Description of Artificial Sequence: Synthetic primer
<400> 604
   tggggctttc ttcccag 17
<210> 605
   <211> 22
   <212> DNA
   <213> Artificial Sequence
<220>
   <223> Description of Artificial Sequence: Synthetic primer
<400> 605
   gataggaggt cctcaataaa ca 22
<210> 606
   <211> 19
   <212> DNA
   <213> Artificial Sequence
<220>
   <223> Description of Artificial Sequence: Synthetic primer
<400> 606
   ctggaagctg aagctgcat 19
<210> 607
   <211> 17
   <212> DNA
   <213> Artificial Sequence
<220>
   <223> Description of Artificial Sequence: Synthetic primer
<400> 607
   ggacggaagg gcagaga 17
<210> 608
   <211> 17
   <212> DNA
   <213> Artificial Sequence
<220>
   <223> Description of Artificial Sequence: Synthetic primer
<400> 608
   tgcaggccgt gtgcttt 17
<210> 609
   <211> 21
   <212> DNA
   <213> Artificial Sequence
<220>
   <223> Description of Artificial Sequence: Synthetic primer
<400> 609
   cacactcttg atgttccagg a 21
<210> 610
   <211> 21
   <212> DNA
   <213> Artificial Sequence
<220>
   <223> Description of Artificial Sequence: Synthetic primer
<400> 610
   gaggtcagga gcaataatga a 21
<210> 611
   <211> 20
   <212> DNA
   <213> Artificial Sequence
<220>
   <223> Description of Artificial Sequence: Synthetic primer
<400> 611
   ggcatcatat aggagctgga 20
<210> 612
   <211> 22
   <212> DNA
   <213> Artificial Sequence
<220>
   <223> Description of Artificial Sequence: Synthetic primer
<400> 612
   caacaaaatc actgatgctg ga 22
<210> 613
   <211> 17
   <212> DNA
   <213> Artificial Sequence
<220>
   <223> Description of Artificial Sequence: Synthetic primer
<400> 613
   tctgtcgtcg aggcgct 17
<210> 614
   <211> 23
   <212> DNA
   <213> Artificial Sequence
<220>
   <223> Description of Artificial Sequence: Synthetic primer
<400> 614
   ggctataaag taactgagac gga 23
<210> 615
   <211> 17
   <212> DNA
   <213> Artificial Sequence
<220>
   <223> Description of Artificial Sequence: Synthetic primer
<400> 615
   cgggtgcgat ttctgtg 17
<210> 616
   <211> 18
   <212> DNA
   <213> Artificial Sequence
<220>
   <223> Description of Artificial Sequence: Synthetic primer
<400> 616
   ctccagaccc ctcgttca 18
<210> 617
   <211> 16
   <212> DNA
   <213> Artificial Sequence
<220>
   <223> Description of Artificial Sequence: Synthetic primer
<400> 617
   tgcccaggca gctgca 16
<210> 618
   <211> 20
   <212> DNA
   <213> Artificial Sequence
<220>
   <223> Description of Artificial Sequence: Synthetic primer
<400> 618
   gcaaatcaga atcacacctg 20
<210> 619
   <211> 20
   <212> DNA
   <213> Artificial Sequence
<220>
   <223> Description of Artificial Sequence: Synthetic primer
<400> 619
   caccattgct aaagtgcaat 20
<210> 620
   <211> 22
   <212> DNA
   <213> Artificial Sequence
<220>
   <223> Description of Artificial Sequence: Synthetic primer
<400> 620
   aacgtggaat ttcctctatg tt 22
<210> 621
   <211> 25
   <212> DNA
   <213> Artificial Sequence
<220>
   <223> Description of Artificial Sequence: Synthetic primer
<400> 621
   gaaaagatca accatgtatt attcg 25
<210> 622
   <211> 17
   <212> DNA
   <213> Artificial Sequence
<220>
   <223> Description of Artificial Sequence: Synthetic primer
<400> 622
   accaggttcc accccag 17
<210> 623
   <211> 19
   <212> DNA
   <213> Artificial Sequence
<220>
   <223> Description of Artificial Sequence: Synthetic primer
<400> 623
   acactcaaaa gatggcggc 19
<210> 624
   <211> 20
   <212> DNA
   <213> Artificial Sequence
<220>
   <223> Description of Artificial Sequence: Synthetic primer
<400> 624
   tcaaatgtcg cagcactttc 20
<210> 625
   <211> 20
   <212> DNA
   <213> Artificial Sequence
<220>
   <223> Description of Artificial Sequence: Synthetic primer
<400> 625
   caccccaaaa tcgaagcact 20
<210> 626
   <211> 20
   <212> DNA
   <213> Artificial Sequence
<220>
   <223> Description of Artificial Sequence: Synthetic primer
<400> 626
   caccccctgg gaagaaattt 20
<210> 627
   <211> 17
   <212> DNA
   <213> Artificial Sequence
<220>
   <223> Description of Artificial Sequence: Synthetic primer
<400> 627
   cctcacgcga gccgaac 17
<210> 628
   <211> 19
   <212> DNA
   <213> Artificial Sequence
<220>
   <223> Description of Artificial Sequence: Synthetic primer
<400> 628
   acgtggattt tcctctatg 19
<210> 629
   <211> 21
   <212> DNA
   <213> Artificial Sequence
<220>
   <223> Description of Artificial Sequence: Synthetic primer
<400> 629
   acaaaagttg cctttgtgtg a 21
<210> 630
   <211> 20
   <212> DNA
   <213> Artificial Sequence
<220>
   <223> Description of Artificial Sequence: Synthetic primer
<400> 630
   gccttctgac tccaagtcca 20
<210> 631
   <211> 23
   <212> DNA
   <213> Artificial Sequence
<220>
   <223> Description of Artificial Sequence: Synthetic primer
<400> 631
   gtggaccatg ttacataggt cag 23
<210> 632
   <211> 23
   <212> DNA
   <213> Artificial Sequence
<220>
   <223> Description of Artificial Sequence: Synthetic primer
<400> 632
   gatgtggacc atattacata cga 23
<210> 633
   <211> 22
   <212> DNA
   <213> Artificial Sequence
<220>
   <223> Description of Artificial Sequence: Synthetic primer
<400> 633
   acagagagct tgcccttgta ta 22
<210> 634
   <211> 22
   <212> DNA
   <213> Artificial Sequence
<220>
   <223> Description of Artificial Sequence: Synthetic primer
<400> 634
   aagtgttgtc cgtgaatgat tc 22
<210> 635
   <211> 20
   <212> DNA
   <213> Artificial Sequence
<220>
   <223> Description of Artificial Sequence: Synthetic primer
<400> 635
   ttctgaccag gcagtgctgt 20
<210> 636
   <211> 23
   <212> DNA
   <213> Artificial Sequence
<220>
   <223> Description of Artificial Sequence: Synthetic primer
<400> 636
   gtcattccta gaaattgttc ata 23
<210> 637
   <211> 19
   <212> DNA
   <213> Artificial Sequence
<220>
   <223> Description of Artificial Sequence: Synthetic primer
<400> 637
   ggccttctga ctccaagtc 19
<210> 638
   <211> 17
   <212> DNA
   <213> Artificial Sequence
<220>
   <223> Description of Artificial Sequence: Synthetic primer
<400> 638
   gaggggcctc agaccga 17
<210> 639
   <211> 17
   <212> DNA
   <213> Artificial Sequence
<220>
   <223> Description of Artificial Sequence: Synthetic primer
<400> 639
   gcagcgcctc acgtttt 17
<210> 640
   <211> 17
   <212> DNA
   <213> Artificial Sequence
<220>
   <223> Description of Artificial Sequence: Synthetic primer
<400> 640
   gggcggacac gacattc 17
<210> 641
   <211> 25
   <212> DNA
   <213> Artificial Sequence
<220>
   <223> Description of Artificial Sequence: Synthetic primer
<400> 641
   taggaaagac agtagattgt atagt 25
<210> 642
   <211> 20
   <212> DNA
   <213> Artificial Sequence
<220>
   <223> Description of Artificial Sequence: Synthetic primer
<400> 642
   gcctggatgc agacttttct 20
<210> 643
   <211> 19
   <212> DNA
   <213> Artificial Sequence
<220>
   <223> Description of Artificial Sequence: Synthetic primer
<400> 643
   aaagctagga ggctgtaca 19
<210> 644
   <211> 20
   <212> DNA
   <213> Artificial Sequence
<220>
   <223> Description of Artificial Sequence: Synthetic primer
<400> 644
   ctcaccatgt tgtttagtgc 20
<210> 645
   <211> 26
   <212> DNA
   <213> Artificial Sequence
<220>
   <223> Description of Artificial Sequence: Synthetic primer
<400> 645
   ggaaagacag tagattgtat agttat 26
<210> 646
   <211> 20
   <212> DNA
   <213> Artificial Sequence
<220>
   <223> Description of Artificial Sequence: Synthetic primer
<400> 646
   ctctgtccac cgcagatatt 20
<210> 647
   <211> 21
   <212> DNA
   <213> Artificial Sequence
<220>
   <223> Description of Artificial Sequence: Synthetic primer
<400> 647
   ggaaggcagt aggttgtata g 21
<210> 648
   <211> 18
   <212> DNA
   <213> Artificial Sequence
<220>
   <223> Description of Artificial Sequence: Synthetic primer
<400> 648
   catggggtcg tgtcactg 18
<210> 649
   <211> 22
   <212> DNA
   <213> Artificial Sequence
<220>
   <223> Description of Artificial Sequence: Synthetic primer
<400> 649
   ggaaagctag aaggttgtac ag 22
<210> 650
   <211> 19
   <212> DNA
   <213> Artificial Sequence
<220>
   <223> Description of Artificial Sequence: Synthetic primer
<400> 650
   aagaattcct cgacggctc 19
<210> 651
   <211> 20
   <212> DNA
   <213> Artificial Sequence
<220>
   <223> Description of Artificial Sequence: Synthetic primer
<400> 651
   aaggcagcag gtcgtatagt 20
<210> 652
   <211> 21
   <212> DNA
   <213> Artificial Sequence
<220>
   <223> Description of Artificial Sequence: Synthetic primer
<400> 652
   caaggaaaca ggttatcggt g 21
<210> 653
   <211> 21
   <212> DNA
   <213> Artificial Sequence
<220>
   <223> Description of Artificial Sequence: Synthetic primer
<400> 653
   gaaagctagg aggccgtata g 21
<210> 654
   <211> 19
   <212> DNA
   <213> Artificial Sequence
<220>
   <223> Description of Artificial Sequence: Synthetic primer
<400> 654
   agaaaagagc ccggctctt 19
<210> 655
   <211> 22
   <212> DNA
   <213> Artificial Sequence
<220>
   <223> Description of Artificial Sequence: Synthetic primer
<400> 655
   gggaaggcaa tagattgtat ag 22
<210> 656
   <211> 20
   <212> DNA
   <213> Artificial Sequence
<220>
   <223> Description of Artificial Sequence: Synthetic primer
<400> 656
   taatgcagca agtctactcc 20
<210> 657
   <211> 24
   <212> DNA
   <213> Artificial Sequence
<220>
   <223> Description of Artificial Sequence: Synthetic primer
<400> 657
   gggaaagaca gtagactgta tagt 24
<210> 658
   <211> 20
   <212> DNA
   <213> Artificial Sequence
<220>
   <223> Description of Artificial Sequence: Synthetic primer
<400> 658
   cagtcggaga agaagtgtac 20
<210> 659
   <211> 18
   <212> DNA
   <213> Artificial Sequence
<220>
   <223> Description of Artificial Sequence: Synthetic primer
<400> 659
   ggcagtggcc tgtacagt 18
<210> 660
   <211> 16
   <212> DNA
   <213> Artificial Sequence
<220>
   <223> Description of Artificial Sequence: Synthetic primer
<400> 660
   ccccacttgg cagctg 16
<210> 661
   <211> 18
   <212> DNA
   <213> Artificial Sequence
<220>
   <223> Description of Artificial Sequence: Synthetic primer
<400> 661
   gcagtagctt gcgcagtt 18
<210> 662
   <211> 18
   <212> DNA
   <213> Artificial Sequence
<220>
   <223> Description of Artificial Sequence: Synthetic primer
<400> 662
   gctgagcatc accagcac 18